# EUROPEAN PATENT SPECIFICATION

(11) **EP 2 137 314 B1**
(45) Date of publication and mention of the grant of the patent: **13.02.2013**
(21) Application number: 08735169.8
(22) Date of filing: 11.04.2008
(51) Int. Cl.: C12N 15/63, C12N 15/85

(54) **NEW MODULATING MOLECULES FOR AN IMPROVED REGULATED EXPRESSION SYSTEM**
NEUE MODULIERENDE MOLEKÜLE FÜR EIN VERBESSERTES REGULIERTES EXPRESSIONSSYSTEM
NOUVELLES MOLÉCULES MODULATRICES DESTINÉES À UN SYSTÈME D'EXPRESSION RÉGULÉE AMÉLIORÉ

(30) Priority: 11.04.2007 US 907615 P
(43) Date of publication of application: 30.12.2009
(73) Proprietor: Bayer Intellectual Property GmbH, 40789 Monheim (DE)
(72) Inventor: BAUZON, Maxine, Hercules, California 94547 (US); DROESCHER, Peter, 10717 Berlin (DE); HARKINS, Richard, N., Alameda, California 94502 (US); HERMISTON, Terry, Corte Madera, California 94925 (US); KRETSCHMER, Peter, San Francisco, California 94111 (US); LEVITSKY, Konstantin, Sausalito, CA 94965 (US); SZYMANSKI, Paul, South San Francisco, California 94080 (US)
(74) Representative: BIP Patents
(86) International application number: PCT/EP2008/002861
(87) International publication number: WO 2008/125280

(56) References cited:
- WO-A-00/31286
- WO-A-02/24899
- WO-A-93/23431
- WO-A-2008/125222
- LEVITSKY KONSTANTIN ET AL: "Development and Validation of an Improved Inducer-Regulator Protein Complex in the pBRES-Regulated Expression System" HUMAN GENE THERAPY, vol. 19, no. 11, November 2008 (2008-11), pages 1273-1282, XP002516080 ISSN: 1043-0342
- SZYMANSKI PAUL ET AL: "Development and validation of a robust and versatile one-plasmid regulated gene expression system." MOLECULAR THERAPY : THE JOURNAL OF THE AMERICAN SOCIETY OF GENE THERAPY JUL 2007, vol. 15, no. 7, July 2007 (2007-07), pages 1340-1347, XP002516079 ISSN: 1525-0024
- KRUEGER CHRISTEL ET AL: "A gene regulation system with four distinct expression levels." THE JOURNAL OF GENE MEDICINE AUG 2006, vol. 8, no. 8, August 2006 (2006-08), pages 1037-1047, XP002516081 ISSN: 1099-498X
- NGAN E S W ET AL: "THE MIFEPRISTONE-INDUCIBLE GENE REGULATORY SYSTEM IN MOUSE MODELS OF DISEASE AND GENE THERAPY" SEMINARS IN CELL AND DEVELOPMENTAL BIOLOGY, ACADEMIC PRESS, GB, vol. 13, no. 2, 1 April 2002 (2002-04-01), pages 143-149, XP001183375 ISSN: 1084-9521
- GALLINARI ET AL: "A Functionally Orthogonal Estrogen Receptor-Based Transcription Switch Specifically Induced by a Nonsteroid Synthetic Ligand" CHEMISTRY AND BIOLOGY, CURRENT BIOLOGY, LONDON, GB, vol. 12, no. 8, 1 August 2005 (2005-08-01), pages 883-893, XP005042259 ISSN: 1074-5521
- SCHWIMMER LAUREN J ET AL: "Creation and discovery of ligand-receptor pairs for transcriptional control with small molecules" PROCEEDINGS OF THE NATIONAL ACADEMY OF SCIENCES OF THE UNITED STATES OF AMERICA, vol. 101, no. 41, 12 October 2004 (2004-10-12), pages 14707-14712, XP002496011 ISSN: 0027-8424
- PANGULURI SIVA K ET AL: "Functional characterization of ecdysone receptor gene switches in mammalian cells" FEBS JOURNAL, vol. 273, no. 24, December 2006 (2006-12), pages 5550-5563, XP002496012 ISSN: 1742-464X(print) 1742-4658(ele
- KINZEL OLAF ET AL: "A structure-guided approach to an orthogonal estrogen-receptor-based gene switch activated by ligands suitable for in vivo studies" JOURNAL OF MEDICINAL CHEMISTRY, vol. 49, no. 18, September 2006 (2006-09), pages 5404-5407, XP002496013 ISSN: 0022-2623
- DOYLE DONALD F ET AL: "Modifying ligand specificity of gene regulatory proteins" CURRENT OPINION IN CHEMICAL BIOLOGY, vol. 4, no. 1, February 2000 (2000-02), pages 60-63, XP002496014 ISSN: 1367-5931
- WANG Y ET AL: "POSITIVE AND NEGATIVE REGULATION OF GENE EXPRESSION IN EUKARYOTIC CELLS WITH AN INDUCIBLE TRANSCRIPTIONAL REGULATOR" GENE THERAPY, MACMILLAN PRESS LTD., BASINGSTOKE, GB, vol. 4, 1 June 1997 (1997-06-01), pages 432-441, XP002916854 ISSN: 0969-7128
- ABRUZZESE RONALD V ET AL: "Ligand-dependent regulation of plasmid-based transgene expression in vivo" HUMAN GENE THERAPY, MARY ANN LIEBERT, NEW YORK ,NY, US, vol. 10, no. 9, 10 June 1999 (1999-06-10), pages 1499-1507, XP002210305 ISSN: 1043-0342
- WANG Y ET AL: "LIGAND-INDUCIBLE AND LIVER-SPECIFIC TARGET GENE EXPRESSION IN TRANSGENIC MICE" NATURE BIOTECHNOLOGY, NATURE PUBLISHING GROUP, NEW YORK, NY, US, vol. 15, 1 March 1997 (1997-03-01), pages 239-243, XP000872866 ISSN: 1087-0156
- BURCIN ET AL: "Adenovirus-mediated regulable target gene expression in vivo" PROCEEDINGS OF THE NATIONAL ACADEMY OF SCIENCES OF USA, NATIONAL ACADEMY OF SCIENCE, WASHINGTON, DC, vol. 96, 1 January 1999 (1999-01-01), pages 355-360, XP002130326 ISSN: 0027-8424
- JENSTER ET AL: "Steroid receptor induction of gene transcription: a two-step model" PROCEEDINGS OF THE NATIONAL ACADEMY OF SCIENCES OF USA, NATIONAL ACADEMY OF SCIENCE, WASHINGTON, DC, vol. 94, 1 July 1997 (1997-07-01), pages 7879-7884, XP002130328 ISSN: 0027-8424

## Description

### FIELD OF THE INVENTION

The present invention relates to an improved expression system for the regulated expression of an encoded protein or nucleic acid therapeutic molecule (TM), for use in the treatment of disease. In particular, the present invention relates to new modulating molecules for such an improved regulated gene expression system, and pharmaceutical compositions and uses thereof for treatment of disease.

### BACKGROUND OF THE INVENTION

The delivery of nucleic acids encoding therapeutic molecules (TMs) for treatment of diseases is thought to provide enormous potential as a therapeutic modality over conventional treatment methods. In particular, the delivery of nucleic acids encoding a therapeutic protein in gene therapy has the potential to provide significant advantages over conventional therapies requiring the administration of bolus protein. These potential advantages include, e.g., the long-term and regulated expression of a TM in the cells of a patient resulting in maximum therapeutic efficacy and minimum side effects and, also, the avoidance of toxic and infectious impurities, and systemic impurities.

For example, the delivery of bolus protein for the treatment of disease is known to result in adverse side effects including, e.g., those related to infectious and toxic impurities, systemic toxicity, injection-site necrosis, influenza-like symptoms, chills, fever, fatigue, anorexia, and weight loss. In some cases these events are dose limiting and may lead to cessation of treatment altogether. Further, it is known that continuous exposure to some protein therapeutics may result in tolerance over time. Thus, there is a need for a regulated, expression system that can provide a sustained or long-term, therapeutically efficacious level of a TM, with the additional feature of a means to rapidly reduce or modulate the level of TM within a dynamic therapeutic window. More particularly, there is a need for a regulated expression system which has the capability to be turned off should the concentration of TM reach a level that is potentially toxic. Moreover, the ability to titrate the level of TM would allow dosing to be adjusted where there is a potential for an increase in tolerance to the TM over time.

Of particular interest and need, is the delivery of a gene encoding a therapeutic protein that can be expressed in target patient cells, to remedy a condition resulting in or caused by a disease, or to stop or slow the progression of a disease. For example, the etiologies of many disease states are the result of the expression of one or more defective gene products or the defective expression of one or more gene products, e.g., the expression of a mutated protein, or the over or under expression of a protein, respectively. Thus, conventional treatment methods include the administration of recombinant proteins to correct for such defective protein expression or expression of a defective protein. However, the administration of protein therapeutics to a patient is known to result in the generation of antibodies against the protein and its rejection by the patient immune system as foreign.

Thus, there is a need for gene-based delivery of therapeutic proteins for the treatment of disease that provides regulated, long-term expression of the protein, resulting in therapeutic efficacy while minimizing dose-limiting toxic side effects. Such a regulated expression system could avoid many of the major limiting factors associated with current protein therapeutics. However, most known nucleic acid delivery systems are not suitable for clinical use and do not afford regulated or long-term expression in cells. Only a few known nucleic acid delivery systems are reported to have an ability to regulate transgene expression under laboratory conditions, but the suitability and workability of these delivery systems for clinical use are not known (*see* e.g., Gossen, M. & Bujard H. (1995) Science 268: 1766-69; No, D. et al. (1996) Proc. Natl. Acad. Sci. USA 93: 3346-51; Amara, J.F. et al. (1997) Proc. Natl. Acad. Sci. USA 94: 10618-23; Wang, Y. (1994) Proc. Natl. Acad. Sci. USA 91: 8180-84; Nordstrom, J.L. (2002) Curr. Opin. Biotechnol.13: 453-58).

### SUMMARY OF THE INVENTION

The present disclosure provides an improved expression system for the regulated expression of an encoded protein or nucleic acid therapeutic molecule (TM) for use in the treatment of disease, wherein therapeutic efficacy of the TM can be maximized and side effects minimized. In particular, the present disclosure provides new modulating molecules, e.g., new activator molecules (AMs), inactivator molecules (IMs), and regulator molecules (RMs) for such an improved regulated gene expression system, and pharmaceutical compositions and methods thereof for treatment of disease.

In particular, the present disclosure provides AMs or IMs that selectively bind to an RM having modifications that enhance this selectivity. Therefore, the new AMs and IMs of the present invention have improved properties that selectively bind or otherwise interact with a new RM of the present invention, and diminish or eliminate cross reactivity with endogenous proteins, particularly endogenous human proteins, e.g., a receptor, more particularly a steroid receptor, and even more particularly a PR or GR. In some aspects, a new AM or IM of the present disclosure an RM modulator, particularly a modified receptor modulator, more particularly a modified steroid receptor modulator, even more particularly a modified PR or GR modulator, e.g., a PR or GR ligand analog.

In one aspect, the AM or IM of the present disclosure is a PR ligand analog that selectively binds to an RM having a modified LBD or more particularly a modified LBP, and has diminished or no side effects relating to e.g., cross reactivity with an endogenous PR or other endogenous steroid receptors, abortifacient activity, or contraceptive activity.

More particularly, the present disclosure provides new AMs, IMs, and RMs having improved properties, including the ability to tightly and specifically regulate the level of TM expression, such that the expression of the TM can be modulated e.g., to increase or decrease, or turn on and off, TM. Importantly, the AMs and IMs of the present disclosure have improved selectivity for an RM of the present disclosure. In some aspects, the LBD, and more particularly the LBP, of the RM is modified to bind the AM or IM and/or be responsive to the AM or IM respectively, with improved specificity and selectivity.

For example, an AM of the present disclosure can be, but is not limited to, an AM that selectively binds to and/or activates an RM, and has little or no effect on an endogenous protein, eg, an endogenous steroid receptor. Also, for example, the RM of the present invention can be, but is not limited to, an RM that is modified to specifically bind and/or otherwise interacts with an AM and thereby is activated by the AM. In one aspect, the AM binds or otherwise interacts with the RM and selectively activates the RM, such that the activated RM increases or turns on expression of the TM. Thereby, an AM and/or RM of the present disclosure, provides the ability to tightly and specifically regulate the level of TM expression and/or activity. In some aspects, the AM binds to a unique RM modified to selectively bind to the AM.

In some aspects, an IM of the present disclosure can be, but is not limited to, an IM that selectively binds to and/or inactivates an RM, and has little or no effect on an endogenous protein, eg, an endogenous steroid receptor. Also, for example, the RM of the present invention can be, but is not limited to, an RM that is modified to specifically bind and/or otherwise interacts with an IM and thereby is inactivated by the IM. In one aspect, the IM binds or otherwise interacts with the RM and selectively inactivates the RM, such that the inactivated RM decreases or turns off expression of the TM. Thereby, an IM and/or RM of the present invention, provides the ability to tightly and specifically regulate or modulate the level of TM expression and/or activity. In some aspects, the IM binds to a unique RM modified to selectively bind to the IM. In preferred aspects, the ligand binding domain (LBD), and more particularly the ligand binding pocket (LBP), of the RM is modified to bind the AM and/or be responsive to the AM, with improved specificity and selectivity. Also in preferred aspects, the AM binds to a unique RM modified to selectively bind to the AM. For example, an AM of the present disclosure can be, but is not limited to, an AM that selectively binds to and/or activates an RM, and has little or no effect on an endogenous protein, e.g., a receptor, more particularly a steroid receptor, and even more particularly a PR or GR. Also, for example, the RM of the present invention can be, but is not limited to, an RM having a modified receptor LBD, or more particularly a modified receptor LBP, to specifically bind and/or otherwise interacts with an AM and thereby is activated by the AM. In one aspect, the AM binds or otherwise interacts with the RM and selectively activates the RM, such that the activated RM increases or turns on expression of the TM. Thereby, an AM and/or RM of the present invention, provides the ability to tightly and specifically regulate the level of TM expression and/or activity.

The encoded TM of the present disclosure can be a nucleic acid or protein that provides a therapeutic benefit to a subject having, or susceptible to, a disease. For example, such therapeutic benefit or activity includes, but is not limited to, the amelioration, modulation, diminution, stabilization, or prevention of a disease or a symptom of a disease.

In one aspect, the present disclosure provides an improved regulated expression system comprising at least a first expression cassette having a nucleic acid sequence encoding a TM, such that, when delivered to cells of a subject, the encoded TM is expressed, and the expression and/or activity of the TM is regulated in the presence of a regulator molecule (RM). Examples of such regulation include, but are not limited to, the induction, repression, increase, or decrease of TM expression and/or activity in the presence of an RM.

In one aspect of the present disclosure, the expression and/or activity of the TM is regulated in a dose-responsive or dose-dependent manner, e.g., according to the amount of a RM present in the cells of the subject or administered to the subject. In other aspects, the expression and/or activity of the TM is regulated in a dose-responsive or dose-dependent manner, e.g., according to the amount of an activator molecule (AM) or inactivator molecule (IM) present in the cells of the subject or administered to the subject.

In another aspect of the present disclosure, the expression and/or activity of the TM is orientation-dependent. For example, in one aspect, the expression and/or activity of the TM in cells is modulated with respect to the 5' to 3' orientation of the expression cassette encoding the TM, or with respect to the 5' to 3' orientation of the transcription or translation of the encoded TM. Consequently, TM expression and/or activity can be modulated by selection of a particular orientation of the expression cassette encoding the TM or the orientation of transcription or translation of the TM.

In another aspect, the regulated expression system of the present disclosure further comprises a second expression cassette encoding an RM, such that, when delivered to cells of a subject, the encoded RM is expressed and the presence thereof regulates the expression and/or activity of the TM. In a preferred aspect, a first expression cassette encoding a TM and a second expression cassette encoding an RM of the present invention are present in a single vector. In a preferred aspect, the single vector is pGT79. In another preferred aspect, the single vector is pGT1003, pGT1004, pGT1005, pGT1006, pGT1007, pGT1008 or pGT1009. In yet another preferred aspect, the single vector is pGT1015, pGT1016, pGT1017, pGT1025, pGT1020, pGT1021, pGT1022, pGT1023 or pGT1024.

A TM of the present disclosure can be an isolated DNA, RNA, or protein, or variant thereof, encoded by a nucleic acid sequence and having a therapeutic activity. More particularly, a TM of the present invention can be a modified, synthetic, or recombinant DNA, RNA or protein. In another aspect of the present disclosure, the encoded TM is a nucleic acid, e.g., a DNA or RNA, having a therapeutic activity. In one aspect of the present invention the encoded TM is an RNA e.g., an siRNA or shRNA. In another aspect of the present disclosure the encoded TM is a protein having a therapeutic activity and, preferably, a human protein or variant thereof. In one aspect the encoded TM is a monoclonal antibody having a therapeutic activity. In one aspect, the encoded TM is the monoclonal antibody, CAMPATH®. In another aspect, the nucleic acid sequence encoding such a protein is a gene or gene fragment. In one aspect, the encoded TM is a granulocyte macrophage colony stimulating factor (GM-CSF) or variant of GM-CSF (e.g., Leukine^{®}). In another aspect, the encoded TM is an interferon, e.g., interferon-alpha (IFN-α) or interferon-beta (IFN-β), and more particularly, is IFN-β-1a.

An RM of the present disclosure can be a naturally-occurring molecule or variant thereof, such as a modified molecule, or an isolated molecule. In some aspects, an RM of the present invention is a synthetic or recombinant molecule. For example, in some aspects, an RM of the present invention is a chemical compound, DNA, RNA, or protein. Further, in some aspects, an RM of the present invention is a modified molecule. In one aspect, the RM is a humanized protein. In some aspects, the RM of the present invention is modified such that the LBD, and more particularly the LBP, is mutated to increase the selectivity of the binding and/or interaction between the RM and AM, or between the RM and IM.

In another aspect, the RM is a human protein or variant thereof having a modified receptor LBD and more particularly a modified receptor LBP, e.g., a modified steroid receptor LBD and more particularly a modified steroid receptor LBP. In one aspect, the RM has a modified PR or GR LBD and more particularly a modified PR or GR LBP. In another aspect, the RM is a transcriptional activator. In another aspect, the RM comprises a transactivation domain (e.g., a VP16 or p65 transactivation domain) or a portion of such a domain. In another aspect, the RM comprises a ligand-binding domain (LBD) or portion of such a domain. Further, in one aspect, an AM binds to the LBD or more particularly the LBP of the RM, thereby activating the RM such that the presence of the activated RM regulates TM expression and/or activity. In another aspect, the RM comprises a DBD, e.g., a GAL-4 DBD. In one aspect, the RM comprises a DBD that binds to a functional sequence (e.g., a promoter sequence) operably linked to a nucleic acid encoding a TM, thereby regulating TM expression (e.g., inducing TM expression).

In another aspect, an RM of the present disclosure is activated and thereby TM expression and/or activity is regulated in the presence of the activated RM. In one aspect, an RM of the present invention is expressed or present in cells of a subject in an inactivated form, and is activated in the presence of an AM, thereby, TM expression and/or activity is regulated by the activated RM. In one aspect, the AM is a biomarker. In a further aspect, the AM is a biomarker for a disease or condition and, more particularly, is a biomarker for a disease state or condition, or symptom thereof. In one aspect, the AM activates the RM by promoting or inhibiting conformational change, enzymatic processing or modification, specific binding, or dimerization of the RM. In a preferred aspect, the AM activates the RM by promoting homodimerization of the RM.

An AM of the present disclosure can be a naturally-occurring molecule or variant thereof, such as a modified molecule, or an isolated molecule. In some aspects, the AM of the present disclosure is a synthetic or recombinant molecule. For example, in some aspects, the AM of the present disclosure is a chemical compound, DNA, RNA, or protein. Further, in some aspects, the AM of the present invention is a modified molecule. In one aspect, the AM is a humanized protein. In another aspect, the AM is a human protein or variant thereof. In preferred aspects, an AM of the present invention selectively binds to and/or otherwise interacts with a unique or specific RM, e.g., an RM having a modified receptor LBD, and more particularly a modified receptor LBP that specifically binds the AM. Also in preferred aspects, an AM of the present invention is an RM modulator, particularly a modified receptor modulator, more particularly a modified steroid receptor modulator, even more particularly a modified PR or GR modulator, even more particularly a PR or GR ligand analog, and yet even more particularly a modified antiprogestin (e.g., a modified MFP) or a modified mesoprogestin (e.g., a modified asoprisnil). In another aspect, an RM of the present disclosure is inactivated and thereby TM expression and/or activity is regulated in the presence of an inactivated RM. In one aspect, an RM of the present disclosure is expressed or present in cells of a subject in an activated form, and is inactivated in the presence of an IM, thereby, TM expression and/or activity is regulated by the inactivated RM. In one aspect, the IM is a biomarker. In a further aspect, the IM is a biomarker for a disease or condition and, more particularly, is a biomarker for a disease state or condition, or symptom thereof. In one aspect, the IM inactivates the RM by promoting or inhibiting conformational change, enzymatic processing, specific binding, or dimerization of the RM. In a preferred aspect, the IM inactivates the RM by inhibiting homodimerization of the RM.
An IM of the present disclosure can be a naturally-occurring molecule or variant thereof, or an isolated molecule. In some aspects, the IM of the present invention is a synthetic or recombinant molecule. For example, in some aspects, the IM of the present disclosure is a chemical compound, DNA, RNA, or protein. Further, in some aspects, the IM of the present invention is a modified molecule. In one aspect, the IM is a humanized protein. In another aspect, the IM is a human protein or variant thereof. In preferred aspects, an IM of the present invention selectively binds to and/or otherwise interacts with a unique or specific RM, e.g., an RM having a modified receptor LBD, and more particularly a modified receptor LBP that specifically binds the IM. Also, in preferred aspects, the IM is an RM modulator, particularly a modified receptor modulator, more particularly a modified steroid receptor modulator, even more particularly a modified PR or GR modulator, e.g. a PR or GR ligand analog, respectively.

The expression of a TM, RM, AM, or IM of the present disclosure can be constitutive or transient. In some aspects, expression of a TM, RM, AM, or IM is regulated or tissue-specific (e.g. muscle-specific). Examples of a regulated RM include, but are not limited to, an RM that is activated by an AM or inactivated by an IM. In one aspect, the expression of a TM, RM, AM, or IM of the present invention is driven by a regulated promoter or a tissue-specific promoter. In a further aspect, the regulated or tissue-specific promoter is regulated in the presence of an RM and, more particularly, by the binding of the RM to the promoter. For example, in one aspect, an RM of the present invention binds to a promoter operably linked to a nucleic acid sequence encoding a TM and thereby, regulates the expression of the encoded TM as described herein, in the cells of a subject. In one aspect, the promoter that is operably linked to a nucleic acid encoding the TM, comprises at least one GAL-4 DNA-binding site (DBS), and preferably comprises 3-18 GAL-4 DBS. In another aspect, the promoter is a Pol II or Pol III promoter. In one aspect, the promoter is the Pol II promoter U6H1. In another aspect, the promoter is a Pol II promoter selected from a group consisting of: a muscle creatine kinase promoter (MCK), a promoter comprising hypoxia responsive element (HRE promoter), endothelial leukocyte adhesion molecule (ELAM) promoter, chimeric promoter (e.g., CMV/actin chimeric promoter), cyclin A promoter, and cdc6 promoter.

The present disclosure also provides pharmaceutical compositions and methods for treatment of disease or condition comprising the improved regulated expression system of the present invention as described herein. In particular aspects, the present invention provides pharmaceutical compositions and methods for treating a disease or condition; regulating the expression of a TM; administering a TM; delivering a TM; or expressing a TM in cells of a subject, where the methods comprise contacting the cells with a regulated expression system of the present invention, such that the encoded TM is expressed in the cells, and such TM expression is regulated in the presence of an RM. In one aspect, the present disclosure provides pharmaceutical compositions and methods for treatment of leukemia, melanoma, hepatitis, and cardiomyopathy. In a preferred aspect, the encoded TM of the regulated expression system of the present invention is an IFN, e.g., an IFN-α or an IFN-β, for treatment of leukemia, melanoma, hepatitis, or cardiomyopathy.

The pharmaceutical compositions of the present disclosure comprise at least one of the expression systems described herein, particularly, at least one of the TM and RM of the present invention, more particularly, at least one of the vectors of the present invention (e.g., pGT79, pGT1003, pGT1004, pGT1005, pGT1006, pGT1007, pGT1008, pGT1009, pGT1015, pGT1016, pGT1017, pGT1025, pGT1020, pGT1021, pGT1022, pGT1023 or pGT1024). In some aspects, the pharmaceutical compositions of the present disclosure comprise at least one AM or IM of the present disclosure. In one aspect, a pharmaceutical composition of the present disclosure comprises one or more vectors encoding at least one TM and/or RM. The TM, RM, AM, and IM of the present invention can be administered to a subject separately or together and ex vivo or in vivo, using any suitable means of administration described herein or known in the art. Examples of such suitable means of administration include, but are not limited to injection (e.g., subcutaneous injection), oral administration, and electroporation. In one aspect, a TM and RM of the present disclosure are present in a single vector, and separately administered from an AM that activates the RM (and thereby, the presence of the activated RM regulates TM expression and/or activity). In a further aspect, the AM is a compound (e.g., an RM modulator, particularly a modified receptor modulator, more particularly a modified steroid receptor modulator, even more particularly a modified PR or GR modulator, even more particularly a PR or GR ligand analog, and yet even more particularly a modified antiprogestin (e.g., a modified MFP) or a modified mesoprogestin (e.g., a modified asoprisnil)) administered orally, and the single vector encoding a TM and RM is a single vector administered by injection or electroporation to cells of a subject (e.g., skeletal muscle cells).

The present disclosure further provides vectors and kits comprising the improved regulated expression system of the present invention. In some aspects, the improved regulated expression system of the present invention comprises one or more vectors, and each vector comprises one or more expression cassettes. In one aspect, the improved regulated expression system of the present invention comprises a single vector having at least one expression cassette and, more preferably, at least two expression cassettes. In a preferred aspect, the improved regulated expression system of the present disclosure comprises a single vector comprising a first expression cassette having at least one cloning site for insertion of a first nucleic acid sequence encoding a TM, and a second expression cassette having at least one cloning site for insertion of a second nucleic acid sequence encoding an RM. In another aspect, the vector is a vector that is used for producing virus, e.g., an adeno-associated virus (AAV) shuttle plasmid and, more particularly, an AAV-1 shuttle plasmid. In one aspect, the vector of the present invention is a nonviral vector (i.e., a vector that does not produce virus), e.g., a plasmid vector that does not produce virus. In a preferred aspect, the vector is a plasmid vector of the present invention comprising a cloning site for insertion of a nucleic acid sequence comprising a sequence encoding a TM. Examples of such plasmid vectors of the present invention include, but are not limited to, pGT79, pGT1003, pGT1004, pGT1005, pGT1006, pGT1007, pGT1008, pGT1009, pGT1015, pGT1016, pGT1017, pGT1025, pGT1020, pGT1021, pGT1022, pGT1023 or pGT1024.

The expression cassettes of the present disclosure comprise functional sequences for expression of an encoded molecule of the present invention, e.g., a TM, RM, AM, or IM. In some aspects, the expression cassette comprises at least one functional sequence operably linked to a nucleic acid sequence encoding a molecule of the present disclosure. Examples of a functional sequence are, but not limited to, a 5' or 3' untranslated region (e.g., UT12), intron (e.g., IVS8), poly(A) site (e.g, SV40 or hGH poly(A) site), or a DNA-binding site (DBS) (e.g., GAL-4 DBS). In one aspect, the functional sequence comprises at least one GAL-4 DBS and preferably comprises multimers of a GAL-4 DBS (e.g., 3-18 GAL-4 DBS). Such functional sequences also include, for example, sequences encoding a regulated promoter or tissue-specific promoter that promotes the regulated or tissue-specific expression, respectively, of a molecule encoded by a nucleic acid sequence operably linked to such functional sequences in an expression cassette of the present invention. In another aspect, the expression cassettes of the present disclosure comprise at least one cloning site and, more preferably, a multiple cloning site (MCS), for the insertion of a nucleic acid sequence encoding a molecule of the present invention, e.g., a TM, RM, AM, or IM.

In one aspect, a first expression cassette of the present disclosure comprises an MCS for insertion of a first nucleic acid sequence encoding a TM, an inducible promoter comprising at least one DBS (e.g., 3-18 GAL-4 DBS), 5' untranslated region (e.g., UT12), an intron (e.g., IVS8), and hGH poly(A) site, such that when the first nucleic acid sequence is inserted at the MCS, these functional sequences are operably linked to this sequence. In another aspect, a second expression cassette of the present disclosure comprises an MCS for insertion of a second nucleic acid sequence encoding a regulated RM and SV40 poly(A) site, such that when the second nucleic acid sequence is inserted at the MCS, these functional sequences are operably linked to this sequence. In a preferred aspect, the first and second expression cassettes are present in a single vector.

The kits of the present disclosure comprise at least one of the expression systems of the present disclosure described herein and, more particularly, at least one of the pharmaceutical compositions, vectors, or molecules (e.g., TM, RM, AM, or IM) of the present disclosure and instructions for their use.

### BRIEF DESCRIPTION OF THE DRAWINGS

The foregoing and other objects of the present invention, the various features thereof, as well as the invention itself may be more fully understood from the following description, when read together with the accompanying drawings in which:

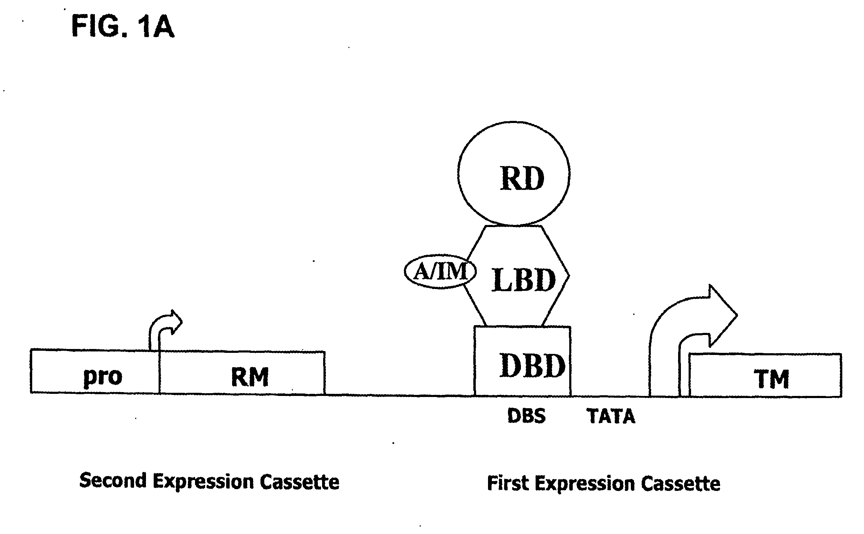

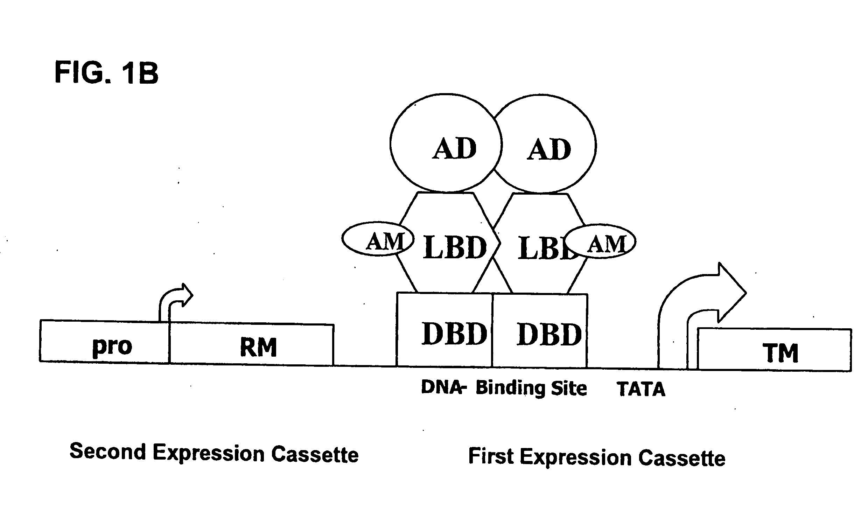

**Figure 1** illustrates unlimiting examples of a regulated expression system of the present invention. Figure 1A illustrates an unlimiting example of a regulated expression system of the present invention comprising: 1) a first expression cassette comprising a first nucleic acid sequence encoding a therapeutic molecule (TM) and a first promoter sequence encoding a DNA-binding site (DBS) and TATA sequence operably linked to the first nucleic acid sequence; 2) a second expression cassette comprising a second nucleic acid sequence encoding a regulator molecule (RM) and a second promoter sequence operably linked to the second nucleic acid sequence; 3) the expressed RM that is a fusion or chimeric protein comprising a DNA-binding domain (DBD), ligand-binding domain (LBD), and regulatory domain (RD); and 4) an activator or inactivator molecule (A/IM) that activates the RM or inactivates the RM, respectively. In one embodiment, an activator molecule (AM) binds to the RM and activates the RM and, thereby, the activated RM binds to the DBS of the promoter sequence operably linked to the TM sequence, resulting in the induction of TM expression in cells (e.g., mammalian cells). In another embodiment, the first and second expression cassettes are present in a single vector.
**Figure 1B** illustrates an unlimiting example of a regulated expression system of the present invention comprising: 1) a first expression cassette comprising a first nucleic acid sequence encoding a TM and a first promoter sequence encoding a DBS and TATA sequence operably linked to the first nucleic acid sequence; 2) a second expression cassette comprising a second nucleic acid sequence encoding a regulator molecule (RM) and a second promoter sequence operably linked to the second nucleic acid sequence; 3) the expressed RM that is a fusion or chimeric protein comprising a DBD, LBD, and activation domain (AD); and 4) an activator or inactivator molecule (A/IM). In one embodiment, an activator molecule (AM) binds to the RM and activates the RM, and thereby, the activated RM forms a homodimer that binds to the DBS of the promoter operably linked to the TM sequence, resulting in the induction of TM expression, in cells (e.g., mammalian cells). In another embodiment, the first and second expression cassettes are present in a single vector.

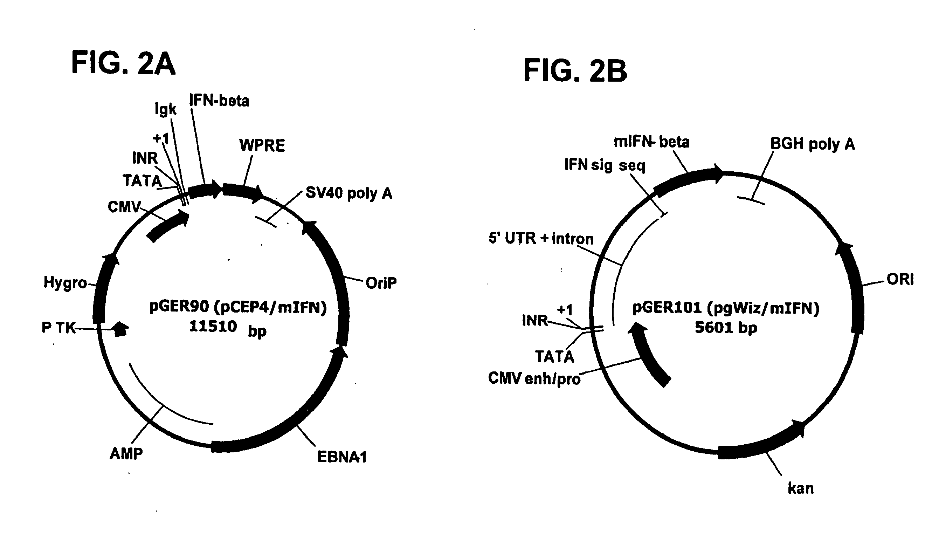

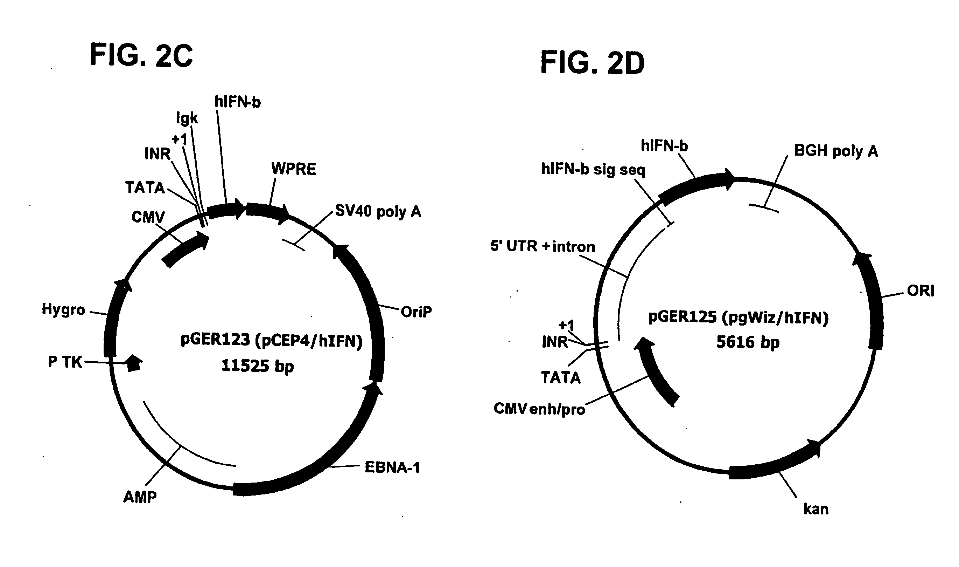

**Figure 2** illustrates murine IFN-ÿ and human IFN-ÿ plasmid vectors for generation of recombinant protein. Figures 2 A and B illustrate murine IFN-ÿ expression vectors for generation of recombinant protein (A, pGER90 (pCEP4/mIFN) and for gene-based delivery studies (B, pGER101 (pgWiz/mIFN). The CMV promoter and enhancer present in pGER90 extends from -831 bp to +1 bp relative to the transcription start site, with no 5' UTR or intron. The CMV sequences present in pGER101 include the promoter, enhancer, 5' UTR, and natural Intron A from -674 bp to +942 bp. Figure 2 C and D illustrate human IFN-ÿ expression vectors for generation of recombinant protein (C, pGER123 (pCEP4/hIFN) and for gene-based delivery studies (D, pGER125 (pgWiz/hIFN).

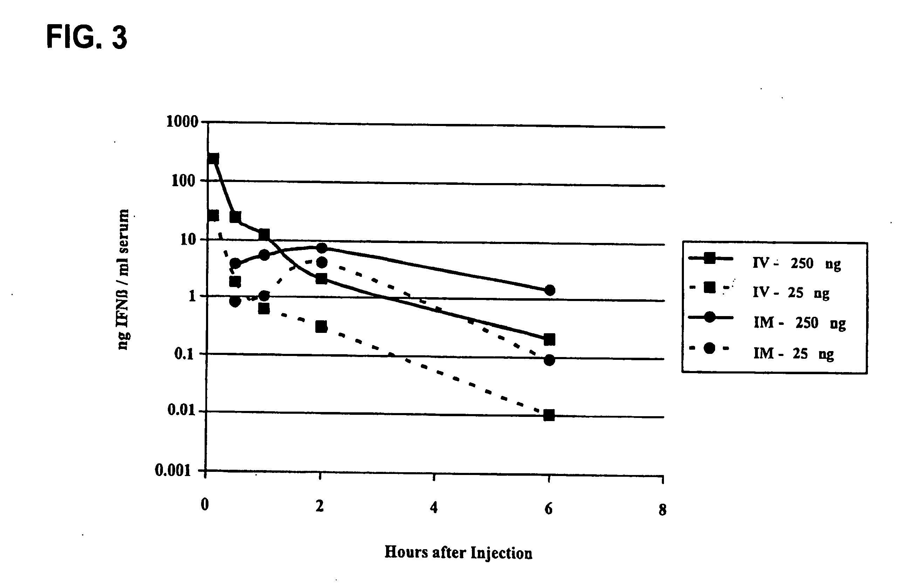

**Figure 3** illustrates the pharmacokinetic profile following injection of human IFN-ÿ1 a protein in mice. C57BI/6 mice were administered either 25 ng (Low Dose) or 250 ng (High Dose) of recombinant hIFN-ÿ 1a protein by either i.v. or i.m. injection. Human IFN-ÿ levels were determined by ELISA (Toray-Fugi Bio, Biosource International) in serum samples obtained following terminal bleeding of mice at the indicated time points post-injection (n=4 mice per time point). Each data point represents the mean value +/- the standard deviation.

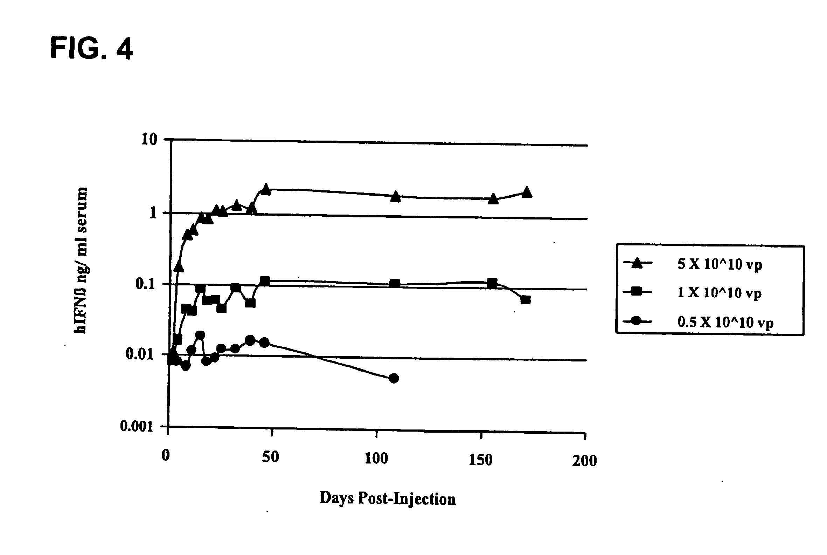

**Figure 4** illustrates the pharmacokinetic profile following intramuscular injection of AAV-1-hIFN-ÿ in mice. C57/BI/6 mice (n=6 per group) were injected i.m. with 0.5x1010, 1.0x1010, or 5.0x1010 viral particles of AAV-1-hIFN-ÿ. Blood samples were taken at the indicated time points following injection and hIFN-ÿ serum levels were determined by ELISA. Each data point represents the mean value +/- the standard deviation.

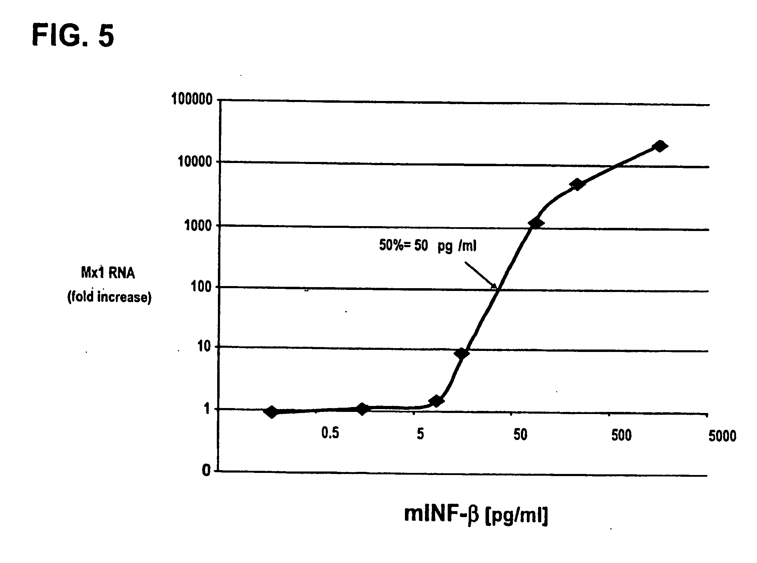

**Figure 5** illustrates Mx1 RNA induction in vitro (in L929 cells) by mIFN-ÿ. L929 cells were seeded at 5x105 cells in 6 well plates and stimulated with increasing amounts of purified recombinant mIFN-ÿ protein. Four hours after treatment the cells were harvested, RNA isolated, and Mx1 RNA quantitated by TaqMan analysis. Mx1 RNA expression is plotted as the fold increase relative to GAPDH RNA.

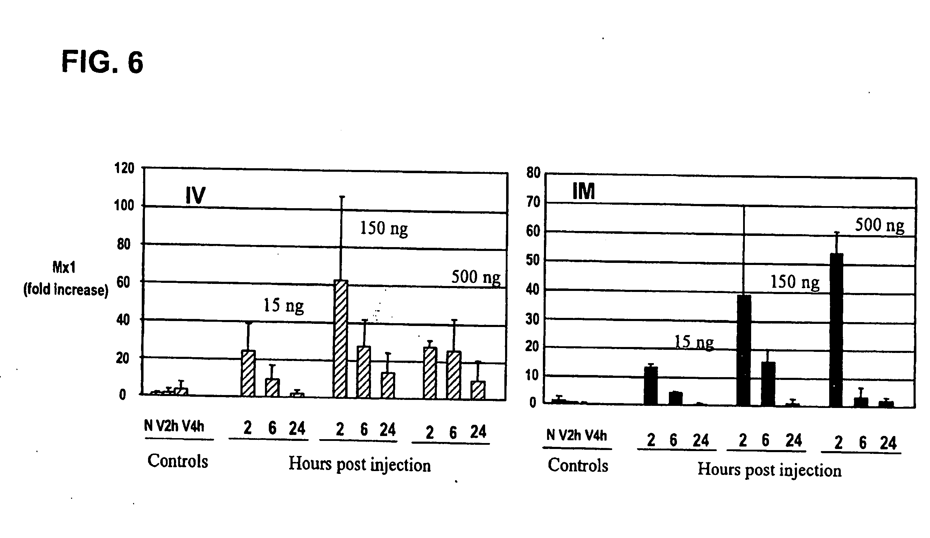

**Figure 6** illustrates Mx1 RNA induction following i.v. (A) or i.m. injection (B) of mIFN-ÿ protein. C57BI/6 mice were administered 15, 150, or 500 ng of purified recombinant mIFN-ÿ protein (specific activity-2.0x108 units/mg) by either i.v. (via tail vein) or i.m. injection (n=3 mice per group). At the specified time points post-injection mice were bled, and RNA was isolated from PBMCs. Mx1 RNA was measured by quantitative RT-PCR. The fold increase in Mx1 RNA is expressed relative to GAPDH values measured in the same samples. The controls include naive mice (N), and mice injected with the vehicle buffer only followed by Mx1 analysis at 2 hours (V2h) or 4h (V4h) post-injection. Each column represents the mean value +/- standard deviation.

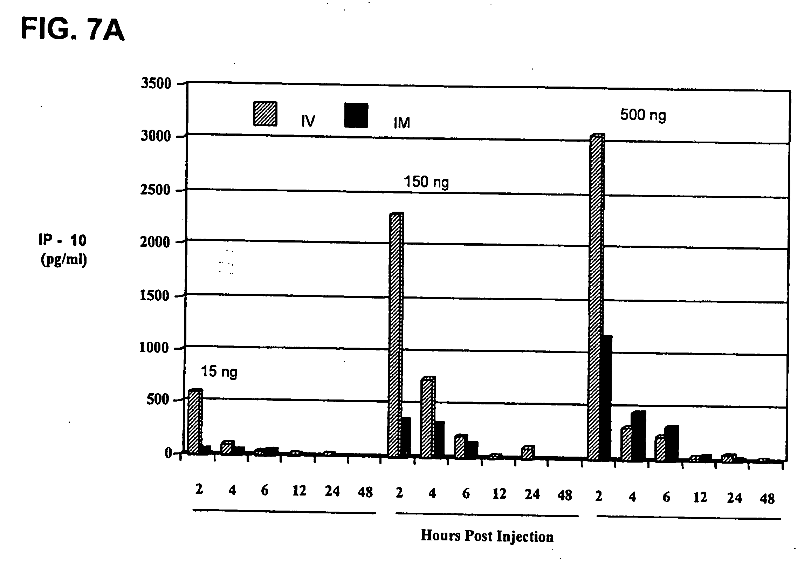

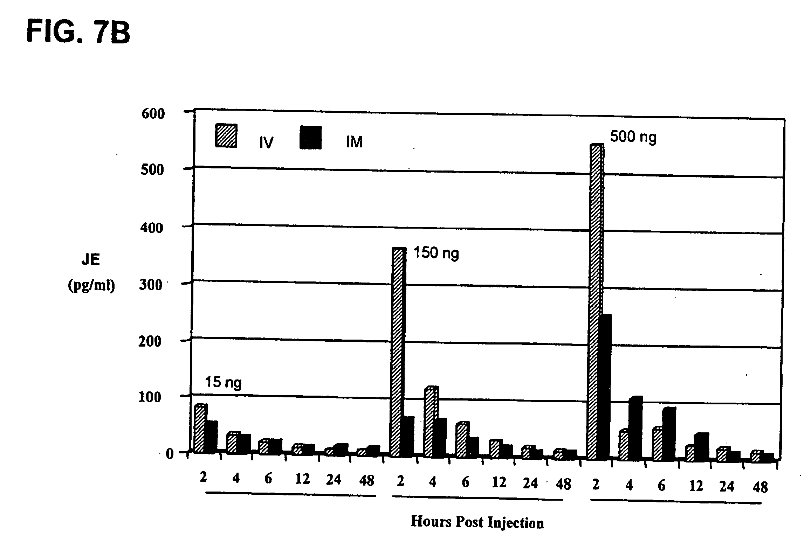

**Figure 7** illustrates induction levels of IP-10 (A) and JE (B) following i.v. or i.m. injection of murine IFN-ÿ protein. C57BI/6 mice were administered 15, 150, or 500 ng of purified recombinant mIFN-ÿ protein (specific activity = 2.0x108 units/mg) by either i.v. (via tail vein) or i.m. injection (n=3 mice per group). The mice were bled at 2, 4, 6, 12, 24, and 48 hours post-injection, and the plasma levels of IP-10 and JE were measured by ELISA (R&D Systems).

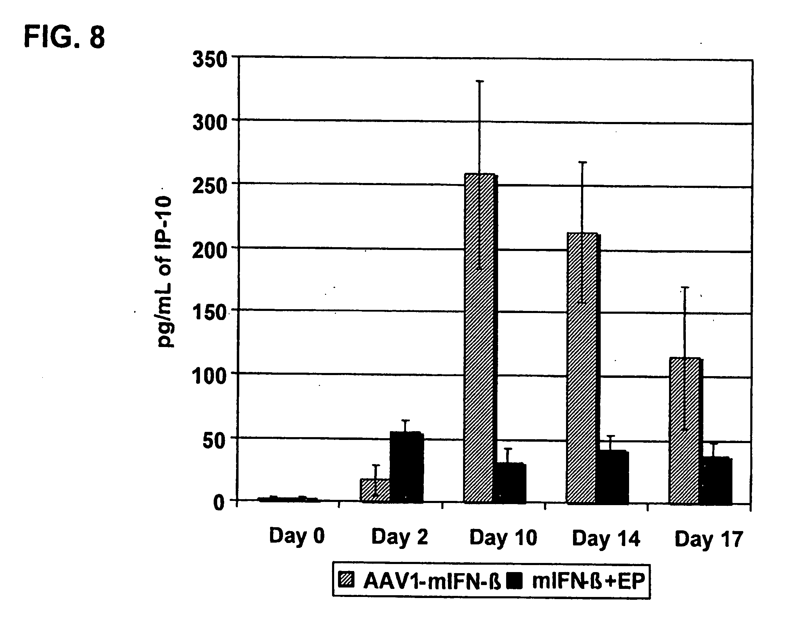

**Figure 8** illustrates the induction of IP-10 following intramuscular injection of AAV-1-mIFN-ÿ DNA or mIFN-ÿ plasmid DNA with electroporation (EP) in mice. Normal mice (C57BI/6) were injected i.m. with either AAV-1-mIFN-ÿ (5x109 viral particles), or mIFN-ÿ plasmid DNA (150 ug) with electroporation. Mice were bled at the indicated time points and IP-10 levels in plasma were determined by ELISA. Each column represents the mean value +/- the standard deviation (n=5 mice per group).

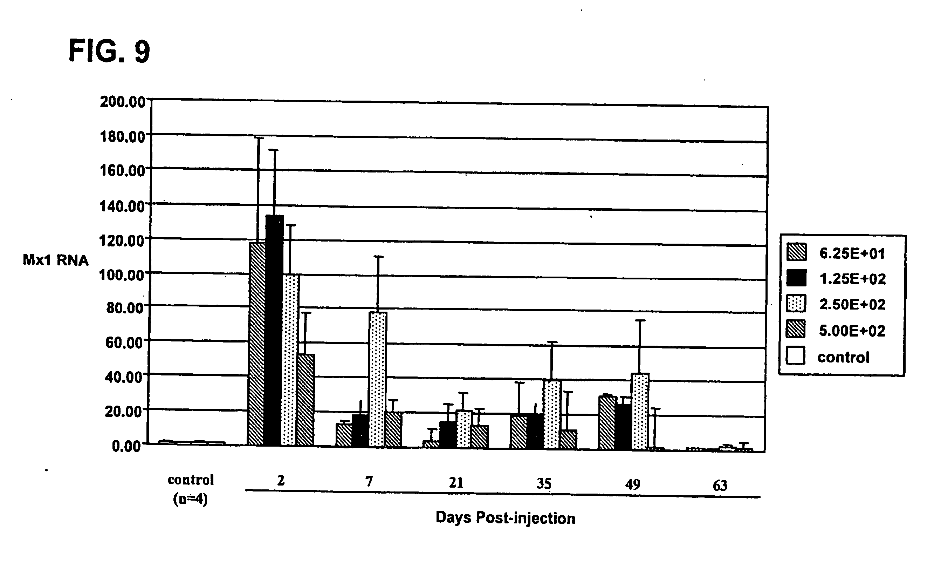

**Figure 9** illustrates the induction of Mx1 mRNA following intramuscular injection of mIFN-ÿ plasmid DNA. Mice were injected i.m. into the hind limb gastrocnemius and tibialis muscles with different amounts of plasmid DNA encoding mIFN-ÿ (62.5, 125, 250, or 500 ug) followed by electroporation (n=5 per group). Mice were bled at the specified time points post-injection, RNA isolated from PBMCs, and Mx1 expression was determined by quantitative RT-PCR. Mx1 RNA levels were normalized to GAPDH expression and are shown as fold induction over background measured at day 0 compared to untreated controls (Controls, n=4). Each column represents the mean value +/- the standard deviation.

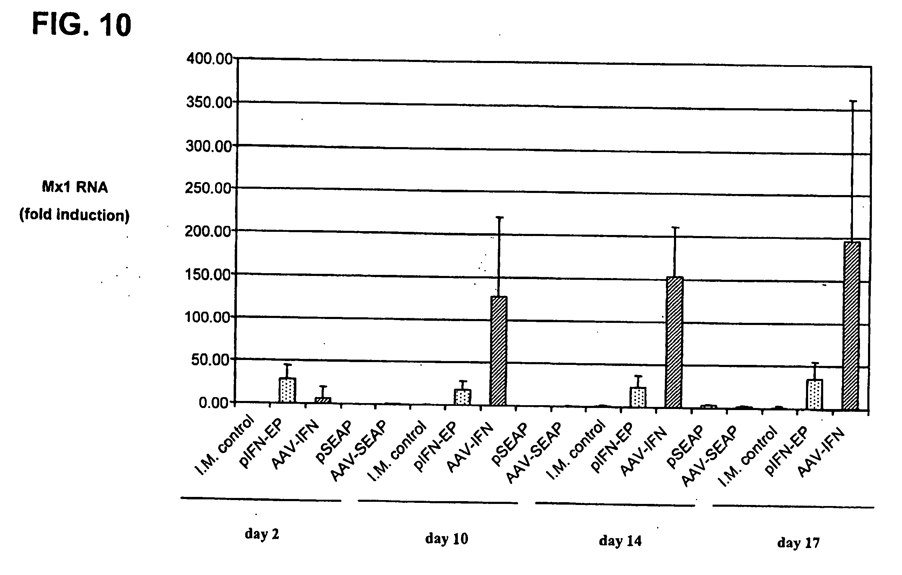

**Figure 10** illustrates the induction of Mx1 mRNA following intramuscular injection of AAV-1-mIFN-ÿ virus or mIFN-ÿ plasmid DNA with electroporation in mice. Normal mice (C57BI/6) were injected i.m. with either AAV-1-mIFN-ÿ (5x1010 viral particles), or mIFN-ÿ plasmid DNA (150 ug) with electroporation. Controls included PBS injected mice (i.m. control), and mice injected with SEAP plasmid (pSEAP) or AAV-1 expressing SEAP (AAV-SEAP). Mice were bled at the indicated time points and Mx1 RNA levels were determined by quantitative RT-PCR in RNA isolated from PBMCs. Mx1 RNA expression was normalized to GAPDH expression and is shown as fold induction over background measured at day 0 in PBS injected control mice. Each column represents the mean value +/- the standard deviation (n=5 mice per group).

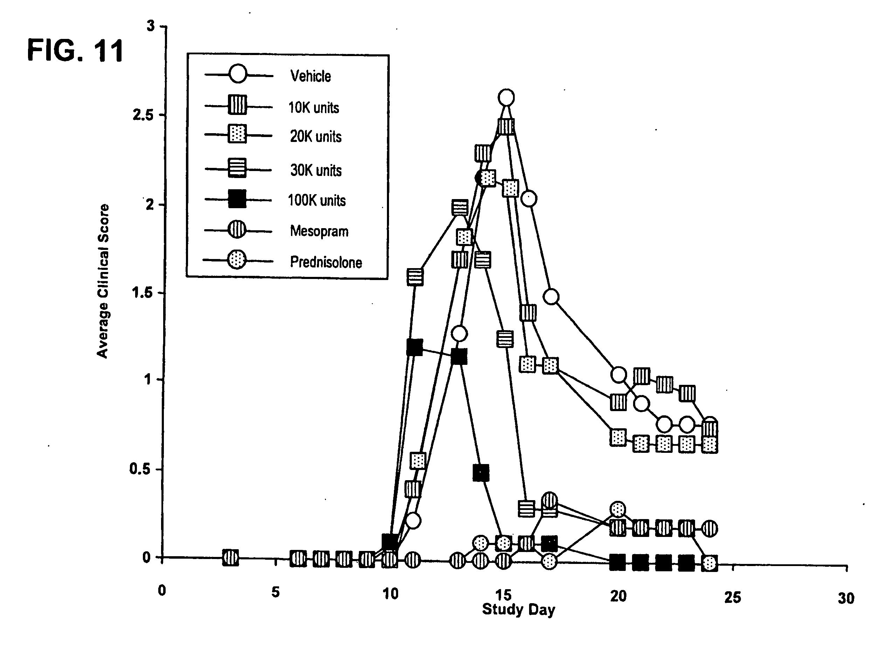

**Figure 11** illustrates the efficacy of IFN-ÿ protein in a mouse acute EAE model (as described in Example 5 and Material and Methods subsection A). Mice treated with 100K units of IFN-ÿ developed significantly decreased clinical scores of EAE compared with vehicle treated mice (p=0.0046). Mice treated with 30K units of IFN-ÿ also developed decreased clinical scores compared to vehicle treated mice, although this decrease did not reach statistical significance. The positive controls in this study, Mesopram and Prednisolone, also significantly decreased clinical scores.

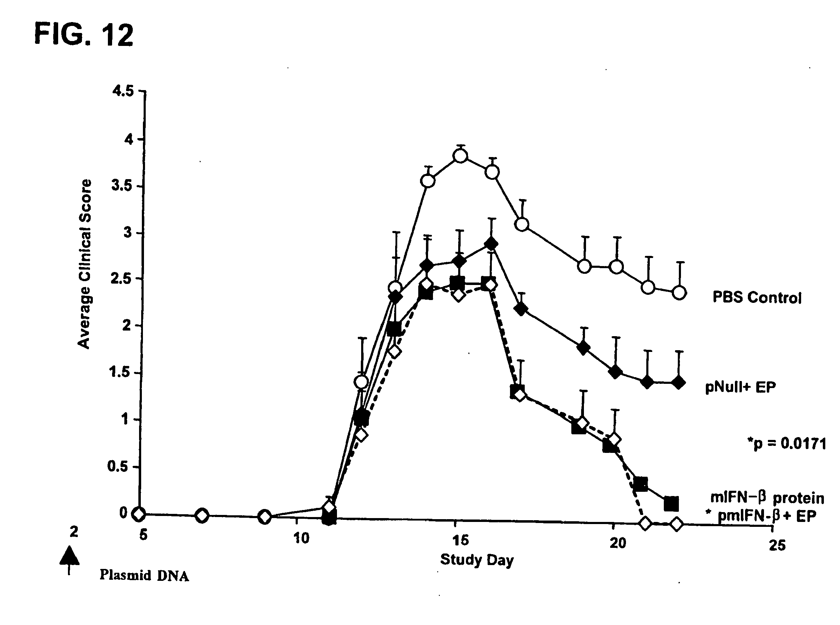

**Figure 12** illustrates the efficacy of gene-based delivery of mIFN-ÿ in a murine acute EAE model. Female SJL mice were immunized with PLP/pertussis toxin on day 1 as fully described in the Materials and Methods. Groups of mice (n=10 per group) were injected with either PBS, a null plasmid (pNull) plus electroporation (EP) (pNull + EP, 120 ug), or plasmid DNA encoding mIFN-ÿ (pmIFN-ÿ) plus EP (pmIFN-ÿ + EP, 120 ug) on day 2 of the study. For protein delivery, recombinant mIFN-ÿ protein (100,000 units) was administered to another group of animals by s.c. injection every other day beginning on day 1 of the study. A significant decrease in disease severity was observed with pmIFN-ÿ+EP versus the pNull+EP control group (p=0.0171). The results of the study are fully described in the Materials and Methods.

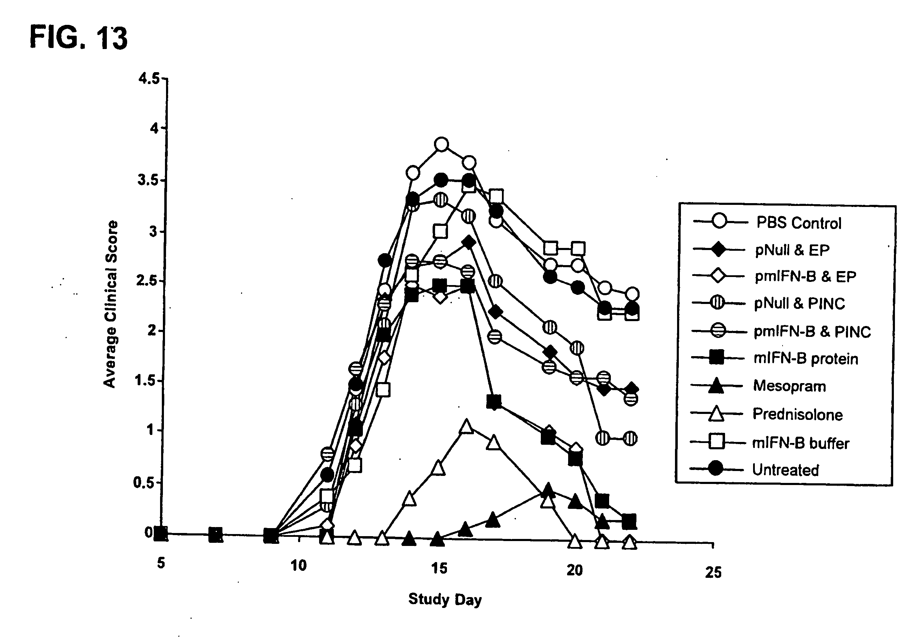

**Figure 13** illustrates the efficacy of IFN-ÿ protein in a mouse acute EAE model as fully described in Example 5 and Materials and Methods.

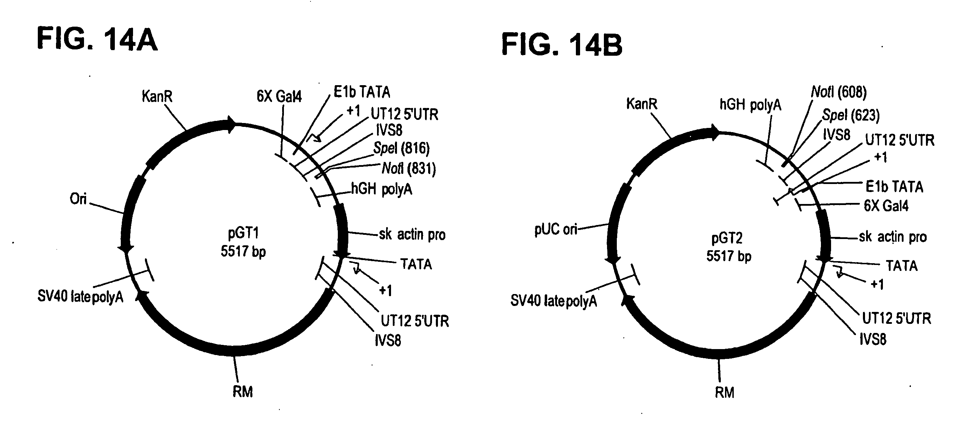

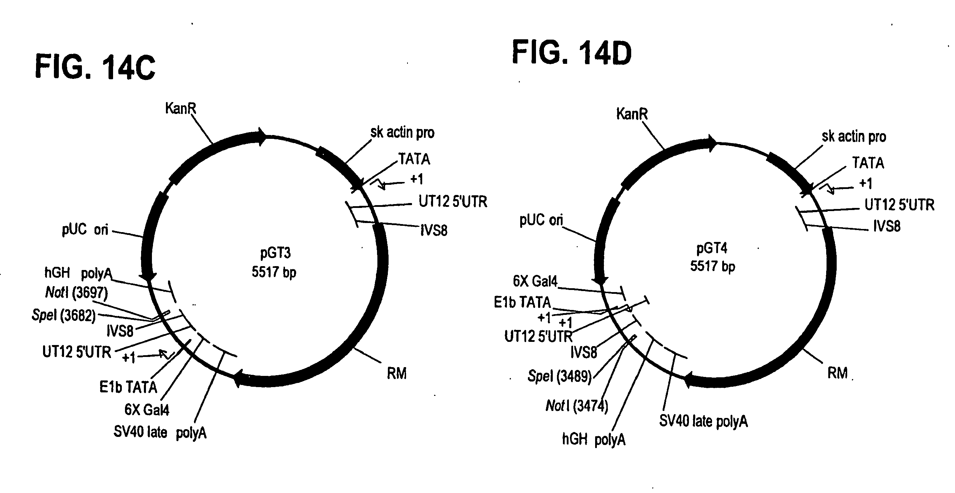

**Figure 14** illustrates plasmid vectors pGT1, pGT2, pGT3, and pGT4 (A, B, C, D, respectively), which are unlimiting examples of one-plasmid regulated expression vectors of the present invention. In these examples, the regulated expression vectors of the present invention contain, in a single plasmid vector: 1) a first expression cassette with a multiple cloning site (MCS) for insertion of a nucleic acid encoding a therapeutic molecule (TM); and 2) a second expression cassette with a cloning site for insertion of a nucleic acid encoding a regulator molecule (RM). These four vectors each provide a different orientation of the first and second expression cassettes relative to each other as described and illustrated. In the first expression cassette, the skeletal muscle promoter (sk actin pro), untranslated region 12 (UT12), intervening sequence 8 (IV8) from the plasmid pLC1674 are located upstream of the MCS and human growth hormone poly (A) site (hGH polyA). A nucleic acid comprising a therapeutic molecule (TM) of interest, e.g., a transgene, can be inserted at the MCS.

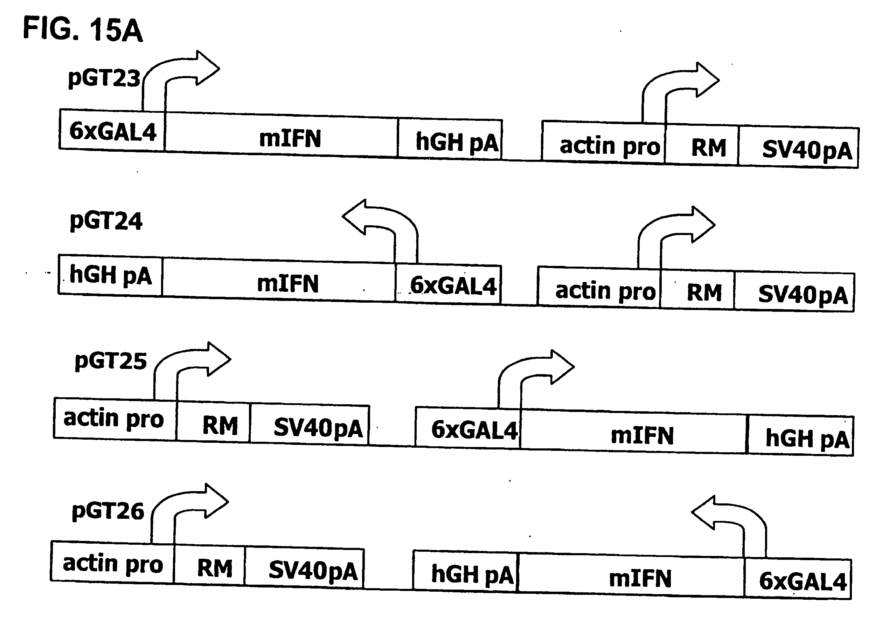

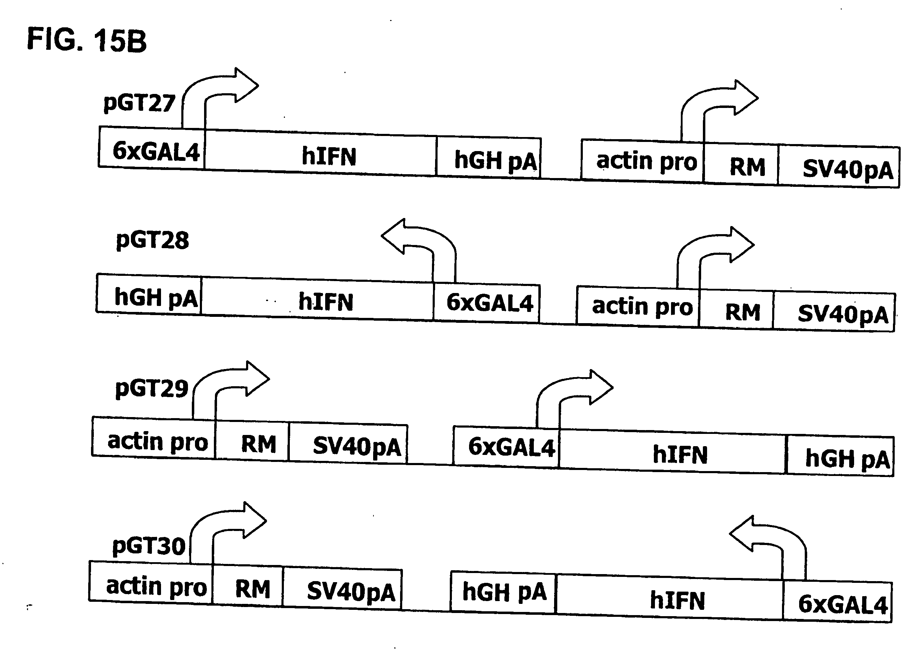

**Figure 15** illustrates unlimiting examples of regulated expression plasmid vectors of the present invention for gene-based delivery of murine IFN-ÿ (pGT23, pGT24, pGT25, and pGT26) (A), or human IFN-ÿ (pGT27, pGT28, pGT29, and pGT30) (B). In these examples, the regulated expression vectors of the present invention contain, in a single plasmid vector: 1) a first expression cassette with a multiple cloning site (MCS) and a nucleic acid inserted at the MCS encoding either a human IFN-ÿ gene or a murine IFN-ÿ gene; and 2) a second expression cassette with a cloning site and a nucleic acid inserted at the site encoding a regulator molecule (RM) that contains the modified LBD of the progesterone receptor (e.g., comprising the amino acid sequence of SEQ ID NO: 22 or encoded by the nucleic acid sequence of SEQ ID NO: 21). These vectors each provide a different orientation of the first and second expression cassettes relative to each other as described and illustrated as fully described in the Materials and Methods, subsection F.

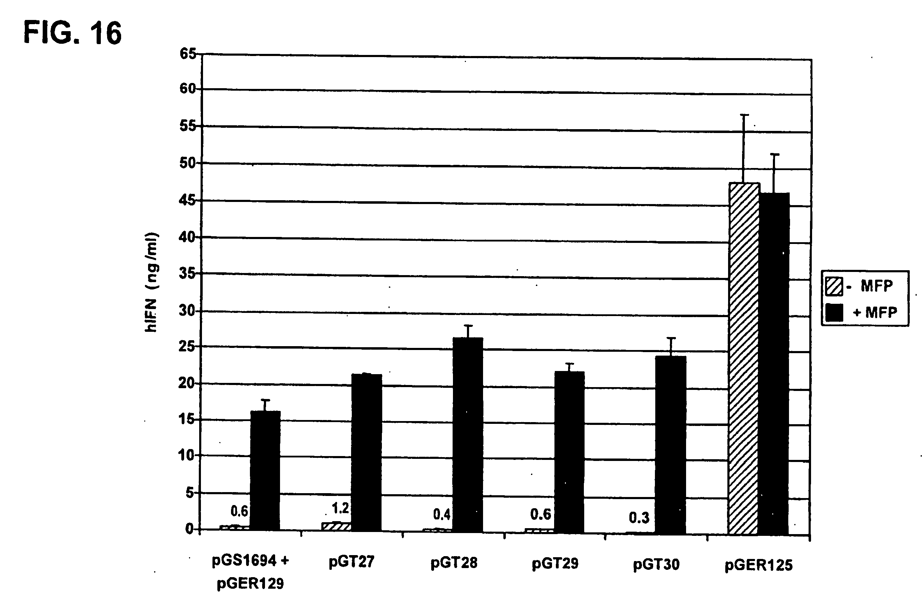

**Figure 16** illustrates the in vitro validation of hIFN-ÿ regulated expression plasmid vectors of the present invention in murine skeletal muscle cells as fully described in Example 6, subsection C. Constitutive (pGER125) and inducible (pGT27, pGT28, pGT29, and pGT30) hIFN-ÿ plasmid vectors were transfected into mouse muscle C2C12 cells, treated with MFP (10 nM), and media collected. Media was assayed for hIFN-ÿ by ELISA. The average of two independent transfections are shown. Plasmid vectors pGS1694 + pGER129 is a two-plasmid system of Valentis in which the present inventors inserted the hIFN-ÿ gene. The regulated expression vectors of the present invention were constructed with the hIFN-ÿ gene in either the forward (hIFN, ÿ) or reverse (hIFNr, ÿ) direction, either upstream or downstream of the RM cassette.

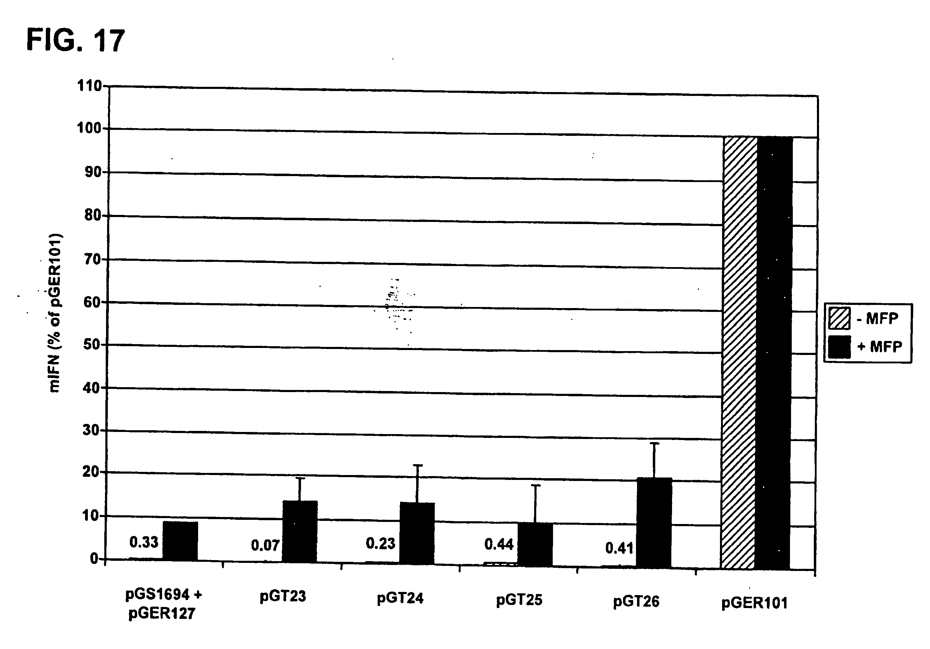

**Figure 17** illustrates the in vitro validation of mIFN-ÿ regulated expression plasmid vectors of the present invention in murine skeletal muscle cells as fully described in Example 6, subsection C. Constitutive (pGER101) and inducible (pGT23, pGT24, pGT25, and pGT26) mIFN-ÿ expression plasmids were transfected into mouse muscle C2C12 cells. Media was replaced 24 hours (hr) after transfection with fresh media with or without MFP (10 nM). Media was collected 24 hr later and assayed for mIFN-ÿ by a reporter gene assay. The chart shows the average of three independent transfections. pGS1694 + pGER127 is a two-plasmid system of Valentis in which the present inventors inserted the mIFN-ÿ gene. The regulated expression vectors of the present invention were constructed with the mIFN-ÿ gene in either the forward (mIFN, ÿ) or reverse (mIFNr, ÿ) direction, either upstream or downstream of the RM cassette..

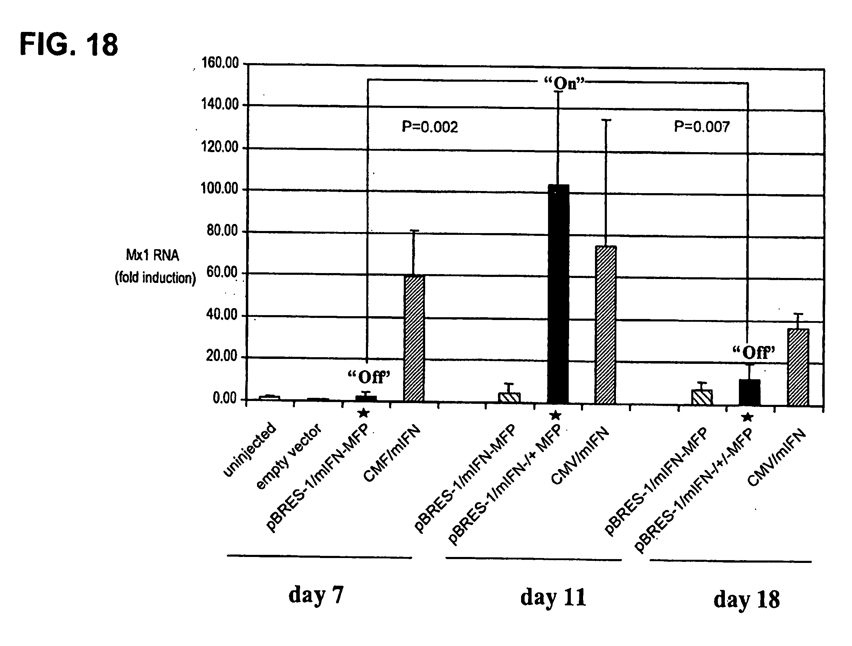

**Figure 18** illustrates Mx1 RNA induction in vivo using a pBRES-1 mIFN-ÿ regulated expression system of the present invention. Constitutive (pGER101) and inducible regulated expression (pGT26) mIFN-ÿ plasmid vectors were injected and electroporated into the tibialis and gastrocnemius muscles of mice (150 ug per animal). Blood was collected at 7 days after injection. Mice were treated with MFP (0.33 mg/kg) by oral gavage once per day 7-10 days after injection. Blood was collected at 11 and 18 days after injection. PBMCs were isolated from the blood and RNA was prepared from PBMCs and assayed by RT-PCR to determine the level of Mx1 RNA. Mx1 expression levels were normalized to GAPDH. The results are shown as the mean (n=5 animals per group) +/- the standard deviation, and show little or no activity of Mx1 RNA using pBRES-1 -mIFN in the absence of MFP at 7 days, and strong induction to levels higher than with CMV-mIFN in the presence of MFP at 11 days, as fully described in the Materials and Methods, subsection C. At 18 days, in the absence of MFP, the Mx1 RNA decreased nearly to baseline.

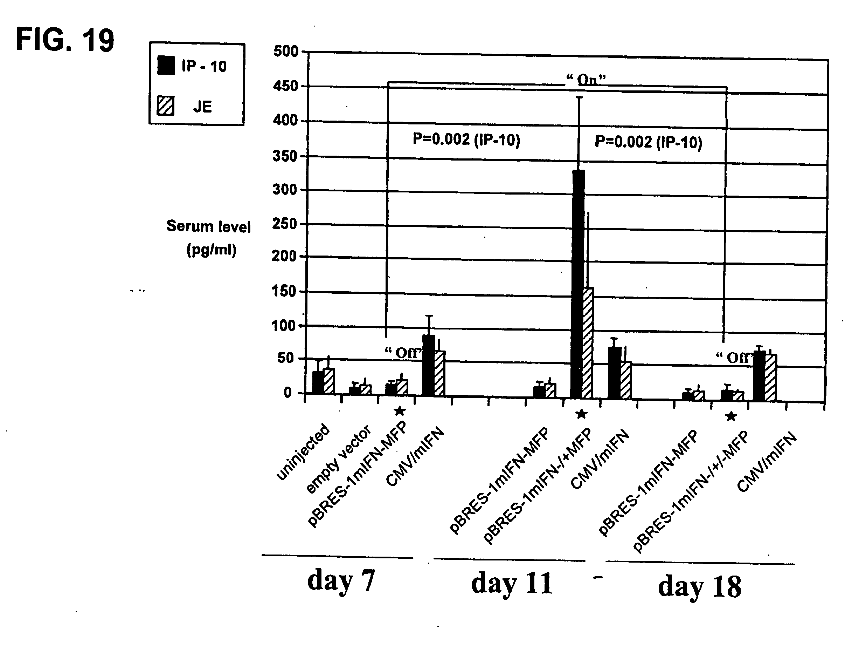

**Figure 19** illustrates IP-10 and JE induction with a pBRES-1 mIFN-ÿ regulated expression system of the present invention. Constitutive (pGER101) and inducible pBRES-1 (pGT26) mIFN expression plasmids were injected and electroporated into hind limb muscles of C57BI/6 mice. Animals were bled and the plasma was assayed for the chemokines IP-10 and JE by ELISA on day 7 (absence of MFP), day 11 (following four consecutive days of oral administration of MFP, and day 18. The results are shown as the mean (n=5 animals per group) +/- the standard deviation, and show little or no activity of chemokines (IP-10 and JE) using pBRES-1-mIFN in the absence of MFP at 7 days, and strong induction to levels higher than with CMV-mIFN in the presence of MFP at 11 days, as fully described in the Materials and Methods, subsection C. At 18 days, in the absence of MFP, the chemokine levels returned to baseline.

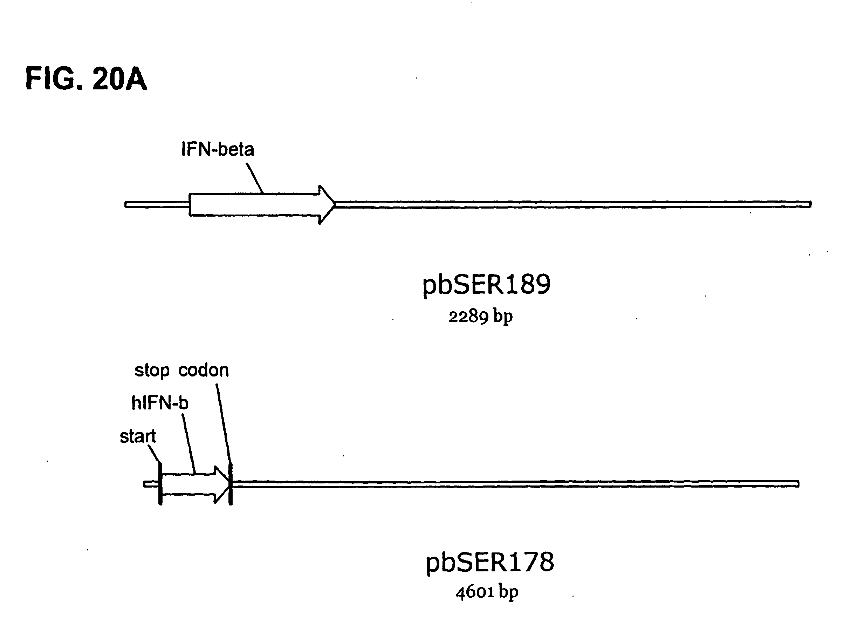

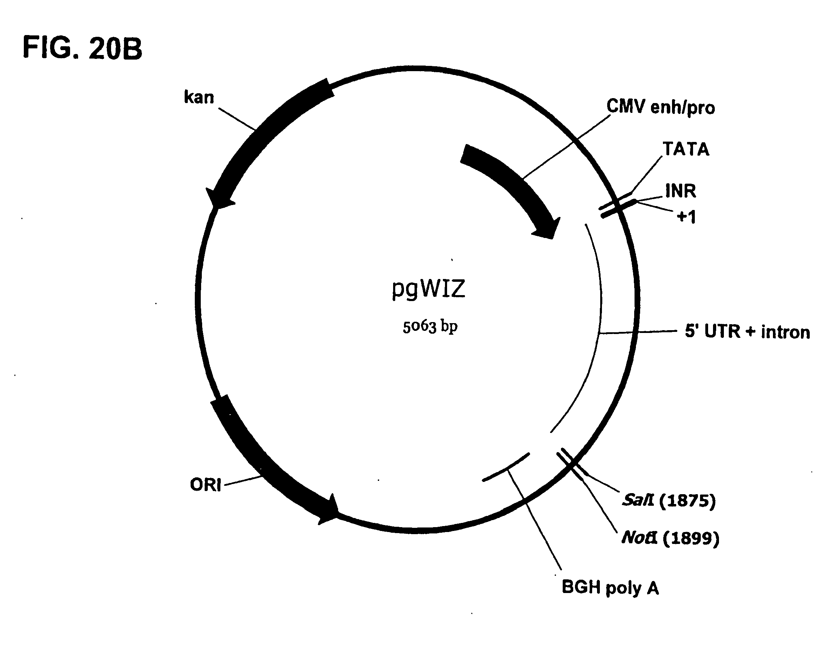

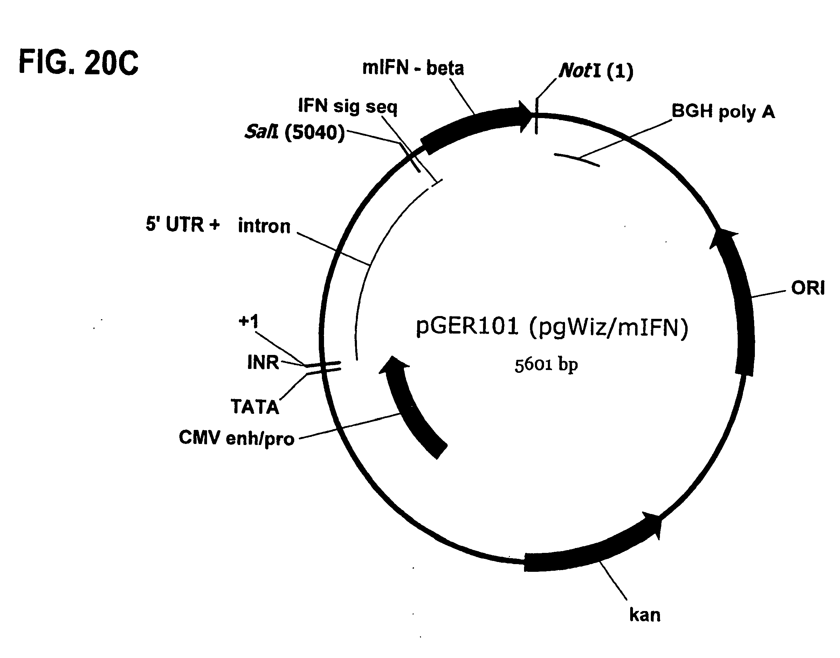

**Figure 20** illustrates plasmid vectors pbSER189 (A) and pgWIZ (B) used in the construction of plasmid vector pGER (pgWiz/mIFN) (C), as fully described in the Materials and Methods, subsection F.

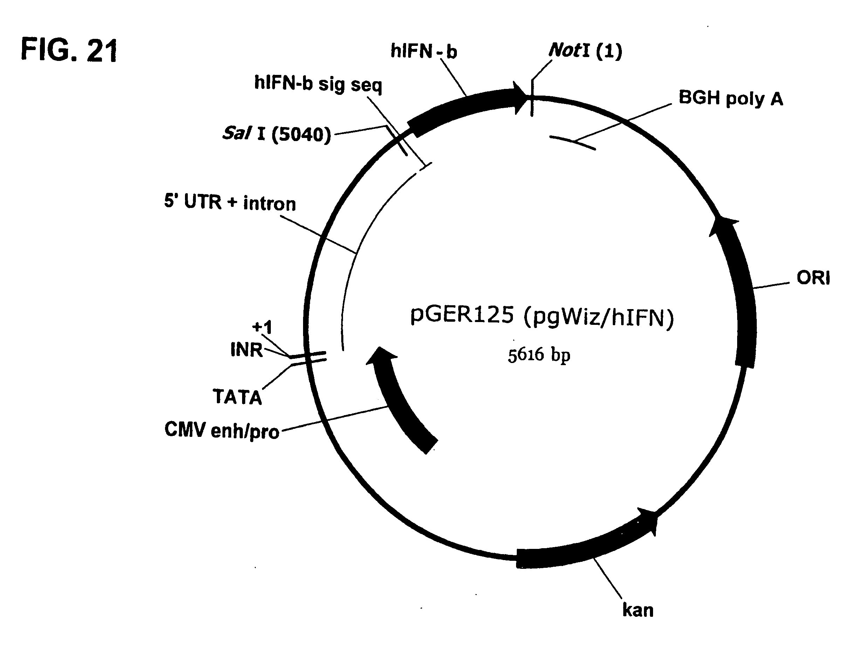

**Figure 21** illustrates the plasmid vector pGER125 (pgWiz/hIFN) as fully described in the Materials and Methods, subsection F.

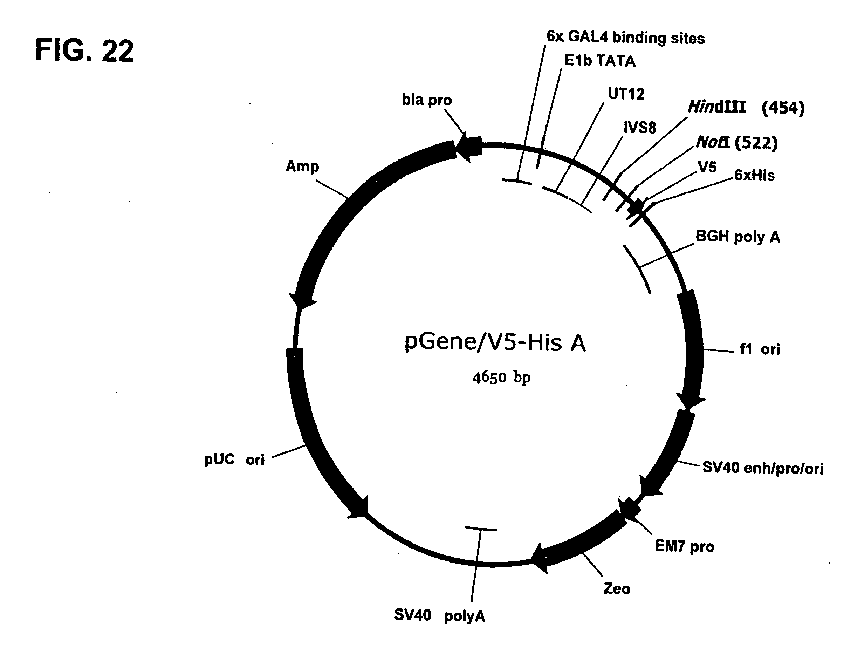

**Figure 22** illustrates the plasmid vector pGene/V5-HisA as fully described in the Materials and Methods, subsection F.

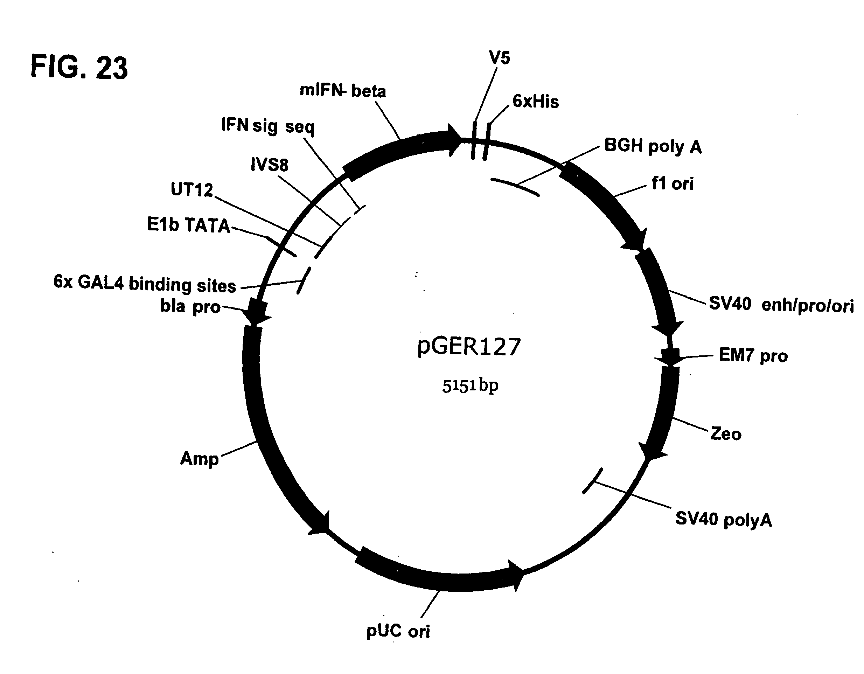

**Figure 23** illustrates the plasmid vector pGene-mIFN (pGER127) as fully described in the Materials and Methods, subsection F.

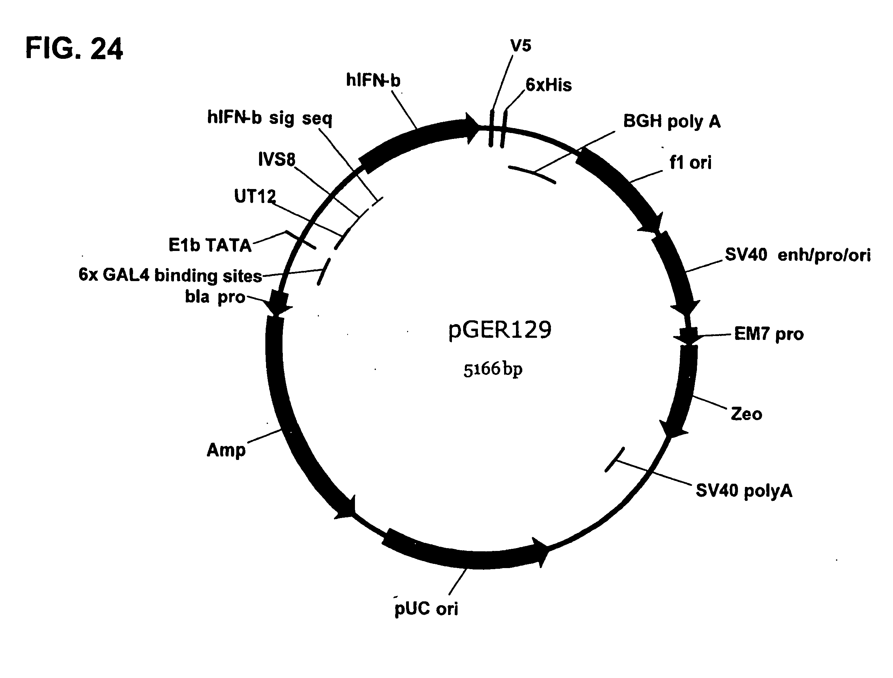

**Figure 24** illustrates the plasmid vector pGene-hIFN (pGER129) as fully described in the Materials and Methods, subsection F.

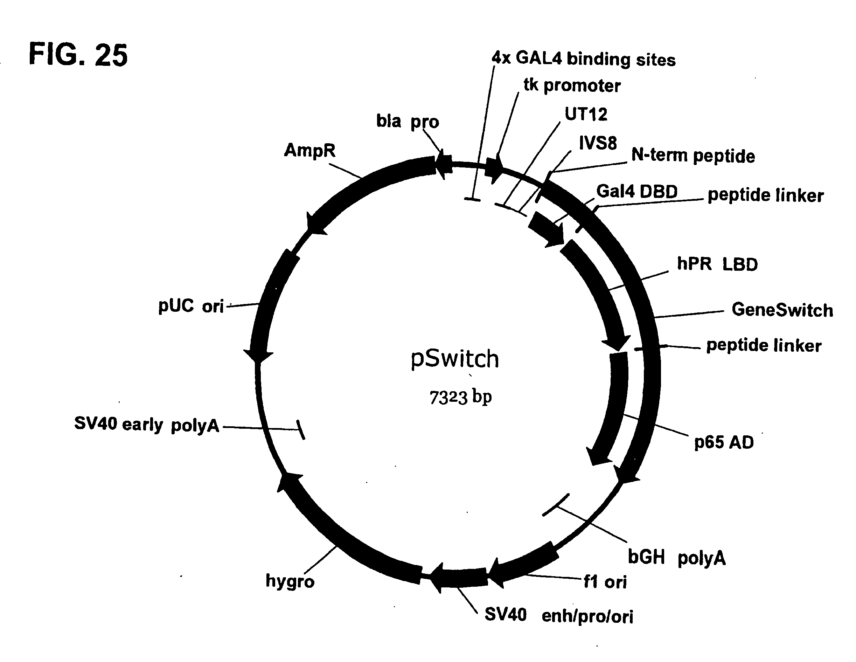

**Figure 25** illustrates the plasmid vector pSwitch (Invitrogen) as fully described in the Materials and Methods, subsection F.

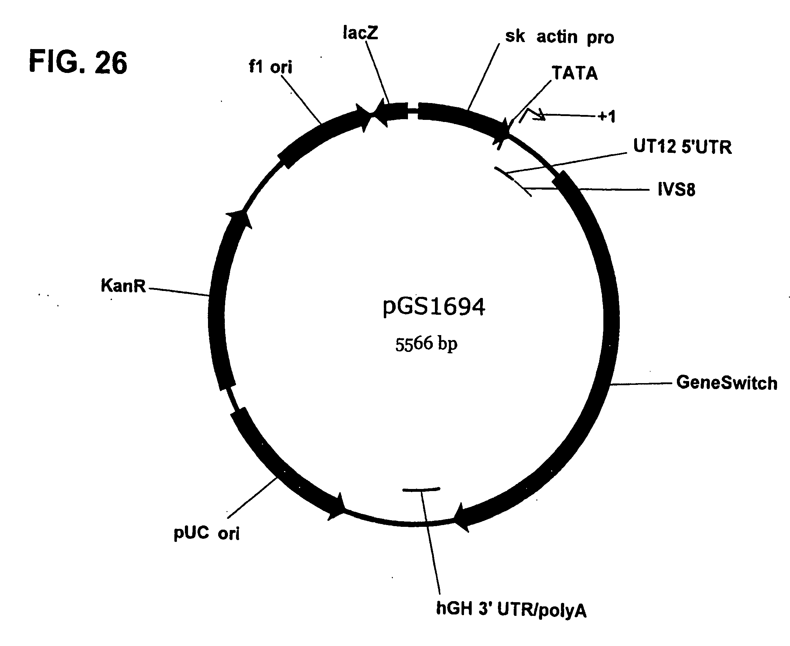

**Figure 26** illustrates the plasmid vector pGS1694 as fully described in the Materials and Methods, subsection F.

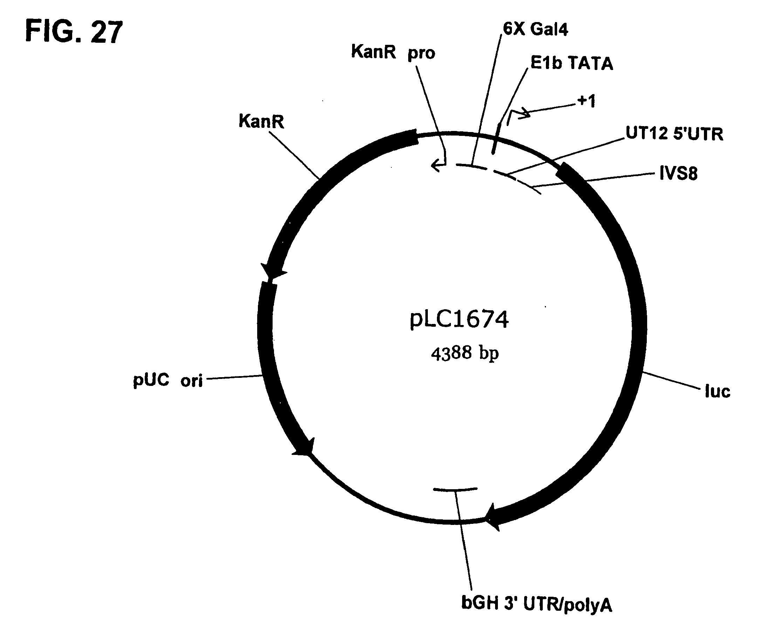

**Figure 27** illustrates the plasmid vector pLC1674 as fully described in the Materials and Methods, subsection F.

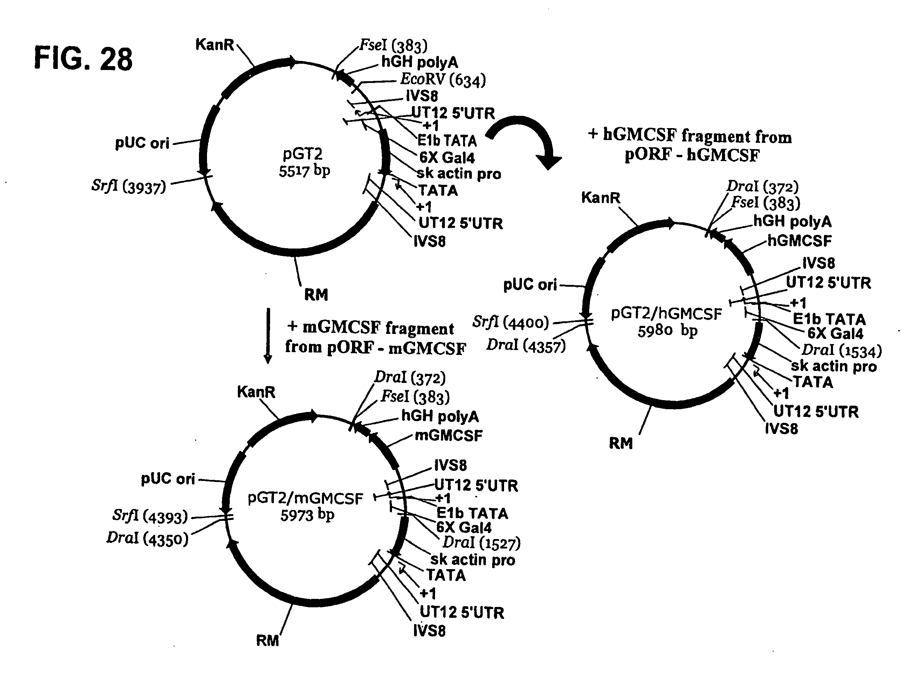

**Figure 28** illustrates the pGT-hGMCSF and pGT-mGMCSF shuttle plasmids and construction thereof, as fully described in the Materials and Methods, subsection F.

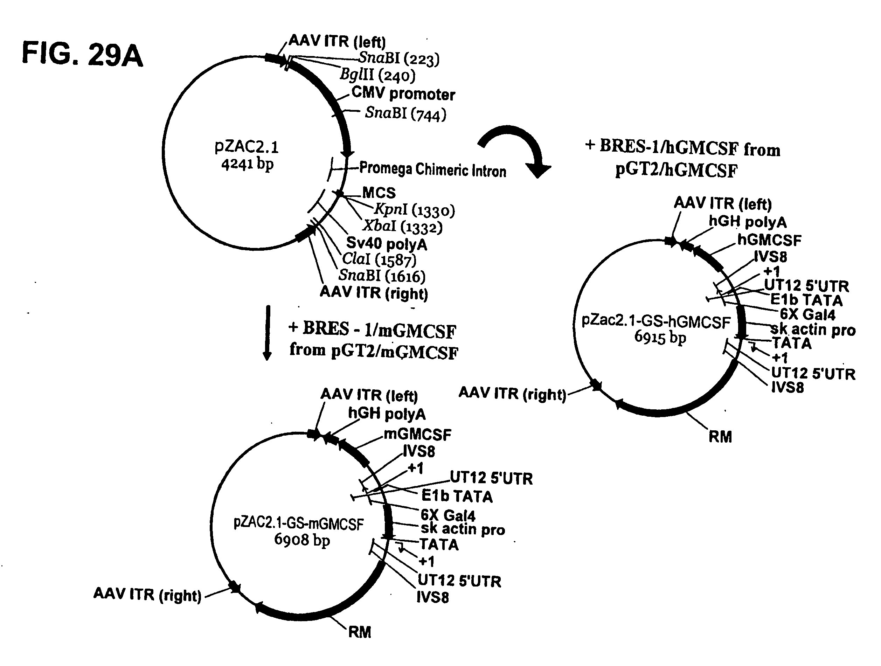

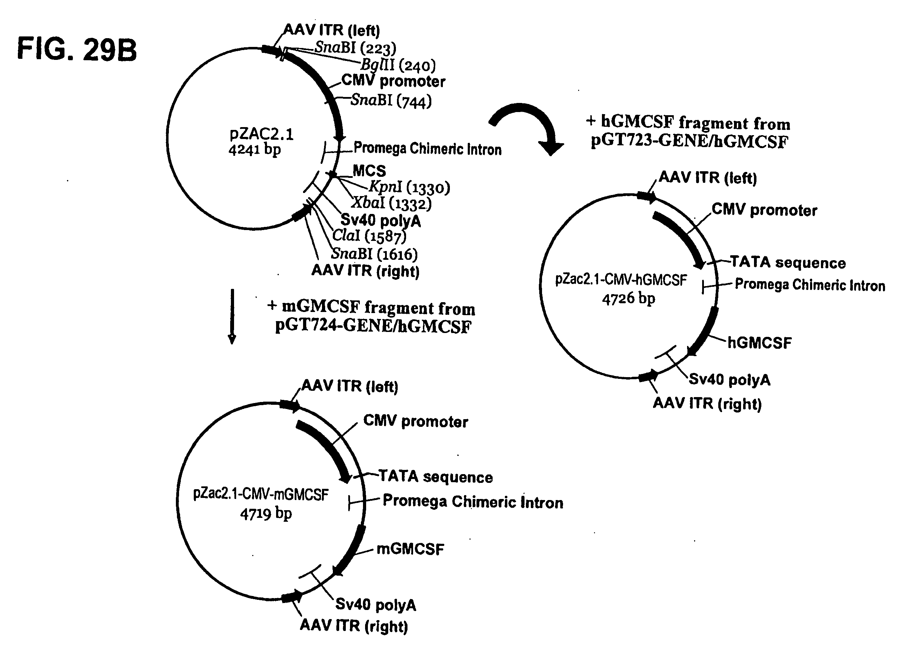

**Figure 29** illustrates the pZac2.1-RM-hGMCSF and pZac2.1-RM-mGMCSF (A) and pZac2.1-CMV-hGMCSF (pGT713) and pZac2.1-CMV-mGMCSF (pGT714) (B) shuttle plasmids and construction thereof, as fully described in the Materials and Methods, subsection F.

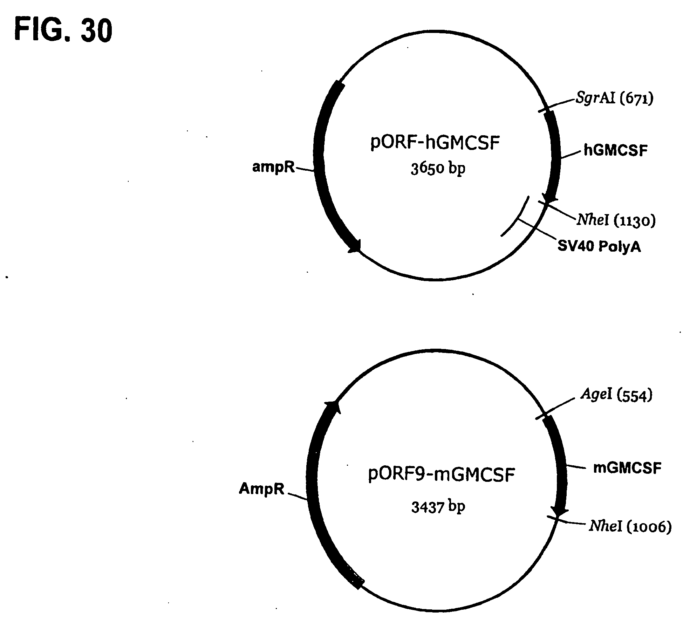

**Figure 30** illustrates the pORF-hGMCSF and pORF9-mGMCSF used in the construction of pZac2.1-RM-hGMCSF and pZac2.1-RM-mGMCSF, respectively, as fully described in the Materials and Methods, subsection F.

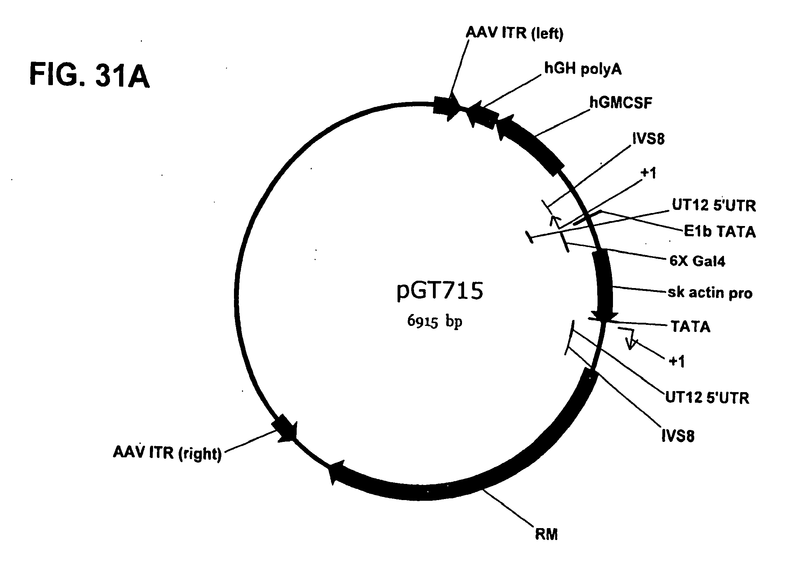

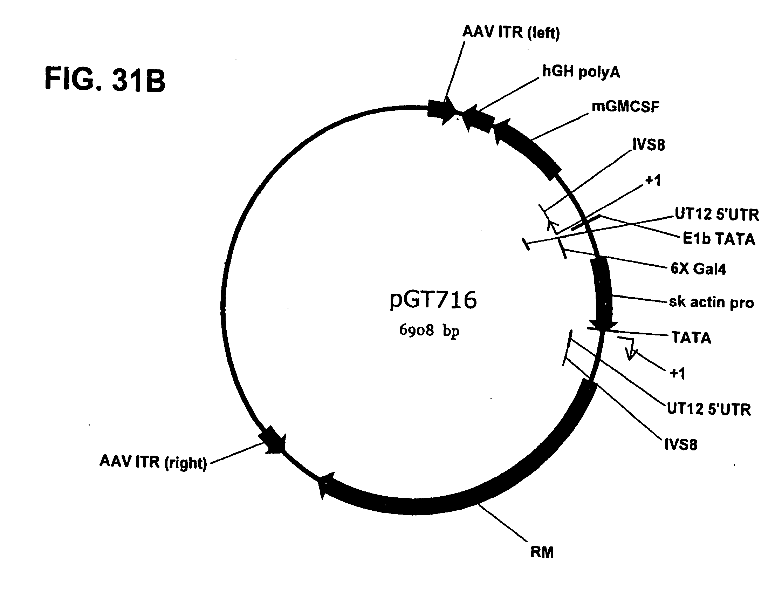

**Figure 31** illustrates the pGT715 (A) and pGT716 (B) shuttle plasmids, as fully described in the Materials and Methods, subsection F.

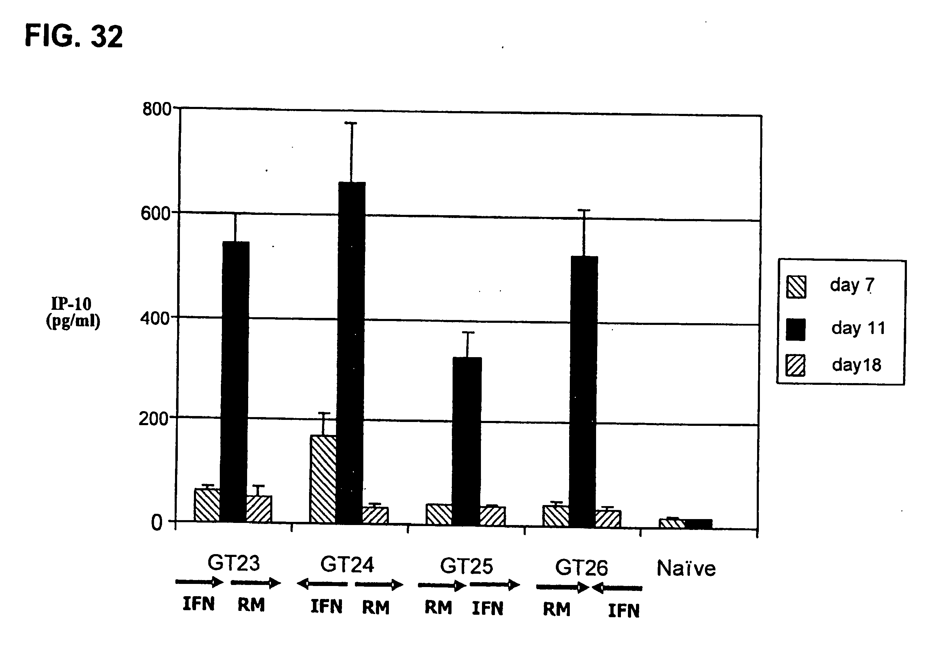

**Figure 32** illustrates IP-10 induction in vivo with mIFN-ÿ regulated expression plasmid vectors of the present invention. Inducible (pGT23, pGT24, pGT25, and pGT26) mIFN-ÿ expression plasmids were injected and electroporated into hind limb muscles of C57BI/6 mice. Animals were bled and the serum was assayed for the chemokine IP-10 by ELISA on day 7 (absence of MFP), day 11 (following four consecutive days of oral administration of MFP, and day 18. The results are shown as the mean (n=5 animals per group) +/- the standard deviation.

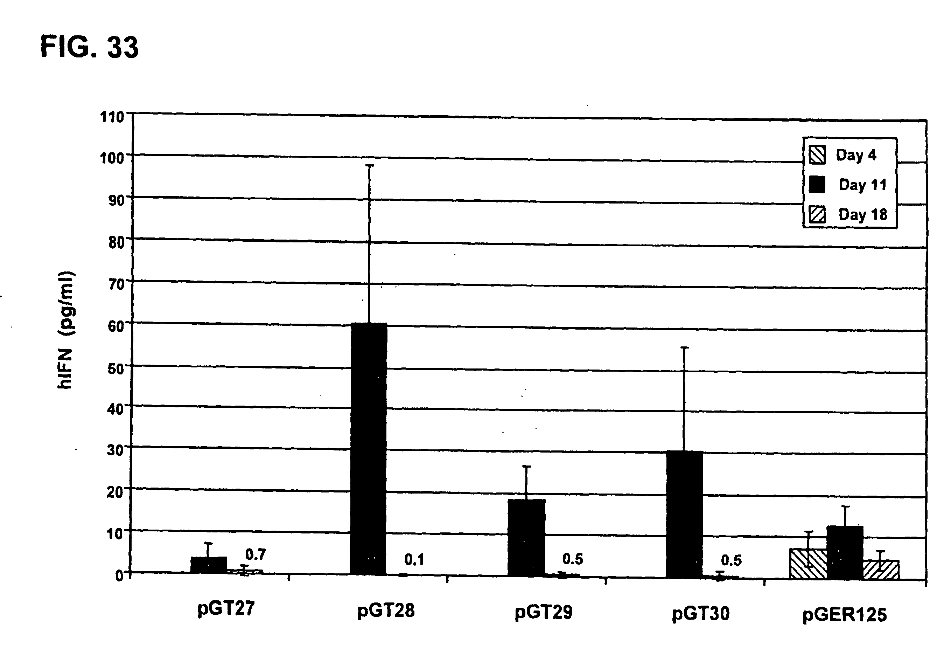

**Figure 33** illustrates hIFN induction in vivo with hIFN-ÿ regulated expression plasmid vectors of the present invention. Constitutive (pGER125) and inducible (pGT27, pGT28, pGT29, and pGT30) hIFN-ÿ expression plasmids were injected and electroporated into hind limb muscles of C57BI/6 mice. Animals were bled and the serum was assayed for hIFN by ELISA on day 7 (absence of MFP), day 11 (following four consecutive days of oral administration of MFP), and day 18. The results are shown as the mean (n=5 animals per group) +/- the standard deviation.

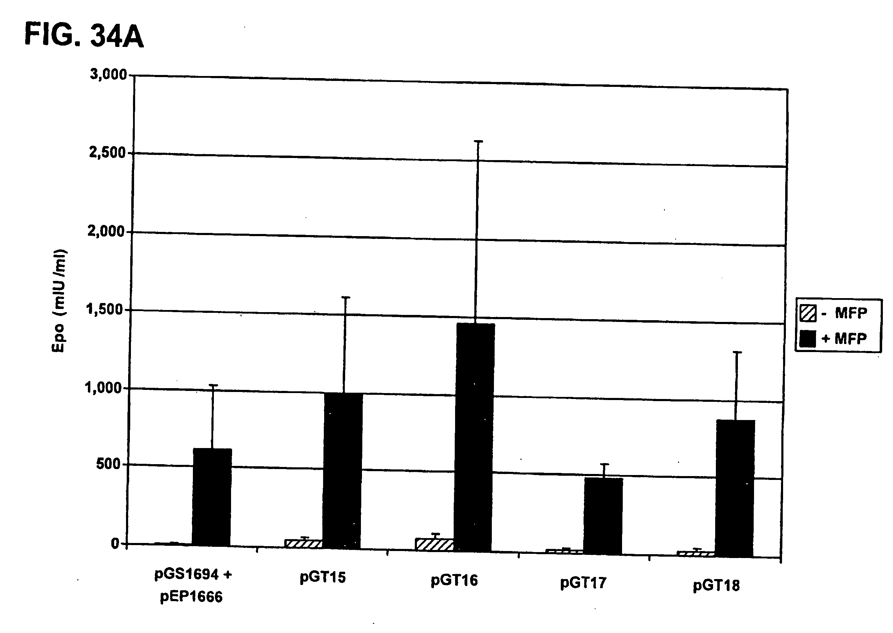

**Figure 34A** illustrates hEPO induction in vivo with hEPO regulated expression plasmid vectors of the present invention. Inducible two-plasmid (pGS1694 + pEP1666) and one-plasmid BRES-1 (pGT27, pGT28, pGT29, and pGT30) hEPO expression plasmids were injected and electroporated into hind limb muscles of C57BI/6 mice. Five animals of each group were administered MFP by i.p. injection for four consecutive days (7-10) and all bled 6 hr after the last MFP injection. The remaining five animals of each group were bled on day 10 in the absence of MFP treatment. Serum was assayed for hEPO by ELISA. The results are shown as the mean (n=5 animals per group) +/- the standard deviation.

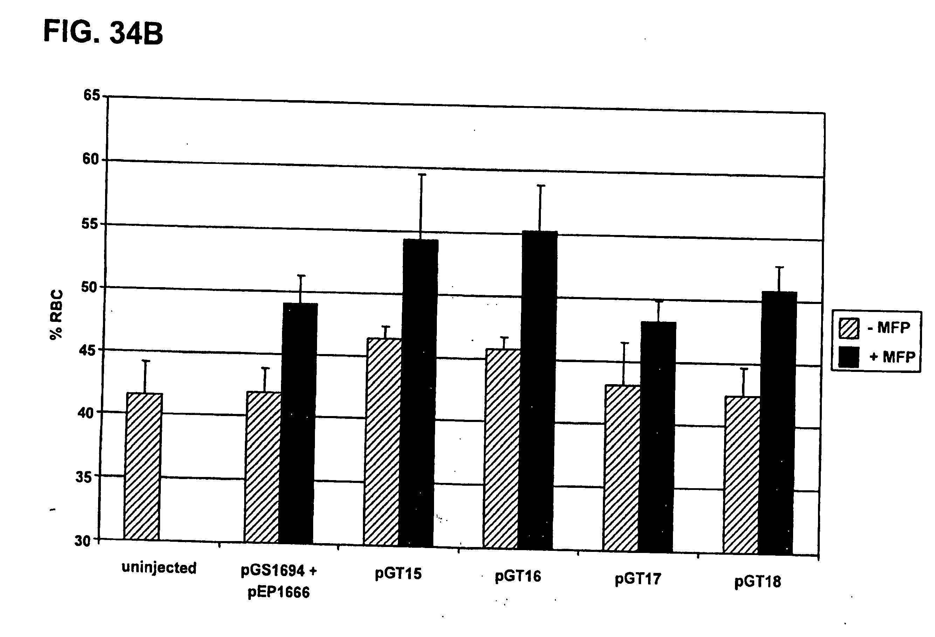

**Figure 34B** illustrates induction of hematocrit count in vivo with hEPO regulated expression plasmid vectors of the present invention. Inducible two-plasmid (pGS1694 + pEP1666) and one-plasmid BRES-1 (pGT27, pGT28, pGT29, and pGT30) hEPO expression plasmids were injected and electroporated into hind limb muscles of C57BI/6 mice and animals were treated with MFP or left untreated and bled as above. Blood was clotted and centrifuged in microcapillary tubes and the % red blood cells (RBC) was measured. The results are shown as the mean (n=5 animals per group) +/- the standard deviation.

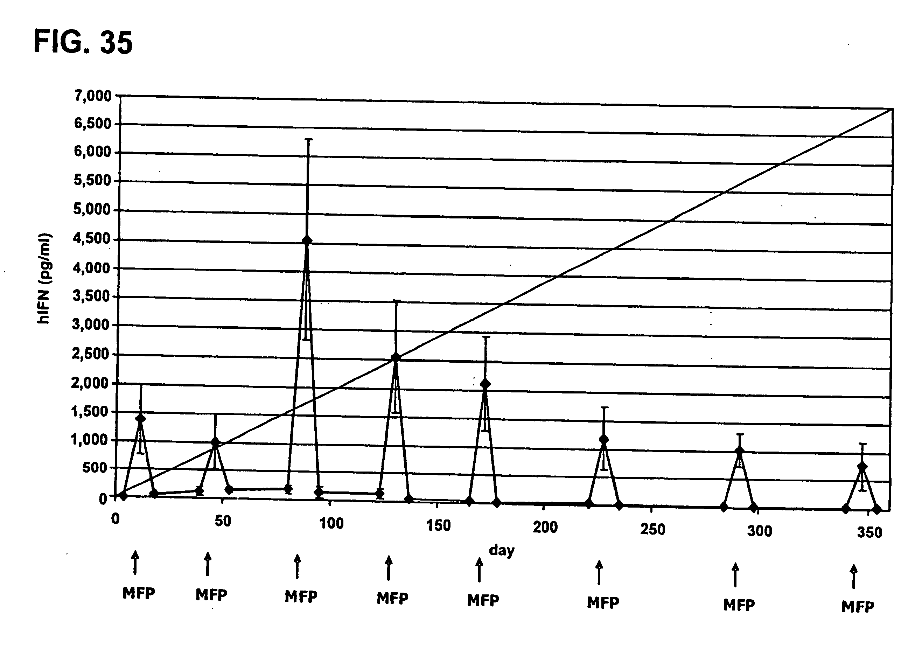

**Figure 35** illustrates long-term, persistent, multiple hIFN inductions in vivo with a hIFN-ÿ regulated expression AAV vector of the present invention. The inducible hIFN-ÿ expression AAV vector AAV-1-GT58 was injected into hind limb muscles of C57BI/6 mice. Animals were bled and the serum was assayed for hIFN by ELISA in the absence or presence of MFP (four consecutive days of i.p. injections) as indicated. The results are shown as the mean (n=5 animals per group) +/- the standard deviation.

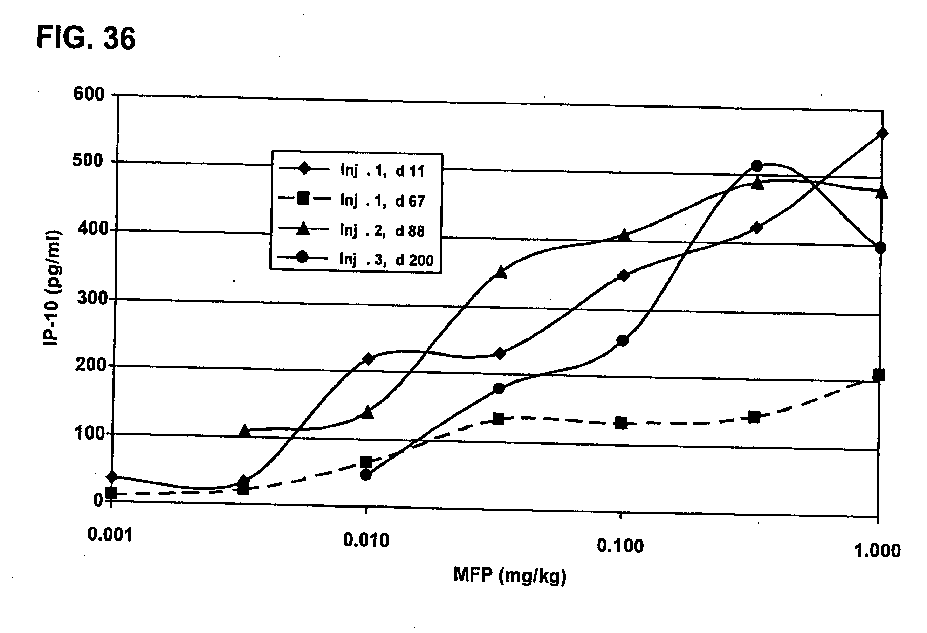

**Figure 36** illustrates long-term, persistent, multiple IP-10 inductions in response to increasing dosages of MFP in vivo with repeated administrations of a mIFN-ÿ regulated expression plasmid vector of the present invention. The inducible mIFN-ÿ expression plasmid pGT26 was injected and electroporated on day 0 into hind limb muscles of C57BI/6 mice. Animals were administered MFP at various concentrations by i.p. injection for four consecutive days (day 7-10 and 63-66) and then bled the following day (day 11 and 67) Plasmid DNA was re-injected on day 77 and 189. MFP treatments after plasmid re-injection were on day 84-87 and 196-199, respectively. Bleeds were taken on day 88 and 200, respectively. Serum was assayed for the chemokine IP-10 by ELISA. The results are shown as the mean (n=5 animals per group).

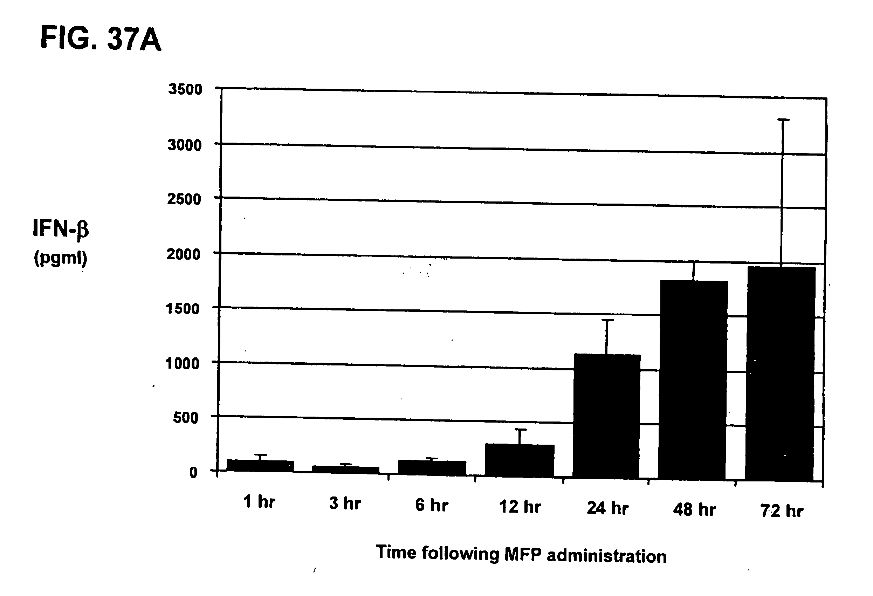

**Figure 37A** illustrates the kinetics of hIFN induction in vivo with a hIFN-ÿ regulated expression AAV vector of the present invention. The inducible hIFN-ÿ expression AAV vector AAV-1GT58 was injected into hind limb muscles of C57BI/6 mice. Animals were administered MFP by i.p. injection for four consecutive days and then bled at various times after the first MFP injection as indicated in the chart. Serum was assayed for hIFN by ELISA. The results are shown as the mean (n=5 animals per group) +/- the standard deviation.

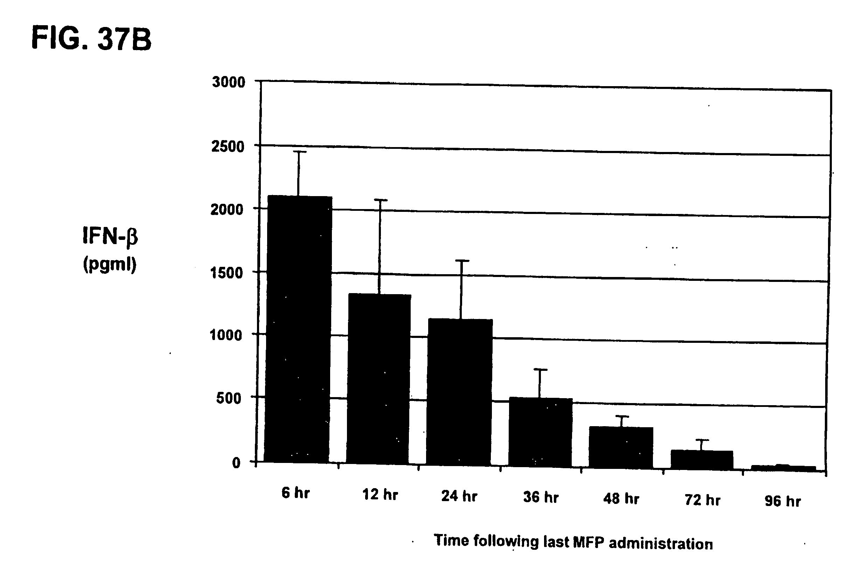

**Figure 37B** illustrates the kinetics of hIFN de-induction in vivo with a hIFN-ÿ regulated expression AAV vector of the present invention. The inducible hIFN-ÿ expression AAV vector AAV-1GT58 was injected into hind limb muscles of C57BI/6 mice. Animals were administered MFP by i.p. injection for four consecutive days and then bled at various times after the last MFP injection as indicated in the chart. Serum was assayed for hIFN by ELISA. The results are shown as the mean (n=5 animals per group) +/- the standard deviation.

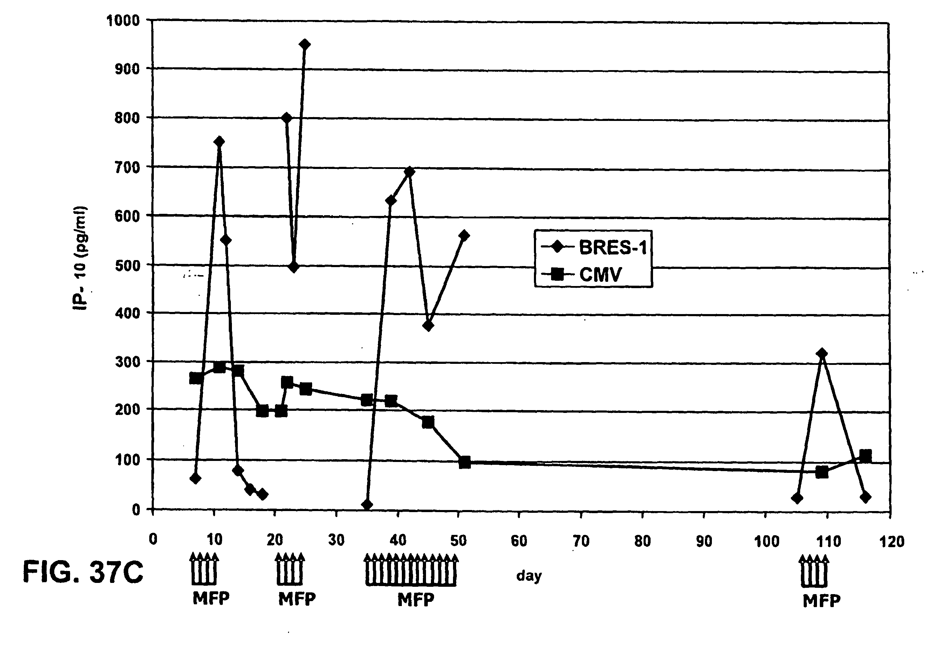

**Figure 37C** illustrates the kinetics of mIFN induction and de-induction, response to pulsatile or chronic MFP treatment, and the persistence of gene expression over several months with a mIFN-ÿ regulated expression plasmid vector of the present invention. Constitutive (pGER101, CMV) and inducible (BRES-1, pGT26) mIFN expression plasmids were injected with electroporation into the hind limb muscles mice, and animals were bled at various time points before, during, or after MFP treatment as indicated on the chart. Serum was assayed for the chemokine IP-10 by ELISA. The results are shown as the mean (n=5 animals per group).

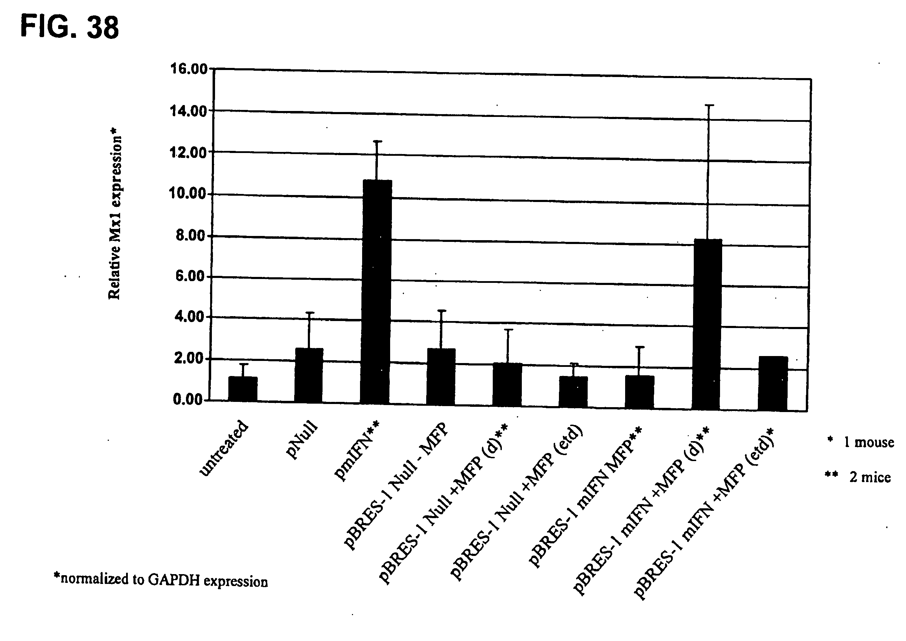

**Figure 38** illustrates Mx-1 induction in vivo with a mIFN-ÿ regulated expression plasmid vector of the present invention. The inducible mIFN-ÿ expression plasmid pBRES-1 mIFN (pGT26) or pBRES-1 Null-MFP (control) plasmid was injected and electroporated into hind limb muscles of SJL mice with acute EAE. Mice were treated with MFP (0.33 mg/kg) by i.p. injection once per day (d) or every third day (etd) after plasmid injection. Blood was collected at day 5 after injection. PBMCs were isolated from the blood and RNA was prepared from and assayed by RT-PCR to determine the level of Mx1 RNA. Mx1 expression levels were normalized to GAPDH. The results are shown as the mean +/- the standard deviation.

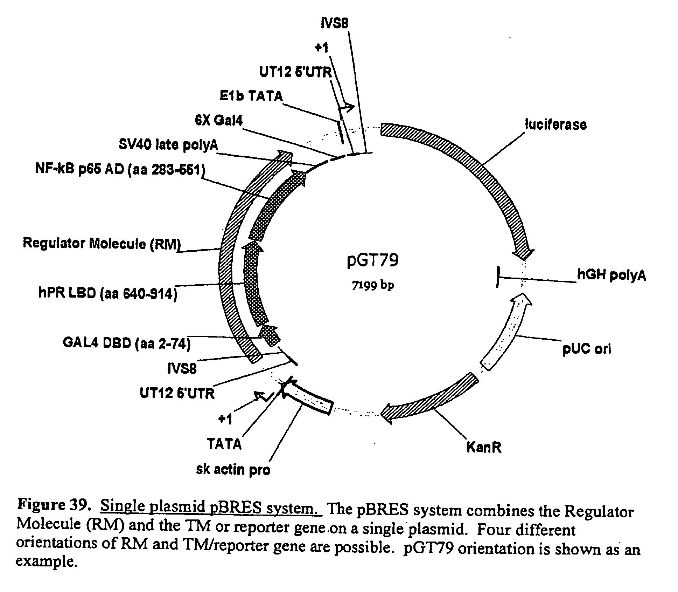

**Figure 39** illustrates a regulated expression system that combines the Regulator Molecule (RM) and the Therapeutic Molecule (TM) or reporter gene on a single plasmid. Four different orientations of RM and TM/reporter gene are possible. pGT79 orientation is shown as an example.

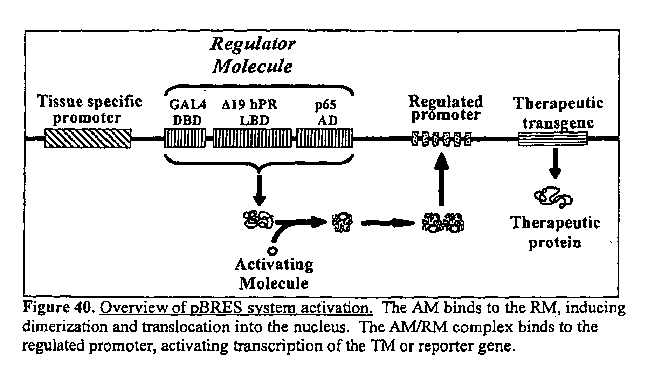

**Figure 40** provides an overview on the activation of the regulated expression system of the present invention. The AM binds to the RM, and induces its dimerization and translocation into the nucleus. The AM/RM complex thus formed binds to the regulated promoter and activates transcription of the TM or reporter gene.

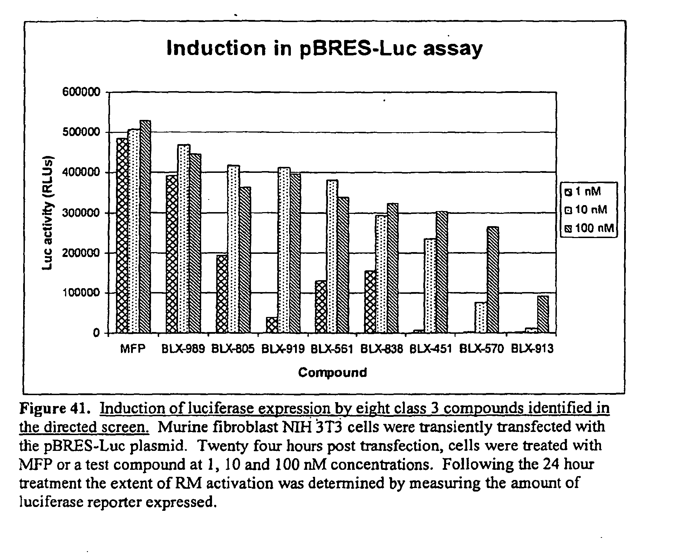

**Figure 41** illustrates the induction of luciferase expression by eight compounds identified in the directed screen (see Examples 10 and 11) as featuring conservative changes in their structure and retaining antiprogestin-like structure similar to more potent antiprogestin compounds. Murine fibroblast NIH 3T3 cells were transiently transfected with the pBRES-Luc plasmid. Twenty four hours post transfection, cells were treated with MFP or a test compound at 1, 10 and 100 nM concentrations. Following the 24 hour treatment, the extent of RM activation was determined by measuring the amount of luciferase reporter expressed.

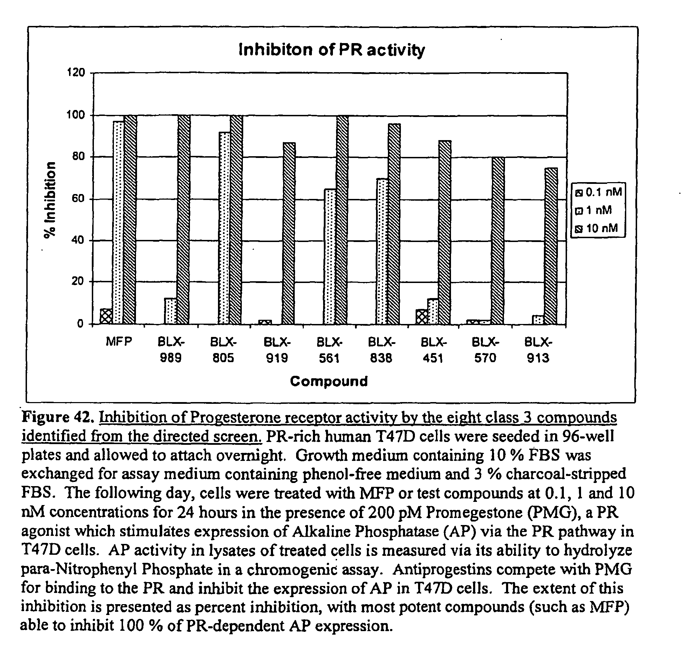

**Figure 42** illustrates the inhibition of progesterone receptor activity by eight compounds identified in the directed screen as featuring conservative changes in their structure and retaining antiprogestin-like structure similar to more potent antiprogestin compounds. PR-rich human T47D cells were seeded in 96-well plates and allowed to attach overnight. Growth medium containing 10 % FBS was exchanged for assay medium containing phenol-free medium and 3 % charcoal-stripped FBS. The following day, cells were treated with MFP or test compounds at 0.1, 1 and 10 nM concentrations for 24 hours in the presence of 200 pM Promegestone (PMG), a PR agonist which stimulates expression of Alkaline Phosphatase (AP) via the PR pathway in T47D cells. AP activity in lysates of treated cells was measured via its ability to hydrolyze para-Nitrophenyl Phosphate in a chromogenic assay. Antiprogestins compete with PMG for binding to the PR and inhibit the expression of AP in T47D cells. The extent of this inhibition is presented as percent inhibition, with most potent compounds (such as MFP) able to inhibit 100 % of PR-dependent AP expression.

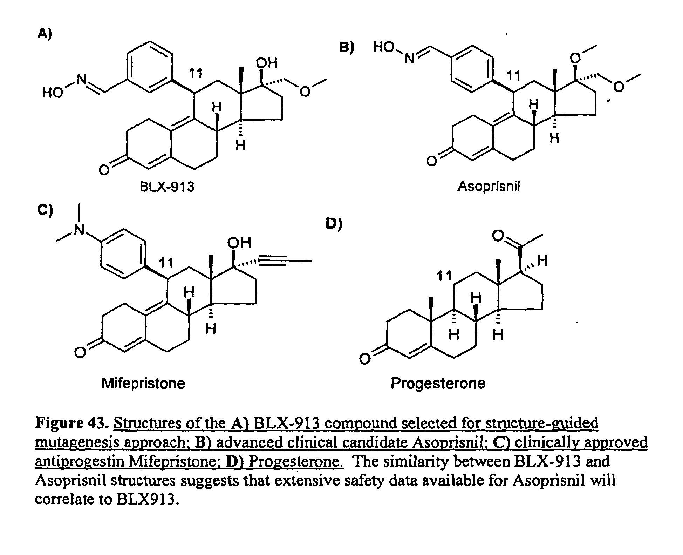

**Figure 43** provides the structures of the A) BLX-913 compound selected for structure-guided mutagenesis approach; B) advanced clinical candidate Asoprisnil; C) clinically approved antiprogestin Mifepristone; D) Progesterone. The similarity between BLX-913 and Asoprisnil structures suggests a strong correlation between the safety data for Asoprisnil and those for BLX913.

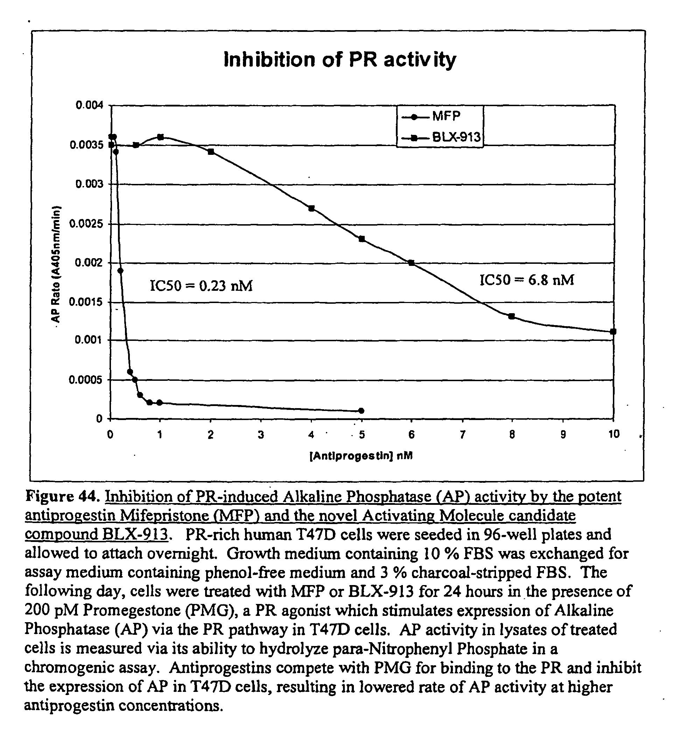

**Figure 44** illustrates the inhibition of PR-induced Alkaline Phosphatase (AP) activity by the potent antiprogestin Mifepristone (MFP) and the new Activating Molecule candidate compound BLX-913. PR-rich human T47D cells were seeded in 96-well plates and allowed to attach overnight. Growth medium containing 10 % FBS was exchanged for assay medium containing phenol-free medium and 3 % charcoal-stripped FBS. The following day, cells were treated with MFP or BLX-913 for 24 hours in the presence of 200 pM Promegestone (PMG), a PR agonist which stimulates expression of Alkaline Phosphatase (AP) via the PR pathway in T47D cells. AP activity in lysates of treated cells is measured via its ability to hydrolyze para-Nitrophenyl Phosphate in a chromogenic assay. Antiprogestins compete with PMG for binding to the PR and inhibit the expression of AP in T47D cells, resulting in lowered rate of AP activity at higher antiprogestin concentrations.

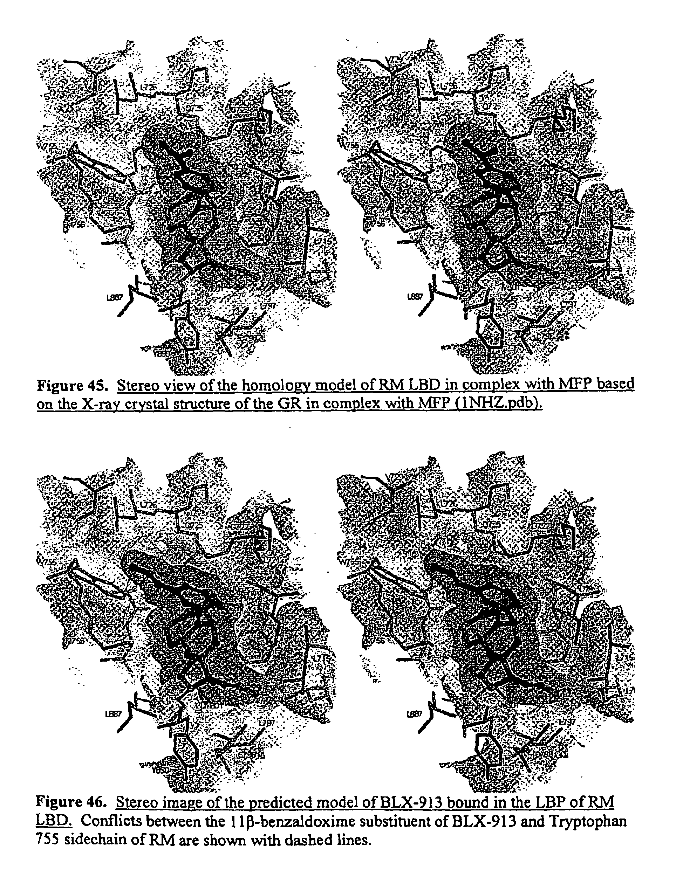

**Figure 45** provides a stereo view of the homology model of RM LBD in complex with MFP based on the X-ray crystal structure of the GR in complex with MFP (1NHZ.pdb).
**Figure 46** provides a stereo image of the predicted model of BLX-913 bound in the LBP of RM LBD. Conflicts between the 11β-benzaldoxime substituent of BLX-913 and Tryptophan 755 sidechain of RM are shown with dashed lines.

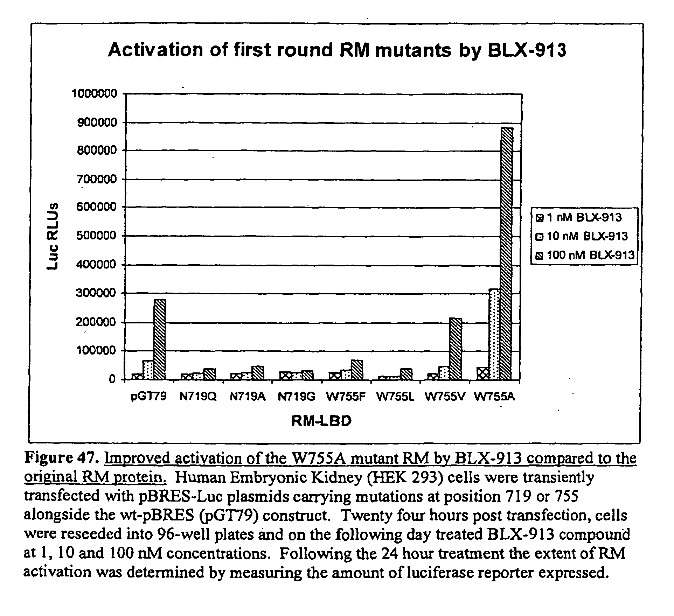

**Figure 47** illustrates improved activation of the W755A mutant RM by BLX-913 compared to activation of the original RM protein. Human Embryonic Kidney (HEK 293) cells were transiently transfected with pBRES-Luc plasmids carrying mutations at position 719 or 755 alongside the wt-pBRES (pGT79) construct. Twenty four hours post transfection, cells were reseeded into 96-well plates and on the following day treated with BLX-913 compound at 1, 10 and 100 nM concentrations. Following the 24 hour treatment the extent of RM activation was determined by measuring the amount of luciferase reporter expressed.

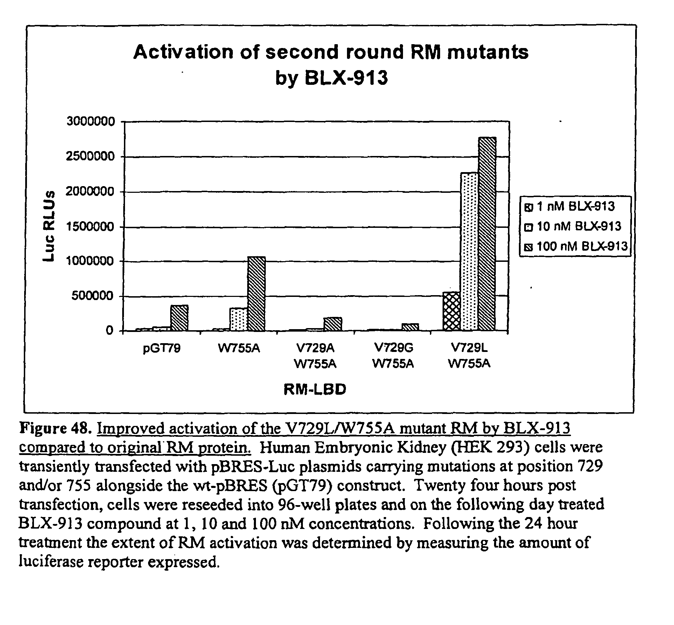

**Figure 48** illustrates improved activation of the V729L/W755A mutant RM by BLX-913 compared to activation of the original RM protein. Human Embryonic Kidney (HEK 293) cells were transiently transfected with pBRES-Luc plasmids carrying mutations at position 729 and/or 755 alongside the wt-pBRES (pGT79) construct. Twenty four hours post transfection, cells were reseeded into 96-well plates and on the following day treated with BLX-913 compound at 1, 10 and 100 nM concentrations. Following the 24 hour treatment the extent of RM activation was determined by measuring the amount of luciferase reporter expressed.

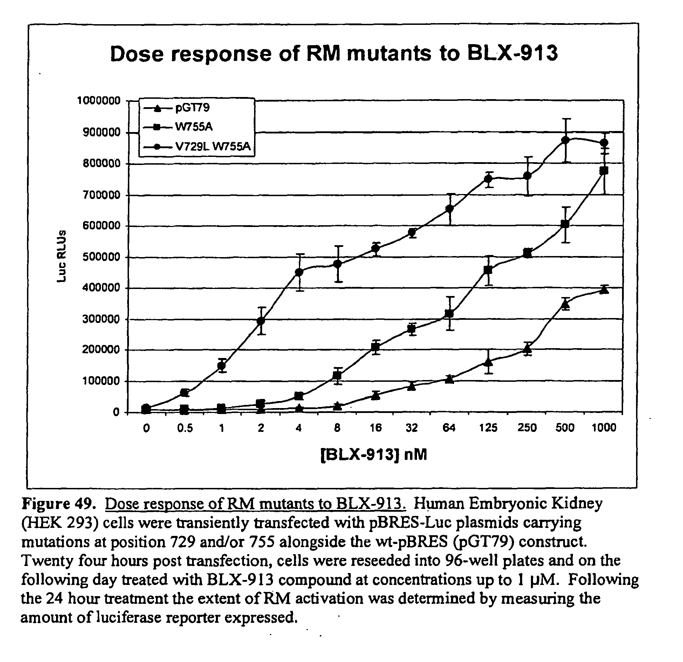

**Figure 49** illustrates the dose response of RM mutants to BLX-913. Human Embryonic Kidney (HEK 293) cells were transiently transfected with pBRES-Luc plasmids carrying mutations at position 729 and/or 755 alongside the wt-pBRES (pGT79) construct. Twenty four hours post transfection, cells were reseeded into 96-well plates and on the following day treated with BLX-913 compound at concentrations up to 1 µM. Following the 24 hour treatment, the extent of RM activation was determined by measuring the amount of luciferase reporter expressed.

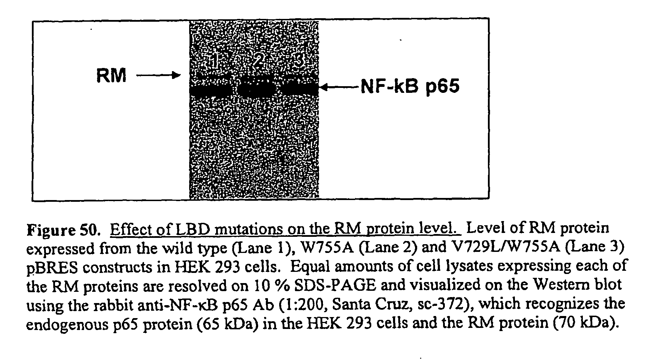

**Figure 50** illustrates the effect of LBD mutations on the RM protein level. The level of RM protein expressed from the wild type (pGT79) (Lane 1), W755A (pGT1009) (Lane 2) and V729L/W755A (pGT1017) (Lane 3) pBRES constructs in HEK 293 cells is shown. Equal amounts of cell lysates expressing each of the RM proteins were resolved on 10 % SDS-PAGE and visualized on the Western blot using the rabbit anti-NF-κB p65 Ab (1:200, Santa Cruz, sc-372), which recognizes the endogenous p65 protein (65 kDa) in the HEK 293 cells and the RM protein (70 kDa).

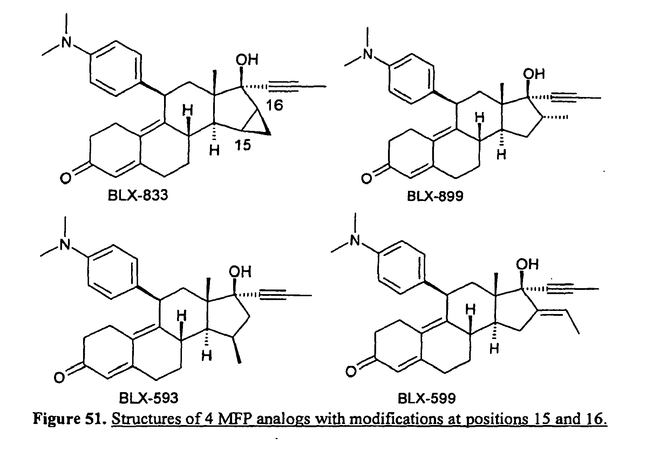

**Figure 51** provides the structures of four MFP analogs with modifications at positions 15 and 16.

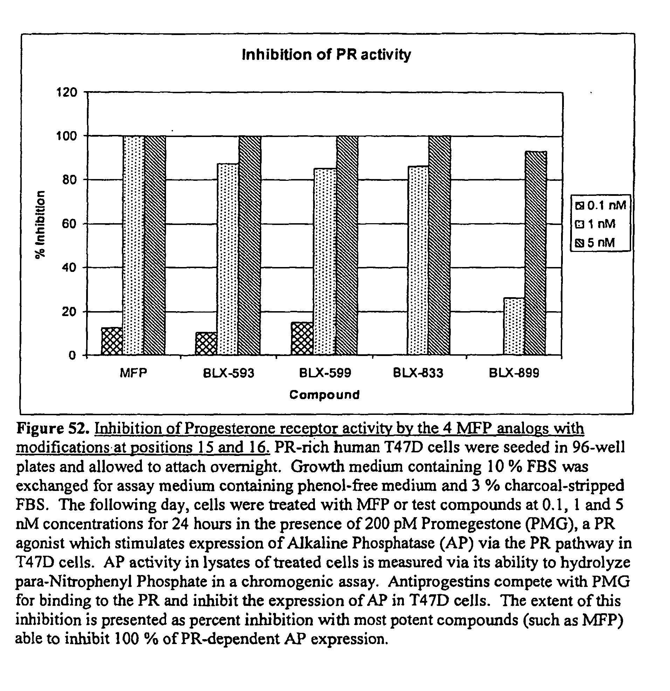

**Figure 52** illustrates the inhibition of Progesterone receptor activity by four MFP analogs with modifications at positions 15 and 16. PR-rich human T47D cells were seeded in 96-well plates and allowed to attach overnight. Growth medium containing 10 % FBS was exchanged for assay medium containing phenol-free medium and 3 % charcoal-stripped FBS. The following day, cells were treated with MFP or test compounds at 0.1, 1 and 5 nM concentrations for 24 hours in the presence of 200 pM Promegestone (PMG), a PR agonist which stimulates expression of Alkaline Phosphatase (AP) via the PR pathway in T47D cells. AP activity in lysates of treated cells was measured via its ability to hydrolyze para-Nitrophenyl Phosphate in a chromogenic assay. Antiprogestins compete with PMG for binding to the PR and inhibit the expression of AP in T47D cells. The extent of this inhibition is presented as percent inhibition with most potent compounds (such as MFP) able to inhibit 100 % of PR-dependent AP expression.

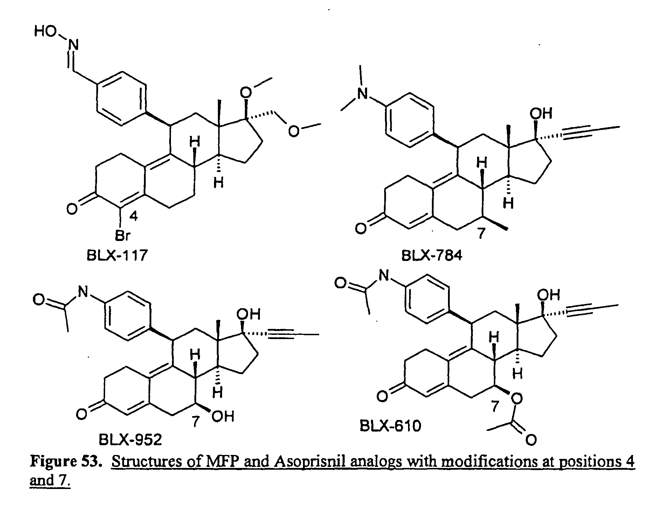

**Figure 53** provides the structures of MFP and Asoprisnil analogs with modifications at positions 4 and 7.

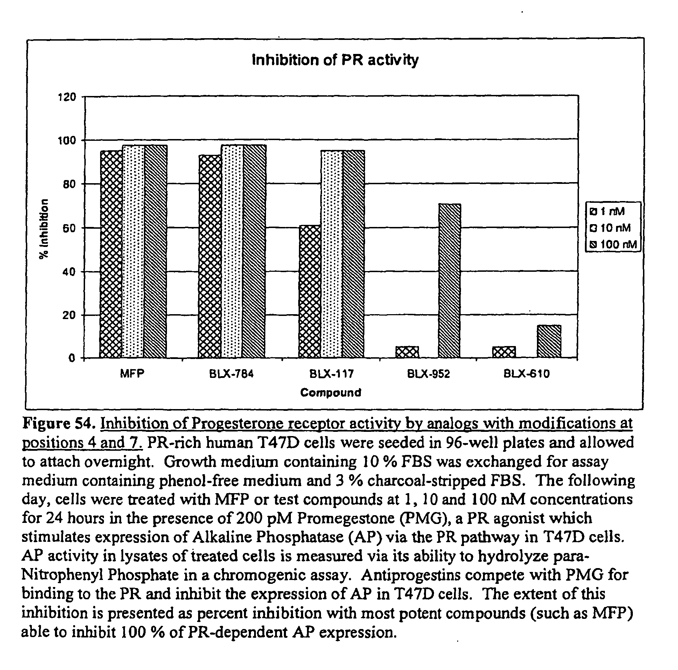

**Figure 54** illustrates the inhibition of Progesterone receptor activity by MFP analogs with modifications at positions 4 and 7. PR-rich human T47D cells were seeded in 96-well plates and allowed to attach overnight. Growth medium containing 10 % FBS was exchanged for assay medium containing phenol-free medium and 3 % charcoal-stripped FBS. The following day, cells were treated with MFP or test compounds at 1, 10 and 100 nM concentrations for 24 hours in the presence of 200 pM Promegestone (PMG), a PR agonist which stimulates expression of Alkaline Phosphatase (AP) via the PR pathway in T47D cells. AP activity in lysates of treated cells was measured via its ability to hydrolyze para-Nitrophenyl Phosphate in a chromogenic assay. Antiprogestins compete with PMG for binding to the PR and inhibit the expression of AP in T47D cells. The extent of this inhibition is presented as percent inhibition with most potent compounds (such as MFP) able to inhibit 100 % of PR-dependent AP expression.

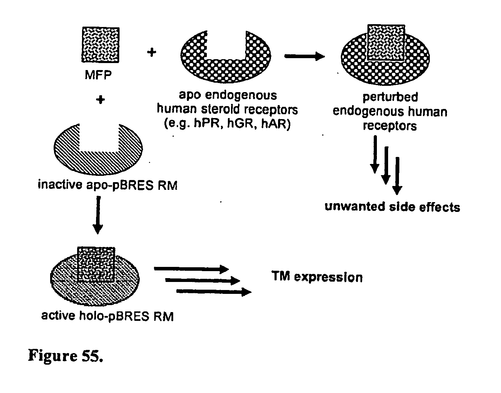

**Figure 55** illustrates an exemplary and non-limiting embodiment of the pBRES expression system. In this embodiment, binding of MFP to the RM activates the RM and results in the subsequent expression of the TM from the pBRES expression system. MFP is a known antagonist for several endogenous human steroid receptors, which can potentially result in unwanted side effects.

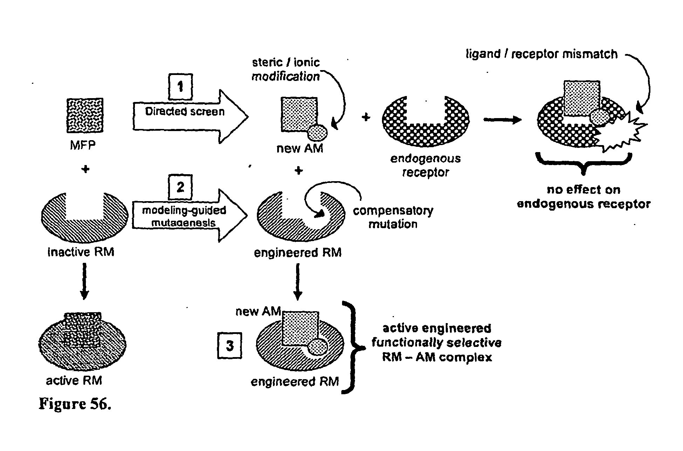

**Figure 56** illustrates an exemplary and non-limiting approach to screening for novel activator molecules and regulator molecules. **(1)** New AMs are identified from a directed screen of PR ligand analogs that do not interact with the endogenous human PR due to the presence of steric or ionic perturbations on the compound. **(2)** Modeling guided mutagenesis is used to introduce compensatory mutations into the LBP of the RM, thereby creating engineered RM molecules. **(3)** The new AM is used in conjunction with the engineered RM to produce an active AM-RM complex with improved properties e.g., specific and tight regulation of TM expression, and a diminution or amelioration of unwanted side effects on or cross reaction with endogenous proteins, particularly endogenous human proteins, e.g., endogenous human steroid receptors. This exemplary and non-limiting approach can also be used to screen for novel inactivator molecules.

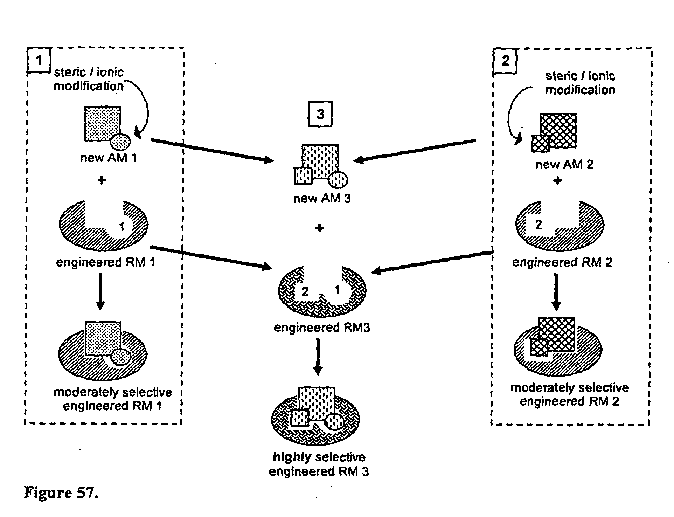

**Figure 57** illustrates an exemplary and non-limiting approach for developing a highly selective AM-RM complex using the SAR from two or more moderately selective AM-RM pairs. **(1)** New AM1 is identified using approach outlined in Fig. 57 and compensatory mutations are installed in region 1 within the LBP of the RM to create a moderately selective AM1-RM1 complex. **(2)** New AM2 is identified using approach outlined in Fig. 57 and compensatory mutations are made in region 2 within the LBP of the RM to create a moderately selective AM2-RM2 complex. **(3)** Using chemical synthesis, selectivity-imparting modifications from AM1 and AM2 are combined in one compound, AM3. The compensatory mutations in regions 1 and 2 within the LBP are combined within a single RM3 construct using site-directed mutagenesis. Taking advantage of two or more selectivity-imparting features, AM3 is used in conjunction with RM3 to yield a AM-RM complex with greater selectivity as compared to AM1 or AM2. This exemplary and non-limiting approach can also be used for developing a highly selective IM-RM complex.

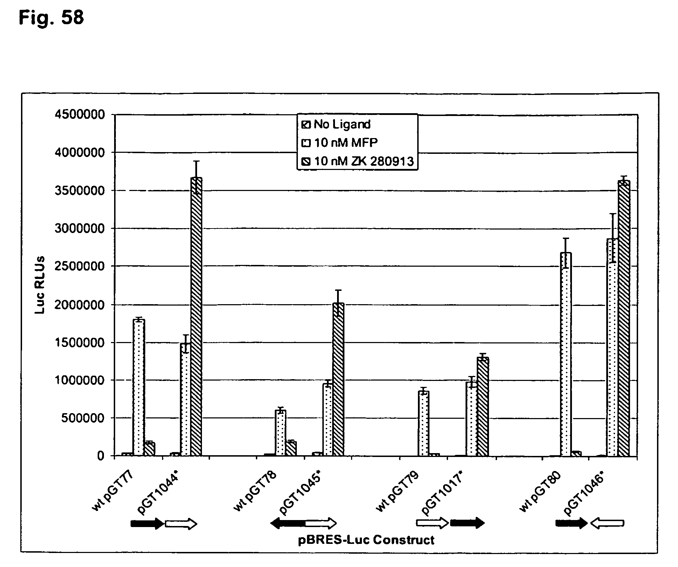

**Figur 58** shows induction of luciferase expression from pBRES with wt or V729L/W755A (*) RP in C2C12 mouse muscle cells. The orientations of the RP (open arrow) and luciferase (filled arrow) genes within each construct are shown.

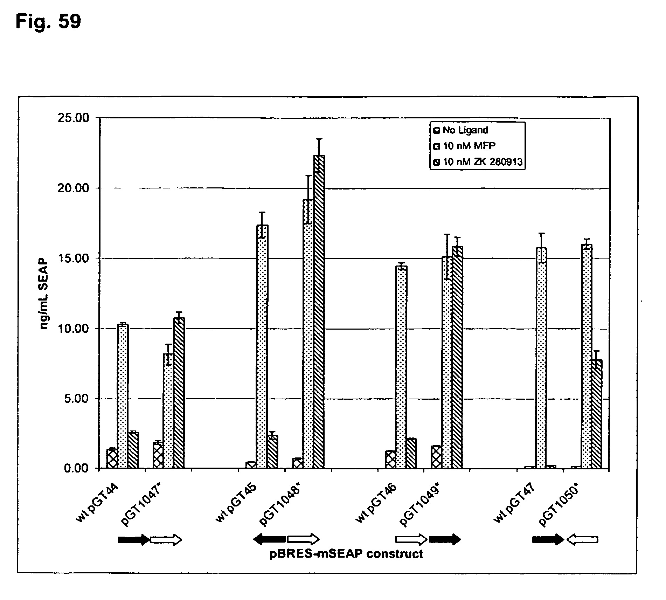

**Figur 59** shows induction of mSEAP expression from pBRES with wt or V729L/W755A (*) RP in C2C12 mouse muscle cells. The orientations of the RP (open arrow) and mSEAP (filled arrow) genes within each construct are shown.

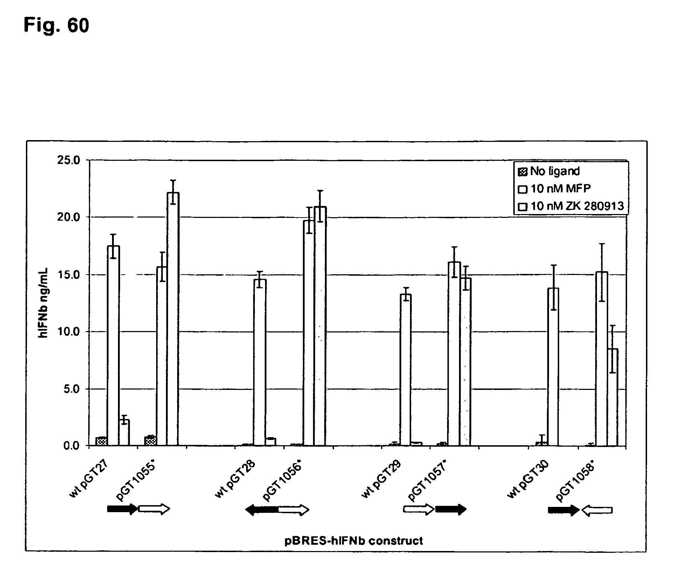

**Figur 60** shows induction of hIFNb expression from pBRES with wt or V729L/W755A (*) RP in C2C12 mouse muscle cells. The orientations of the RP (open arrow) and hIFNb (filled arrow) genes within each construct are shown.

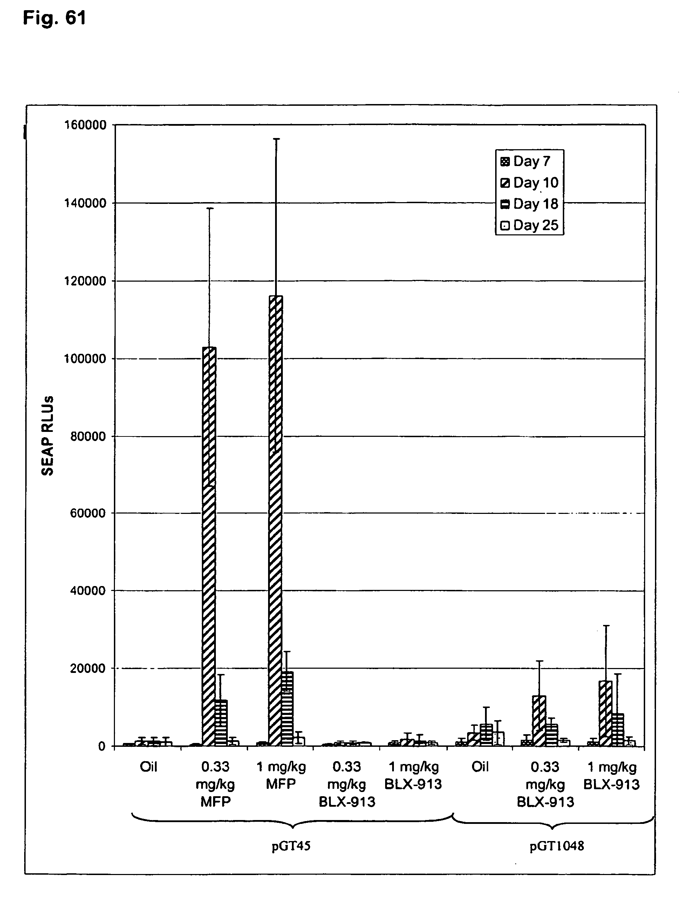

**Figur 61** shows results of in vivo testing of the BLX-913/engineered RP system.

### DETAILED DESCRIPTION OF THE INVENTION

The references cited herein, including e.g., patents, patent applications, journals, books, and Web-site publications, are incorporated herein by reference, in their entirety.

### Abbreviations

AAV (adeno-associated virus)
AAV-1 (adeno-associated virus, serotype 1)
AAV-2 (adeno-associated virus, serotype 2)
AM (activator molecule)
AMP (ampicillin)
bp (base pairs)
BRES-1 (Berlex Regulated Expression System-1)
BGH (bovine growth hormone)
CMV (cytomegalovirus)
DBD (DNA-binding domain)
DNA (deoxyribonucleic acid)
EAE (Experimental Allergic Encephalomyelitis)
enh (enhancer)
E1b TATA (Adenovirus E1 b gene promoter TATA box)
EBNA-1 (Epstein-Barr virus Nuclear Antigen)
EDTA (ethylene diamine tetraacetic acid)
EF-1α (elongation factor-1alpha)
ELAM (endothelial leukocyte adhesion molecule)
ELISA (enzyme-linked immunosorbent assay)
EP (electroporation)
EPO (erythropoietin)
GAL-4 (yeast GAL-4 protein)
6x GAL-4 (six copies of the GAL-4 DNA binding site)
GAPDH (glyceraldehyde 3-phosphate dehydrogenase)
GMCSF (granulocyte macrophage stimulating factor)
hGMCSF (human granulocyte macrophage colony-stimulating factor)
hIFN (human interferon)
hIFN-β (human interferon-β)
hr (hour)
HR (hormone receptor)
HRE (hypoxia responsive element)
hGH (human growth hormone)
hPR (human progesterone receptor)
HTLV (human T-cell lymphotropic virus)
HSV (herpes simplex virus)
Hygro (hygromycin)
IFN-β (interferon-β)
IFN-β1a (interferon-β 1a)
IFNβ1b (interferon-β 1b)
IFN sig seq (interferon signal sequence)
IgK (immunoglobulin kappa) i.m. or IM (intramuscular)
inj. (injected)
INR or inr (transcription initiator element)
IP-10 or IP-10 (interferon-alpha inducible protein 10)
ITR (inverted terminal repeats)
i.p. or IP (intraperitoneal)
IVS8 (intervening sequence or intron 8)
i.v. or IV (intravenous)
JE (murine analog of MCP-1)
kDA (kilodalton)
kan (kanamycin)
KanR (Kanamycin resistance gene)
LBD (ligand-binding domain)
LBP (ligand-binding pocket)
MCP-1 (monocyte chemoattractant protein)
MCS (multiple cloning site)
MFP (mifepristone)
mg (milligram)
mGMCSF (mouse granulocyte macrophage colony-stimulating factor)
mIFN (murine interferon)
mIFN-β (murine interferon-beta)
ml (milliliter)
min (min)
MCK (muscle creatine kinase)
Mx1 (murine homologue of MxA)
MxA (human myxovirus protein)
ng (nanogram)
ORF (open reading frame)
Ori (origin of replication)
OriP (replication origin of Epstein Barr Virus)
pBRES (plasmid Berlex Regulated Expression System)
p65 (transcription regulatory domain of NFkappaB p65 protein)
PBS (phosphate buffered saline)
PEG (polyethylene glycol)
PINC (Protective Interacting Non-Condensing polymer)
pg (picogram)
pk (pharmacokinetics)
polyA or poly(A) (polyadenylation site)
PR (progesterone receptor)
pro (promoter)
P TK (promoter of the Herpes Simplex Virus thymidine kinase gene)
pUC ori (replication origin of pUC plasmids)
r (reverse)
RM (regulator molecule)
RNA (ribonucleic acid)
rpm (revolutions per minute)
RT (room temperature)
s.c. or SC (subcutaneous)
SEAP (Secreted Alkaline Phosphatase)
SHR (steroid hormone receptor)
shRNA (short hairpin RNA)
siRNA (small interfering RNA)
sk actin pro (skeletal muscle promoter)
SkM or Sk (skeletal muscle)
SV40 (simian virus 40)
TK (thymidine kinase)
TKpA (thymidine kinase poly A)
TM (therapeutic molecule)
UbiB (Ubiquitin B)
ug (microgram)
5'UTR (5' untranslated region)
UT12 (untranslated region 12)
VP-16 (herpes virus VP-16 transactivation domain)
vol. (volume)
WPRE (Woodchuck Post-Transcriptional Regulator Element)

### Technical and Scientific Terms

Technical and scientific terms used herein have the meanings commonly understood by one of ordinary skill in the art to which the present invention pertains, unless otherwise defined. Reference is made herein to various methodologies known to those of ordinary skill in the art. Standard reference works setting forth the general principles of recombinant DNA technology include Sambrook, J., et al. (1989) Molecular Cloning,: A Laboratory Manual, 2d Ed., Cold Spring Harbor Laboratory Press, Planview, N. Y.; McPherson, M. J., Ed. (1991) Directed Mutagenesis: A Practical Approach, IRL Press, Oxford; Jones, J. (1992) Amino Acid and Peptide Synthesis, Oxford Science Publications, Oxford; Austen, B. M. and Westwood, O. M. R. (1991) Protein Targeting and Secretion, IRL Press, Oxford. Any suitable materials and/or methods known to those of ordinary skill in the art can be utilized in carrying out the present invention; however, preferred materials and/or methods are described. Materials, reagents and the like to which reference is made in the following description and examples are obtainable from commercial sources, unless otherwise noted.

### Regulated Expression System

The improved regulated, expression system of the present invention is a highly innovative technology which provides for nucleic acids that encode a therapeutic molecule (TM) that can be delivered to and expressed in the cells of a subject, such that the expression and/or activity of the expressed TM is regulated and provides a therapeutic benefit to the subject, for the treatment of disease. In particular, the present invention provides new activator molecules (AMs), inactivator molecules (IMs), and regulator molecules (RMs) having improved properties.

For example, an advantage of the new AMs and IMs of the present invention is that they selectively bind to an RM having modifications that enhance this selectivity, e.g., modifications in the RM LBD and more particularly the RM LBP. Therefore, the new AMs and IMs of the present invention have improved properties that selectively bind or otherwise interact with an RM of the present invention, and diminish or eliminate cross reactivity with endogenous proteins, particularly endogenous human proteins, e.g., a receptor, more particularly a steroid receptor, and even more particularly a PR or glucocorticoid receptor (GR). In some embodiments, a new AM or IM of the present invention is an RM modulator, particularly a modified receptor modulator, more particularly a modified steroid receptor modulator, even more particularly a modified PR or GR modulator, e.g., a PR or GR ligand analog, respectively. In one embodiment, the PR ligand analog selectively binds to an RM having a modified LBD, or more particularly a modified LBP, and has diminished or no side effects relating to e.g., cross reactivity with endogenous PR or other endogenous steroid receptors, abortifacient activity, or contraceptive activity.

More particularly, an advantage of the regulated expression system of the present invention is that the new AMs, IMs, and RMs of the present invention provide for the tightly modulated expression of a TM, e.g., a protein or nucleic acid, in cells of a subject, such that the expression and/or activity of the TM can be modulated e.g., to increase or decrease, or turn on and off, TM expression and/or activity. Importantly, the AMs and IMs of the present invention have improved selectivity for an RM, over an endogenous protein.

The ligand binding domain (LBD), and more particularly the ligand binding pocket (LBP), of the RM is modified to bind an AM and/or be responsive to the AM, with improved specificity and selectivity. In some embodiments, the AM binds to a unique RM modified to selectively bind to the AM. For example, an AM of the present invention can be, but is not limited to, an AM that selectively binds to and/or activates an RM, and has little or no effect on an endogenous protein, e.g., a receptor, more particularly a steroid receptor, and even more particularly a PR or GR. Also, for example, the RM of the present invention can be, but is not limited to, an RM having a modified receptor LBD, or more particularly a modified receptor LBP, to specifically bind and/or otherwise interacts with an AM and thereby is activated by the AM. In one embodiment, the AM binds or otherwise interacts with the RM and selectively activates the RM, such that the activated RM increases or turns on expression of the TM. Thereby, an AM and/or RM of the present invention, provides the ability to tightly and specifically regulate the level of TM expression and/or activity.

The ligand binding domain (LBD), and more particularly the ligand binding pocket (LBP), of the RM is modified to bind an IM and/or be responsive to the IM, with improved specificity and selectivity. In some embodiments, the IM binds to a unique RM modified to selectively bind to the IM. For example, an IM of the present invention can be, but is not limited to, an IM that selectively binds to and/or inactivates an RM, and has little or no effect on an endogenous protein, e.g., a receptor, more particularly a steroid receptor, and even more particularly a PR or GR. Also, for example, the RM of the present invention can be, but is not limited to, an RM having a modified receptor LBD, or more particularly a modified receptor LBP, to specifically bind and/or otherwise interacts with an IM and thereby is activated by the AM. In one embodiment, the AM binds or otherwise interacts with the RM and selectively activates the RM, such that the activated RM increases or turns on expression of the TM. Thereby, an AM and/or RM of the present invention, provides the ability to tightly and specifically regulate the level of TM expression and/or activity.

A further advantage of the present invention is that it provides for the expression and/or activity of a TM, in the cells of a subject, in a dose-dependent or orientation-dependent manner (as described herein), e.g., depending on the amount of a regulator molecule (RM) present in or administered to a subject, or the orientation of a nucleic acid encoding a TM, respectively. Consequently, another advantage of the compositions and methods of the present invention is that it can be used to optimize therapy in a manner specific to a disease or disease state of a subject. A further advantage of the present expression system is that it can comprise a single nucleic acid vector, which can be administered to a subject via a single injection. Thus, the present expression system provides significant advantages over known nucleic acid-based therapy or bolus protein-based therapy.

In particular, the expression system of the present invention provides for the regulated, long-term expression of a TM (e.g., a protein or nucleic acid) in the cells of a subject, resulting in therapeutic efficacy while minimizing dose-limiting side effects. More particularly, gene therapy, using the expression system of the present invention, can provide regulated, long-term expression of a protein and thereby minimize dose-limiting side effects and maximize therapeutic efficacy of the protein for the treatment of disease in a subject. For example, Interferon beta (IFN-β) has been shown to be an effective protein drug for subjects with multiple sclerosis (MS) in reducing the severity of the disease and slowing its progression. However, IFN-β is known to have a short half-life in circulation. Further, frequent, local administration of the protein may cause dose-dependent side effects. However, using the regulated expression system of the present invention, a nucleic acid encoding an IFN-β (e.g., IFN-β-1a) can be administered to the cells of a subject, and the expression of the encoded IFN-β in the cells can be regulated long-term, and optimized, to achieve maximum therapeutic efficacy and minimum dose-limiting side effects of the IFN-β drug, for treatment of MS.

An AM that is a small molecule activator, in the form of an orally available pill, controls promoter induction and subsequent expression of a TM encoded by a nucleic acid sequence of the regulated, expression system of the present invention. In this manner the level of the expressed TM (e.g., a protein or nucleic acid) in circulation in a subject can be tightly regulated in an on/off manner and/or in a dose-dependent manner. An AM of the present invention can directly or indirectly control expression of a TM. For example, in one embodiment, the AM activates an RM, and the presence of the activated RM thereby modulates (e.g., induces) expression of the TM in the cells of a subject. Thus, another advantage of the regulated expression system of the present invention is that it allows for the option for continuous versus pulsatile therapy of a TM expressed in the cells of a subject (e.g., a protein or nucleic acid), and the modulation of expression levels of the TM, in order to optimize therapeutic efficacy of the TM while minimizing any side effects thereof. In particular, the regulated expression system of the present invention allows for the first time the option for continuous and durable, versus pulsatile, IFN-β protein therapy in MS subjects. Further, another advantage of the present invention is that it can provide renewable expression of a TM in the cells of a subject, by repeated administration of a nucleic acid vector encoding the TM.

More particularly, the present regulated expression system allows for the subject-specific or disease-specific therapy, by modulating and optimizing the expression level of a TM in the cells of a subject, to achieve maximum therapeutic efficacy and minimum side effects. As used herein, "subject-specific" or "disease-specific" therapy refers to treatment that is specific to a subject having a specific disease, stage of disease, or disease condition or symptom. For example, using the regulated expression system of the present invention, the level of IFN-β expressed in the cells of a subject having MS can be modulated and optimized to achieve maximum therapeutic efficacy and minimum side effects, for treatment of a specific condition, symptom, or stage of MS (e.g., relapsing remitting, primary progressive, or secondary progressive); or according to a subject's response or tolerance to IFN-β.

More specifically, the present invention provides an improved regulated gene expression system, and pharmaceutical compositions and methods thereof for treatment of disease. The encoded TM can be a nucleic acid or protein that provides a therapeutic benefit to a subject having, or susceptible to, a disease. As used herein, "therapeutic benefit" or "therapeutic activity" includes, but is not limited to, the amelioration, modulation, diminution, repression, stabilization, or prevention, delay, or slowing of the onset or progression of a disease or symptom or condition of a disease. As used herein, "subject" refers to a mammal (e.g., a human), and more particularly, refers to a mammal in need of treatment for a disease. "Treatment", "treating", "treat", or grammatical equivalents thereof, refers to providing a therapeutic benefit to a subject for a disease, including a stage, symptom or condition of a disease. "Disease" as used herein encompasses a stage, symptom, condition, or pathology of a disease, or genetic predisposition for a disease. Such diseases can be autoimmune or inflammatory diseases. In some embodiments the disease is a cancer. In some embodiments, the disease is e.g., multiple sclerosis, leukemia, melanoma, hepatitis, or cardiomyopathy. Further, the improved regulated expression system of the present invention provides a new approach for engineering changes in an animal genome (e.g., a murine genome) so that gene function in an animal model can be accurately analyzed and credible animal models (e.g., murine models) of human diseases can be generated. In particular, the improved regulated expression system of the present invention provides an invaluable tool for biomedical research because using the present system, expression of a target molecule e.g., a target gene in an animal genome (or other molecule of the present invention) can be regulated temporally and in a spatial-specific manner.

Further, the improved regulated expression system of the present invention provides a new approach for the selective or unique expression of target shRNA both in vitro and in vivo. For example, using the regulated expression system of the present invention, a polymerase II (POL II) based expression system can be modified to generate a target shRNA selectively or uniquely. For example to uniquely generate a target shRNA, the present regulated, expression system can be modified and used to generate the shRNA by operably linking a POL II promoter to an intron-containing gene, and the resulting spliced intron processed by the inclusion of MIR sequences to express the target shRNA. Also for example, the RM protein-targeted GAL-4 binding sites of the present vectors and expression cassettes described herein could be inserted upstream of a U6 promoter to create an RM-responsive system, with the additional potential modification of exchanging the p65 transactivator with a polymerase III (POL III) activator (e.g., Oct-2^{Q}).

The present invention provides an improved regulated expression system comprising at least a first expression cassette having a nucleic acid sequence encoding a TM, such that, when delivered to cells of a subject, the encoded TM is expressed, and the expression and/or activity of the TM is regulated in the presence of a regulator molecule (RM). "Regulation" of the activity and/or expression of a molecule of the present invention (e.g., a TM) as used herein refers to the modulation of the expression and/or an activity of the molecule resulting in e.g., the induction, repression, increase, or decrease of an activity and/or the expression of such a molecule. Further examples of such regulation include, but are not limited to, the modulation of an amount, conformation, signal transduction, binding specificity, half-life, stability, or other cellular modification or processing of a molecule of the present invention (e.g., a TM). In preferred embodiments, the TM of the present expression system is regulated. However, examples of other molecules that are suitable for regulation in the present expression system, include, but are not limited to an RM, activator molecule (AM), and inactivator molecule (IM) as described herein.

The expression and/or activity of the TM is regulated in a dose-responsive or dose-dependent manner, e.g., according to the amount of a RM present in the cells of the subject or administered to the subject. In other embodiments, the expression and/or activity of the TM is regulated in a dose-responsive or dose-dependent manner, e.g., according to the amount of an activator molecule (AM), or inactivator molecule (IM) present in the cells of the subject or administered to the subject. In one embodiment, the expression and/or activity of the TM is regulated in a dose-responsive or dose-dependent manner according to the amount of the same TM or different TM present in the cells of the subject or administered to the subject. As used herein "dose-responsive" or "dose-dependent" refers to the correlation of the expression and/or activity of a molecule of the present invention (e.g. a TM), with the presence in, or administration to, the cells of a subject, a particular dose or amount of a second molecule. Examples of such a second molecule include, but are not limited to, an RM, AM, IM, or TM. Further examples, of a second molecule include a cellular molecule e.g., a biomarker (e.g., a biomarker associated with a disease).

As used herein "cells of a subject" refer to autologous cells from a subject, or heterologous cells (or donor cells) that are not from a subject but are delivered or administered to a subject as described herein. Preferably, the autologous cells are present in a subject, and the heterologous cells are delivered to and present in a subject. In preferred embodiments, a composition of the present invention, e.g., a vector encoding a TM and/or RM, is delivered in vivo to autologous cells of a subject, such that the encoded molecule is expressed in cells present in the subject. In one embodiment, a composition of the present invention, e.g., a vector encoding a TM and/or RM, is delivered ex vivo to autologous or heterologous cells of a subject and then the treated cells are delivered to the subject, such that the encoded molecule is expressed in cells present in the subject.

The expression and/or activity of the TM is orientation-dependent. As used herein "orientation-dependent" refers to the 5' to 3' orientation of an expression cassette encoding a TM of the present invention, or the 5' to 3' direction of transcription or translation of an encoded TM of the present invention, and in some embodiments the orientation is: with respect to a vector comprising the expression cassette or encoding the TM; with respect to the orientation of another expression cassette on the same vector; or with respect to the orientation of the expression of another molecule encoded by the same vector. For example, in one embodiment, the expression and/or activity of the TM in cells is modulated with respect to the 5' to 3' orientation of the expression cassette encoding the TM, or with respect to the 5' to 3' orientation of the transcription or translation of the encoded TM. Consequently, TM expression and/or activity can be modulated by selection of a particular orientation of the expression cassette encoding the TM or the orientation of transcription or translation of the TM.

The regulated expression system of the present invention comprises at least one expression cassette encoding a TM and can comprise additional expression cassettes encoding one or more of the molecules of the present invention, e.g., a TM, RM, AM, or IM. Further, one or more expression cassettes can be present in a single vector, or in more than one vector. Further, the present invention is not limited to a single TM, RM, AM, or IM and encompasses embodiments having one or more or multiples of a TM, RM, AM, or IM of the present invention, which can be present alone or together in a single vector or in more than one vector. As used herein, "vector" refers to a nucleic acid suitable for inserting and expressing in cells a nucleic acid sequence encoding one or more molecules of the present invention, e.g., a TM, RM, AM, or IM. "Expression cassette", as used herein refers to a nucleic acid encoding the requisite components or functional sequences for the expression in cells of a molecule of the present invention (e.g., a protein or nucleic acid TM, RM, AM, or IM), where the molecule is encoded by a nucleic acid sequence operably inserted into the expression cassette (e.g., at a cloning site in the expression cassette) and operably linked to the functional sequences of the expression cassette. "Operably linked" or "operably inserted" sequence or sequences, as used herein, refers to a sequence or sequences fused, joined, attached or otherwise brought together with another sequence such that the respective sequences function as intended, known, and/or to achieve a particular outcome (e.g., a promoter sequence operably linked to a gene sequence to promote transcription of the encoded gene).

Figures 1A and 1B illustrate unlimiting examples of a regulated expression system of the present invention comprising: 1) a first expression cassette comprising a first nucleic acid sequence encoding a therapeutic molecule (TM) and a first promoter sequence encoding a DNA-binding site (DBS) and TATA sequence operably linked to the first nucleic acid sequence; 2) a second expression cassette comprising a second nucleic acid sequence encoding a regulator molecule (RM) and a second promoter sequence operably linked to the second nucleic acid sequence, wherein the RM comprises a DNA-binding domain (DBD), ligand-binding domain (LBD), and regulatory domain (RD) and more specifically, in Figure 1B, an activation domain (AD); and 4) an activator or inactivator molecule (AM/IM) that activates the RM or inactivates the RM, respectively, such that the presence of the activated or inactivated RM regulates the expression and/or activity of the TM.

FIG. 39 illustrates a regulated expression system that combines the regulator molecule (RM) and the therapeutic molecule (TM) or reporter gene on a single plasmid. The regulated expression system of the invention, represented in the pGT79 orientation, comprises: 1) a first expression cassette comprising a first nucleic acid sequence encoding a therapeutic molecule (TM) or a reporter gene and a first promoter sequence encoding a DNA-binding site (DBS) and TATA sequence operably linked to the first nucleic acid sequence; 2) a second expression cassette comprising a second nucleic acid sequence encoding a regulator molecule (RM) and a second promoter sequence operably linked to the second nucleic acid sequence, wherein the RM comprises a DNA-binding domain (DBD), ligand-binding domain (LBD), and a VP16 or p65 transactivation domain (AD). An activator molecule (AM) activates the RM, such that the presence of the activated or inactivated RM regulates the expression and/or activity of the TM (FIG. 40).

A first expression cassette comprises the following operably linked functional sequences: 6x GAL-4 DBS, E1b TATA, and transcription start site (e.g., SEQ ID NO: 1), 5' untranslated region UT12 (e.g., SEQ ID NO: 2), synthetic intron IVS8 (e.g, SEQ ID NO: 3), multiple cloning site (e.g, SEQ ID NO: 4 or SEQ ID NO: 5), human growth hormone (hGH) polyadenylation (poly(A)) site (e.g., SEQ ID NO: 6); and a second expression cassette comprises the following operably linked functional sequences: chicken skeletal muscle alpha-actin promoter (e.g., SEQ ID NO: 7) and SV40 poly(A) site (e.g., SEQ ID NO: 8). In a preferred embodiment, the first and second expression cassettes are present in a single vector (e.g., as schematically illustrated in Figures 1A-B). In another preferred embodiment, the vector is a single plasmid vector e.g., pGT1 (comprising the sequence of SEQ ID NO: 9 and SEQ ID NO: 4), pGT2 (comprising the sequence of SEQ ID NO: 10 and SEQ ID NO: 4), pGT3 (comprising the sequence of SEQ ID NO: 11 and SEQ ID NO: 4), or pGT4 (SEQ ID NO: 12),wherein each vector comprises a multiple cloning site (SEQ ID NO: 4) located 3' of the IVS8 and 5' of the hGH poly(A) site (e.g., as schematically depicted in Figures 14A-D), or pGT11 (comprising the sequence of SEQ ID NO: 9 and SEQ ID NO: 5), pGT12 (comprising the sequence of SEQ ID NO: 10 and SEQ ID NO: 5), pGT13 (comprising the sequence of SEQ ID NO: 11 and SEQ ID NO: 5), or pGT14 (comprising the sequence of SEQ ID NO: 12 and SEQ ID NO: 5), wherein each vector comprises a multiple cloning site (SEQ ID NO:5) located 3' of the IVS8 and 5' of the hGH poly(A) site. In a preferred embodiment, the vector is a single plasmid vector, e.g., pGT79 (SEQ ID NO: 59), or a single plasmid vector derived from pGT79, e.g., pGT1003 (comprising a nucleic acid sequence comprising SEQ ID NO: 60 encoding an amino acid sequence represented by SEQ ID NO: 61), pGT1004 (comprising a nucleic acid sequence comprising SEQ ID NO: 62 encoding an amino acid sequence represented by SEQ ID NO: 63), pGT1005 (comprising a nucleic acid sequence comprising SEQ ID NO: 64 encoding an amino acid sequence represented by SEQ ID NO: 65), pGT1006 (comprising a nucleic acid sequence comprising SEQ ID NO: 66 encoding an amino acid sequence represented by SEQ ID NO: 67), pGT1007 (comprising a nucleic acid sequence comprising SEQ ID NO: 68 encoding an amino acid sequence represented by SEQ ID NO: 69), pGT1008 (comprising a nucleic acid sequence comprising SEQ ID NO: 70 encoding an amino acid sequence represented by SEQ ID NO: 71), pGT1009 (comprising a nucleic acid sequence comprising SEQ ID NO: 72 encoding an amino acid sequence represented by SEQ ID NO: 73), pGT1015 (comprising a nucleic acid sequence comprising SEQ ID NO: 74 encoding an amino acid sequence represented by SEQ ID NO: 75), pGT1016 (comprising a nucleic acid sequence comprising SEQ ID NO: 76 encoding an amino acid sequence represented by SEQ ID NO: 77), pGT1017 (comprising a nucleic acid sequence comprising SEQ ID NO: 78 encoding an amino acid sequence represented by SEQ ID NO: 79), pGT1025 (comprising a nucleic acid sequence comprising SEQ ID NO: 80 encoding an amino acid sequence represented by SEQ ID NO: 81), pGT1020 (comprising a nucleic acid sequence comprising SEQ ID NO: 82 encoding an amino acid sequence represented by SEQ ID NO: 83), pGT1021 (comprising a nucleic acid sequence comprising SEQ ID NO: 84 encoding an amino acid sequence represented by SEQ ID NO: 85), pGT1022 (comprising a nucleic acid sequence comprising SEQ ID NO: 86 encoding an amino acid sequence represented by SEQ ID NO: 87), pGT1023 (comprising a nucleic acid sequence comprising SEQ ID NO: 88 encoding an amino acid sequence represented by SEQ ID NO: 89), or pGT1024 (comprising a nucleic acid sequence comprising SEQ ID NO: 90 encoding an amino acid sequence represented by SEQ ID NO: 91).

Further, the TM is an IFN-β or GM-CSF. For example, in one embodiment, the first nucleic acid sequence encodes a TM that is human IFN-β 1 a comprising the amino acid sequence of SEQ ID NO: 13, and is encoded by the nucleic acid sequence of SEQ ID NO: 14. In another embodiment, the first nucleic acid sequence encodes a TM that is a mouse IFN-β comprising the amino acid sequence of SEQ ID NO:15, and is encoded by the nucleic acid sequence of SEQ ID NO: 16. In another embodiment, the first nucleic acid sequence encodes a TM that is a human GM-CSF comprising the amino acid sequence of SEQ ID NO: 17, and is encoded by the nucleic acid sequence of SEQ ID NO: 18. In another embodiment, the first nucleic acid sequence encodes a TM that is a mouse GM-CSF comprising the amino acid sequence of SEQ ID NO: 19, and is encoded by the nucleic acid sequence of SEQ ID NO: 20. Further, in preferred embodiments, the RM is a variant of a wild-type or naturally-occurring progesterone receptor (PR). For example, in one embodiment, the second nucleic acid sequence encodes an RM that is a mutated PR comprising the amino acid sequence of SEQ ID NO: 22, and is encoded by the nucleic acid sequence of SEQ ID NO: 21.

The nucleic acid sequence encoding a TM is inserted or cloned into the Spe I and Not I restriction enzyme sites of an MCS of a first expression cassette, of a single plasmid vector. In one embodiment, the single plasmid vector comprising a nucleic acid sequence encoding a TM is e.g., pGT23 (SEQ ID NO: 23), pGT24 (SEQ ID NO: 24), pGT25 (SEQ ID NO: 25), or pGT26 (SEQ ID NO: 26), where the encoded TM is a mouse IFN-β (e.g., comprising the amino acid sequence of SEQ ID NO: 15 and/or encoded by a nucleic acid sequence comprising SEQ ID NO: 16), and the 5'-3' orientation of transcription of the encoded TM and of the inserted nucleic acid sequence is schematically illustrated in Figure 15A (see arrow). In another embodiment, the single plasmid vector comprising a nucleic acid sequence encoding a TM is e.g., pGT27 (SEQ ID NO: 27), pGT28 (SEQ ID NO: 28), pGT29 (SEQ ID NO: 29), or pGT30 (SEQ ID NO: 30), where the encoded TM is a human IFN-β (e.g., comprising the amino acid sequence of SEQ ID NO: 13 and/or encoded by a nucleic acid sequence comprising SEQ ID NO: 14). In a preferred embodiment, the single plasmid vector comprising a nucleic acid sequence encoding a TM is e.g., pGT79 (SEQ ID NO: 59), or a single plasmid vector derived from pGT79, e.g., pGT1003 (comprising a nucleic acid sequence comprising SEQ ID NO: 60 encoding an amino acid sequence represented by SEQ ID NO: 61), pGT1004 (comprising a nucleic acid sequence comprising SEQ ID NO: 62 encoding an amino acid sequence represented by SEQ ID NO: 63), pGT1005 (comprising a nucleic acid sequence comprising SEQ ID NO: 64 encoding an amino acid sequence represented by SEQ ID NO: 65), pGT1006 (comprising a nucleic acid sequence comprising SEQ ID NO: 66 encoding an amino acid sequence represented by SEQ ID NO: 67), pGT1007 (comprising a nucleic acid sequence comprising SEQ ID NO: 68 encoding an amino acid sequence represented by SEQ ID NO: 69), pGT1008 (comprising a nucleic acid sequence comprising SEQ ID NO: 70 encoding an amino acid sequence represented by SEQ ID NO: 71), pGT1009 (comprising a nucleic acid sequence comprising SEQ ID NO: 72 encoding an amino acid sequence represented by SEQ ID NO: 73), pGT1015 (comprising a nucleic acid sequence comprising SEQ ID NO: 74 encoding an amino acid sequence represented by SEQ ID NO: 75), pGT1016 (comprising a nucleic acid sequence comprising SEQ ID NO: 76 encoding an amino acid sequence represented by SEQ ID NO: 77), pGT1017 (comprising a nucleic acid sequence comprising SEQ ID NO: 78 encoding an amino acid sequence represented by SEQ ID NO: 79), pGT1025 (comprising a nucleic acid sequence comprising SEQ ID NO: 80 encoding an amino acid sequence represented by SEQ ID NO: 81), pGT1020 (comprising a nucleic acid sequence comprising SEQ ID NO: 82 encoding an amino acid sequence represented by SEQ ID NO: 83), pGT1021 (comprising a nucleic acid sequence comprising SEQ ID NO: 84 encoding an amino acid sequence represented by SEQ ID NO: 85), pGT1022 (comprising a nucleic acid sequence comprising SEQ ID NO: 86 encoding an amino acid sequence represented by SEQ ID NO: 87), pGT1023 (comprising a nucleic acid sequence comprising SEQ ID NO: 88 encoding an amino acid sequence represented by SEQ ID NO: 89), or pGT1024 (comprising a nucleic acid sequence comprising SEQ ID NO: 90 encoding an amino acid sequence represented by SEQ ID NO: 91), where the encoded TM is a mouse IFN-β (e.g., comprising the amino acid sequence of SEQ ID NO: 15 and/or encoded by a nucleic acid sequence comprising SEQ ID NO: 16), or a human IFN-β (e.g., comprising the amino acid sequence of SEQ ID NO: 13 and/or encoded by a nucleic acid sequence comprising SEQ ID NO: 14).

Furthe the single plasmid vector comprises the nucleic acid sequence of a vector backbone (e.g., SEQ ID NO: 12), MCS (e.g., SEQ ID NO: 31), and Spel-Notl fragment (SEQ ID NO: 31), wherein the fragment encodes a TM that is a mouse IFN-β, has an Spel sequence at the 5' end and Notl sequence at the 3' end compatible for insertion of the fragment at the Spel-Notl site in the MCS, and is inserted at the Spel-Notl site of the MCS. Further, in one embodiment, the single plasmid vector comprises the nucleic acid sequence of a vector backbone (e.g., SEQ ID NO: 12), MCS (e.g., SEQ ID NO: 32), and Spel-Notl fragment (SEQ ID NO: 31), wherein the fragment encodes a TM that is a human IFN-β, has an Spel sequence at the 5' end and Notl sequence at the 3' end compatible for insertion of the fragment at the Spe l-Notl site in the MCS, and is inserted at the Spel-Notl site of the MCS.

Further an AM binds to the RM and activates the RM, thereby, the activated RM binds to the DBS of the promoter sequence operably linked to the TM sequence, resulting in the induction of TM expression and/or activity, in cells (e.g., mammalian cells). However, in another embodiment, an inactivator molecule (IM) binds to the RM and inactivates the RM, thereby, the inactivated RM does not bind to the DBS of the TM promoter, resulting in the repression or in the lack of induction of TM expression and/or activity. In one embodiment of the example illustrated in Figure 1B, an activator molecule (AM) binds to the LBD of the RM and activates the RM, thereby, the activated RM forms a homodimer that binds to the DBS of the promoter operably linked to the TM sequence, resulting in the induction of TM expression and/or activity, in cells (e.g., mammalian cells). In another embodiment of the examples illustrated in Figures 1 A and 1B, the first and second expression cassettes are present in a single vector. In another embodiment, a first expression cassette encoding a TM and a second expression cassette encoding an RM of the present invention are present in a single vector (e.g., pGT23 (SEQ ID NO: 23), pGT24 (SEQ ID NO: 24), pGT25 (SEQ ID NO: 25), pGT26 (SEQ ID NO: 26), pGT27 (SEQ ID NO: 27), pGT28 (SEQ ID NO: 28), pGT29 (SEQ ID NO: 29), or pGT30 (SEQ ID NO: 30)). In one embodiment of the example illustrated in Figure 40, an activator molecule (AM) binds to the LBD of the RM and activates the RM, thereby, the activated RM forms a homodimer that binds to the DBS of the promoter operably linked to the TM sequence, resulting in the induction of TM expression and/or activity, in cells (e.g., mammalian cells). In another embodiment of the examples illustrated in Figure 39, the first and second expression cassettes are present in a single vector. In another preferred embodiment, a first expression cassette encoding a TM and a second expression cassette encoding an RM of the present invention are present in a single vector (e.g., pGT79, pGT1003, pGT1004, pGT1005, pGT1006, pGT1007, pGT1008, pGT1009, pGT1015, pGT1016, pGT1017, pGT1025, pGT1020, pGT1021, pGT1022, pGT1023 or pGT1024).

A "therapeutic molecule" or "TM" as used herein refers to a molecule having a therapeutic activity or providing a therapeutic benefit. A TM of the present invention can be an isolated DNA, RNA, or protein, or variant thereof, encoded by a nucleic acid sequence and having a therapeutic activity. "Variants" as used herein, include muteins, e.g., muteins of an isolated DNA, RNA, protein, or chemical compound. More particularly, a TM of the present invention can be a modified, synthetic, or recombinant DNA, RNA or protein. "Modified" as used herein, encompasses molecules modified chemically, synthetically, or by recombinant technology, including e.g., mutated, fusion, or chimeric molecules. In one embodiment, the encoded TM is a protein that is expressed and cleaved or processed in the cells of a subject and thereby results in multiple TMs, or an activated TM, or a TM that differs from the expressed and uncleaved or unprocessed TM. In another embodiment of the present invention, the encoded TM is a nucleic acid (e.g., an RNA) having a therapeutic activity. In one embodiment, the encoded RNA encodes multiple splice sites that are multiply or differentially spliced in the cells of a subject. In some embodiments, the multiply- or differentially-spliced RNAs encode for different or variant proteins, or comprise different or variant RNAs, having a similar or separate therapeutic activity. In some embodiments the multiply- or differentially-spliced RNAs are spliced in response to the presence of a specific factor, disease, condition, or tissue.

The encoded TM is a protein having a therapeutic activity and, preferably, a human protein or variant thereof. In a further embodiment, the nucleic acid sequence encoding such a protein is of a gene or gene fragment. In one embodiment, the TM is a granulocyte macrophage colony stimulating factor (GM-CSF). In another embodiment, the TM is an interferon, e.g., interferon-beta (IFN-β), and more particularly, is IFN-β 1a or IFN-β 1 b. In some embodiments the encoded TM is an antibody, and preferably a monoclonal antibody (e.g., CAMPATH). Suitable sequences encoding a monoclonal antibody can be identified and made using methods known in the art, and inserted into a vector of the regulated expression system of the present invention as described herein. The therapeutic activity of monoclonal antibodies has been reported (see e.g., Gatto, B. (2004) 4:411-414; Groner *et al.* (2004) 4:539-547).

"Regulator molecule" or "RM" as used herein refers to a molecule that regulates the expression and/or activity of a TM of the present invention. Examples of such regulation by an RM of the present invention, include, but are not limited to, the modulation of TM expression and/or activity, and more particularly, an increase, decrease, activation (or induction), or inactivation (or repression) of TM expression and/or activity, by an RM of the present invention. Further such modulation of TM expression and/or activity by an RM of the present invention can be direct (e.g., by direct contact of an RM with a TM) or indirect (e.g., where the RM effects a molecule in a signal transduction pathway that results in the modulation of TM expression and/or activity). Further examples of RMs suitable for use in the regulated, expression system of the present invention include, but are not limited to, molecules that effect cellular expression, activity, or processing of a TM of the present invention. Examples of such suitable RMs, include, but are not limited to, transcriptional regulatory molecules (e.g., that activate, inactivate, decrease, or increase transcription of an RNA of an expressed TM); RNA processing molecules (e.g., molecules that activate, inactivate, decrease, or increase RNA processing such as RNA splicing, polyadenylation, or cleavage of an RNA of an expressed TM); or molecules that effect protein translation or post-translational processing of a protein (e.g., enzymes that activate, inactivate, decrease, or increase the phosphorylation, cleavage, or formation of a particular conformation or multimeric form of a protein of an expressed TM).

An RM of the present disclosure can be a naturally-occurring, modified or isolated molecule, or variant thereof. In some embodiments, an RM of the present invention is a synthetic or recombinant molecule. For example, in some embodiments, an RM of the present disclosure is a chemical compound, DNA, RNA, or protein. Further, in some embodiments, an RM of the present invention is a modified molecule. In one embodiment, the RM is a humanized protein. In another embodiment, the RM is a human protein or variant thereof. For example, in one embodiment, the RM is a transcriptional activator e.g., a steroid receptor and, more particularly, a progesterone receptor. In one embodiment, the RM comprises a transactivation domain (e.g., a VP16 or p65 transactivation domain, see e.g., Schmitz et al. (1991) EMBO J 10:3805-3817; Moore et al. (1993) Molec and Cell Biol 13:1666; Blair et al. (1994) Molec and Cell Biol 14:7226-7234), and/or other functional domain (e.g., a basal factor interaction domain) of a co-activator (e.g., p300/CBP), a basal transcription factor (e.g. TFIIB), or a histone acetyltransferase (e.g. p300/CBP or P/CAF, Latchman, D. (2004) Eukaryotic Transcription Factors, Elsevier Academic Press, London; Goodman et al. (2000) Genes & Devl 14:1553-1577; Shikama et al. (1997) Trends in Cell Bio 7:230-236). In another embodiment, the RM comprises a ligand-binding domain (LBD). Further, in one embodiment, an AM binds to the LBD of the RM, thereby activating the RM such that the presence of the activated RM regulates TM expression and/or activity. In another embodiment, the RM comprises a DBD, e.g., a GAL-4 DBD. In one embodiment, the RM comprises a DBD that binds to a functional sequence (e.g., a promoter sequence) operably linked to a nucleic acid encoding a TM, thereby regulating TM expression (e.g., inducing TM expression).

An RM of the present disclosure is activated by an activator molecule (AM) and, thereby, TM expression and/or activity is regulated in the presence of the activated RM. "Activator molecule" or "AM" as used herein refers to a molecule that induces or increases the expression and/or activity of an RM of the present invention. Examples of such activation by an AM include, but are not limited to the induction or increase in expression and/or activity of an RM of the present invention. Further such activation in RM expression and/or activity by an AM of the present invention can be direct (e.g., by direct contact of an AM with a RM) or indirect (e.g., where the AM affects a molecule in a signal transduction pathway that results in the modulation of RM expression and/or activity). Further examples of AMs suitable for use in the regulated expression system of the present invention include, but are not limited to, molecules that effect cellular processing of an RM of the present invention (examples of such cellular processing are described herein, e.g., above).

The AM is a biomarker. In a further embodiment, the AM is a biomarker for a disease or condition and, more particularly, is a biomarker for a disease state or condition, or symptom thereof. In one embodiment, the AM activates the RM by promoting or inhibiting conformational change, enzymatic processing or modification, specific binding, or dimerization of the RM. In a preferred embodiment, the AM activates the RM by promoting homodimerization of the RM. In one embodiment, the AM activates the RM by binding to the RM and, more particularly, to a functional domain of the RM, e.g., a LBD of the RM.

An AM of the present disclosure can be a naturally-occurring or isolated molecule, or variant thereof. In some embodiments, the AM of the present invention is a synthetic or recombinant molecule. For example, in some embodiments, the AM of the present invention is a chemical compound, DNA, RNA, or protein. Further, in some embodiments, the AM of the present invention is a modified molecule. In one embodiment, the AM is a humanized protein. In another embodiment, the AM is a human protein or variant thereof. In one embodiment, the AM is a chemical compound, e.g., an antiprogestin. In one embodiment, the AM is mifepristone. In a preferred embodiment, the AM is a PR ligand analog , e.g., BLX-913, BLX-833, BLX-899, BLX-593, BLX-599, BLX-952, BLX-610, BLX-117, or BLX-784.

The regulated expression system of the present invention comprises: 1) a first expression cassette having a first nucleic acid sequence encoding a TM, and at least one GAL-4 DNA-binding site (DBS) and, more particularly, six GAL-4 DBS (6XGAL-4 DBS), located upstream and operably linked to the first nucleic acid sequence; 2) a second expression cassette having a second nucleic acid sequence encoding an RM that is a modified progesterone receptor comprising a VP-16 AD or p65 AD (e.g., a p65 AD comprising the nucleic acid sequence of SEQ ID NO: 39 or amino acid sequence of SEQ ID NO: 40), progesterone (PR) LBD, and GAL-4 DBD, and an actin promoter sequence located upstream and operably linked to the second nucleic acid sequence; and 3) an AM that is a small molecule inducer, e.g., PR ligand analog, that when orally administered to a subject, activates the expressed RM in the cells of the subject and, thereby, the activated RM forms a dimer that binds to the 6XGAL-4 DBS and induces expression of the encoded TM. In a preferred embodiment, the first and second expression cassettes are present in a single vector.

The RM of the present invention is a transcriptional regulator and more particularly, a mutated steroid receptor. In one embodiment, the RM is a mutated human PR (hPR) and comprises a mutated hPR receptor LBD, (e.g., having a C-terminal deletion of about 19-66 amino acids), wherein the RM is activated in the presence of an AM that is an antagonist of the wild-type PR from which the mutant PR was derived. In a preferred embodiment, the RM LBD comprises a mutated human PR LBD, or more particularly a mutated human PR LBP, comprising one or more site-directed mutations within the LBD or more particularly, the LBP. In another embodiment, the RM of the present invention comprises a regulatory domain (RD), e.g., an activation domain (AD), and more particularly, a transactivation domain (TD). Examples of suitable regulatory domains for use in the RM of the present invention, include, but are not limited to, those known in the art or described herein (e.g., TAF-1, TAF-2, TAU-1, and TAU-2).

An RM of the present invention is inactivated and thereby TM expression and/or activity is regulated in the presence of an inactivated RM. "Inactivator molecule" or "IM" as used herein refers to a molecule that inactivates the expression and/or activity of an RM of the present invention. Examples of such inactivation by an IM include, but are not limited to the repression or decrease in expression and/or activity of an RM of the present invention. Further such inactivation in RM expression and/or activity by an IM of the present invention can be direct (e.g., by direct contact of an IM with a RM) or indirect (e.g., where the IM affects a molecule in a signal transduction pathway that results in the inactivation of RM expression and/or activity). Further examples of IMs suitable for use in the regulated, expression system of the present invention include, but are not limited to, molecules that effect cellular processing of an RM of the present invention (examples of such cellular processing are described herein, e.g., above).

An RM of the present invention is expressed or present in cells of a subject in an activated form, and is inactivated in the presence of an inactivator molecule (IM), thereby, TM expression and/or activity is regulated by the inactivated RM. In another embodiment, the IM is a biomarker. In a further embodiment, the IM is a biomarker for a disease or condition and, more particularly, is a biomarker for a disease state or condition, or symptom thereof. In one embodiment, the IM inactivates the RM by promoting or inhibiting conformational change, enzymatic processing, specific binding, or dimerization of the RM. In a preferred embodiment, the IM inactivates the RM by inhibiting homodimerization of the RM. In one embodiment, the IM inactivates the RM by binding to the RM and, more particularly, to a functional domain of the RM, e.g., an AD of the RM.

An IM of the present disclosure can be a naturally-occurring or isolated molecule, or variant thereof. In some embodiments, the IM of the present invention is a synthetic or recombinant molecule. For example, in some embodiments, the IM of the present invention is a chemical compound, DNA, RNA, or protein. Further, in some embodiments, the IM of the present invention is a modified molecule. In one embodiment, the IM is a humanized protein. In another embodiment, the IM is a human protein or variant thereof. In a preferred embodiment, the IM is a chemical compound.

The expression of a TM, RM, AM, or IM of the present disclosure can be constitutive or transient. In some embodiments, expression of a TM, RM, AM, or IM is regulated or tissue-specific (e.g. muscle-specific). Examples of a regulated RM include, but are not limited to, an RM that is activated by an AM or inactivated by an IM. In one embodiment, the expression of a TM, RM, AM, or IM of the present invention is driven by a regulated promoter or a tissue-specific promoter. In a further embodiment, the regulated or tissue-specific promoter is regulated in the presence of an RM and, more particularly, by the binding of the RM to the promoter. In one embodiment, the tissue-specific promoter is a muscle-specific promoter and, more particularly, an actin promoter. In one embodiment, an RM of the present invention binds to a promoter operably linked to a nucleic acid sequence encoding a TM and thereby regulates the expression of the encoded TM as described herein, in the cells of a subject.

The TM, RM, AM, or IM of the present disclosure can be isolated, produced, and modified using known methods and assays for nucleic acids, proteins, and chemical compounds, as described herein, e.g., below.

### Pharmaceutical Compositions And Treatment Methods

The present disclosure also provides pharmaceutical compositions and methods for treatment of a variety of diseases comprising the improved regulated expression system of the present invention as described herein.

The present invention provides pharmaceutical compositions and methods for treating a disease or condition; regulating the expression of a TM; administering a TM; delivering a TM; or expressing a TM in cells of a subject, where the methods comprise contacting the cells of a subject with a regulated expression system of the present invention, such that the encoded TM is expressed in the cells of the subject, and such TM expression is regulated in the presence of an RM.

The pharmaceutical compositions of the present disclosure comprise at least one TM, RM, AM, or IM of the present invention present and, in some embodiments, the nucleic acid sequence encoding such molecules are present alone or together in a single vector or in more than one vector. In other embodiments, the pharmaceutical compositions of the present invention can comprise more than one of each TM, RM, AM, or IM, and more than one kind thereof (e.g., a first and second TM, RM, AM, and/or IM). More particularly, the pharmaceutical compositions of the present invention can comprise nucleic acid sequences encoding more than one of each TM, RM, AM, or IM, and more than one kind thereof. In one embodiment, a pharmaceutical composition of the present invention comprises at least one of the vectors of the present invention (e.g., pGT23 (SEQ ID NO: 23), pGT24 (SEQ ID NO: 24), pGT25 (SEQ ID NO: 25), pGT26 (SEQ ID NO: 26), pGT27 (SEQ ID NO: 27), pGT28 (SEQ ID NO: 28), pGT29 (SEQ ID NO: 29), or pGT30 (SEQ ID NO: 30), pGT79 (SEQ ID NO: 59), pGT1003 (comprising SEQ ID NO: 60), pGT1004 (comprising SEQ ID NO: 62), pGT1005 (comprising SEQ ID NO: 64), pGT1006 (comprising SEQ ID NO: 66), pGT1007 (comprising SEQ ID NO: 68), pGT1008 (comprising SEQ ID NO: 70), pGT1009 (comprising SEQ ID NO: 72), pGT1015 (comprising SEQ ID NO: 74), pGT1016 (comprising SEQ ID NO: 76), pGT1017 (comprising SEQ ID NO: 78), pGT1025 (comprising SEQ ID NO: 80), pGT1020 (comprising SEQ ID NO: 82), pGT1021 (comprising SEQ ID NO: 84), pGT1022 (comprising SEQ ID NO: 86), pGT1023 (comprising SEQ ID NO: 88), or pGT1024 (comprising SEQ ID NO: 90)).

An pharmaceutical composition of the present invention comprises at least one AM or IM of the present invention. In one embodiment, a pharmaceutical composition of the present invention comprises one or more vectors encoding at least one TM and/or RM. The TM, RM, AM, and IM of the present invention can be administered to a subject separately or together, and ex vivo or in vivo, using any suitable means of administration described herein or known in the art. Examples of such suitable means of administration include, but are not limited to injection (e.g., intramuscular or subcutaneous injection), oral administration, and electroporation. In one embodiment, a TM and RM of the present invention are present in a single vector, and separately administered from an AM that activates the RM (and thereby, the presence of the activated RM regulates TM expression and/or activity). In a further embodiment, the AM is a compound e.g., a PR or GR ligand analog, more particularly a modified antiprogestin (e.g., a modified MFP) or a modified mesoprogestin (e.g., a modified asoprisnil)) administered orally to a subject, and the single vector encoding a TM and RM is a single vector administered by injection or by electroporation to cells of a subject (e.g., skeletal muscle cells). Further examples of a suitable means for administering a composition of the present inventions include the ex vivo delivery of the composition, e.g., a nucleic acid vector encoding a TM and/or RM, to cells of a subject and then the delivery of the treated cells to the subject, such that the encoded molecule is expressed in cells in the subject (see e.g., Studeny et al. (2004) J Natl Cancer Inst 96(21):1593-1603; Studeny et al. (2002) Cancer Res 62(13):3603-3608).

The regulated expression system of the present invention comprises a nucleic acid sequence encoding a therapeutic gene (e.g., IFN-β gene) that is administered to a subject by injection. As used herein "therapeutic gene" refers to a gene encoding a TM, e.g., a protein having a therapeutic activity (e.g., IFN-β 1a or 1b). In a particular embodiment, the gene is IFN-β 1a and is administered as a single intramuscular injection periodically, e.g., 3 to 6 months, using a vector of the present invention. In other embodiments, a therapeutic gene is administered every 1-3 months, 3 to 6 months, 6 to 9 months, or 9 to 12 months. In another embodiment, the regulation of the circulating levels of the expressed protein is achieved by controlled induction of a promoter driving expression of the encoded protein in the target subject cells or tissue.

The RM is a small molecule activator, in the form of an orally available pill that controls promoter induction and subsequent expression of a TM and, more particularly, a therapeutic gene. In this manner the level of expressed TM (e.g., a protein or nucleic acid), in the circulation can be tightly regulated in an on/off manner and/or in a dose-dependent manner. Thus, the regulated, expression system of the present invention allows for the first time the option for continuous versus pulsatile therapy of a TM (e.g., a protein or nucleic acid), and the modulation of expression levels of a TM, in order to optimize therapeutic efficacy of a TM while minimizing any side effects thereof. In particular, the regulated expression system of the present invention allows for the first time the option for continuous versus pulsatile TM therapy in subjects and, more particularly, allows for subject-specific therapy by modulating and optimizing expression levels of a TM in cells of the subject to achieve maximum therapeutic efficacy and minimum side effects, for treatment of a disease.

Using the regulated expression system of the present invention, nucleic acids encoding a TM (e.g., a protein or nucleic acid) can be delivered to target cells of a subject, for treatment of disease. More particularly, using the regulated, expression system of the present invention, nucleic acids encoding a TM can be delivered to target cells of a subject, such that the expressed TM is provided in a therapeutically effective dose or amount. As used herein, a "therapeutically effective dose" or "therapeutically effective amount" of a TM of the present invention is a dose or amount that, when present in the cells of a subject in need of treatment of a disease, results in a therapeutic benefit to the subject (i.e., results in treatment of the disease). Further, a suitable amount or dose of a nucleic acid encoding a TM administered to a subject or present in the cells of a subject; or an amount or dose of an RM, AM, or IM, or nucleic acid encoding an RM, AM, or IM, that is administered to and/or present in the cells of a subject, that results in the presence of a TM in the cells of a subject and/or a therapeutically effective amount of the TM, can be determined empirically by one skilled in the art. For example, in one embodiment a suitable dose or amount of an RM or a nucleic acid encoding an RM, administered to a subject, is a dose or amount that regulates (e.g., induces) the expression and/or activity of a TM in the cells of a subject such that a therapeutically effective dose is achieved.

Factors influencing the amount of TM that constitutes a therapeutically effective dose include, but are not limited to, the severity and history of the disease to be treated, and the age, health, and physical condition of the subject undergoing therapy. A therapeutically effective dose of a TM of the present invention can also depend upon the dosing frequency and severity of the disease in the subject undergoing treatment. The dosing regimen of a TM of the present invention can be continued for as long as is required to achieve the desired effect, i.e., for example, prevention and/or amelioration of the disease, symptoms associated with the disease, disease severity, and/or periodicity of the recurrence of the disease, as described herein. In one embodiment, the dosing regimen is continued for a period of up to one year to indefinitely, such as for one month to 30 years, about three months to about 20 years, about 6 months to about 10 years.

Examples of suitable nucleic acids for use in the regulated expression system of the present invention include, but are not limited to, those nucleic acids encoding a gene for a hormone, growth factor, enzyme, cytokine, receptor, or MHC molecule having a therapeutic activity. Additionally, suitable genes for use in the compositions and methods of the present invention, include nucleic acid sequences that are exogenous or endogenous to cells into which the nucleic acid encoding the gene of interest can be introduced. Of particular interest and suitability for use in the compositions and methods of the present invention for treatment of disease are those genes encoding a polypeptide that is either absent, produced in diminished quantities, or produced in a mutant form in those subjects having or are susceptible to a genetic disease. Examples of such genetic diseases include, but are not limited to, retinoblastoma, Wilms tumor, adenosine deaminase deficiency (ADA), thalassemias, cystic fibrosis, Sickle cell disease, Huntington's disease, Duchenne's muscular dystrophy, Phenylketonuria, Lesch-Nyhan syndrome, Gaucher's disease, and Tay-Sach's disease.

Also of particular interest and suitability for use in the compositions and methods of the present invention for treatment of disease are nucleic acids encoding a tumor suppressor gene. Examples of such suitable tumor suppressor genes include, but are not limited to, retinoblastoma, GM-CSF, G-CSF, M-CSF, human growth hormone (HGH), TNF, TGF-β, TGF-α, hemoglobin, interleukins, co-stimulatory factor B7, insulin, factor VIII, factor IX, PDGF, EGF, NGF, EPO, and β-globin, as well as biologically or therapeutically active muteins of the proteins encoded by such genes. Suitable genes for delivery to target cells can be from any species, but preferably a mammalian species, and more preferably a human. Further, preferred species, as sources of suitable genes, are those species into which the gene of interest is to be delivered using the methods and compositions of the present invention, e.g., a mammalian species and preferably a human.

Further examples of suitable nucleic acids for use in the compositions and methods of the present invention include, but are not limited to, those that encode a protein or nucleic acid TM having an anti-inflammatory, antiviral, or anticancer activity. Examples of such suitable nucleic acids include, but are not limited to, those encoding a granulocyte macrophage stimulating colony factor (GM-CSF) or variant thereof (e.g., Leukine^{®} or human GMCSFLeu²³Asp²⁷Glu³⁹⁾), having an anticancer activity (see e.g., the GM-CSF mutants of US Patent Nos. 5,032,676; 5,391,485; and 5,393,870). Also, for example, suitable nucleic acids include, but are not limited to, those encoding an interferon having an anti-inflammatory or antiviral activity, e.g., an inteferon, particularly IFN-β, and more particularly, an IFN-β 1a or IFN-β 1b.

The compositions and methods of the present invention are used to treat MS by delivering to a subject in need of treatment, a nucleic acid encoding a TM that is an IFN-β and, more particularly, is IFN-β 1a, such that the IFN-β is expressed in the cells of the subject and the expression and/or activity of the IFN-β is regulated by an RM, as described herein.

MS is a chronic and severe disease characterized by focal inflammation in the central nervous system (CNS) (see *e.g*., Hemmer et al. (2002) Neuroscience 3: 291-301; Keegan et al. (2002) Ann. Rev. Med. 53: 285-302; Young, V. Wee (2002) Neurology 59: 802-808; Goodin et al. (2001) Am. Academy of Neurology 58: 169-178). An associated loss of the insulating myelin sheath from around the axons of the nerve cells (demyelination) and a degeneration of the axons are also prominent features of the disease. Resulting from the focal inflammation, an astrocytotic gliosis leads to the formation of sclerotic lesions in the white matter *(see e.g.,* Prineas (1985) Demyelinating Diseases, Elsvevier: Amsterdam; Raine (1983) Multiple Sclerosis, Williams and Wilkins: Baltimore; Raine et al. (1988) J. Neuroimmunol. 20: 189-201; and Martin (1997) J. Neural Transmission (Suppl) 49: 53-67).

There are two major types of MS subject populations at the onset of the disease: those subjects with relapsing-remitting MS and those subjects with primary progressive MS. Relapsing-remitting MS is characterized by episodes (the so called relapses or exacerbation) where new neurologic deficits emerge or preexisting neurologic deficits worsen and periods of remission where the clinical symptoms are stabilized or diminished, whereas, primary progressive MS subjects suffer from progressive neurological deterioration without exacerbations. A large proportion of subjects with relapsing-remitting MS also experience during the course of their disease a worsening of neurologic symptoms independent of relapses, with or without superimposed relapses. Once this stage of the disease is reached, it is called secondary progressive MS.

The clinical symptoms of MS are thought to result from a focal breakdown in the blood-brain barrier (BBB) which permits the entry of inflammatory infiltrates into the brain and spinal cord. Further, these infiltrates are thought to consist of various lymphocytes and macrophages that lead to demyelination, axonal degeneration and scar tissue formation, and the degeneration of oligodendrocytes imperative to CNS myelin production (*see e.g*., Martin (1997) J. Neural Transmission (Suppl) 49:53-67). Consequently, the nerve-insulating myelin and the ability of oligodendroglial cells to repair damaged myelin are seriously compromised *(see e.g.,* Scientific American 269(1993):106-114). These symptoms of MS include pain and tingling in the arms and legs, localized and generalized numbness, muscle spasm and weakness, difficulty with balance when standing or walking, difficulty with speech and swallowing, cognitive deficits, fatigue, and bowel and bladder dysfunction.

Although there is no known cure for MS, immunomodulatory therapy with interferons has proven to be successful in reducing the severity of the underlying disease in subjects with MS. Interferons are important cytokines characterized by antiviral, antiproliferative, and immunomodulatory activities. These activities form a basis for the clinical benefits that have been observed in the treatment of subjects with multiple sclerosis. The interferons are divided into the type I and type II classes. IFN-β belongs to the class of type I interferons, which also includes interferons alpha, tau and omega, whereas interferon gamma is the only known member of the distinct type II class.

Human IFN-β is a regulatory polypeptide with a molecular weight of 22 kDa consisting of 166 amino acid residues. The polypeptide can be produced by most cells in the body, in particular fibroblasts, in response to viral infection or exposure to other biologics. Further, IFN-β binds to a multimeric cell surface receptor, and productive receptor binding results in a cascade of intracellular events leading to the expression of IFNB inducible genes which in turn produces effects which can be classified as antiviral, antiproliferative and immunomodulatory.

Human IFN-β is a well-characterized polypeptide. The amino acid sequence of human IFN-β is known (*see e.g.,* Gene 10:11-15,1980, and in EP 83069, EP 41313 and U.S. Pat. No. 4,686,191). Also, crystal structures have been reported for human and murine IFN-β, respectively (*see e.g.,* Proc. Natl. Acad. Sci. USA 94:11813-11818, 1997. J. Mol. Biol. 253:187-207, 1995; reviewed in Cell Mol. Life Sci. 54:1203-1206, 1998). In addition, protein-engineered variants of IFN-β have been reported (see *e.g*., WO 9525170, WO 9848018, U.S. Pat. No. 5,545,723, U.S. Pat. No. 4,914,033, EP 260350, U.S. Pat. No. 4,588585, U.S. Pat. No. 4,769,233, Stewart et al, DNA Vol. 6 No. 2 1987 pp. 119-128, Runkel et al, 1998, Jour. Biol. Chem. 273, No. 14, pp. 8003-8008). Also, the expression of IFN-β in CHO cells has been reported (see *e.g*., U.S. Pat. No. 4,966,843, U.S. Pat. No. 5,376,567 and U.S. Pat. No. 5,795,779). Further, IFN-β fusion proteins are reported, *e.g*., in WO 00/23472.

Commercial preparations of IFN-β are approved for the treatment of subjects with MS and are sold under the names Betaseron® (also termed Betaferon® or IFN-β 1 bₛₑᵣ₁₇, which is non-glycosylated, produced using recombinant bacterial cells, has a deletion of the N-terminal methionine residue and the C17S mutation), Avonex® and Rebif® (also termed IFN-β 1a, which is glycosylated, produced using recombinant mammalian cells. Further, a comparison of IFN-β 1a and IFN-β 1 b with respect to structure and function has been presented in Pharm. Res. 15:641-649, 1998.

IFN-β is the first therapeutic intervention shown to delay the progression of MS. In addition, the approved dose of IFN-β has been shown to be effective in reducing the exacerbation rate of MS, and more subjects remain exacerbation-free for prolonged periods of time as compared with placebo-treated subjects. Furthermore, the accumulation rate of disability is reduced (*see e.g*., Neurol. 51:682-689, 1998).

IFN-β has inhibitory effects on the proliferation of leukocytes and antigen presentation. Furthermore, IFN-β may modulate the profile of cytokine production towards an anti-inflammatory phenotype. Finally, IFN-β can reduce T-cell migration by inhibiting the activity of T-cell matrix metalloproteases. Such IFN-β activities are likely to act in concert to account for the beneficial effect of IFN-β in the treatment of subjects with MS *(see e.g.,* Neurol. 51:682-689, 1998).

In a preferred embodiment, the compositions and methods of the present invention are for use in the treatment of subjects suffering from various clinically recognized forms of MS, including but not limited to, relapsing-remitting MS, different types of progressive MS (including, but not limited to, *e.g*., primary and secondary progressive MS, progressive-relapsing MS) and, also, clinically isolated syndromes suggestive of MS.

As used herein, "relapsing-remitting" MS is a clinical course of MS that is characterized by clearly defined, sporadic exacerbations or relapses, during which existing symptoms become more severe and/or new symptoms appear. Such exacerbations or relapses, may be followed by partial recovery, or full recovery and remission. The length of time between these sporadic exacerbations or relapses may be months or years, during which time inflammatory lesions, demyelination, axonal loss, and scar formation may still proceed. Relapsing-remitting MS is the most common beginning phase of MS, and it has been reported that about 50% of the cases have progression within 10 to 15 years, and another 40% within 25 years of onset.

As used herein, "primary-progressive" MS is a clinical course of MS that is characterized from the beginning by progressive disease, with no plateaus or remissions, or an occasional plateau and very short-lived, minor improvements. As the disease progresses, the subject may experience difficulty in walking, the steadily decline in motor skills, and an increase in disabilities over many months and years, generally, in the absence of those distinct inflammatory attacks characteristic of relapsing-remitting MS.

As used herein, "secondary-progressive" MS is a clinical course of MS that initially is relapsing-remitting and then becomes progressive at a variable rate independent of relapses. Although subjects experiencing this type of MS may continue to experience inflammatory attacks or exacerbations, eventually the exacerbations and periods of remission may diminish, with the disease taking on the characteristic decline observed with primary-progressive MS.

As used herein "progressive-relapsing" MS is a clinical course of MS that may show permanent neurological deterioration from the onset of the disease, but with clear, acute exacerbations or relapses that look like relapsing-remitting MS. For these subjects, lost functions may never return. It has been reported that this type of MS has a high mortality rate if untreated.

Clinically isolated syndromes suggestive of MS include, but are not limited to, early onset multiple sclerosis and monosymptomatic MS. For purposes of the present invention, the term "multiple sclerosis" is intended to encompass each of these clinical manifestations of the disease and clinically isolated syndromes suggestive of MS unless otherwise specified. For example, a subject having MS or symptoms associated with MS is a subject in need of treatment of MS or associated symptoms of MS. In one embodiment, when a subject suffering from MS undergoes treatment in accordance with the pharmaceutical compositions and methods of the present invention, treatment can result in the prevention and/or amelioration of MS disease symptoms, disease severity, and/or periodicity of recurrence of the disease, *i.e*., treatment of MS using the compositions and methods of the present invention can result in lengthening the time period between episodes in which symptoms flare, and/or can suppress the ongoing immune or autoimmune response associated with the disease, which, left untreated, can enhance disease progression and disability.

Further, a subject can be pre-treated with a pharmaceutical composition or can be a naive subject who has not been pre-treated with a pharmaceutical composition, prior to treatment using a pharmaceutical composition or method of the present invention. For example, for the treatment of MS, a pre-treated subject can be one who has been pretreated with an IFN-β protein drug (e.g., IFN-β 1a) or IFN-β variant (e.g., IFN-β 1b), prior to treatment with the compositions or methods of the present invention. For example, an approved dose of Betaseron®, Avonex®, or Rebif® can be used to pre-treat subjects. Thus, the pharmaceutical compositions and methods of the present invention are suitable for use in the treatment of pre-treated and naive subjects.

The pharmaceutical compositions and methods of the present invention can also be used to block or reduce the physiological and pathogenic deterioration associated with a disease, e.g., inflammatory response in the brain and other regions of the nervous system, breakdown or disruption of the blood-brain barrier, appearance of lesions in the brain, tissue destruction, demyelination, autoimmune inflammatory response, acute or chronic inflammatory response, neuronal death, and/or neuroglial death. Beneficial effects of the pharmaceutical compositions and methods of the present invention include, but are not limited to, preventing the disease, slowing the onset of an established disease, ameliorating symptoms of a disease, reducing an exacerbation rate, slowing the progression of the disease, and postponing or preventing disability including cognitive decline, loss of employment, hospitalization, and finally death. The episodic recurrence of a particular type of disease (e.g. MS), can be treated, *e.g*., by decreasing the severity of the symptoms (such as the symptoms described above) associated with the episode, or by lengthening the time period between the occurrence of episodes, *e.g*., by days, weeks, months, or years, where the episodes can be characterized by the flare-up and exacerbation of disease symptoms, or preventing or slowing the appearance of brain inflammatory lesions e.g. in MS (see, *e.g*., Adams (1993) Principles of Neurology, page 777, for a description of a neurological inflammatory lesion).

Further, suitable nucleic acids for use in the compositions and methods of the present invention, can encode a TM that is a fusion or chimeric protein, or a fusion or chimeric nucleic acid (e.g., RNA). In some embodiments, a TM of the present invention can regulate expression of a gene product or block one or more steps in a biological pathway (e.g., a sepsis pathway) and, thereby, provide a therapeutic benefit. Further, the nucleic acid can encode a toxin fused to a TM (e.g., a receptor ligand gene or an antibody that directs the fused toxin to a target such as a tumor cell or a virus) and, thereby, have a therapeutic effect. Standard methods for operably inserting and/or fusing, nucleic acid sequences, or inserting and/or amino acid sequences into amino acid sequences, of the present invention are described herein and in the art (see, e.g., Sambrook et al., Molecular Cloning: A Laboratory Manual, 2nd ed., Cold Spring Harbor Laboratory, Cold Spring Harbor, N.Y. (1989); Ausubel et al. (eds.), Current Protocols In Molecular Biology, John Wiley and Sons (1987)).

Adverse effects due to some disease treatment regimens are known in the art (see, e.g., Munschauer et al., (1997) Clinical Therapeutics 19(5): 883-893; Walther et al. (1999) Neurology 53: 1622-1627; Lublin *et al.* (1996) 46: 12-18; Bayas *et al.* (2000) 2: 149-159; Ree *et al.* (2002) *8*: 15-18; Walther *et al.* (1998) *5(2)*: 65-70). For example, some of the adverse effects due to treatment of MS include, but are not limited, *e.g*., flu-like symptoms; increased spasticity or deterioration of neurological symptoms; menstrual disorders; laboratory abnormalities (*e.g*., abnormal blood count/value for hemoglobin, leukocytes, granulocytes, lymphocytes, or thrombocytes); abnormal laboratory value for liver enzymes (*e.g*. bilirubin, transaminases, or alkaline phosphatases); injection site reactions, (*e.g*., inflammation, pain, or erythema); cutaneous or subcutaneous necroses; and depression. Suitable co-medications and the use of these co-medications, in conjunction with the compositions and methods of the present invention, for treating adverse effects due to treatment of a disease (e.g., MS), can be determined according to co-medications generally known in the art for treatment of such effects (*see*, e.g., Munschauer et al., (1997) Clinical Therapeutics 19(5): 883-893; Walther et al. (1999) Neurology 53: 1622-1627; Lublin et al. (1996)46: 12-18; Bayas et al. (2000) 2: 149-159; Ree *et al.* (2002) *8*: 15-18; Walther *et al.* (1998) *5(2)*: 65-70). Doses and dosing regimens for such co-medications are also generally known. Such co-medications are well known in the art and may include, but are not limited to, *e.g*., those that help alleviate or mitigate adverse effects due to a disease or due to treatment of a disease. Examples of such co-medications include, but are not limited to, analgesics, non-steroidal anti-inflammatory drugs (NSAIDs), and steroids.

Other suitable examples of co-medications also include, but are not limited to, *e.g*., ibuprofen, acetaminophen, acetylsalicyclic acid, prednisone, pentoxifylline, baclofen, steroids, antibacterial agents, and antidepressants (see *e.g.,* Walther et al. (1999) Neurology 52: 1622-1627). For example, flu-like symptoms can be treated with NSAIDs (e.g., ibuprofen or acetylsalicylic acid) or with paracetamol or with pentoxifylline; increased spasticity or deterioration of neurological symptoms can also be treated with NSAIDs and/or muscle relaxants (*e.g*., baclofen); menstrual disorders can be treated with oral contraceptives; injection site reactions can be treated with systemic NSAIDs and/or steroids (*e.g*., hydrocortisone); cutaneous or subcutaneous necrosis can be treated with antibacterial agents and depression can be treated with antidepressants (see *e.g*., Walther et al. (1999) Neurology 53: 1622-1627).

Combination therapies with other drugs, which are effective in the treatment of a particular disease and have a different adverse event profile may increase the treatment effect and level out the adverse event profile. For treatment of MS, examples of combination therapies include, but are not limited to, *e.g*., glatiramer acetate (Copaxone), mitoxantrone, cyclophosphamide, cyclosporine A, cladribine, monoclonal antibodies (*e.g*., Campath-H1® or Antegren®/Natazulimab®), and statins.

Effective treatment of disease in a subject using the methods of the invention can be examined in several alternative ways including, for example, EDSS (extended disability status scale) score, Functional Composite Score, cognitive testing, appearance of exacerbations, or MRI e.g., for the treatment of MS. The EDSS is a means to grade clinical impairment due to MS *(see e.g.,* Kurtzke (1983) Neurology 33:1444). Eight functional systems, the walking range, the ability to walk, and the ability to maintain self-care functions are evaluated for the type and severity of neurologic impairment. For example, prior to treatment, impairment in the following systems is evaluated: pyramidal, cerebellar, brainstem, sensory, bowel and bladder, visual, cerebral, and other. Together with the assessment of the walking range, of the ability to walk with or without assistive devices, and of the ability to maintain self-care functions the final EDSS score is calculated. Follow-up scores are then obtained at defined intervals of treatment. The grade scale may range, *e.g*., from 0 (normal) to 10 (death due to MS). An increase of one full step (or a one-half step at the higher baseline EDSS scores) may define disease progression *(see e.g.,* Kurtzke (1994) Ann. Neurol. 36:573-79, Goodkin (1991) Neurology. 41:332*.).*

For treatment of MS, exacerbations can be defined as the appearance of a new symptom that is attributable to MS and accompanied by an appropriate new neurologic abnormality (see *e.g*., IFN-β MS Study Group). Exacerbations typically last at least 24 hours, and are preceded by stability or improvement for at least 30 days or a separation of at least 30 days from onset of the last event. Standard neurological examinations may result in the exacerbations being classified as either mild, moderate, or severe according to changes in a Neurological Rating Scale (see *e.g*., Sipe et al. (1984) Neurology 34:1368), and/or changes in EDSS score or evaluating physician opinion. An annual exacerbation rate (or other measures for the frequency of relapses, like e.g., a hazard ratio for recurrent relapses), the proportion of exacerbation-free subjects, and other relapse-based measures for disease activity are then determined, and the effectiveness of therapy is assessed between the treated group and the placebo group, for any of these measurements.

Further, suitable vectors for use in the compositions and methods of the present invention for the treatment of disease are those having minimal immunological toxicity, e.g., plasmid or AAV vectors. For example, plasmid vectors encoding either TGF-β or IL-4, under control of a CMV promoter, reportedly protect mice from myelin basic protein (MBP) induced EAE with minimal immunolgical toxicity (*see* e.g., C.A. Piccirillo and G.J. Prud'homme (1999) Human Gene Therapy 10: 1915-22)). Further, a variety of vectors and target tissues are reportedly suitable for use for expressing cytokines in an EAE model, including a non-replicative herpes simplex (HSV) type-1 vector expressing IL-4, IL-10, or IL-1 antagonist following intrathecal administration (see e.g., G. Martino et al. (2000) J. Neuroimmunol 107: 184-90).

A number of new technologies can also be used to diagnose and manage treatment of disease (e.g., MS). For example, magnetic resonance imaging (MRI) scanning can be used as a concomitant indicator of disease and disease activity, and can also be used as a diagnostic tool (see *e.g.,* Paty et al (1993) Neurology 43: 662-667; Frank et al. (1994) Ann. Neurology 36(suppl.): S86-S90; (1995) Neurology 45: 1277-1285; Filippi et al. (1994) Neurology 44: 635-641). For example, for the treatment of MS, MRI can be used to measure active lesions using, e.g., gadolinium-DTPA-enhanced T1-weighted imaging (see *e.g*., McDonald et al. (2001) Ann. Neurol. 50: 121-127) or the location and extent of lesions using T2-weighted and T1-weighted techniques. Baseline MRIs can be obtained and thereafter, the same imaging plane and subject position can be used for each subsequent study. For MS, areas of lesions can be outlined and summed slice by slice for total lesion area, and various criteria may be examined, *e.g*.: 1) evidence of new lesions; 2) rate of appearance of active or new lesions; and 3) change in lesion area or lesion volume (see *e.g*., Paty et al. (1993) Neurology 43:665). Thus, improvement due to therapy may then be established, *e.g*., when there is a statistically significant improvement in an individual subject compared to baseline or in a treated group versus a placebo group.

### Formulation and Administration of Compositions

In preferred embodiments, the nucleic acid compositions of the present invention are formulated for administration or delivery to the cells of a subject. The nucleic acid compositions of the present invention are formulated with non-ionic and/or anionic polymers. Such polymers can enhance transfection efficiency and expression of molecules encoded by the nucleic acid, and protect the nucleic acid from degradation. Thus, in some embodiments where transfection efficiency or expression is enhanced using formulated nucleic acid compositions of the present invention, lower amounts of the nucleic acid composition (e.g., a vector encoding a molecule of the present invention, e.g., a TM and/or RM of the present invention) can be used. As used herein, "biodegradable polymers", refers to polymers that can be metabolized or cleared in vivo by a subject and having no or minimal toxic effects or side effects on the subject.

"Anionic polymers" as used herein refers to polymers having a repeating subunit which includes, for example, an ionized carboxyl, phosphate or sulfate group having a net negative charge at neutral pH. Examples of anionic polymers suitable for use in the present invention include, but are not limited to, poly-amino acids (e.g., poly-glutamic acid, poly-aspartic acid and combinations thereof), poly-nucleic acids, poly-acrylic acid, poly-galacturonic acid, and poly-vinyl sulfate. In some embodiments, where the polymer is a polymeric acid, the polymer is utilized as a salt form. Examples of other polymers include, but are not limited to PVP, PVA, and chitosan. As used herein, "poly-L-glutamic acid" is used interchangeably herein with "poly-L-glutamic acid, sodium salt", "sodium poly-L-glutamate" and "poly-L-glutamate." "Poly- L-glutamate" as used herein refers to a sodium salt of poly-L-glutamic acid. Further, in preferred embodiments the L stereoisomer of polyglutamic acid is used in the compositions of the present invention, but, in other embodiments, other stereoisomer or racemic mixtures of isomers are suitable for use in the compositions of the present invention. Further, in some embodiments other salts of anionic amino acid polymers are suitable for use in the compositions of the present invention.

The term "anionic amino acid polymers" as used herein refers to polymeric forms of a given anionic amino acid, for example, a poly-glutamic acid or poly-aspartic acid. In some embodiments, polymers formed of a mixture of anionic amino acids, for example glutamic acid and aspartic acid, may be equally suitable for use in compositions of the present invention.

Methods for formulating pharmaceutical compositions are generally known in the art. For example, see Remington's Pharmaceutical Sciences 18. sup. ed.: Mack Pub. Co.: Eaton, Pa. 1990, for a thorough discussion on the formulation and selection of pharmaceutically acceptable carriers, stabilizers, and isomolytes (also *see, e.g.,* U.S. Patent Nos. 4,588,585; 5,183,746; 5,795,779; and 5,814,485; U.S. Application Nos. 10/190,838, 10/035,397; and PCT International Application Nos. PCT/US02/21464 and PCT/US01/51074).

A pharmaceutically acceptable carrier and other components (*e.g*., co-medications) may be used in the pharmaceutical compositions and methods of the present invention. As used herein, "pharmaceutically acceptable carrier" is a carrier or diluent that is conventionally used in the art to facilitate the storage, administration, and/or the desired effect of the therapeutic ingredients of the pharmaceutical composition. A carrier may also reduce undesirable side effects of administering or delivering to a subject a pharmaceutical composition of the present invention. A suitable carrier is preferably stable, e.g., incapable of reacting with other ingredients in the formulation. Further, a suitable carrier preferably does not produce significant local or systemic adverse effect in a subject at the doses and concentrations employed for therapy. Such carriers are generally known in the art.

Suitable pharmaceutically acceptable carriers are, *e.g*., solvents, dispersion media, antibacterial and antifungal agents, microcapsules, liposomes, cationic lipid carriers, isotonic and absorption delaying agents and the like which are not incompatible components of the pharmaceutical compositions of the present invention. The use of such media and agents for therapeutically effective or active substances is well known in the art. Supplementary active ingredients may also be incorporated into the pharmaceutical compositions of the present invention and used in the methods of the present invention.

Additional examples of pharmaceutically suitable carriers for use in the pharmaceutical compositions of the present invention are large stable macromolecules such as albumin, gelatin, collagen, polysaccharide, monosaccharides, polyvinylpyrrolidone, polylactic acid, polyglycolic acid, polymeric amino acids, fixed oils, ethyl oleate, liposomes, glucose, sucrose, lactose, mannose, dextrose, dextran, cellulose, mannitol, sorbitol, polyethylene glycol (PEG), heparin alginate, and the like. Slow-release carriers, such as hyaluronic acid, may also be suitable.

Stabilizing agents such as human serum albumin (HSA), mannitol, dextrose, trehalose, thioglycerol, and dithiothreitol (DTT), may also be added to the pharmaceutical compositions of the present invention to enhance their stability. Suitable stabilizing agents include but are not limited to ethylenediaminetetracetic acid (EDTA) or one of its salts such as disodium EDTA; polyoxyethylene sorbitol esters e.g., polysorbate 80 (TWEEN 80), polysorbate 20 (TWEEN 20); polyoxypropylene-polyoxyethylene esters e.g., Pluronic F68 and Pluronic F127; polyoxethylene alcohols *e.g*., Brij 35; semethicone; polyethylene glycol *e.g*., PEG400; lysophosphatidylcholine; and polyoxyethylene-p-t-octyphenol *e.g*., Triton X-100. Stabilization of pharmaceutical compositions by surfactants is generally known in the art (*see e.g*., Levine et al. (1991) J. Parenteral Sci. Technol. 45(3):160-165*).*

Other acceptable components of the pharmaceutical compositions of the present invention may include, but are not limited to, buffers that enhance isotonicity such as water, saline, phosphate, citrate, succinate, acetic acid, aspartate, and other organic acids or their salts. Preferably, pharmaceutical compositions of the present invention comprise a non-ionic tonicifying agent in an amount sufficient to render the compositions isotonic with body fluids. The pharmaceutical compositions of the present invention can be made isotonic with a number of non-ionic tonicity modifying agents generally known to those in the art, *e.g*., carbohydrates of various classifications (see, *e.g*., Voet and Voet (1990) Biochemistry (John Wiley & Sons, New York); monosaccharides classified as aldoses (*e.g*., glucose, mannose, arabinose), and ribose, as well as those classified as ketoses (*e.g*., fructose, sorbose, and xylulose); disaccharides (*e.g*., sucrose, maltose, trehalose, and lactose); and alditols (acyclic polyhydroxy alcohols) *e.g*., glycerol, mannitol, xylitol, and sorbitol. In a preferred embodiment, non-ionic tonicifying agents are trehalose, sucrose, and mannitol, or a combination thereof.

Preferably, the non-ionic tonicifying agent is added in an amount sufficient to render the formulation isotonic with body fluids. When incorporated into a pharmaceutical composition of the present invention (including, *e.g*., an HSA-free pharmaceutical composition), the non-ionic tonicifying agent is present at a concentration of about 1 % to about 10 %, depending upon the agent used (see *e.g*., U.S. Patent Application Nos. 10/190,838, 10/035,397; and PCT International Application Nos. PCT/US02/21464 and PCT/US01/51074).

Further, preferred pharmaceutical compositions of the present disclosure may incorporate buffers having reduced local pain and irritation resulting from injection, or improve solubility or stability of a component of the pharmaceutical compositions of the present invention (e.g., comprising and/or encoding a TM, RM, AM, and/or IM). Such buffers include, but are not limited to, *e.g*., low-phosphate, aspartate, and succinate buffers.

The pharmaceutical compositions of the present invention may additionally comprise a solubilizing compound or formulation that is capable of enhancing the solubility of the components of the compositions. Suitable solubilizing compounds include, *e.g*., compounds containing a guanidinium group, preferably arginine. Additional examples of suitable solubilizing compounds include, but are not limited to, *e.g*., the amino acid arginine, or amino acid analogues of arginine that retain the ability to enhance the solubility of an IFN-β mutein of the present invention. Examples of such amino acid analogues include but are not limited to, *e.g*., dipeptides and tripeptides that contain arginine. Further examples of suitable solubilizing compounds are discussed in, *e.g*., U.S. Patent Nos. 4,816,440; 4,894,330; 5,005,605; 5,183,746; 5,643,566; and in Wang et al. (1980) J. Parenteral Drug Assoc. 34: 452-462).

The pharmaceutical compositions of the present invention (e.g., comprising and/or encoding a TM, RM, AM, and/or IM) are formulated in a unit dosage and in an injectable form such as a solution, suspension, or emulsion, or in the form of lyophilized powder, which can be converted into solution, suspension, or emulsion prior to administration. The pharmaceutical compositions of the present invention may be sterilized by membrane filtration, which also removes aggregates, and stored in unit-dose or multi-dose containers such as sealed vials, ampules or syringes.

An AM or IM of the present invention is formulated for oral administration. In one embodiment, the nucleic acids encoding a TM and/or RM of the present invention are formulated for administration by injection or electroporation, and an AM and/or IM of the present invention is formulated for oral administration. For example, in a preferred embodiment, the regulated expression system of the present invention comprises a single vector encoding at least a TM and an RM formulated for delivery by injection or electroporation to the cells of a subject, and an AM that is formulated for oral administration to the subject, such that the presence of the AM in the cell of the subject activates the RM and thereby the RM induces expression of the TM in the cells of the subject.

Liquid, lyophilized, or spray-dried pharmaceutical compositions of the present disclosure may be prepared as known in the art, *e.g*., as an aqueous or nonaqueous solution or suspension for subsequent administration to a subject in accordance with the methods of the present invention. Each of these pharmaceutical compositions may comprise a therapeutically or prophylactically effective or active component. As used herein, a therapeutically or prophylactically "effective" or "active" component is an amount of a molecule of the present invention (e.g., comprising and/or encoding a TM, RM, AM, and/or IM) that is included in the pharmaceutical composition of the present invention to bring about a desired therapeutic or prophylactic response with regard to treatment, prevention, or diagnosis of a disease or condition in a subject in need of treatment, using the pharmaceutical compositions and methods of the present invention. Preferably the pharmaceutical compositions of the present invention comprise appropriate stabilizing agents, bulking agents, or both to minimize problems associated with loss of biological or therapeutic activity during preparation and storage.

Formulation of the pharmaceutical compositions of the present invention are preferably stable under the conditions of manufacture and storage and preserved against the contaminating action of microorganisms such as bacteria and fungi. Methods of preventing microorganism contamination are well known, and can be achieved *e.g*., through the addition of various antibacterial and antifungal agents.

Suitable forms of the pharmaceutical composition of the present invention may include sterile aqueous solutions or dispersions, and sterile powders for the extemporaneous preparation of sterile injectable solutions or dispersion. Suitable forms are preferably sterile and fluid to the extent that they can easily be taken up and injected via a syringe. Typical carriers may include a solvent or dispersion medium containing, for example, water buffered aqueous solutions (*i.e*., biocompatible buffers), ethanol, polyols such as glycerol, propylene glycol, polyethylene glycol, suitable mixtures thereof, surfactants, or vegetable oils. Sterilization can be accomplished by any art-recognized technique, including but not limited to filtration or addition of antibacterial or antifungal agents, for example, paraben, chlorobutanol, phenol, sorbic acid or thimerosal. Further, isotonic agents such as sugars or sodium chloride may be incorporated in the subject compositions.

Production of sterile injectable solutions containing a pharmaceutical composition of the present invention may be accomplished by incorporating the composition in the desired amount, in an appropriate formulation with various ingredients (*e.g*., those enumerated herein) as desired, and followed by sterilization. To obtain a sterile powder, the above solutions can be vacuum-dried or freeze-dried as necessary.

The pharmaceutical compositions of the present invention can thus be compounded for convenient and effective administration in pharmaceutically effective amounts with a suitable pharmaceutically acceptable carrier in a therapeutically effective dose. The precise therapeutically effective amount of the compositions and methods of the present invention for application to humans can be determined by the skilled artisan with consideration of individual differences in age, weight, extent of cellular infiltration by inflammatory cells and condition of the MS subject.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier.

The principal active ingredients may be compounded for convenient and effective administration in therapeutically effective amounts with a suitable pharmaceutically acceptable carrier in dosage unit form as described herein. Further, the co-medications are contained in a unit dosage form in amounts generally known in the art. In the case of compositions containing supplementary active ingredients, *e.g*. co-medications, the dosages may be determined, *e.g*., by reference to the known dose and manner of administration of the ingredients.

The pharmaceutical compositions of the present invention may be administered in a manner compatible with the dosage formulation and in such an amount as will be therapeutically effective. Further, the pharmaceutical compositions of the present invention may be administered in any way which is medically acceptable and which may depend on a specific type or stage of disease or associated symptoms being treated. Possible administration routes include injections, by parenteral routes such as intravascular, intravenous, intra-arterial, subcutaneous, intramuscular, intratumor, intraperitoneal, intraventricular, intraepidural or others, as well as oral, nasal, ophthalmic, rectal, topical, or by inhalation. In a preferred embodiment, the administration route is intramuscular. In a preferred embodiment, the pharmaceutical composition of the present invention is administered intramuscularly, every 3-12 months. In another preferred embodiment, the intramuscular administration is via automated or manual injection (*e.g*., using a syringe) of the pharmaceutical composition. Sustained release administration is also contemplated, *e.g*., using erodible implants.

In particular, the nucleic acid pharmaceutical compositions of the present invention can be delivered to cells of a subject using any means described herein or known in the art, including e.g., by injection or other suitable means. For example, known methods of delivery of nucleic acids to cells by physical means are suitable for use and include, but are not limited to, electroporation, sonoporation, and pressure. In some embodiments, delivery of a nucleic acid composition of the present invention is by electroporation and comprises the application of a pulsed electric field to create transient pores in the cellular membrane and, thereby, an exogenous molecule, e.g., a nucleic acid composition of the present invention, is delivered to the cell. It is known that adjusting the electrical pulse generated by an electroporetic system, nucleic acid molecules can find their way through passageways or pores in the cell that are created during such a procedure (*see e.g*., U. S. Patent No. 5, 704,908, U.S. Patent No. 5,704,908).

As used herein, "pulse voltage device", or "pulse voltage injection device" refers to an apparatus that is capable of causing or causes uptake of nucleic acid molecules into the cells of a subject by emitting a localized pulse of electricity to the cells, thereby, causing the cell membrane to destabilize and result in the formation of passageways or pores in the cell membrane. Conventional devices of this type are suitable for use for the delivery of a nucleic acid composition of the present invention. In some embodiments, the device is calibrated to allow one of ordinary skill in the art to select and/or adjust the desired voltage amplitude and/or the duration of pulsed voltage and therefore. A pulse voltage nucleic acid delivery device can include, for example, an electroporetic apparatus as described e.g. in U.S. Patent No. 5,439,440, U.S. Patent No. 5,704,908, U.S. Patent No. 5,702,384, and in international patent publications No. WO96/12520, No. WO 96/12006, No. WO 95/19805, or No. WO 97/07826.

Packaging material used to contain the active ingredient of a pharmaceutical composition of the present invention can comprise glass, plastic, metal or any other suitable inert material and, preferably, is packaging material that does not chemically react with any of the ingredients contained therein. In one embodiment, the pharmaceutical composition is packaged in a clear glass, single-use vial; and a separate vial containing diluent is included for each vial of drug. In another preferred embodiment, the diluent is provided in a syringe (i.e., the syringe is pre-filled with the diluent). In yet another preferred embodiment, the pharmaceutical composition of the present invention is provided in solution in a syringe (i.e., the syringe is pre-filled with the pharmaceutical composition in solution) and is ready for use. In one embodiment, the pharmaceutical composition of the present invention can be stored under refrigeration, between 2° to 8°C (36° to 46°F). In a preferred embodiment, the pharmaceutical composition is stored at room temperature.

### Vectors And Kits

The present disclosure further provides vectors and kits comprising the improved regulated expression system of the present invention for treatment of disease. In some embodiments, the improved regulated expression system of the present invention comprises one or more vectors, and each vector comprises one or more expression cassettes. In one embodiment, the improved regulated expression system of the present invention comprises a single vector having at least one expression cassette and, more preferably at least two expression cassettes.

Suitable vectors for use in the regulated, expression system of the present invention, include, but are not limited to, those that are capable of expressing an encoded TM, and/or other encoded molecule of the present invention (e.g., RM, AM, or IM), when administered to the cells of a subject. Examples of suitable vectors include, but are not limited to, those described herein and those known in the art, including vectors for producing virus and nonviral vectors (vectors that do not produce virus). For example, one class of suitable vectors utilize DNA elements which provide autonomously replicating extra-chromosomal plasmids, derived from animal viruses such as bovine papilloma virus, polyoma virus, adenovirus, or SV40 virus. Further, known vectors for producing virus (*see e.g*., Wang, et al., Gene Therapy, 4: 432- 441,1997; Oligino, et al., Gene Therapy 5: 491-496,1998) may be modified and adapted for use in the regulated expression system of the present invention. Further, the vectors of the present invention can be modified to include additional functional and operably linked sequences for optimal expression of an encoded molecule. Examples of suitable functional sequences include, but are not limited to, splicing, polyadenylation and other types of RNA processing sequences; and transcriptional promoter, enhancer, and termination sequences. Suitable cDNA expression vectors incorporating such functional sequences include those described by Okayama, H., Mol. Cell. Biol. 3:280 (1983), and others.

"Plasmid" as used herein refers to a composition comprising extrachromosomal genetic material, usually of a circular duplex of DNA that can replicate independently of chromosomal DNA. Plasmids may be used as vectors, as described herein. "Vector" as used herein refers to a composition (e.g., a nucleic acid construct) comprising genetic material designed to direct transformation or transfection of a targeted cell. Further, a vector may contain multiple functional sequences positionally and sequentially oriented with respect to other sequences of the vector such that an encoded molecule of the present invention can be transcribed and when necessary translated in the transfected or transformed cells. Where a vector or expression cassette encodes a molecule of the present invention (e.g., a TM, RM, AM or IM), it comprises the essential components (e.g., promoter, poly(A) site, transcription start and stop sites) for expression of the encoded molecule in a heterologous cell (e.g., cells of a subject) according to the regulated expression system of the present invention, described herein.

The improved regulated expression system of the present invention comprises a single vector comprising a first expression cassette having at least one cloning site for insertion of a first nucleic acid sequence encoding a TM, and a second expression cassette having at least one cloning site for insertion of second nucleic acid sequence encoding an RM. In another embodiment, the vector is a vector for producing virus encoding a molecule of the present invention (e.g., a TM and/or RM) for delivery to cells of a subject as described herein, e.g., a shuttle plasmid and more particularly, an AAV-1 shuttle plasmid (see e.g., Table 8).

The vector is a single plasmid vector e.g., pGT1 (comprising the sequence of SEQ ID NO: 9 and SEQ ID NO: 4), pGT2 (comprising the sequence of SEQ ID NO: 10 and SEQ ID NO: 4), pGT3 (comprising the sequence of SEQ ID NO: 11 and SEQ ID NO: 4), or pGT4 (SEQ ID NO: 12), wherein each vector comprises a multiple cloning site (SEQ ID NO: 4) located 3' of the IVS8 and 5' of the hGH poly(A) site (e.g., as schematically depicted in Figures 14A-D), or pGT11 (comprising the sequence of SEQ ID NO: 9 and SEQ ID NO: 5), pGT12 (comprising the sequence of SEQ ID NO: 10 and SEQ ID NO: 5), pGT13 (comprising the sequence of SEQ ID NO: 11 and SEQ ID NO: 5), or pGT14 (comprising the sequence of SEQ ID NO: 12 and SEQ ID NO: 5), wherein each vector comprises a multiple cloning site (SEQ ID NO:5) located 3' of the IVS8 and 5' of the hGH poly(A) site. The single plasmid vector is e.g., pGT79 (SEQ ID NO: 59), or a single plasmid vector derived from pGT79, e.g., pGT1003 (comprising SEQ ID NO: 60), pGT1004 (comprising SEQ ID NO: 62), pGT1005 (comprising SEQ ID NO: 64), pGT1006 (comprising SEQ ID NO: 66), pGT1007 (comprising SEQ ID NO: 68), pGT1008 (comprising SEQ ID NO: 70), pGT1009 (comprising SEQ ID NO: 72), pGT1015 (comprising SEQ ID NO: 74), pGT1016 (comprising SEQ ID NO: 76), pGT1017 (comprising SEQ ID NO: 78), pGT1025 (comprising SEQ ID NO: 80), pGT1020 (comprising SEQ ID NO: 82), pGT1021 (comprising SEQ ID NO: 84), pGT1022 (comprising SEQ ID NO: 86), pGT1023 (comprising SEQ ID NO: 88), or pGT1024 (comprising SEQ ID NO: 90).

The expression cassettes of the present invention comprise functional sequences for expression of an encoded molecule of the present invention, e.g., a TM, RM, AM, or IM. In some embodiments, the expression cassette comprises at least one functional sequence operably linked to a nucleic acid sequence encoding a molecule of the present invention. As used herein "functional sequence" refers to a nucleic acid or amino acid sequence having a function or activity in a cell, e.g., a function or activity relating to the cellular expression, processing, or cloning of a molecule, or to the biological or cellular activity or function of a molecule. Examples of a functional sequence include a sequence encoding a molecule of the present invention (e.g., a TM, RM, AM, or IM), promoter, protein or nucleic acid binding site, splice site, transcription stop site, regulatory domain (e.g., activation domain), transcription start site, protein or nucleic acid stabilization site, intervening sequence, restriction enzyme site or cloning site, viral packaging signal, or other cellular, protein, or nucleic acid processing or regulatory signal (e.g., a signal transduction sequence or tissue-specific sequence). Further examples of a functional sequence are, but not limited to, a 5' or 3' untranslated region (e.g., UT12, SEQ ID NO: 2), intron (e.g., IVS8, SEQ ID NO: 3), polyadenylation (poly(A)) site (e.g, SV40, SEQ ID NO: 8 or hGH poly(A) site, SEQ ID NO: 6), or a DNA-binding site (DBS) (e.g., SEQ ID NO: 49).

Such functional sequences also include, for example, sequences encoding a regulated promoter or tissue-specific promoter that promotes the regulated or tissue-specific expression, respectively, of a molecule encoded by a nucleic acid sequence operably linked to such functional sequences in an expression cassette of the present invention. Examples of suitable promoters include, but are not limited to, a CMV promoter, muscle-specific promoter (e.g., actin promoter or muscle creatine kinase (MCK) promoter), condition-specific (e.g., hypoxia or inflammation) promoter or element (e.g. ELAM promoter or HRE), constitutive promoter (e.g., ubiquitin, PGK, or EF1α promoter), synthetic or chimeric promoter (e.g., CMV/actin promoter), cell-cycle specific promoter (e.g., cyclin A or cdc6 promoter). In one embodiment, the promoter is a physiologically responsive promoter, e.g., a promoter responsive to inflammation and, preferably responsive to the presence of cytokines or chemokines or other cellular or biological molecules indicative of the onset or presence of a disease or condition. Further examples of suitable promoters are provided in Table 1 below.

**Table 1**

| Promoter | Plasmid/Source | Description | Reference(s) |
|---|---|---|---|
| EF-1α | pdrive-chef1 (InvivoGen) | Chimpanzee EF-1α promoter | e.g., KimDW, et al.1990 Gene. 91(2):217-23; Guo ZS, et al. 1996 Gene Ther. 3(9):802-810 |
| EF-1α/RU5 | pdrive-chef1ru5 (InvivoGen) | Chimpanzee EF-1α promoter +HTLV 5'UTR | e.g., KimDW, et al. 1990 Gene. 91(2):217-23; Guo ZS, et al. 1996 Gene Ther. 3(9):802-810; Takebe Y. et al 1998 Mol Cell Bio. 8(1):466-472 |
| UbiB | pdrive-hubib (InvivoGen) | Human Ubiquitin B promoter | e.g., Ciechanover A.and Schwartz AL. 1998 PNAS 95(6):2727-30; Yew NS, et al. 2001 Mol Ther. 4(1):75-82 |
| Sk promoter | pGS1694 (Valentis) | Skeletal muscle actin promoter+UT12 5'UTR+IVS8 intron | e.g., PCT application no. PCT/US01/30305 |
| MCK promoter | Mouse genomic DNA | Mouse Muscle Creatine Kinase | e.g., Jaynes JB et al, 1986 Mol Cell Biol. Aug;6(8):2855-64. Hauser et al 2000, Mol Ther 2:16-25 |
| EF-1α/RU5 | pORF-htrail (InvivoGen) | Human EF-1α + HTLV 5'UTR and bacterial promoter | e.g., KimDW, et al. 1990 Gene. 91(2):217-23; Guo ZS, et al. 1996 Gene Ther. 3(9):802-810; Takebe Y. et al 1998 Mol Cell Bio. 8(1):466-472 |
| TK promoter | phRL-TK (Promega) | HSV Thymidine Kinase promoter | e.g., Wagner EF et al, 1985 EMBO J (4) 663-6; Stewart CL et al, 1987 EMBO J (6) 383-8 |

In one embodiment, the regulated expression system of the present invention comprises one or more vectors comprising at least one expression cassette having a CMV promoter sequence that is operably linked to a nucleic acid sequence encoding a molecule of the present invention that is a fusion or chimeric protein, and the promoter drives the expression of the encoded molecule (e.g., TM, RM, AM, or IM). In some embodiments, a suitable promoter would be one that would provide a durable level of expression of the encoded molecule in the cells of a subject. In one embodiment, a nucleic acid vector encodes a molecule of the present invention (e.g., TM, RM, AM, or IM) that is operably linked to a promoter that provides durable expression in the cells of a subject, and the nucleic acid vector is administered to the cells of the subject via electroporation, such that the molecule is expressed in the cells, preferably, at the site of administration. In a preferred embodiment, the encoded molecule is a TM.

In another embodiment, the regulated expression system of the present invention comprises one or more vectors comprising a first expression cassette having a promoter sequence comprising at least one GAL-4 DBS, operably linked to a nucleic acid sequence encoding a TM of the present invention. In one embodiment, the promoter sequence comprises multimers of a GAL-4 DBS, e.g., 3-18 GAL-4 DBS.

Further, the expression cassettes of the present invention can be suitably modified to comprise cloning sites for the insertion of a desired nucleic sequence. In another embodiment, the expression cassettes of the present invention comprise at least one cloning site and, more preferably a multiple cloning site (MCS), for the insertion of a nucleic acid sequence encoding a molecule of the present invention, e.g., a TM, RM, AM, or IM. As used herein, "cloning site" refers to an enzyme site or other site in a nucleic acid wherein a nucleic acid sequence can be inserted, operably linked, or otherwise attached using conventional methods known in the art e.g., such that the sequence functions for its intended purpose. In one embodiment, a first expression cassette of the present invention comprises an MCS for insertion of a first nucleic acid sequence encoding a TM, an inducible promoter comprising at least one DBS (e.g., 3-18 GAL-4 DBS), 5' untranslated region (e.g., UT12, SEQ ID NO: 2), an intron (e.g., IVS8, SEQ ID NO: 3), and hGH poly(A) site (e.g., SEQ ID NO: 6), such that when the first nucleic acid sequence is inserted at the MCS (e.g., SEQ ID NO: 4, or SEQ ID NO: 5), these functional sequences are operably linked to the first nucleic acid sequence. In another embodiment, a second expression cassette of the present invention comprises an MCS for insertion of a second nucleic acid sequence encoding a regulated RM and SV40 poly(A) site (e.g., SEQ ID NO: 8), such that when the second nucleic acid sequence is inserted at the MCS, these functional sequences are operably linked to the second nucleic acid sequence. In a preferred embodiment, the first and second expression cassettes are present in a single vector as described above.

The kits of the present invention comprise at least one of the expression systems of the present invention described herein and, more particularly, at least one of the pharmaceutical compositions, vectors, or molecules (e.g., TM, RM, AM, or IM) of the present invention, and instructions for their use.

### Isolation and Construction of Compositions

The compositions of the present invention include, for example, chemical compounds, proteins, and nucleic acids (e.g., DNA or RNA molecules), particularly, nucleic acids encoding a protein or RNA. The chemical, nucleic acid, and protein compositions of the present invention can be isolated, constructed, and/or tested using conventional methods and assays, as described herein or in the art.

"Protein" or "amino acid molecule" as used herein refers to a peptide, full-length protein, or fragment or portion of a full-length protein. Further, a protein of the present invention can be a fused, chimeric, modified, isolated, synthetic, or recombinant amino acid molecule. In particular, examples of proteins suitable for use in the compositions and methods of the present invention, include, but are not limited to, a wild-type, full-length protein (including a secreted form thereof), or an analog, derivative, or variant thereof having a biological or therapeutic activity. More particularly, protein variants of the present invention can be muteins i.e., comprising a mutation e.g., a single or multiple amino acid substitution, deletion, or addition such that the variant retains or has a biological or therapeutic activity. Sequences encoding a protein may include, e.g., codon-optimized versions of wild-type protein sequences, or humanized sequences. Optimal codon usage in humans can be identified from codon usage frequencies for expressed human genes and may be determined by methods known in the art e.g., program "Human High.codN" from the Wisconsin Sequence Analysis Package, Version 8.1, Genetics Computer Group, Madison, WI. For example, codons that are most frequently used in highly expressed human genes may be optimal codons for expression in the cells of a human subject, and, thus, can be used as a basis for constructing a synthetic coding sequence.

"Nucleic acid" as used herein with reference to a molecule of the present invention, refers to a nucleic acid molecule, e.g., a DNA or RNA, or fused, chimeric, modified, isolated, synthetic, or recombinant form thereof. In particular, examples of nucleic acids suitable for use in the compositions and methods of the present invention, include, but are not limited to, a wild-type, full-length DNA or RNA (e.g., mRNA) encoding a protein, or other nucleic acid molecule having a biological or therapeutic activity (e.g., shRNA, siRNA, ribozyme, antisense RNA or DNA, RNA or DNA oligonucleotide), or an analog, derivative, or variant thereof. More particularly, nucleic acid variants of the present invention can be muteins i.e., comprising a mutation, e.g., a single or multiple nucleic acid substitution, deletion, or addition such that the variant retains or has a biological or therapeutic activity.

Further, modifications of the regulated, expression system of the present invention can be carried out and tested using conventional methods and assays, as described herein or in the art.

"Modified" as used herein, with reference to a molecule of the present invention (e.g., comprising and/or encoding a TM, RM, AM, and/or IM) refers to any reaction or manipulation resulting in a change or alteration of a reference nucleic acid, amino acid, or chemical molecule to arrive at a desired composition or molecule of the present invention (e.g., mutation of a wild-type protein or nucleic acid to arrive at a desired variant thereof having a specific biological and/or therapeutic activity; mutation of a protein or nucleic acid sequence to arrive at a desired humanized sequence; or mutation of a chemical compound to arrive at a desired chemical structure, and/or biological and/or therapeutic activity). For example, the functional domains or functional sequences of a molecule of the present invention, including any sequence operably linked to or encoding a molecule of the present invention (including e.g., a vector sequence or transcription control sequence), can be modified to arrive at a desired composition or molecule of the present invention.

In particular, the regulated expression system of the present invention can be modified or optimized to achieve a particular specificity (e.g., specific to a particular tissue, condition, disease, or biomarker or other molecule), stringency, or amount of regulation, expression, and/or activity for use in the treatment of disease, as described herein. For example, the regulated, expression system of the present invention can be modified or optimized to achieve such objectives by isolating or constructing: 1) new or variant AMs and IMs having a desired binding specificity for the LBD of an RM; 2) RMs having a new or variant AD, LBD (e.g., that binds a new or variant AM or IM), and/or DBD (e.g., that binds a new or variant promoter sequence); 3) promoters having activity that is highly specific and responsive to the presence of a particular RM (e.g., that are specifically, activated or inactivated in the presence of, e.g., by the binding of, a particular RM); 4) fully humanized sequences (e.g., modifying sequences encoding a GAL-4 DBD and GAL-4 DBS such that the sequences are fully humanized); 5) an expression cassette for expression of an RNA (e.g., shRNA, siRNA, ribozyme, or antisense RNA), particularly, a RNA TM; and 6) modifying promoter or other functional sequences to reduce non-specific expression, particularly, of a TM encoded by a sequence operably linked to the promoter or other functional sequences.

The regulated expression system of the present invention is modified such that the basal expression of a TM is significantly reduced in order to increase reliance on administration of an RM and, thereby, provide an increased margin of safety by virtue of extrinsically controlled TM expression rather than through dependence on the dose of plasmid administrated. For example, a promoter sequence operably linked to a nucleic acid coding sequence encoding a TM, may be modified and optimized for the number of copies of a GAL-4 DBS, such that the responsiveness of the promoter (and resulting TM expression) can be modulated by the presence of (e.g., binding of) an RM having a GAL-4 DBD. Also for example, a minimal promoter can be constructed and modified using standard methods and operably linked to a TM to reduce the basal expression of the TM.

Further the TM is encoded by a nucleic acid sequence that when delivered to and/or is present in the cells of a subject, the TM is expressed at a low level. In some embodiments, it is preferable to regulate the level of TM expression by inherent properties of the nucleic acid encoding the TM that is delivered to and/or present in the cells of the subject. For example, in some embodiments, as the level of basal TM expression in the cells of a subject increases with an increasing amount of nucleic acid encoding the TM, it may be desirable to reduce the amount of expressed TM protein in the cells of the subject by utilizing a weak promoter.

Utilizing unique restriction endonuclease sites in the promoter region, different regions of the promoter and 5' UTR can be modified to delete sequences that may have an effect on the expression of a TM in the presence or absence of an RM of the present invention. For example, in another embodiment a deletion is made in a sequence encoding the transcription initiation region (inr) such that the intrinsic activity of an inducible promoter that is operably linked to a sequence encoding a TM, is modified, e.g., the activity is decreased or is increased. In another embodiment, downstream of the transcription initiation region (inr), is an operably-linked sequence encoding the UT12 (5' untranslated region of CMV, +1 to +112).

The TM is encoded by a nucleic acid sequence that is operably linked to an inducible promoter sequence (e.g., SEQ ID NO: 1) having 6X GAL-4 DBS operably linked to a TATA box sequence. The sequence from -33 to -22, which contains the TATA box from the EIb region of Adenovirus type 2 (residues 1665-1677 of NCBI accession no. J01917) is suitable for use in such an embodiment of the present invention. In another embodiment, the promoter sequence comprises 6X GAL-4 DBS operably linked to an Ad EIb TATA box sequence and a CMV sequence that contains the putative initiator (inr) region of the CMV promoter (Macias et al., Journ. of Virol. 70(6):3628 (1996)), such that these functional sequences are operably linked to a nucleic acid encoding a TM.

The TM is encoded by a nucleic acid sequence that is operably linked to multiple copies of a GAL-4 DBS comprising a 17 nucleotide sequence 5'-TGGAGTACTGTCCTCCG-3' or 5'-CGGAGTACTGTCCTCCG-3' (e.g., of the consensus GAL-4 DBS of SEQ ID NO: 49). In one embodiment, the TM is encoded by a nucleic acid sequence that is operably linked to 4 copies of a GAL-4 DBS each comprising the 17 nucleotide sequence 5'-CGGAGTACTGTCCTCCG-3' separated by a 10 nucleotide spacer having the nucleotide sequence 5'-AGTTTAAAAG-3' as in e.g., SEQ ID NO: 50. In another embodiment, the TM is encoded by a nucleic acid sequence that is operably linked to 6 copies of a GAL-4 DBS arranged in two groups with 3 copies each of a GAL-4 DBS, and wherein: 1) in each group (containing 3 copies of a GAL-4 DBS) the second copy of the GAL-4 DBS comprises the 17 nucleotide sequence 5'-TGGAGTACTGTCCTCCG-3' and the first and third copy of the GAL-4 DBS each comprise the 17 nucleotide sequence 5'-CGGAGTACTGTCCTCCG-3; 2) each copy within the group of 3 copies is separated by two nucleotides 5'-AG-3'; and 3) between the two groups of 3 copies there is a longer spacer sequence 5'-AGTCGAGGGTCGAAG-3' (e.g., the sequence of SEQ ID NO: 51 comprising 6 copies of a GAL-4 DBS as described).

Where the expression in a particular tissue is desired, the regulated expression system of the present invention may be modified to comprise tissue-specific promoters. For example, if the target tissue for TM expression is muscle, the nucleic acid sequence encoding a TM may be operably linked to a muscle-specific promoter, e.g., an actin promoter sequence. Tissue-specific promoters, (e.g., muscle-specific promoters) may increase the fidelity of expression of the encoded TM. In some embodiments, tissue-specific promoters may provide the advantage of reduced expression in dendritic and other antigen-presenting cells, thus avoiding immune responses to the expressed TM (e.g., protein or nucleic acid).

The regulated expression system of the present invention is modified to impose a lag time between delivery of a nucleic acid encoding a TM (e.g., a vector) and the induction of TM expression, particularly where there is an inflammatory response to the nucleic acid delivery. In this embodiment, TM expression can be delayed until there is a reduction in the inflammatory response. For example, in some embodiments, by increasing the length of the lag period (time before inducing TM expression) from e.g., 12 to 54 days, the incidence of anti-TM antibody production can be decreased. Further, in some embodiments it is desirable to lengthen the delay between the introduction of the nucleic acids encoding a TM, and the administration of an AM (e.g., an RM modulator, particularly a modified receptor modulator, more particularly a modified steroid receptor modulator, even more particularly a modified PR or GR modulator, even more particularly a PR or GR ligand analog, and yet even more particularly a modified antiprogestin (e.g., a modified MFP) or a modified mesoprogestin (e.g., a modified asoprisnil)) that regulates the expression and/or activity of the TM in the cells of a subject (e.g., where the AM activates an RM and the presence of the activated RM thereby regulates TM expression and/or activity). In one embodiment, the lag period is 12 days, preferably 20 days, and more preferably 55 days or until the immune response has decreased.

The regulated expression system is modified such that the specificity, selectivity, precise timing, and/or level of TM expression and/or activity is modulated in the presence of an RM. In a further embodiment, the RM has a rapid clearance in a subject administered an RM of the present invention.

The RM is a protein and is modified such that it is activated in the presence of a specific or cognate ligand and, thereby, the presence of the activated RM regulates the expression and/or activity of a TM. Further, the specificity and stringency of activation of the RM can be optimized by mutation of the GAL-4 DBD of the RM to minimize any propensity to form dimers in the absence of an AM. More specifically, to minimize any non-specific activation and/or dimer formation of the RM (e.g., in the absence of an AM), the RM can be modified by mutation of the GAL-4 domain by deleting or otherwise mutating the C-terminal portion of the GAL-4 DBD (e.g., 20 C-terminal residues) and, thereby, reducing the length of a coiled-coil structure that is predicted to contribute to GAL-4 homodimer formation.

The GAL-4 DNA Binding Domain ("GAL-4 DBD") comprises a portion or fragment of amino acids 1-93 of the N-terminal DBD of GAL-4 (where e.g., the sequence of amino acids 2-93 is SEQ ID NO: 37 and amino acid 1 is a methionine). For example, in some embodiments, the GAL-4 DBD comprises amino acids 2-93 of the N-terminal DBD of GAL-4 (e.g., comprising the amino acid sequence of SEQ ID NO: 38, or encoded by the nucleic acid sequence of SEQ ID NO: 37). In one embodiment, the GAL-4 DBD comprises amino acids 2-93 of the N-terminal DBD of GAL-4 and an operably linked N-terminal peptide sequence e.g. as in SEQ ID NO: 46 (or as encoded by the nucleic acid sequence of SEQ ID NO: 45), wherein, e.g., the N-terminal peptide sequence is immediately followed by amino acids 2-93 of the N-terminal DBD of GAL-4. In other embodiments, the GAL-4 DBD comprises amino acids 2-74 of the N-terminal DNA binding domain of GAL-4 (e.g., comprising the amino acid sequence of SEQ ID NO: 48, or encoded by the nucleic acid sequence of SEQ ID NO: 47). In some embodiments, a suitable GAL-4 DBD has a modification in a nucleic acid sequence or amino acid sequence that results in a mutation of the GAL-4 DBD such that it retains the ability to bind to a canonical 17-mer binding site, CGGAAGACTCTCCTCCG, but has a reduced ability to form a helical tertiary structure needed for autodimerization. In some embodiments, mutations or deletions are made to the region spanning amino acids 75 to 93 and/or 54 to 74 of the GAL-4 DBD sequence. For example, in one embodiment, a deletion is made of the amino acids 54 to 64 or 65 to 75 of the GAL-4 DBD sequence, such that autodimerization is minimized through the coiled-coil region of an RM comprising the mutated GAL-4 DBD.

The nucleic acid sequence of the RM is modified to encode a fusion or chimeric protein comprising one or more functional domains, e.g., a DNA binding domain (DBD), ligand-binding domain (LBD), and/or regulatory domain (RD) (e.g, an activation domain). Examples of suitable functional domains for use in the fusion or chimeric proteins of the present invention include, but are not limited to the GAL-4 DBD, human progesterone receptor (hPR) LBD, and NFKappaB p65 AD.

Examples of suitable regulatory domains (RD) for use in an RM of the present invention, include, but are not limited to, NFkappaBp65, VP-16, TAF- 1, TAF-2, TAU-1, TAU-2, ORF-10, TEF-1, and any other nucleic acid or amino acid sequences having a regulatory function (e.g., regulates the expression and/or activity of a molecule of the present invention) and, more particularly, a transcriptional regulatory function (*see e.g*., Pham et al. (1992) Mol. Endocrinol. 6(7):1043-50; Dahlman-Wright et al. (1994) Proc. Natl. Acad. Sci. U.S.A. 91: 1619-1623; Milhon et al. (1997) Mol. Endocrinol. II(12):1795-805; Moriuchi et al. (1995) Proc. Natl. Acad. Sci. USA 92(20):9333-7); Hwang et al. (1993) EMBO J 12(6):2337-48). In one embodiment, the preferred RD is a human transactivation domain (e.g., NFkappaB p65). In another embodiment, the RM, particularly a functional domain of the RM (e.g., an RD), is humanized.

The LBD of an RM of the present invention is derived from an amino acid sequence correlating to a wild-typed LBD of a receptor in the steroid-receptor family, e.g., a progesterone receptor (PR) and more particularly, a human progesterone receptor (hPR). In another embodiment, the RM is a steroid receptor, and the amino acid sequence of the LBD of the steroid receptor (e.g., a PR, and more particularly an hPR) is mutated to result in a mutated steroid-receptor LBD (e.g., a mutated hPR LBD) that selectively binds to an AM that is an antiprogestin instead of progestin. Thus, in this embodiment, an RM that is a mutated steroid-receptor LBD (e.g., a mutated hPR LBD) can be selectively activated by an AM that is an antiprogestin, instead of a naturally-occurring progestin. In particular, in one embodiment, the antiprogestin binds to a natural PR, but acts as an antagonist.

The progestin binds to a wild-type PR and acts as an agonist, and does not bind to a truncated or mutated PR. In another embodiment, a mutated PR retains the ability to bind antiprogestins, but responds to them as agonists. In a preferred embodiment, when the antiprogestin binds to a mutated PR that is an RM, the mutated PR protein is activated and forms a dimer. In this embodiment, the dimer-antiprogestin complex then binds to the DBS of a promoter sequence and, thereby, induces transcription of a nucleic acid sequence encoding a TM, where the nucleic acid sequence is operably linked to the promoter.

In the presence of the anti-progestin MFP (RU486), the chimeric RM binds to a 17-mer GAL-4 DBS operably linked to a nucleic acid sequence encoding a TM, and results in an efficient ligand-inducible transactivation of TM expression. The modified steroid-hormone LBD of the RM may also be modified by deletion of carboxy terminal amino acids, preferably, from about 1 to 120 carboxy terminal amino acids. The extent of deletion desired can be obtained using standard molecular biological techniques to achieve both selectivity for the desired ligand and high inducibility when the ligand is administered. In one embodiment, the mutated steroid hormone receptor LBD is mutated by deletion of about 1 to about 60 carboxy terminal amino acids. In another embodiment 42 carboxy terminal amino acids are deleted. In yet another embodiment, having both high selectively and high inducibility, 19 carboxy terminal amino acids are deleted.

The nucleic acid sequence of an RM comprises a sequence encoding a truncated GAL-4 DBD, a mutated progesterone receptor having a C-terminal deletion of 19 amino acids, and a p65 transactivation domain (e.g., SEQ ID NO: 39). In another embodiment, the nucleic acid sequence of an RM comprises a sequence encoding a chimeric receptor having a mutated progesterone-receptor ligand-binding domain, a truncated GAL-4 DNA binding domain, and a VP16 or p65 transregulatory domain, where the p65 transregulatory domain is part of the activation domain of the human p65 protein; a component of the NFkappaB complex. By replacing VP16 with a variety of human-derived activation domains, e.g., residues 286-550 of the human p65, the potent inducibility of the chimeric receptor can be retained while "humanizing" the protein or reducing the potential for a foreign protein immune response due to the viral VP16 component.

A DBD of an RM of the present invention is not limited to a modified GAL-4 DBD as described herein. For example, in some embodiments, a suitable DBD is one that has been modified to remove sequences that are not essential for recognition of binding sites but may be predicted to contribute to autodimerization by virtue of their secondary structure. Other DBDs that may be so modified and suitable, include e.g., the known DBD of a member of the steroid-receptor family (e.g., glucocorticoid receptor, progesterone receptor, retinoic acid receptor, thyroid receptor, androgen receptor, ecdysone receptor) or other cellular DNA-binding proteins such as the cAMP Response Element Binding protein (CREB) or zinc finger DNA binding proteins, such as SP1.

The steroid-receptor family of gene regulatory proteins is also suitable for the construction of an RM of the present invention. Steroid receptors are ligand-activated transcription factors whose ligands can range from steroids to retinoids, fatty acids, vitamins, thyroid hormones, and other presently unidentified small molecules. These compounds bind to receptors and either up-regulate or down-regulate the expression of steroid-regulated genes. The compounds are reportedly cleared from the body by existing mechanisms and are usually non-toxic. In the present invention, a ligand of a steroid receptor may be any compound or molecule that activates the steroid receptor e.g., by binding to, or otherwise interacting with, the LBD of the steroid receptor.

The term "steroid-hormone receptor" as used herein refers to steroid-hormone receptors in the superfamily of steroid receptors. Representative examples of the steroid-hormone receptor family, include, but are not limited to, the estrogen, progesterone, glucocorticoid, mineralocorticoid, androgen, thyroid hormone, retinoic acid, retinoid X, Vitamin D, COUP-TF, ecdysone, Nurr-1 and orphan receptors. The receptors for hormones in the steroid/thyroid/retinoid supergene family, for example, are transcription factors that bind to target sequences in the regulatory regions of hormone-sensitive genes to enhance or suppress their transcription. These receptors have evolutionarily conserved similarities in a series of discrete structural domains, including a ligand binding domain (LBD), a DNA binding domain (DBD), a dimerization domain, and one or more transactivation domain(s).

Various mutations or changes in the amino acid sequences of the different structural domains may be generated to form a variant steroid receptor or more specifically a mutated steroid receptor. In one embodiment, the mutated steroid receptor is capable of preferentially binding to a non-natural or non-native ligand rather than binding to the wild-type or naturally-occurring hormone receptor ligand. In one embodiment, a mutated hormone receptor is generated by deletion of amino acids at the carboxy terminal end of a reference hormone receptor (e.g., a wild-type or naturally-occurring hormone receptor) e.g., a deletion of from about 1 to about 120 amino acids from the carboxy terminal end of the reference hormone receptor. In another embodiment, a mutated progesterone receptor of the present invention comprises a carboxy terminal deletion of from about 1 to about 60 amino acids of a reference progesterone receptor (e.g., a wild-type or naturally-occurring hormone receptor). In another embodiment, a mutated progesterone receptor comprises a carboxy terminal deletion of 19 amino acids of a reference progesterone receptor (e.g., a wild-type or naturally-occurring hormone receptor). In one embodiment, the RM comprises aprogesterone receptor LBD or portion thereof, having one or more site-directed mutations within the LBD or portion thereof that comprises amino acids 690-914 of the human progesterone receptor (hPR). In some embodiments, the mutated progesterone receptor LBD or portion thereof comprises one site-directed mutation at position 719, as in pGT1003, pGT1004 and pGT1005; or one site-directed mutation at position 755, as in pGT1006, pGT1007, pGT 1008 and pGT1009; or two site-directed mutations at positions 729 and 755, as in pGT1015, pGT1016, pGT1017 and pGT1025; or two site-directed mutation at positions 726 and 755, as in pGT1020, pGT1021, pGT1022, pGT1023 and pGT1024.

Further examples of modified or mutated steroid-hormone receptors for modification and/or use in the compositions and methods of the present invention are described in, for example: (1) "Adenoviral Vector-Mediated Delivery of Modified Steroid Hormone Receptors and Related Products and Methods" International Patent Publication No. WO 0031286 (PCTAJS99/26802); (2) "Modified Glucocorticoid Receptors, Glucocorticoid Receptor/Progesterone Receptor Hybrids" International Patent Publication No. WO 9818925 (PCTAJS97/19607); (3) "Modified Steroid Hormones for Gene Therapy and Methods for Their Use" International Patent Publication No. WO 9640911 (PCT/US96/0432); (4) "Mutated Steroid Hormone Receptors, Methods for Their Use and Molecular Switch for Gene Therapy" International Patent Publication No. WO 9323431 (PCTAJS93/0439); (5) "Progesterone Receptors Having C-Terminal Hormone Binding Domain Truncations", U.S. Patent No. 5,364,791; (6) "Modified Steroid Hormone Receptors, Methods for Their Use and Molecular Switch for Gene Therapy" U.S. Patent No. 5,874,534; and (7) "Modified Steroid Hormone Receptors, Methods for Their Use and Molecular Switch for Gene Therapy" U.S. Patent No. 5,935,934.

Furthermore, a mutated steroid-hormone receptor LBD may be selected based on the ability of an antagonist of a wild-type steroid-hormone receptor to activate the mutant receptor even in the presence of an agonist for the wild-type receptor. For example, in one embodiment, progesterone is the normal ligand for the progesterone receptor and functions as a strong agonist for the receptor. The anti-progestin, MFP (RU486), is a non-natural or non-native ligand for the progesterone receptor. MFP is considered an "anti-progestin" because, although it is able to exert an agonist effect on the wild-type progesterone receptor, MFP inhibits the agonistic effects of progesterone. Thus, MFP may be considered an "antagonist" for the wild-type progesterone receptor when in the presence of the normal agonist, i.e., when both MFP and progesterone are together in the presence of the wild-type progesterone receptor. However, in some embodiments of the present invention, the mutated progesterone steroid-hormone receptor is not activated by progesterone (agonist for the wild type receptor) but is activated in the presence of MFP ("antagonist" for the wild type receptor). In addition, in one embodiment, progesterone does not block the activation of the mutated steroid-hormone receptor by MFP. Thus, the mutated receptor may be characterized as activated when bound to an antagonist (e.g, MFP) for the wild-type receptor even in the presence of an agonist (e.g., progesterone) for the wild-type progesterone receptor.

The mutated steroid receptor activates the transcription of a desired TM in the presence of an antagonist for a wild-type steroid hormone receptor. In some embodiments, the antagonist is a non-naturally-occurring or non-wild-type ligand that acts as an antagonist of a wild-type steroid receptor (e.g., a wild-type steroid hormone receptor). In one embodiment, an antagonist of a wild-type steroid hormone receptor is a molecule that interacts with or binds to the wild-type steroid hormone receptor and blocks the activity of an agonist of the receptor. In another embodiment, an agonist of a wild-type steroid hormone receptor is a molecule that interacts with the wild-type steroid hormone receptor to regulate the expression and/or activity of a TM in the cells of a subject. Examples of such agonists include, but are not limited to, progesterone or progestin for the progesterone receptor 10, where progesterone binds to a wild-type progesterone receptor to activate the transcription of progesterone-regulated genes.

Suitable progesterone receptor agonists are chemical compounds that mimic progesterone. For example, Mifepristone (MFP), otherwise known as RU486, is a non-natural ligand that also binds to the wild-type progesterone receptor and competes with progesterone for binding. In preferred embodiments of this invention, the AM is a PR ligand analog that binds with low affinity or does not bind, interact with, or otherwise have an effect on the wild-type progesterone receptor or any other endogenous steroid receptor, and binds selectively and/or specifically to an RM having a modified LBD that is selective or specific for the AM. In a preferred embodiment, the PR ligand analog is e.g., BLX-913, BLX-833, BLX-899, BLX-593, BLX-599, BLX-952, BLX-610, BLX-117, or BLX-784. The progesterone receptor (PR) may be modified, e.g. in the LBD of the progesterone receptor, such that it only binds to MFP and not to progesterone. For example, mutation of the LBD of the progesterone receptor may be such that binding of the MFP activates the progesterone receptor. In one embodiment, the mutated PR LBD, or more generally any other mutated steroid receptor LBD, is fused with a particular DBD (e.g., the GAL-4 DBD), such that binding of MFP selectively activates the RM to transactivate TM expression and/or activity that is driven by a promoter recognized by the DBD of the PR. Thus, in some embodiments, the mutated steroid receptor of the present invention is not activated in the presence of agonists for the wild-type steroid receptor, but instead the mutated steroid receptor is activated in the presence of non-natural ligands.

The term "non-natural ligands" or "non-native ligands" refers to compounds which are non-wild-type or not naturally-occurring ligands that bind to the ligand binding domain of a receptor. Examples of non-natural ligands are Selective Progesterone Receptor Modulators (SPRMs) or mesoprogestins (see e.g., Chwalisz et al. (2002) Ann NY Acad Sci 955:373-388; Elger et al. (2000) Steroids 65(10-11):713-723; Chwalisz et al. (2004) Semin Reprod Med 22(2):113-119; DeManno et al. (2003) 68(10-13):1019-1032; Fuhrmann et al. (2000) J Med Chem 43(26):5010-5016). Examples of SPRMs or mesoprogestins are described and illustrated in Table 2 below (compounds 1-16).

**Table 2**

| 1. name: 17ß-Methoxy-17α-(methoxymethyl)-11ß-methoxyphenyl-4,9-estra-dien-3-one structure: | |
|---|---|
| 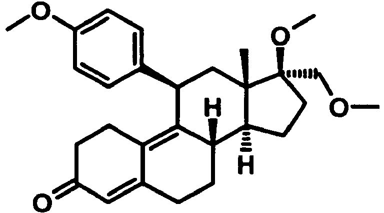 | |
| formula: C₂₈ H₃₆ O₄ | |
| molecular mass: 436,60 g/mol | |
| mp.: 184 - 186 ° C (dichloromethane) | |

| 2. name: 4-[17ß-Methoxy-17α-(methoxymethyl)-3-oxoestra-4,9-dien-11ß-yl]benzaldehyde-1 (E)-[O-(ethoxy)carbonyl]oxime | |
|---|---|
| structure: | |
| 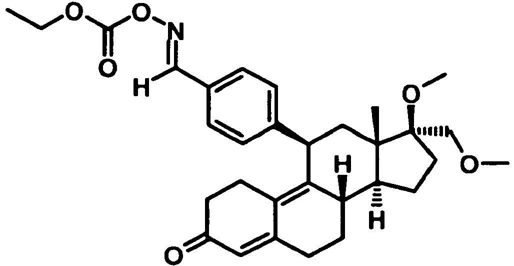 | |
| formula: C₃₁ H₃₉ N O₆ | |
| molecular mass: 521,66 g/mol | |
| mp.: 143-151 ° C (decomp. methanol) | |

| 3. name: 4-[17ß-Methoxy-17α-(methoxymethyl)-3-oxoestra-4,9-dien-11ß-yl]benzaldehyde-1 (E)-oxime acetate | |
|---|---|
| structure: | 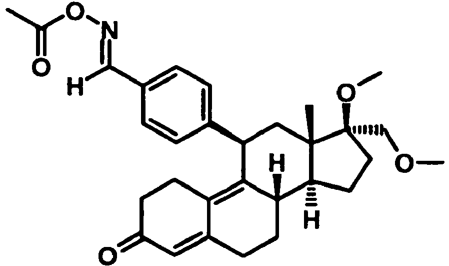 |
| formula: C₃₀ H₃₇ N O₅ | |
| molecular mass: 491,63 g/mol | |
| mp.: 110-119 ° C (ethyl acetate) | |
| | |

| 4. name: 4-[17α-(Ethoxymethyl)-17ß-methoxy-3-oxoestra-4,9-dien-11ß-yl] benzaldehyde-1 (E)-oxime | |
|---|---|
| structure: | 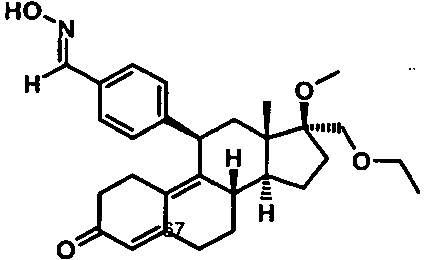 |
| formula: C₂₉ H₃₇ N O₄ | |
| molecular mass: 463,62 g/mol | |
| mp.: 90- 95 °C (methyl *tert.* butyl ether) | |
| purity HPLC: 98,9 area % (264 nm) | |
| | |

| 5. name: 4-[17ß-Methoxy-17α-(methoxymethyl)-3-oxoestra-4,9-dien-11ß-yl]benzaldehyde-thiosemicarbazone | |
|---|---|
| structure: | 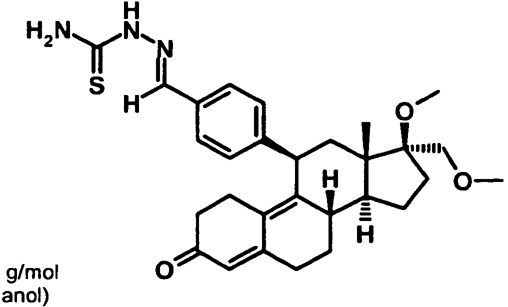 |
| formula: C₂₉ H₃₇ N₃ O₃ S | |
| molecular mass: 507,69 | |
| mp.: 217-236 ° C (methanol) | |
| | |

| 6. name: 4-[17ß-Hydroxy-17α-(methoxymethyl)-3-oxoestra-4,9-dien-11ß-yl]benzaldehyde-1 (E)-oxime acetate | |
|---|---|
| structure: | 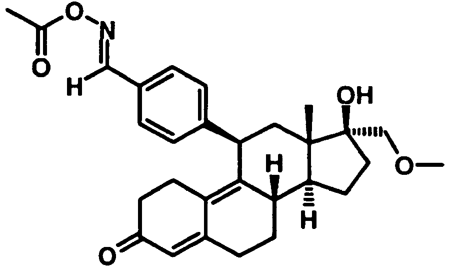 |
| formula: C₂₉H₃₅NO₅ | |
| molecular mass: 477,61 | |
| m.p: 112 °C (acetone, decomp.), α_{D} = +209 ° (CHCl₃) | |
| | |

| 7. name: 4-[17ß-Hydroxy-17α-(methoxymethyl)-3-oxoestra-4,9-dien-11ß-yl]benzaldehyde-1-(E)-[O-(ethylamino)carbonyl]oxime | |
|---|---|
| structure: | 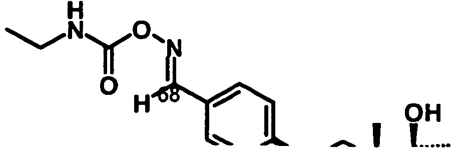 |
| formula: C₃₀ H₃₈ N₂ O₅ | |
| molecular mass: 506,65 g/mol | |
| mp.: 184-190 ° C (methylenechloride / ethyl acetate) | |
| | |

| 8. name: 4-[17ß-Methoxy-17α-(methoxymethyl)-3-oxoestra-4,9-dien-11ß-yl]benzaldehyde-1-(E)-[O-(methoxy)carbonyl]oxime (ZK280993) | |
|---|---|
| structure: | 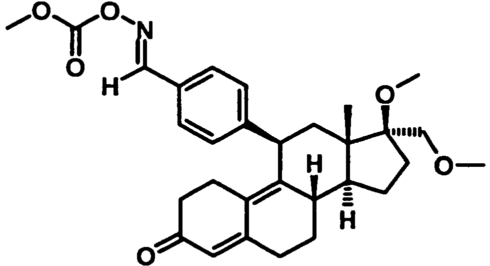 |
| formula: C₃₀ H₃₇ N O₆ | |
| molecular mass: 507,63 g/mol | |
| mp.: 110 ° C (mtbe, decomp.) | |
| | |

| 9. name: 4-(17ß-Hydroxy 17α-methyl-3-oxoestra-4,9-dien-11ß-yl] benzaldehyde-1(E)-oxime (ZK280999) | |
|---|---|
| structure: | 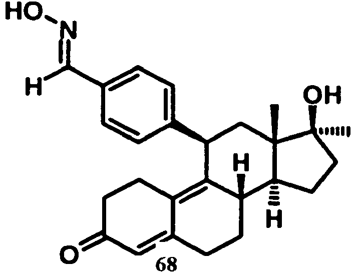 |
| formula: C₂₆ H₃₁ N O₃ | |
| molecular mass: 405,53 g/mol | |
| mp.: 163-165 °C (ethanol/water) | |
| | |

| 10. name: 4-[17ß-Methoxy-17α-(methoxymethyl)-3-oxoestra-4,9-dien-11ß-yl]benzaldehyde-1(E)-[O-(ethylthio)carbonyl]oxime (ZK190425) | |
|---|---|
| structure: | 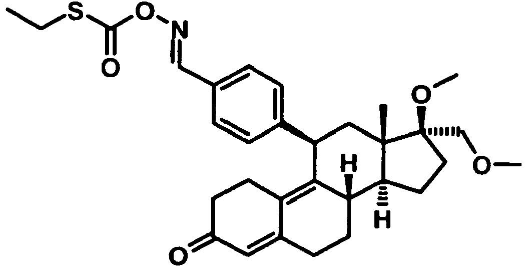 |
| formula: C₃₁ H₃₉ N O₅ S | |
| molecular mass: 537.72 g/mol | |
| mp.: 148-155°Cdecomp. (acetone/ n-hexane) | |
| | |

| 11. name: 4-[17ß-Hydroxy-17α-(2-propoxymethyl)-3-oxoestra-4,9-dien-11ß-yl]benzaldehyde-1(E)-oxime (ZK281100) | |
|---|---|
| structure: | 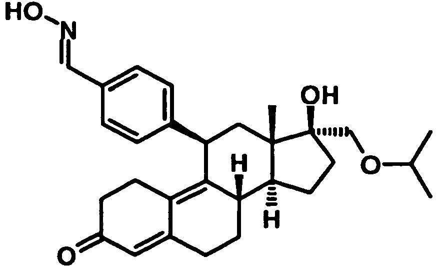 |
| formula: C₂₉ H₃₇ N O₄ | |
| molecular mass: 463,62 g/mol | |
| mp.: 192-196 ° C (diethylether, decomp.) | |
| | |

| 12. name: 4-(4'-Brom-17ß-methoxy17α-(methoxymethyl)-3-oxoestra-4,9-dien-11ß-yl]benzaldehyde-1(E)-oxime (ZK281117) | |
|---|---|
| structure: | |
| | 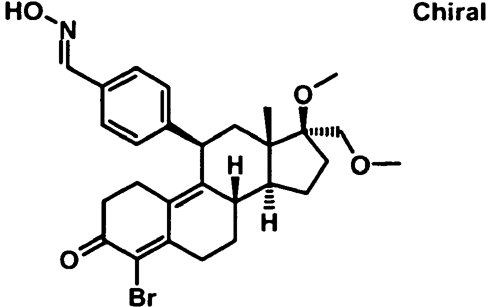 |
| formula: C₂₈ H₃₄ Br N O₄ | |
| molecular mass: 528,48 g/mol | |
| mp.: 138-141 °C (diethylether/n-hexane) | |
| | |

| 13. name: 4-[17ß-Methoxy-17α-(methoxymethyl)-3-oxoestra-4,9-dien-11ß-yl]acetophenon11ß-(4-Acetylphenyl)17ß-methoxy-17α-(methoxymethyl)-4,9-estradien-3-one (ZK139905) | |
|---|---|
| structure: | 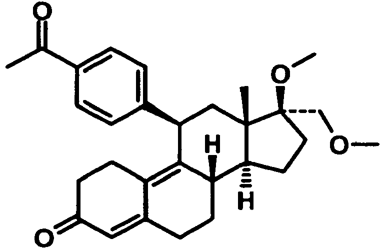 |
| formula: C₂₉ H₃₆ O₄ | |
| molecular mass: 448,61 g/mol | |
| mp.: 133-137 °C (diethylether/methylenechloride) | |
| | |

| 14. name: 11ß-[4-(Dimethylamino)phenyl]-17ß-methoxy-17α-(methoxymethyl)-4,9-estradien-3-one (ZK281317) | |
|---|---|
| structure: | 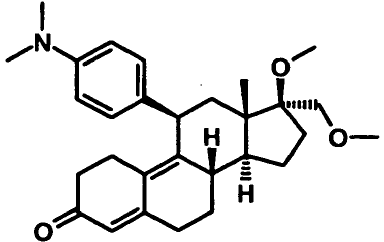 |
| formula: C₂₉H₃₉NO₃ | |
| molecular mass: 449,64 | |
| foam (hexane), α_{D} = +184 ° (CHCl₃) | |
| | |

| 15. name: 4-[17α-Ethinyl-17ß-methoxy-3-oxoestra-4,9-dien-11ß-yl] benzaldehyde-1 (E)-oxime (ZK281527) | |
|---|---|
| structure: | 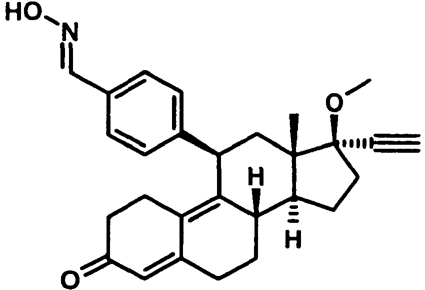 |
| formula: C₂₈ H₃₁ N O₃ | |
| molecular mass: 429,56 g/mol | |
| mp.: 149-157 ° C (acetone) | |
| | |

| 16. name: 4-[9α,10α-epoxy-17ß-hydroxy-17α-(methoxymethyl)-3-oxoestr- 4-en-11ß-yl] benzaldehyde-(E)-oxime (ZK234965) | |
|---|---|
| structure: | 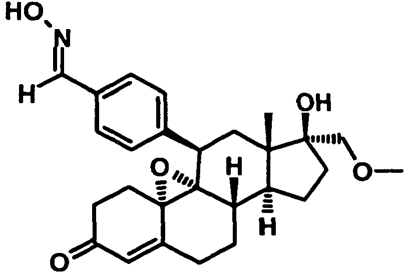 |
| formula: C₂₇H₃₃NO₅ | |
| molecular mass: 451,57 | |
| mp.: 110° C decomp. (acetone / methyl tert.-butyl ether) | |

Also, examples of non-natural ligands and non-native ligands are anti-hormones that may include the following: 11-(4-dimethylaminophenyl)-17-hydroxy-17c- propynyl-4, 9- estradiene-3-one (RU38486 or Mifepristone); 11-(4-dimethylaminophenyl)-17o-hydroxy-17-(3-hydroxypropyl)-13-methyl-4, 9-gonadiene- 3-one (ZK98299 or Onapristone); 11-(4-acetylphenyl)-17-hydroxy-17c-(1- propynyl)- 4,9-estradiene-3-one (ZK112993); 11-(4-dimethylaminophenyl)- 17-hydroxy-17(z-(3- hydroxy- 1 (Z)-propenyl-estra-4,9-diene-3-one (ZK98734); (7, 11, 17)-11-(4- dimethylaminophenyl)-7-methyl-4',5'- dihydrospiro [ester-4,9-diene-17,2' (3'H)-furan]-3- one (Org31806); (11, 14, 17c) -4', 5 '-dihydro- 11-(4-dimethylaminophenyl)- [spiroestra- 4, 9-diene-17,2'(3'H)-furan]-3-one (Org31376); 5-c-pregnane-3,2-dione, Org 33628 (Kloosterboer et al. (1995) Ann N Y Acad Sci Jun 12; 761:192-201), Org 33245 (Schoonen et al. (1998) J Steroid Biochem Mol Biol Feb; 64(3-4): 157- 70). A further example of such ligands are non-steroidal progesterone receptor-binding ligands e.g., that act as an inducer of an RM of the present invention.

Preferred non-natural ligands are PR ligand analogs as described herein, e.g., BLX-913 (illustrated in Figure 43), BLX-833, BLX-899, BLX-593, BLX-599 (illustrated in Figure 51), and BLX-952, BLX-610, BLX-117, or BLX-784 (illustrated in Figure 53). These and other new, functionally selective PR ligand analogs for the regulated expression system of the present invention may be identified by combining compound screening data with protein engineering to develop a unique AM-RM complex. The use of engineering specificity and selectivity into nuclear hormone receptor-ligand pairs involving regulated expression systems based on the Estrogen receptor (Kinzel, O. et al. (2006) J. Med. Chem. 49: 5404-5407; Whelan, J. et al. (1996) J. Steroid Biochem. Molec. Biol. 58: 3-12; Gallinari, P. et al. (2005) Chem. Biol. 12: 883-893; Shi, Y. et al. (2001) Chem. Biol. 8: 501-510; Chockalingam, K. et al. (2005) Proc. Natl. Acad. Sci. USA 102: 5691-5696; Tedesco, R. et al. (2001) Chem. Biol. 8: 277-287; Roscilli, G. et al. (2002) Mol. Ther. 6: 653-663), the Ecdysone receptor (Kumar, M.B. (2004) J. Biol. Chem. 279: 27211-27218), the Retinoid X receptor (Doyle, D.F. et al. (2001) J. Am. Chem. Soc. 123: 11367-11371; Schwimmer, L.J. et al. (2004) Proc. Natl. Acad. Sci. USA 101: 14707-14712) and the Thyroid Receptor (Hassan, A.Q. et al. (2006) J. Am. Chem. Soc. 128: 8868-8874) has been described.

Proprietary antiprogestin-like steroidal compounds, many sharing significant structural similarity to MFP, may be identified and tested using the pBRES-based luciferase screening assay developed by the present inventors. Structure-activity relationship (SAR) data derived from this directed screen may be used to identify compounds which do not activate the pBRES expression system, presumably due to steric or electronic conflicts between the compound and the amino acids lining the ligand binding pocket (LBP) within the RM LBD. Compounds thus identified are further screened in a T47D cell based secondary assay to confirm that the pBRES-inactive compounds also lack anti-PR activity. Starting with these inactive candidate compounds a functionally selective, i.e. orthogonal in respect to other physiological targets, AM-RM complex can be developed using a modeling-guided LBD engineering approach to re-establish pBRES activation.

Since no structural data for the interaction between MFP-like compounds and the hPR-derived LBD is available, homology models can be developed for the MFP-RM LBD complex based on the X-ray crystal structure of the GR complexed with MFP (Kauppi B. et al. (2003) J. Biol. Chem. 278: 22748-22754). The inactive compounds identified in the directed screen are docked into the model of the RM LBD and the conflicts between each compound and the LBP residues can be identified. A series of mutations aimed at alleviating the conflicts may be then modeled and installed into the RM LBP.

As described herein, the ability of candidate compounds to activate the engineered pBRES expression system can be measured in the pBRES-based luciferase assay. Those mutations that improve the interaction between previously inactive compounds and the altered RM LBD may be docked into the model and used to propose further mutations designed to better fit the ligand binding pocket to a new AM. Through iterations of the mutagenesis and testing in cell-based assays cycle, new mutated RM LBDs can be identified which are more active, in response to an AM of the present invention, than the original RM. For example, in some embodiments the new mutated RM is at least 10-50 fold, 50-100 fold, 100-200 fold, 200-300 fold, 300-400 fold, 400-500 fold, 500-600 fold, 600-700 fold, or 700-800 fold more active in response to an AM of the present invention, as compared to the original or reference RM that is not mutated. In one embodiment, the new mutant RM is 30-50 fold more active in response to an AM of the present invention. These studies demonstrate the clinical applicability of the pBRES expression system.

Using standard methods for amino acid or nucleic acid modifications (including, e.g., deletions, insertions, point mutations, fusions), a protein domain (e.g., AD, LBD, DBD) or functional nucleic acid sequence (e.g., sequence encoding a protein, RNA, promoter, splice site, intron, DNA binding site, or poly(A) site) of a molecule of the present invention (e.g., a TM, RM, AM, IM, or nucleic acid encoding a TM, RM, AM, or IM) can be modified or optimized for such regulation, expression, and/or activity. Further, a molecule of the present invention (e.g., a TM, RM, AM, IM, or nucleic acid encoding a TM, RM, AM, or IM) can be modified such that the expression and/or activity of the molecule is transient or constitutive, and/or is regulated by the presence of a particular condition, disease, biomarker or other molecule, or is self-regulated. More particularly, the primary, secondary, or tertiary structure of a nucleic acid or amino acid molecule, or chemical compound, of the present invention can be modified to achieve a particular stringency, specificity, or amount of binding, activation, inactivation, or conformation (e.g., to form a homo- or hetero-dimer or other multimer, or bind a specific or cognate ligand or site).

For example, a transcribed portion of an expression cassette of the present invention can be modified to include post-transcriptional elements (e.g., a UTR, splice site, intron, and/or poly(A) signal) that optimize or improve the specificity, level and fidelity of expression and/or activity of an operably linked, encoded, and expressed molecule (e.g., a TM, RM, AM, or IM). Further, the promoter sequence of an operably linked sequence encoding a molecule of the present invention can be modified such that the expression of the encoded molecule is, e.g., transient or constitutive, inducible or repressible, and/or modulated or otherwise regulated by the presence of a specific condition or molecule.

Various functional sequences of an expression cassette of the present invention can be modified to optimize for the expression and/or activity of an encoded molecule (e.g., TM, RM, AM, or IM). For example, intron sequences can be modified to optimize for the highly efficient and accurate splicing of RNA transcripts from such sequences. Thereby, cryptic splicing can be minimized and expression can be maximized of the desired molecule (e.g., TM, RM, AM, or IM) encoded by a nucleic acid of the present invention. Examples of suitable synthetic introns for use in the compositions of the present invention include, but are not limited to, consensus sequences for a 5' splice site, 3' splice site, and/or branch point. The 5' splice site is reported to pair with U1 snRNA. A suitable 5' splice site consensus sequence is one that is optimized to minimize the free energy of helix formation between U1 RNA and the synthetic 5' splice site e.g., 5' splice site sequence comprising 5'-CAGGUAAGU-3'. Further, the branch point (BP) sequence, except for a single bulged A residue, is reported to pair with U2 snRNA. Thus, a branch point sequence can be optimized to minimize the free energy of helix formation between U2 RNA and the sequence. The BP is typically located 18-38 nts upstream of the 3' splice site. In one embodiment, the BP sequence of the synthetic intron is located 24 nts upstream from the 3' splice site and is e.g., BP sequence comprising 5' UACUAC 3'. Further, the polypyrimidine tract of the consensus sequence for 3' splice sites can be optimized for 3' splice site function. For example, it has been reported that at least 5 consecutive uracil residues are optimal for 3' splice site function, and thus, in some embodiments the polypyrimidine tract of a synthetic intron of the present invention, has 7 consecutive uracil residues.

Similarly, the length of an intron can be optimized. For example, it is known that naturally-occurring introns may be 90-200 nt in length. In one embodiment, the length of the resultant internal exons are less than 300 nucleotides. In one embodiment, a synthetic intron is IVS8 (e.g., SEQ ID NO: 3) and comprises restriction enzyme sites, Bbsl and Earl (located within the synthetic intron), and Pstl and Nhel. The restriction enzyme Bbsl may be used to cleave the DNA precisely at the 5' splice site, and Earl may be used to cleave the DNA precisely at the 3' splice site. Further, a synthetic intron may be inserted at multiple locations of a nucleic acid sequence encoding a molecule of the present invention. For example, in some embodiments, a nucleic acid sequence encoding a molecule of the present invention is modified to comprise multiple introns.

In addition to the synthetic intron, IVS8 (e.g., SEQ ID NO: 3) an expression cassette of the present invention is modified to comprise a nucleic acid sequence encoding a CMV 5' UTR termed UT12, an expression control element (e.g., SEQ ID NO: 2). In another embodiment, an expression cassette of the present invention is modified to comprise a nucleic acid sequence encoding a SV40 poly(A) signal (e.g., SEQ ID NO: 8). In yet another embodiment, an expression cassette of the present invention is modified to comprise a nucleic acid sequence encoding a human growth hormone ("hGH") poly (A) signal (e.g., SEQ ID NO: 6). These and other modifications described herein may be employed to optimize the level and fidelity of expression of an encoded molecule (e.g., TM, RM, AM, IM) that is encoded by a nucleic acid sequence of the present invention (e.g., encoded by a nucleic acid sequence of an expression cassette, or vector), as described herein.

The term "intron" as used herein refers to a sequence encoded in a DNA sequence that is transcribed into an RNA molecule by RNA polymerase but is removed by splicing to form the mature messenger RNA. A "synthetic intron" refers to a sequence that is not initially replicated from a naturally-occurring intron sequence and generally will not have a naturally-occurring sequence, but will be removed from an RNA transcript during normal post-transcriptional processing. Such synthetic introns can be designed to have a variety of different characteristics, in particular such introns can be designed to have a desired strength of splice site and a desired length. In a preferred embodiment of the present invention, both the molecular switch expression cassette and the therapeutic gene expression cassette include a synthetic intron. The synthetic intron includes consensus sequences for the 5' splice site, 3' splice site, and branch point. When incorporated into eukaryotic vectors designed to express therapeutic genes, the synthetic intron will direct the splicing of RNA transcripts in a highly efficient and accurate manner, thereby minimizing cryptic splicing and maximizing production of the desired gene product.

Further, using known methods, a functional sequence encoding a domain of a protein can be modified to optimize for the activity of the protein. For example, using molecular modeling a truncation mutant can be designed such that there is lower dimerization potential while retaining sequence-specific DNA binding activity of a protein having a GAL-4 DBD. The GAL-4 DBD is reported to bind as a dimer to the palindromic 17-mer GAL-4 DBS (CGGAAGACTCTCCTCCG) and such dimer binding reportedly results in the activation of an inducible promoter having the GAL-4 DBS. Thus, the nucleic acid sequence encoding a protein having a GAL-4 DBD can be modified such that the tertiary structure of the GAL-4 DBD is optimized to reduce any unregulated (e.g., AM-independent) or undesired dimerization, resulting in activation of an inducible promoter.

The structure and function of the GAL-4 DBD sequences are known (e.g., see PCT/US01/30305). For example, the cysteine (C) may be involved in chelating zinc; the coiled-coil structures that form the dimerization elements comprise residues 54-74 and 86-93; the generally hydrophobic amino acids are reportedly at the first and fourth positions of each heptad repeat sequence; residue Ser 47 and Arg 51 are reported to form a hydrogen bond between the protein chains forming the dimer; and residues 8-40 reportedly form the Zn binding domain or the DNA recognition unit. This DNA recognition unit has two alpha helical domains that form a compact globular structure and in the presence of Zn resulting in a structure that reportedly is a binuclear metal ion cluster rather than a zinc finger, i.e., the cysteine-rich amino-acid sequence (CysXa-Xaa2-Cys14-Xaaa-CysZ1-Xaa6-CysZS-Xaa2-Cys31-Xaaa-Cys38) binds two Zn(II) ions (Pan and Coleman (1990) PNAS 87: 2077-81). The Zn cluster may be responsible for making contact with the major groove of the 3 bp at extreme ends of the 17-met binding site; and a proline at 26 (cis proline) reportedly forms the loop that joins the two alpha-helical domains of the zinc cluster domain and is also critical for this function. Further, residues 41-49 reportedly join the DNA recognition unit and the dimerization elements, residues 54-74 and 86-93.

Once dimerized, residues 47-51 of the dimer can also interact with phosphates of the DNA target. Residues 50-64 may be involved in weak dimerization. The dimers consist of a short coiled-coil that forms an amphipathic alpha-helix and wherein two alpha-helices are packed into a parallel coiled-coil similar to a leucine zipper. In addition to hydrophobic interactions of 3 pairs of leucines and a pair of valines found within residues 54-74, there are two pairs of Arg-Glu 20 salt links, and hydrogen bonds between Arg 51 of one monomer to Ser 47 of the other monomer. Residues 65-93 may form a strong dimerization domain. The structure of residues 65-71 is a continuation of the coiled-coil structure for one heptad repeat. Residues 72-78 contain a proline and therefore disrupt the amphipathic helix. Residues 79-99, however, contain three more potentially alpha-helical heptad sequences (Marmorstein et al (1992) Nature 356: 408-414). Further, the Kd for binding of GAL-4 residues 1-100 is reported to be 3 nM (Reece and Ptashne (1993) Science 261: 909-911).

In view of the structure and function of the GAL-4 DBD sequences a number of possible modifications can be made to the regions of the GAL-4 domain. In some embodiments, the GAL-4 regions are modified to optimize for the elimination or reduction of any basal expression and retention of sequence-specific DNA binding. More particularly, in some embodiments, the length of the region that contains the interacting coiled-coil sequences of the GAL-4 DBD (e.g., residues 54-74 and residues 86-93) can be shortened by deletion e.g., by deleting amino acid sequence 54-64, 65-74, 54-74, or 86-93. Also, GAL-4 mutants with only one coiled-coil region can be constructed by deleting one of the coiled-coil regions. In addition, mutant or artificial sequences may be inserted into the GAL-4 domain using unique restriction sites positioned at, e.g., the junctions of each of the alpha-helical heptad sequences. Thus, modified versions of the GAL-4 domain can be produced that have progressively reduced alpha-helical heptad sequences.

The native GAL-4 sequence is modified to remove the N-terminal methionine and additional amino acids are added to the N-terminal end of the sequence. In these embodiments, the modifications to the N-terminal amino acids of the native GAL-4 sequence are not of consequence as long as they do not affect the tertiary structure of residues 8-40 of the Zn binding domain. Further, in some embodiments, the specific binding of a small molecule AM (e.g., a PR ligand analog, particularly a modified antiprogestin such as a modified MFP, or a modified mesoprogestin such as a modified asoprisnil), to a mutated hPR LBD of a protein having a GAL-4 DBD (e.g., an RM) triggers a conformational change in the protein so as to initiate dimerization of the protein. Additionally, in some embodiments, an expression cassette of the present invention comprises a nucleic acid sequence encoding residues 2-93 of the GAL-4 DBD sequence of SEQ ID NO: 37. Further, in one embodiment, the DNA recognition sequence of the GAL-4 DBD comprises residues 9-40 of the GAL-4 DBD sequence of SEQ ID NO: 37.

In one embodiment of the present invention, the GAL-4 domain is truncated by deletion of 19 amino acids at the C- terminal portion of the GAL-4 DBD and comprises residues 75-93 of the GAL-4 DBD sequence of SEQ ID NO: 37. An RM of the present invention is a chimeric protein comprising a mutated progesterone receptor comprising residues 2-74 of the GAL-4 DBD sequence of SEQ ID NO: 37 and a mutated progesterone receptor LBD that is specifically activated in the presence of an AM. Further, in the absence of the AM there is little or no RM activation and resulting induction or activation of transcription of a nucleic acid sequence operably linked to a promoter having a GAL-4 DBS.

As mentioned, nucleic acids encoding variants of a native molecule (e.g., protein or nucleic acid) are also suitable for use in the compositions and methods of the present invention. For example, a variant of IFN-β (e.g., IFN-β 1 b) is suitable for use as a TM in the compositions and methods of the present invention, particularly, for the treatment of MS. Preferably, the IFN-β variant is a variant of a native human IFN-β. Variants of native human IFN-β, which may be naturally-occurring (*e.g*., allelic variants that occur at the IFN-β locus) or recombinantly or synthetically produced, have amino acid sequences that are similar to, or substantially similar to a mature native IFN-β sequence. Nucleic acids encoding a native human IFN-β (e.g., comprising the amino acid sequence of SEQ ID NO: 13) are suitable for use in the compositions and methods of the present invention e.g., IFN-β 1 a (e.g., SEQ ID NO: 14). Also, nucleic acids encoding a human IFN-β variant are suitable for use in the compositions and methods of the present invention e.g., IFN-β 1b (*see e.g*., US Patent Ser. Nos. 4,588,585, 4,737,462, and 4,959,314). Variants also encompass nucleic acids encoding fragments or truncated forms of a native molecule (e.g., protein or nucleic acid) that retain a biological or therapeutic activity. For example, nucleic acids encoding these biologically active fragments or truncated forms of a native protein. Further, in some embodiments, the expressed protein of the present invention may be glycosylated or not glycosylated.

Further, suitable protein or nucleic acid variants for use in the compositions and methods of the present invention can be variants of a native or wild-type protein or nucleic acid, respectively, of any mammalian species including, but not limited to, avian, canine, bovine, porcine, equine, and human. Non-limiting examples of IFN-β variants encompassed by the present invention (e.g., encoded by a nucleic acid , *e.g*., Nagata et al. (1980) Nature 284:316-320; Goeddel et al. (1980) Nature 287:411-416; Yelverton et al. (1981) Nucleic Acids Res. 9:731-741; Streuli et al. (1981) Proc. Natl. Acad. Sci. U.S.A. 78:2848-2852; EP028033B1, and EP109748B1. *See* also, *e.g*., U.S. Pat. Nos. 4,518,584; 4,569,908; 4,588,585; 4,738,844; 4,753,795; 4,769,233; 4,793,995; 4,914,033; 4,959,314; 5,545,723; and 5,814,485. These citations also provide guidance regarding residues and regions of the IFN-β protein that can be altered without the loss of biological activity.

Changes or modifications of expressed proteins and nucleic acids (e.g., RNA) of the present invention can be introduced by mutation into the nucleotide sequences encoding them, thereby leading to changes in the amino acid sequence of the expressed protein or nucleic acid sequence without altering the biological or therapeutic activity of the expressed molecule. For example, an isolated nucleic acid molecule encoding a variant protein having a sequence that differs from the amino acid sequence for a reference or starting protein can be created by introducing one or more nucleotide substitutions, additions, or deletions into the corresponding nucleotide sequence (for IFN-β variants, see, *e.g*., U.S. Pat. No. 5,588,585, U.S. Pat. No. 4,959,314; 4,737,462; L. Lin (1998) Dev. Biol. Stand. 96: 97-104), such that one or more amino acid substitutions, additions or deletions are introduced into the sequence encoding a reference or starting protein and thereby resulting in a variant protein when the encoding protein is expressed. For example, mutations can be introduced by standard techniques for modifying nucleic acid or amino acid sequences, such as site-directed mutagenesis and PCR-mediated mutagenesis.

Further, nucleic acid sequences encoding a protein can be modified to encode conservative amino acid substitutions at one or more predicted, preferably nonessential amino acid residues. As used herein, a "nonessential" amino acid residue is a residue that can be altered from a reference sequence of a protein without altering its biological or therapeutic activity, whereas an "essential" amino acid residue is required for such activity. As used herein, a "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine), and aromatic side chains (*e.g*., tyrosine, phenylalanine, tryptophan, histidine). In preferred embodiments, such substitutions are not made for conserved amino acid residues, or for amino acid residues residing within a conserved motif.

Further, the nucleotide sequences of a variant molecule can be made by introducing mutations randomly along all or part of the coding sequence of a reference molecule, such as by saturation mutagenesis, and the resultant mutants can be screened for biological or therapeutic activity. Following mutagenesis, the encoded protein can be expressed recombinantly, and the activity of the protein can be determined using standard assay techniques described herein or known in the art. In preferred embodiments, biologically or therapeutically active protein variants have at least 80 %, more preferably about 90 % to about 95 % or more, and most preferably about 96 % to about 99 % or more amino acid sequence identity to the amino acid sequence of a reference protein, which serves as the basis for comparison or reference. As used herein "sequence identity" is the same amino acid residues that are found within a variant protein and a protein molecule that serves as a reference when a specified, contiguous segment of the amino acid sequence of the variant is aligned and compared to the amino acid sequence of the reference molecule.

For the optimal alignment of two sequences for the purposes of sequence identity determination, the contiguous segment of the amino acid sequence of the variant may have additional amino acid residues or deleted amino acid residues with respect to the amino acid sequence of the reference molecule. The contiguous segment used for comparison to the reference amino acid sequence will comprise at least 20 contiguous amino acid residues. Corrections for increased sequence identity associated with inclusion of gaps in the amino acid sequence of the variant can be made by assigning gap penalties. Methods of sequence alignment are well known in the art.

For example, the determination of percent identity between any two sequences can be accomplished using a mathematical algorithm. One preferred, non-limiting example of a mathematical algorithm utilized for the comparison of sequences is *e.g.*, the algorithm of Myers and Miller (1988) Comput. Appl. Biosci. 4:11-7. Such an algorithm is utilized in the ALIGN program (version 2.0), which is part of the GCG alignment software package. A PAM120 weight residue table, a gap length penalty of 12, and a gap penalty of 4 can be used with the ALIGN program when comparing amino acid sequences. Another preferred, non-limiting example of a mathematical algorithm for use in comparing two sequences is the algorithm of Karlin and Altschul (1990) Proc. Natl. Acad. Sci. USA 90:5873-5877, modified as in Karlin and Altschul (1993) Proc. Natl. Acad. Sci USA 90:5873-5877. Such an algorithm is incorporated into the NBLAST and XBLAST programs of Altschul et al. (1990) J. Mol. Biol. 215:403-410. BLAST amino acid sequence searches can be performed with the XBLAST program, score=50, word length=3, to obtain amino acid sequence similar to the protein of interest.

To obtain gapped alignments for comparison purposes, gapped BLAST can be utilized as described in Altschul et al. (1997) Nucleic Acids Res. 25:3389-3402. Alternatively, PSI-BLAST can be used to perform an integrated search that detects distant relationships between molecules (see *e.g.*, Altschul *et al.* (1997) supra.). When utilizing BLAST, gapped BLAST, or PSI-BLAST programs, the default parameters can be used (*see e.g*., www.ncbi.nlm.nih.gov). Also see the ALIGN program (Dayhoff (1978) in Atlas of Protein Sequence and Structure 5:Suppl. 3, National Biomedical Research Foundation, Washington, D.C.) and programs in the Wisconsin Sequence Analysis Package, Version 8 (available from Genetics Computer Group, Madison, Wis.), for example, the GAP program, where default parameters of the programs are utilized.

When considering percentage of amino acid sequence identity, some amino acid residue positions may differ as a result of conservative amino acid substitutions, which do not affect properties of protein function. In these instances, percent sequence identity may be adjusted upwards to account for the similarity in conservatively substituted amino acids. Such adjustments are well known in the art (*see*, *e.g.,* Myers and Miller (1988) Comput. Appl. Biosci. 4:11-17).

Further, in embodiments where the RM, AM, or IM is a protein, the protein can be covalently linked with, *e.g*., polyethylene glycol (PEG) or albumin. These covalent hybrid molecules can have certain desirable properties such as an extended serum half-life after administration to a subject. Methods for creating PEG-IFN adducts involve chemical modification of monomethoxypolyethylene glycol to create an activated compound that will react with a protein of the present invention. Methods for making and using PEG-linked proteins are reported, *e.g.*, in Delgado et al. (1992) Crit. Rev. Ther. Drug. Carrier Syst. 9:249-304 (and as described herein in the Background). Methods for creating albumin fusion proteins involve fusion of the coding sequences for the protein of interest and albumin and are reported, *e.g*., in U.S. Pat. No. 5,876,969.

Biologically or therapeutically active protein or nucleic acid variants encompassed by the invention preferably retain or have a biological or therapeutic activity. In some embodiments, the variant retains at least about 25 %, about 50 %, about 75 %, about 85 %, about 90 %, about 95 %, about 98 %, about 99 % or more of the biologically or therapeutic activity of the reference molecule (e.g., protein or nucleic acid). Variants whose activity is increased in comparison with the activity of the reference molecule (e.g., protein or nucleic acid) are also encompassed. The biological or therapeutic activity of variants can be measured by any method known in the art (*see e.g.,* assays described in Fellous et al. (1982) Proc. Natl. Acad. Sci USA 79:3082-3086; Czerniecki et al. (1984) J. Virol. 49(2):490-496; Mark et al. (1984) Proc. Natl Acad. Sci. USA 81:5662-5666; Branca et al. (1981) Nature 277:221-223; Williams et al. (1979) Nature 282:582-586; Herberman et al. (1979) Nature 277:221-223; Anderson et al. (1982) J. Biol. Chem. 257(19):11301-11304).

Generally, for cloning, testing, and other uses described herein, the nucleic acid, protein and chemical compositions of the present invention can be produced or synthesized using methods known in the art. For example, proteins can be produced by culturing a host cell transformed with an expression vector comprising a nucleotide sequence that encodes a protein or nucleic acid of the present invention. The host cell is one that can transcribe the nucleotide sequence and produce the desired protein or nucleic acid, and can be prokaryotic (see, *e.g*., *E. coli)* or eukaryotic (*e.g*., a yeast, insect, or mammalian cell). Examples of recombinant production of IFN-β, including suitable expression vectors, are provided in, *e.g*., Mantei et al. (1982) Nature 297:128; Ohno et al. (1982) Nucleic Acids Res. 10:967; Smith et al. (1983) Mol. Cell. Biol. 3:2156, and U.S. Pat. No. 4,462,940, 5,702,699, and 5,814,485; U.S. Pat. No. 5,795,779).

Further, genes have been cloned using recombinant DNA ("rDNA") technology and can be produced and tested in e.g., animal or plant cells, or transgenic animals (*see e.g.,* Nagola et al. (1980) Nature 284:316; Goeddel et al. (1980) Nature 287:411; Yelverton et al. (1981) Nuc. Acid Res. 9:731; Streuli et al. (1981) Proc. Natl. Acad. Sci. U.S.A. 78:2848). Proteins may also be produced with rDNA technology, e.g., by extracting poly-A-rich 12S messenger RNA from virally induced human cells, synthesizing double-stranded cDNA using the mRNA as a template, introducing the cDNA into an appropriate cloning vector, transforming suitable microorganisms with the vector, harvesting the microorganisms, and extracting the protein therefrom (*see*, *e.g.,* European Patent Application Nos. 28033 (published May 6, 1981); 32134 (published Jul. 15, 1981); and 34307 (published Aug. 26, 1981)),

Also, proteins can be synthesized chemically and tested, by any of several techniques that are known to those skilled in the peptide art (*see e.g.,* Li et al. (1983) Proc. Natl. Acad. Sci. USA 80:2216-2220, Steward and Young (1984) Solid Phase Peptide Synthesis (Pierce Chemical Company, Rockford, III.), and Baraney and Merrifield (1980) The Peptides: Analysis, Synthesis, Biology, ed. Gross and Meinhofer, Vol. 2 (Academic Press, N. Y., 1980), pp. 3-254, *Discussing Solid-Phase Peptide Synthesis Techniques;* and Bodansky (1984) Principles of Peptide Synthesis (Springer-Verlag, Berlin) and Gross and Meinhofer, eds. (1980), The Peptides. Analysis, Synthesis, Biology, Vol. 1 (Academic Press, New York, discussing classical solution synthesis). A protein of the present invention can also be chemically prepared e.g., by the method of simultaneous multiple peptide synthesis. See, for example, Houghten (1984) Proc. Natl. Acad. Sci. USA 82:5131-5135; and U.S. Pat. No. 4,631,211.

Further, one skilled in the art would know how to test the compositions of the present invention for the treatment of disease using accepted and appropriate animal models and methods known in the art. For example, it has been reported that gene delivery systems can be used to deliver cytokines in several animal autoimmune disease models, e.g., including experimental allergic encephalomyelitis (EAE), arthritis, lupus, and NOD diabetes models (*see* e.g., G.C. Tsokos and G.T. Nepom (2000) Clin. Invest. 106: 181-83; G.J. Prud'homme (2000) J. Gene Med. 2: 222-32). EAE is a model of central nervous system inflammation that ensues after immunization with certain CNS auto-antigens, for example brain derived proteolipid or myelin basic protein. Its course and clinical manifestations are similar to multiple sclerosis (MS) in humans and it has become an accepted model to study MS. There have been several reports that describe the testing of Type I IFN's, delivered as a protein (15-20) and by a vector (*see* e.g., K. Triantaphyllopoulos et al. (1998) Gene Therapy 5: 253-63; J.L. Croxford et al. (1998) J. Immunol. 160: 5181-87), in murine and rat EAE models. Yu *et al*. have shown that mice administered mIFN-β protein, at the time of EAE disease induction, exhibit delayed progression to disability (as measured by clinical score), delayed onset of relapse, and a decrease in exacerbation frequency compared to normal mice (see e.g., M. Yu et al. (1996) J. Immunol. 64: 91-100). This result closely resembles the human results with IFN-β treatment. Plasmid based vectors were used by Triantaphyllopoulos et al. in a gene therapy-based approach to deliver IFN-β to the CNS under the control of a neuron specific promoter (see e.g., K. Triantaphyllopoulos et al. (1998) Gene Therapy 5: 253-63). These vectors were injected intra-cranially into mice during the effecter phase of EAE, and reduced or prevented the clinical signs of disease. The results of the studies performed to date using IFN-β in the murine EAE model demonstrate that IFN is an effective therapy in delaying the onset, and reversing the manifestations of disease in the EAE model. Gene therapy methods for delivering IFN-β in this model (local intra-cranial administration) have been shown to be effective.

### EXAMPLES

The following examples are offered by way of illustration and are not intended to limit the invention in any way.

As described in the following Examples, human IFN-β (hIFN-β) and murine IFN-β (mIFN-β) expression vectors were constructed, assays developed to measure IFN-β directly in serum, and biomarkers were identified that correlate with IFN-β expression *in vivo.* Further, pharmacokinetic studies were performed in normal mice comparing gene-based delivery of IFN-β with bolus protein delivery and a superior pharmacokinetic profile was demonstrated using intramuscular injection of non-viral plasmid DNA or an adeno-associated viral (AAV) vector encoding IFN-β. In addition, long term, stable expression of hIFN-β was observed for nearly 6 months with an AAV-1 vector expressing hIFN-β. Also, single intramuscular injection of plasmid DNA encoding mIFN-β was shown to be efficacious in a murine model of experimental allergic encephalomyelitis (EAE), and equally as effective as an every-other-day injection of mIFN-β protein. As described below, examples of the regulated expression system of the present invention were constructed and tested. For example, regulated expression of IFN-β was demonstrated in normal mice using a regulated expression system of the present invention, where a TM and RM are contained in a single plasmid vector.

The data from the studies described in these Examples demonstrate the potential of the regulated, expression system of the present invention for delivery of a nucleic-acid encoded TM, for treatment of disease. In particular, these Examples demonstrate the potential for the use of the regulated expression system of the present invention for delivery of a nucleic acid encoding IFN-β (e.g., IFN-β 1a) for long term, regulated expression of the protein for the treatment of MS. In one embodiment, the delivery vector is a single plasmid vector comprising a first and second expression cassette encoding a TM (e.g., IFN-β) and RM, respectively which provides persistent (e.g., greater than 3 months) and renewable expression through oral administration of an AM e.g., a small molecule inducer (e.g., MFP) and, further, the vector is capable of repeat administration by intramuscular injection.

### EXAMPLE 1: Construction of Vectors for Use in IFN-β or GMCSF Gene Therapy

**A. Plasmid Vectors:** The murine IFN-β (mIFN-β) gene from the bacterial expression vector pbSER189 was PCR amplified, with immunoglobulin kappa (IgK) (for protein purification) or mIFN-β (for gene therapy) signal sequence added on the 5' primer. The PCR products were inserted downstream of the cytomegalovirus (CMV) promoter in the expression vectors pCEP4/WPRE, to generate pGER90 (Figure 2A) for recombinant protein expression and purification, and pgWiz, to generate pGER101 (Figure 2B) for gene therapy.

The human IFN-β gene from the bacterial expression vector pbSER178 was PCR amplified by the same procedure as the mIFN-β gene (except with the hIFN signal sequence for the gene therapy vector) and inserted into pCEP4/WPRE to generate pGER123 (Figure 2C) for recombinant protein expression and purification, and pgWiz to generate pGER125 (Figure 2D) for gene therapy.

The construction of plasmid vectors is fully described in the Methods and Materials section, subsection F.

**B. AAV-1 Vectors:** An AAV-1-hIFN-β shuttle plasmid encoding hIFN-β was constructed by inserting the blunted HinclI/NotI fragment of pGWIZ/hIFN-β encoding hIFN-β into the blunted AgeI-SalI site of the AAV-1 vector, pTReGFP. The resulting shuttle plasmid was named pGT62 (SEQ ID NO: 44) and used to produce AAV-1 virus encoding hIFN-β. Two batches of the AAV-1 virus were prepared as described herein using standard methods and used in pharmacokinetic studies (Figure 4). The expression levels of hIFN-β in these two viral batches were validated by ELISA.

Also, AAV-1-GM-CSF shuttle plasmids pGT714 and pGT713 (Figure 30B), encoding mGMCSF or hGMCSF, were constructed by inserting a fragment encoding mGMCSF or hGMCSF into the vector pGENE/V5HisA (Invitrogen). The resulting vectors were named pGT723-GENE/hGMCSF and pGT724-GENE/mGMCSF. A fragment encoding mGM-CSF was then excised from pGT724-GENE/mGMCSF by digesting the vector with KpnI-XbaI. Similarly, a fragment encoding hGM-CSF was excised from pGT723-GENE/hGMCSF by digesting the vector with KpnI-XbaI. The vector pZac2.1 was digested with KpnI-XbaI and treated with calf intestinal phosphatase (CIP) and then the excised fragment encoding either mGMCSF or hGMCSF was inserted into pZac2.1 at the KpnI-XbaI site. The resulting shuttle plasmids were named pGT713 (pZac2.1-CMV-hGMCSF) and pGT714 (pZac2.1-CMV-mGM-CSF) (Figure 30B).

The construction of the vectors of the present invention is fully described in the Methods and Materials section, subsection F.

### EXAMPLE 2: Pharmacokinetic Studies of IFN-β Gene Delivery

**A. Pharmacokinetic Studies with Human IFN-β:** Pharmacokinetic studies were performed in normal mice to compare bolus protein versus gene-based delivery of human IFN-β (hIFN-β).

**1) Human IFN-β1a Protein Phamacokinetic Study:** A pharmacokinetic study was carried out in C57/BI6 mice using bolus injection of recombinant hIFN-β1a delivered either by intramuscular (i.m.) or intravenous (i.v.) injection and using a commercially available ELISA to detect serum levels of hIFN-β. Figure 3 shows the pharmacokinetic profile of hIFN-β1a protein in serum of mice following a single i.m. or i.v. injection of either 25 ng (1 ug/kg) or 250 ng (10 ug/kg) of hIFN-β1a protein. Following i.v. injection, hIFN-β1a was detected in serum in a dose dependent manner at the first time point (30 min), and was rapidly cleared such that the levels were near the limit of detection (LOD) of the assay (LOD = 12.5 pg/ml) by 6 hours. Following i.m. injection of recombinant hIFN-β1a protein, the hIFN-β1a serum level reached a maximum level at 2 hours post-injection and then decreased by approximately 10-fold by 6 hours. The amount of hIFN-β1a remaining in the serum at 6 hours was higher with the i.m. injection compared to the i.v. injection. With both the i.v. and i.m. injections, a 10-fold difference in serum hIFN-β1a level was seen between the high and low dose. The highly transient kinetics displayed following bolus injection of recombinant hIFN-β1a is very similar to the results previously reported in humans and in other animal species (Buchwalder, P-A et al. (2000) J Interferon Cytokine Res 20: 57-66; Pepinsky, RB et al. (2001) J Pharm Exp Ther 297: 1059-55).

**2) Pharmacokinetic Study of Gene-Based Delivery of AAV-1-CMV hIFN-β1a:** An AAV-1 vector was constructed to constitutively express human IFN-β (AAV-1-CMV hIFN-β1a) and delivered by i.m injection at three doses (0.5x10¹⁰, 1.0x10¹⁰, or 5.0 x10¹⁰ viral particles) into C57BI/6 mice. The results are shown in Figure 4. Human IFN-β1a expression in the serum of mice was low on day 2 but increased rapidly up to day 10 at which time serum levels in all three dose-groups reached a plateau or gradually increased. A clear dose response was observed with increasing amounts of AAV-1-CMV hIFN-β1a vector administered. At the two higher doses steady levels of hIFN-β1a expression was detected in the serum 171 days post-injection. In contrast to the study using bolus i.m. injection of recombinant hIFN-β1a protein, this study using gene-based delivery of an AAV-1 vector encoding hIFN-β1a, demonstrates long-term expression of hIFN-β1a in serum after a single injection of the vector.

### EXAMPLE 3: Identification and Use of IFN-β Biomarkers for Gene Therapy

**A. Development of mIFN-β Biomarkers:** For higher sensitivity in detection of murine IFN-β (mIFN-β) activity in vivo, biomarkers for mIFN-β activity were identified in mice after injection of mIFN-β protein or mIFN-β encoded gene therapy vectors. Biomarkers can be used to follow human IFN-β activity in clinical samples from patients treated with Betaseron (IFN-β 1b) (see e.g., Arnason, BG (1996) Clin Immunol Immunopathol 81: 1-11; Deisenhammer, F et al. (2000) Neurology 54: 2055-60; Knobler, RL et al. (1993) J Interferon Res 13: 333-40.; Kracke, A et al. (2000) Neurology 541: 193-9). One of the primary biomarkers used in the IFN-β clinical studies is MxA (see e.g., Kracke, A et al. (2000) Neurology 541: 193-9; Bertoloto, A et al. (2001) J Imm Meth 256: 141-152) since it is specifically induced by type I IFN's (see e.g., von Wussow, P et al (1990) J Imm 20:2015-19). In the present study, the expression of the MxA mouse homologue, Mx1, (see e.g., Hug, H et al. Mol Cell Biol 8: 3065-79; Pavlovic, J (1993) Ciba Found Symp 176: 233-43) isolated from murine peripheral blood monocytes (PBMC's) was used to detect the presence of biologically active mIFN-β.

A quantitative PCR/RT-PCR assay was developed to quantitate the levels of Mx1 mRNA in murine PBMC's. Specifically, peripheral blood mononuclear cells (PBMCs) were isolated from mice treated with either mIFN-β protein or following gene-based delivery of the mIFN-β gene. Blood samples obtained from treated mice were centrifuged on a ficoll cushion for 25 minutes at 2,000 rpm. Purified PBMCs were pelleted and RNA for the Mx1 assay was purified using the "RNAeasy" mini extraction kit from Qiagen. The RNA was stored in H₂O at -80 °C. Mx1 RT-PCR was performed using TaqMan^{®} chemistry and analysis was done on the Applied Biosystems (ABI) PRISM^{®} 7700 Sequence Detection instrument. For reverse transcription of the RNA and amplification of cDNA, the "One-step TaqMan^{®} RNA" kit from ABI was used. RNA was reverse transcribed for 30 minutes at 48 °C and the amplification was done in 40 cycles with a denaturation step at 95 °C for 30 seconds, and an annealing/elongation step at 60 °C for 1 minute. The samples were analyzed with an Mx1-specific probe/primer combination. Mx1 expression was normalized to GAPDH expression measured in parallel using a standard assay from ABI.

The assay was validated *in vitro* by examining Mx1 RNA induction in murine L929 cells treated with purified recombinant mIFN-β protein (Figure 5). A dose dependent increase in the expression of Mx1 RNA was observed with an EC50 of approximately 50 pg/ml mIFN-β resulting in a 100-fold increase in the level of Mx1 RNA.

**B. Bolus Injection of mIFN-β Protein Induces Transient Biomarker Response *In Vivo***: The biomarker response after bolus injection of purified recombinant mIFN-β was measured in these studies. Induction of Mx1 RNA expression (25- to 50-fold relative to vehicle injected mice) was observed with each mIFN-β concentration tested whether administered i.v. or i.m. (Figure 6). The highest Mx1 expression levels were measured 2 hours after injection. At that time point a clear dose response was observed when mIFN-β was delivered i.m. Mx1 expression dropped rapidly and beyond 12 hours after injection no Mx1 RNA levels above background were detected. When mIFN-β was injected i.v., the highest induction was observed with 150 ng. At the 500 ng dose there was a diminished level of Mx1 RNA induction. This saturating effect has been observed in other studies in which high IFN-β levels lead to an apparent down-regulation of the bioresponse, perhaps due to down regulation of the IFN type I receptor (see e.g., Mager, DE and Jusko, WJ (2002) Pharm Res 19:1537-43). Mx1 RNA expression in the i.v. injected mice also peaked at approximately 2 hours post-injection and dropped rapidly thereafter.

This is the first time that the expression of Mx1 RNA has been used as a biomarker to follow the activity of mIFN-β in mice. The results clearly show that Mx1 RNA is expressed constitutively in mouse PBMC's at a low level and can be strongly upregulated by mIFN-β treatment. However, the upregulation is short term and rapidly drops from high expression levels to background within 12-24 hours. The rapid kinetics correspond with the short half-life time reported for type I interferons in humans (see e.g., Salmon, P et al. (1996) J Interferon Cytokine Res 16: 759-64; Buchwalder, P-A et al. (2000) J Interferon Cytokine Res 20: 57-66) and other animals species (Pepinsky, RB et al. (2001) J Pharm Exp Ther 297: 1059-55; Mager, DE et al. (2003) J Pharm Exp Ther 306: 262-70).

**C. Chemokines IP-10 and JE**: During the analysis of plasma samples from mice treated with mIFN-β protein, two murine chemokines, IP-10 and JE the murine homologue of MCP-1 (monocyte chemoattractant protein, see e.g., Yoshimura, T (1989) FEBS Lett 244: 487-93), were also identified to have a similar response and activation to mIFN-β as Mx1 RNA (Figures 7 and 8). A strong induction of IP-10 and JE was seen 2 hours after administration of mIFN-β protein either i.v. or i.m. IP-10 levels increased 3000-fold at the high dose delivered i.v. With each of the three mIFN-β doses tested, a rapid drop to background in the plasma levels of IP-10 and JE was observed by 24 hours. A clear dose response for IP-10 and JE was observed with both routes of administration.

Such a strong dose dependent induction of IP-10 and JE by IFN-β has not previously been known until demonstrated by the present inventors, as described herein. Although IP-10 is known as a biological marker for IFN-γ by virtue of the interferon responsive element (ISRE) in the promoter region (see e.g., Luster, AD et al. (1985) Nature 315: 672-76), it is has not previously been shown to be a specific biomarker for mIFN-β in mice.

**D. Long-term Biomarker Response Following mIFN-β Gene Delivery *In Vivo***: These studies demonstrate the measurement of the induction of mIFN-β biomarkers in mice following intramuscular delivery of plasmid DNA or an AAV-1 vector encoding mIFN-β.

### EXAMPLE 4: Delivery of mIFN-β Gene

**A. Plasmid Delivery of mIFN-β Gene**: For plasmid delivery different doses of plasmid DNA encoding mIFN-β were injected i.m. into mice followed by electroporation of the injected muscle. Mx1 expression was measured from PBMCs isolated from each individual animal and expressed as the fold increase over background levels of the control group (Figure 9). There was a strong up-regulation of Mx1 RNA (40- to 130-fold induction) in all four groups receiving mIFN-β plasmid DNA at day 2 post-injection. Mx1 expression in all four groups was significantly above background (p <0.002). The Mx1 expression data showed that there is a dose response with 250 µg as the optimal DNA concentration. There was an initial peak at the first day after electroporation followed by a drop in expression and at later time points the expression levels increased again. This variation in biomarker response was also reflected in the levels of the chemokines IP-10 and JE (data not shown) and appears to be a reproducible phenomenon in other studies using plasmid delivery of IFN-β. Significant levels of Mx1 induction were observed out to day 49 of the study.

**B. AAV-1 Delivery of mIFN-β Gene**: C57BI/6 mice were injected with the DNA of pGT61 encoding mIFN-β, or with the virus produced from pGT61 encoding mIFNβ, or the DNA of pGER75 encoding SEAP (see Materials and Methods, subsection G).

The mice were bled at days 2, 10, 14 and 17 post-injection. Mice that received the pGT61 DNA showed an approximately 15-fold induction of Mx1 RNA over background at day 2 (Figure 10). Mx1 expression continued to increase to greater than 100-fold over background by day 10. By day 17 Mx1 RNA expression level was about 180-fold above background. No increased Mx1 expression was observed in the control group that received the pGER75 DNA.

The Mx1 RNA expression levels in the mice injected with the virus produced from pGT61 were about 5-fold higher on day 10, 14 and 17 than in mice that received the pGT61 DNA. This was supported by IFN-β RNA RT-PCR analysis performed on the injected muscles. At day 17 when the animals were sacrificed the mIFN-β RNA expression in the muscle was 9.0x10⁵ copies/µg RNA in the DNA-injected muscles compared with 2.0x10⁶ copies/µg RNA in the virus-injected muscles (data not shown).

The plasma samples were also analyzed for IP-10 and JE (Figures 7 and 8). The results obtained were very similar to that obtained for Mx1 RNA induction. At day 2 the mice that received the DNA by electroporation showed higher IP-10 plasma level compared to the mice that were injected with the AAV-1-mIFN-β expressed virus. However, by day 10 the IP-10 levels in the mice injected with AAV-1-mIFN-β showed a strong increase and averaged approximately 5- to 10- fold greater than plasmid mIFN-β injected mice.

**C. Summary and Conclusions**: The pharmacokinetic profile following bolus injection of human IFN-β1a protein is very similar to previously published reports of studies using hIFN-β1a administered to normal human volunteers and patients (see e.g., Buchwalder, P-A (2000) J Interferon Cytokine Res 20: 57-66). Human IFN-β1a protein injected i.v. is rapidly cleared, and by 6 hours the serum levels are below the detection limit of the assay. Following i.m. injection of the protein the peak values are lower but the serum half-life is prolonged. However, the kinetics are still very rapid and serum levels fall below the limit of detection within hours. Recent pharmacokinetic studies in mice, rats and monkeys using a PEGylated form of IFN-β1a show that the attachment of a 20-kDa polymer of polyethylene glycol (PEG) extends the half-life (t_{1/2}) from approximately 1 hour to 10 hours (see e.g., Pepinsky, RB et al. (2001) J Pharm Exp Ther 297: 1059-66).

Attempts to measure serum levels of hIFN-β following plasmid DNA delivery have been unsuccessful presumably due to low expression of the transgene, even though detectable levels of hIFN-β protein have been measured by ELISA in lysates of the injected muscles (data not shown). However, using an AAV-1 vector encoding hIFN-β, very high serum levels of hIFN-β protein were detected by the hIFN-β ELISA in a dose dependent manner following i.m. injection. Moreover, very stable and persistent levels were measured out to nearly 6 months post-injection. It is interesting to note the lack of an apparent immunogenic response to hIFN-β expression as a foreign transgene in this model, though the blood was not analyzed for the presence of anti-hIFN-β antibodies. It has been reported that bolus protein delivery of hIFN-β is highly immunogenic in other animal models (e.g. monkeys, see ref. 33). The results suggest that i.m. administration of an AAV-1 vector encoding hIFN-β could be developed as a platform to achieve high transgene expression over extended periods of time.

Three mIFN-β biomarkers were identified and validated to perform pharmacokinetic studies using murine IFN-B protein or gene delivery. A highly sensitive quantitative RT-PCR assay was developed to measure the induction of Mx1 RNA isolated from PBMC's of mice administered mIFN-β. Two murine chemokines, IP-10 and JE, were also identified as sensitive IFN-β biomarkers and commercial ELISA's allowed the means to rapidly quantitate and support the results obtained with the Mx1 TaqMan assay. Two different types of gene delivery vectors were tested, plasmid DNA (plus electroporation) and AAV-1. Administration of bolus mIFN-β protein either i.v. or i.m. resulted in a strong but transient induction of all three biomarkers, with a Tₘₐₓ of approximately 2 hours. Biomarker levels rapidly dropped to background within 12 to 24 hours post-injection. A dose response was observed for Mx1, IP-10 and JE when mIFN-β was injected i.m. The rapid drop in the biomarker levels directly reflects the rapid systemic clearance of IFN-β following bolus protein administration.

Gene-based delivery of mIFN-β using plasmid plus electroporation or an AAV-1 vector resulted in biomarker responses that were greater than those observed when mIFN-β protein was injected. With plasmid DNA a biomarker response was measured out to 49 days and was down to background levels at day 63. These data demonstrated for the first time that a mIFN-β expression plasmid is capable of expressing biologically active mIFN-β for at least 7 weeks. The kinetics of the biomarker response was slightly different when mIFN-β DNA was delivered by an AAV-1 vector. The response was relatively low early after infection and increased over the first week. Stabilization in the biomarker response at a high level was observed in the second and third week after infection.

In summary, a superior pharmacokinetic profile has been demonstrated for gene-based delivery for both human and murine IFN-β compared to bolus protein administration in mice. First, the level of IFN-β expressed is equal to or greater than the levels achieved with protein delivery, as measured directly in the serum or as reflected by the induction of IFN-β biomarkers. Second, the duration of IFN-β expression from a single injection of an IFN-β vector is far longer (stable expression for weeks to months) compared to the transient kinetics observed with protein administration (hours).

### EXAMPLE 5: Efficacy Studies using Gene-based Delivery of mIFN-β

These studies demonstrate that gene-based delivery is efficacious in an animal model of MS. The rodent EAE model is an accepted model of MS and there are several reports in which IFN-β has been shown to be active in these models (Yu, M et al. (1996) J Imm 64: 91-100). There have also been reports that gene-based delivery of IFN-B is efficacious in some of these models (see e.g., Triantaphyllopoulos, K et al., (1998) Gene Therapy 5: 253-63). The results of these studies validate a murine EAE model with mIFN-β protein; and compare gene-based delivery and protein delivery of mIFN-β in the model.

**A. Efficacy of mIFN-β Protein in Mouse Acute EAE Model**: Eight-week old female SJL mice were immunized with proteolipid protein (PLP) on day 1 and then treated every other day through the course of the study with subcutaneous (s.c.) injections of different doses (10,000, 20,000, 30,000, or 100,000 units per group) of purified recombinant murine IFN-β protein The positive controls used for this study were Mesopram and Prednisolone, administered intraperitoneally (i.p.), twice daily.

Specifically, the gene encoding mIFN-β was cloned into a pCEP4 expression vector (Invitrogen). The expression plasmid encoding mIFN-β was transiently transfected into 293E cells (Edge Biosystems) using X-tremeGene Ro-1539 Transfection Reagent (Roche). Murine IFN-β protein was purified from the medium by ion-exchange chromatography and by hydrophobic-interaction chromatography. The product was sialyzed and concentrated against dilution buffer (50 mM sodium acetate, pH 5.5, 150 mM sodium chloride, and 5% polypropylene glycol) and sterile filtered. Aliquots of the purified protein were stored at -80 °C. The activity of the purified protein was assessed using a luciferase reporter gene assay (Hardy et al. (2001) Blood 97:473-482), using a commercial mIFN-β reference standard from Access Biomedical (San Diego, CA). The specific activity of the purified protein was 2x10⁸ units/mg.

For animal studies, purified mIFN-β was diluted to 100 ug/mL in dilution buffer. Immediately prior to injection of the animals, the mIFN-β stock solution was diluted to the desired concentration of mIFN-β. The vehicle control used in these studies was the dilution buffer minus mIFN-β.

The results of the study are shown in Figure 11. Mice treated with 100,000 units of IFN-β (approximately 500 ng, 20 ug/kg) developed significantly decreased clinical scores of EAE compared with vehicle treated mice (p=0.0046). Mice treated with 30,000 units of IFN-β also demonstrated decreased clinical scores compared to vehicle treated mice, although this decrease did not reach statistical significance. The positive controls in this study, Mesopram and Prednisolone, also significantly decreased clinical scores.

**B. Gene-based Delivery of mIFN-β is Efficacious in Murine Acute EAE Model:** Based upon the results of the previous study demonstrating that mIFN-β protein is efficacious in the murine Acute EAE model a second study was performed to test and compare plasmid delivery of mIFN-β with protein delivery. As in the first study, the mice were injected on day 1 with PLP. For gene delivery, the mice received an intramuscular injection on day 2 of the study with either PBS, null plasmid DNA (pNulI) with electroporation (EP), mIFN-β plasmid DNA (plus EP), or mIFN-β plasmid DNA formulated with a polymer formulation called "PINC" (Mumper, RJ et al (1998) J Controlled Release 52: 191-203). For protein delivery mice were injected every other day with murine IFN-β protein (100,000 units, s.c. injection) or vehicle.

The results of this study are shown in Figure 12. As in the previous study the mice treated with 100,000 Units of mIFN-β protein had significantly decreased clinical scores compared to the vehicle control treated mice (p=0.045). Gene delivery of the mIFN-β + EP also significantly decreased clinical scores, compared to gene delivery of pNulI & EP (p= 0.0171). Gene delivery using the PINC formulation of IFN-β did not statistically decrease clinical scores compared to pNulI (data not shown). The full results of this study are described in the Materials and Methods, subsection A, and in Figure 13.

**C. Summary and Conclusions**: A murine acute EAE model has been validated using recombinant mIFN-β protein by demonstrating that every other day injection of 100,000 units of mIFN-β during the course of the study significantly decreased the severity of the disease compared to a vehicle control. Gene-based delivery of a plasmid encoding mIFN-β with electroporation was shown to significantly decrease the clinical scores in diseased mice. A single injection of the plasmid on day 2 of the study was as effective in reducing the scores as an every other day injection of IFN-β protein. These results demonstrate that gene-based delivery of IFN-β is efficacious in an animal model of MS.

### EXAMPLE 6: Regulated Expression of IFN-β In Vivo Using a Regulated Expression System

**A. Design, Construction and *In Vitro* Validation**: The regulated expression systems of the present invention has advantages over known expression systems. In a preferred embodiment, the system of the present invention solves several development and manufacturing issues by having in a single vector a first expression cassette encoding a therapeutic molecule of interest (TM) (e.g., an IFN-β transgene) and a second expression cassette encoding a regulator molecule (RM) that regulates the expression of the TM.

As an example of one preferred embodiment, the present inventors provide a new and improved regulated expression system. In this embodiment, the expression cassettes of the regulated, expression system of the present invention are present in a single plasmid vector called BRES-1. The BRES-1 single vector has a number of versatile features incorporated into its design, including multiple cloning sites (MCS) for the insertion of different transgenes as well as different promoters to drive expression of the regulatory protein. In addition, the size of the BRES-1 expression cassettes is compatible with many different delivery vectors, including plasmid and AAV vectors.

**B. Construction of mIFN-β and hIFN-β Inducible Expression Vectors for Gene Therapy**: The mIFN-β and hIFN-β genes from pGER101 and pGER125, respectively, were transferred to a series of four BRES-1 vectors (Figure 14A-D). The resulting plasmids have either the expression cassette encoding the murine or human IFN-β gene and the expression cassette encoding the RM gene in four different orientations relative to each other (Figure 15A-B). The resulting BRES-1 plasmids encoding the mIFN-β were designated as pGT23, pGT24, pGT25, and pGT26 (Figure 15A), and the resulting BRES-1 plasmids encoding the hIFN-β were designated as pGT27, pGT28, pGT29, and pGT30 (Figure 15B). See the Materials and Methods, subsection F for a complete description of the construction of these plasmids.

**C. *In Vitro* Validation of IFN-β Expression Vectors**: Constitutive (pGER125) and inducible (pGT27, pGT28, pGT29, and pGT30) hIFN-β expression plasmids were transfected into murine muscle C₂C₁₂ cells. The cells were treated with the inducer, MFP, and the media was assayed for hIFN-β by ELISA (Figure 16). The results indicate little hIFN-β expression in the absence of MFP. Human IFN-β expression from the BRES-1-hIFN-β plasmids is induced by MFP approximately 20- to 90-fold, to levels up to about 50% of that expressed from the CMV promoter (pGER125). Compared to a two-plasmid expression system (pGS1694 plus pGER129), all four plasmid orientations of the BRES-1 system displayed comparable basal activity in the absence of MFP and induced activities equal to or greater than the two-plasmid in the presence of MFP. A similar *in vitro* study was performed with the mIFN-β BRES-1 plasmids (Figure 17) and the results were very similar to those described with the hIFN-β BRES-1 plasmids.

**D. Regulated Expression of IFN-β In Vivo**: A study was performed in naïve C57BI/6 mice using the mIFN-β BRES-1 plasmid vector pGT26 which was constructed by digestion of pGER101 with Sal I, blunt-ending by filling in the 5' overhang with Klenow DNA polymerase, ligation of Spe I linkers, digestion with Spe I and Not I, and insertion of the resulting fragment carrying the mIFN gene between the Spe I and Not I sites of pGT4.

The plasmid vector pGT26 was used to test whether the expression of mIFN-β could be regulated in an off/on/off pulsatile manner through oral administration of the inducer, MFP. Constitutive and inducible BRES-1 mIFN-β expression plasmids were injected with electroporation into the hind limb muscles of mice. Mice were treated with MFP for four consecutive days, beginning on day 7 after plasmid injection. Blood was collected at days 11 and 18 post-injection, PBMCs were isolated and Mx1 RNA levels were determined by RT-PCR. Plasma samples were also assayed for the chemokines IP-10 and JE. The results of the study for biomarker analysis of Mx1 RNA and chemokine analysis are shown in Figures 18 and 19, respectively. In the absence of MFP little or no biomarker induction is observed at 7 days. Following oral administration of MFP, all biomarkers were strongly induced, to levels higher than with CMV-mIFN-β at day 11. By day 18 the chemokine levels had returned to baseline and the Mx1 RNA level had decreased nearly to baseline (see Materials and Methods, subsection G below for description of the study and controls).

**E. Summary and Conclusions**: The present inventors have identified two vectors, a non-viral plasmid DNA and an adeno-associated virus type 1 (AAV-1), that are suitable for delivery of a therapeutic molecule (TM), e.g. an IFN-β gene, to treat a chronic disease e.g., MS. Further, the present inventors have shown that both vectors can be delivered to skeletal muscle by intramuscular injection and generate IFN-β expression levels that are measurable and persistent in murine animal models. In the case of plasmid DNA the present inventors have demonstrated that a single injection of a mIFN-β encoded plasmid (with electroporation) is efficacious and as effective as an every other day injection of mIFN-β protein in an animal model of MS. The present inventors developed biomarkers of mIFN-β to show that plasmid encoded mIFN-β expression persists for at least 45 days following a single plasmid injection. In the case of the AAV-1 vector the present inventors have shown that a single intramuscular injection of a human IFN-β encoded AAV-1 vector results in high serum levels of the human IFN-β protein that persists for 6 months. Both plasmid and AAV-1 vectors have been shown to be compatible with the BRES-1 regulated expression system of the present invention. For example, the present inventors demonstrated the regulated expression of IFN-β in mice using a single-plasmid vector BRES-1 regulated expression system of the present invention.

In one embodiment of the new and improved BRES-1 regulated expression system, the expression cassettes are present in a single vector, e.g., a single plasmid vector. In this embodiment, the BRES-1 single plasmid vector contains the expression cassettes for both the Regulator molecule (RM) (e.g., a transcriptional activator such as a modified steroid hormone receptor) and the therapeutic molecule (TM) (e.g., human or murine IFN-β) on a single shuttle plasmid expression vector. The single vector of the BRES-1 regulated expression system contains multiple cloning sites (MCS) to simplify the insertion or replacement of promoters, regulatory elements and transgenes into the plasmid backbone. As demonstrated herein by the present inventors, BRES-1 mIFN-β and BRES-1 hIFN-β single-plasmid vectors were constructed and tested *in vitro,* and shown to have low background activity in the absence of the activator molecule (AM), the small molecule inducer MFP, and showed high inducibility (comparable to a two-plasmid system) in the presence of MFP.

An *in vivo* study was conducted in normal mice using a BRES-1 mIFN-β plasmid vector, and oral administration of MFP. Using biomarkers to monitor mIFN-β expression levels the results showed low background expression of mIFN-β biomarkers in the absence of MFP, strong induction following MFP administration, and a return to basal levels of expression upon withdrawal of MFP (Figures 18 and 19). Based upon these results the present inventors have achieved the regulated expression of IFN-β in vivo using the regulated expression system of the present invention.

Thus, an outcome of these studies and the compositions and methods of the present invention is a gene-based delivery system for IFN-β that will provide long-term, regulated expression of IFN-β for the treatment of a disease or condition, e.g., an anti-inflammatory disease or condition, and more preferably MS. The gene therapy vectors of the present invention can incorporate one or more expression cassettes for delivery of a therapeutic molecule (TM) of interest (e.g., IFN-β) for treatment of a disease or condition. In one embodiment, the regulated expression system as described herein can provide long-term, renewable expression through oral administration of the small molecule inducer, MFP. The single-vector BRES-1 system is capable of repeat administration, e.g., by intramuscular injection, and will allow the testing of continuous versus pulsatile IFN-β therapy in the clinic.

### EXAMPLE 7: Selection of Candidate Vector

### BRES-1 Regulated expression system

**A. BRES-1 Orientation**: The *in vivo* studies performed utilized one of the four BRES-1 orientations that were constructed as described in Figure 15. As described herein, this was based upon *in vitro* data that showed that the construct, pGT26, had the highest level of transgene expression in the presence of MFP. These four BRES-1 orientations can be tested *in vivo* using the protocols described herein to determine which one provides the best "window" of transgene expression (e.g., the lowest basal expression level minus MFP, and highest induced expression level plus MFP).

**i. Orientation-dependent effects on target gene expression in BRES-1 plasmids**: A study was performed in naive C57BI/6 mice using the mIFN-β BRES-1 plasmid vectors pGT23, 24, 25, and 26, to determine the level of mIFN expression as assayed by the level of the chemokine IP-10, which serves as a biomarker for mIFN expression. pGT23, pGT24, pGT25, and pGT26 were injected with electroporation into the hind limb muscles of mice, with 15 animals per group. Five mice from each group were bled at day 7 in the absence of MFP. The remaining 10 mice in each group were treated with MFP for four consecutive days, beginning on day 7 after plasmid injection. Blood was collected at days 11 and 18 post-injection, and plasma samples were assayed for IP-10 (See "Experimental Design" for details). The results show that pGT26 offered the best combination of low expression - MFP and high expression + MFP, consistent with the in vitro results (Figure 32). pGT24 had the highest induction of IP-10 expression, but the IP-10 levels in the absence of MFP were higher than the other orientations. pGT25 had lower IP-10 levels both - and + MFP, and pGT23 had IP-10 levels + MFP about the same as pGT26. The IP-10 levels with pGT24 - MFP, however, were considerably higher than for pGT26. These results in total indicate an orientation-dependent effect on both basal and induced target gene expression.

**Experimental Design**: In vivo transfection of BRES-1/mIFN plasmids and comparison of the four orientations of pBRES-1/mIFN was performed as follows. Normal C57BI/6 mice were injected and electroporated with single-vector BRES-1 mouse IFN expression plasmids. mIFN expression was monitored by biomarker response and mIFN RNA analysis. The mice went through one off/on/off cycle of MFP treatment.

**DNA solutions**: Each mouse in all injected groups received 250 ug of plasmid DNA in 150 µl PBS.

**Table 3**

| **Group** | **plasmid** | **Description** | **n*** |
|---|---|---|---|
| 1 | pGT23 | mIFN-RM | 15 |
| 2 | pGT24 | mIFN rev-RM | 15 |
| 3 | pGT25 | RM-mIFN | 15 |
| 4 | pGT26 | RM-mIFN rev | 15 |

| | | | |
|---|---|---|---|
| *n=number of animals | | | |

**DNA delivery**: Adult male C57BI/6 mice were injected bilaterally on day 0 with 250 ug plasmid DNA in 150 µl PBS. The DNA solution was injected 25 µl into the tibialis muscle and 50 µl into the gastrocnemius muscle of each hind leg, followed by electroporation with a caliper (8 pulses at 200 V/cm, 1 Hz, 20 msec/pulse).

**MFP treatment**: Groups 1-4 (all injected mice) were administered MFP by oral gavage at 0.33 mg/kg (100 µl of 0.083 mg/ml in sesame oil, made fresh) as indicated in the table below.

**Table 4**

| **Group/ plasmid** | **n/ mouse #** | **day 0** | **day 7** | **day 7-10** | **day 11** | **day 18** |
|---|---|---|---|---|---|---|
| 1 pGT23 | 15 101-115 | inject 101-115 | Terminal bleed + muscles 101-105 | MFP 106-115 | Terminal bleed + muscles 106-110. | Terminal bleed + muscles 111-115 |
| | | | | | Tail bleed 111-115 | |
| 2 pGT24 | 15 201-215 | inject 201-215 | Terminal bleed + muscles 201-205 | MFP 206-215 | Terminal bleed + muscles 206-210. | Terminal bleed + muscles 211-215 |
| | | | | | Tail bleed 211-215 | |
| 3 pGT25 | 15 301-315 | inject 301-315 | Terminal bleed + muscles 301-305 | MFP 306-315 | Terminal bleed + muscles 306-310. | Terminal bleed + muscles 311-315 |
| | | | | | Tail bleed 311-315 | |
| 4 pGT26 | 15 401-415 | inject 401-415 | Terminal bleed + muscles 401-405 | MFP 406-415 | Terminal bleed + muscles 406-410 | Terminal bleed + muscles 411-415 |
| | | | | | Tail bleed 411-415. | |
| 5 uninjected | 5 501-515 | | Terminal bleed + muscles 501-505 | | | |

| | | | | | | |
|---|---|---|---|---|---|---|
| Day 7: Compares baseline level of expression in the absence of MFP. Day 11: Compares induced level of expression after MFP treatment. Day 18: Compares expression after 7 days without MFP treatment. | | | | | | |

**Blood collection and Endpoint Analysis/Assay Procedure**: Mice were bled by tail nick or cardiac puncture (terminal bleed) at the time points indicated in the table above. Blood was collected into Microtainer tubes (containing EDTA) at RT (room temperature). Plasma was collected from the tail bleeds for IP-10 assays. PBMC's were separated and collected from the terminal bleeds, and the remaining plasma was assayed for IP-10 by ELISA.

**ii. Orientation-dependent effects on target gene expression in BRES-1 plasmids**: BRES-1/hIFN plasmids were also tested in a similar manner as described above. Constitutive (pGER125) or inducible BRES-1/hIFN (pGT27, pGT28, pGT29, and pGT30) plasmids were injected with electroporation into the hind limb muscles of naive C57BI/6 mice. Mice were bled at day 4 in the absence of MFP, then treated with MFP for four consecutive days, beginning on day 7 after plasmid injection. Blood was collected at days 11 and 18 post-injection. Serum was collected from clotted blood and samples were assayed for hIFN by ELISA (See "Experimental Design" below for details).

The results show that pGT28 offered the best combination of low expression - MFP and high expression + MFP, consistent with the in vitro results (Figure 33). Expression of hIFN - MFP at day 4 was undetectable for all BRES-1/hIFN plasmids. Expression of HIFN + MFP at day 7 was less than with the CMV promoter for pGT27, but was higher for pGT28, 29, and 30. Expression of hIFN from pGT28 was as much as 5-fold higher as that from CMV. Expression had fallen to nearly undetectable for all BRES-1/hIFN plasmids by day 18, with pGT28 having the lowest expression at that point. These results in total indicate an orientation-dependent effect on both basal and induced target gene expression.

**Experimental Design**: In vivo transfection of BRES-1/hIFN plasmids and comparison of the four orientations of pBRES-1/hIFN was performed as follows. Normal adult C57BI/6 mice were injected with plasmid vectors carrying the BRES-1/hIFN or CMV-hIFN expression cassettes. Human IFN expression was assayed through one off/on/off cycle of MFP treatment.

**Plasmid DNA solutions**: Group 2-6 mice (plasmid) received 250 ug DNA per mouse in a volume of 150 ul.

**Table 5**

| **Group** | **vector** | **description** | **n*** |
|---|---|---|---|
| 2 | pGT27 | hIFN-RM in plasmid | 5 |
| 3 | pGT28 | HIFN rev-RM in plasmid | 5 |
| 4 | pGT29 | RM-hIFN in plasmid | 5 |
| 5 | pGT30 | RM-hIFN rev in plasmid | 5 |
| 6 | pGER125 | CMV-hIFN in plasmid | 5 |

| | | | |
|---|---|---|---|
| *n=number of animals | | | |

DNA delivery: For Groups 2-6 (plasmid), 25 µl of the DNA solution was injected into the tibialis muscle and 50 µl into the gastrocnemius muscle of each hind leg, followed by electroporation with a caliper (8 pulses at 200 V/cm, 1 Hz, 20 msec/pulse).

MFP treatment: Groups 2-5 were administered MFP by i.p. injection at 0.33 mg/kg (100 µl of 0.083 mg/ml in sesame oil, made fresh) on day 7-10, as indicated in the table below.

**Table 6: Cycle 1**

| **Group Plasmid** | **n/ mouse #** | **day 0** | **day 4** | **day 7-10** | **day 11** | **day 18** |
|---|---|---|---|---|---|---|
| 2 pGT27 | 5 201-205 | inject and EP plasmid | tail bleed | MFP | tail bleed | terminal bleed + muscles |
| 3 pGT28 | 5 301-305 | inject and EP plasmid | tail bleed | MFP | tail bleed | terminal bleed + muscles |
| 4 pGT29 | 5 401-405 | inject and EP plasmid | tail bleed | MFP | tail bleed | terminal bleed + muscles |
| 5 pGT30 | 5 501-505 | inject and EP plasmid | tail bleed | MFP | tail bleed | terminal bleed + muscles |
| 6 pGER125 | 5 601-605 | inject and EP plasmid | tail bleed | | tail bleed | terminal bleed + muscles |
| 7 Uninjected | 5 701-705 | | | | | terminal bleed + muscles |

Blood collection and Endpoint Analysis/Assay Procedure: Mice were bled by tail nick or cardiac puncture (terminal bleed) at the time points indicated in the table above. Blood was collected in Microtainer tubes (no anti-coagulant). Serum was separated and collected from the blood, and then assayed for hIFN by ELISA.

**iii. Orientation-dependent effects on target gene expression in BRES-1 plasmids**: BRES-1/hEPO plasmids were also tested in a similar manner as above. Inducible one-plasmid BRES-1/hEPO (pGT15, pGT16, pGT17, and pGT18) or two-plasmid (pGS1694 + pEP1666) vectors were injected with electroporation into the hind limb muscles of naive C57BI/6 mice, with 10 animals per group. Five mice from each group were treated with MFP for four consecutive days, beginning on day 7 after plasmid injection, and five mice from each group did not receive MFP. Blood was collected at day 10 post-injection, 6 hr after the last MFP treatment. Serum was collected from clotted blood and samples were assayed for hEPO by ELISA (See Experimental Design below for details). The results show that the induced expression levels are higher than the two plasmid system for 3 of the 4 BRES-1/EPO plasmids and that the expression levels vary with orientation, consistent with the mIFN and hIFN BRES-1 plasmids (Figure 34A). The results show a good correlation between EPO expression and hematocrit levels (Figure 34B), demonstrating a physiological effect of inducible EPO gene expression. These results in total indicate an orientation-effect on both basal and induced target gene expression.

**Experimental Design**: In vivo transfection of BRES-1/hEPO plasmids and comparison of the four orientations of pBRES-1/hEPO with the two-plasmid regulated expression system was performed as follows. Normal adult C57BI/6 mice were injected with BRES-1/hEpo plasmid vectors or the two-plasmid regulated expression system with Epo as the GS-responsive target gene. Human Epo expression was assayed in the absence and presence of MFP treatment. The following protocol consists of five groups of mice (N=10, where "N" is the number of animals). For each group, all ten mice were injected/electroporated with one of the 4 pBRES-1 plasmids or the two plasmids. An additional group of N=10 (Group 6) was uninjected for negative controls.

For each tail bleed or terminal bleed below, 10 µl of blood was immediately collected for a hematocrit assay, and serum from the remainder of the blood was also collected. See below under "Blood Collection and Endpoint Analysis/Assay Procedure". For each of groups 1-5, at day 7 five mice were injected with MFP days 7-10 in the morning, and tail bled on day 10 in the afternoon, about 6 hrs after the last MFP injection (induced samples). The remaining five mice in each group were not induced with MFP, and were terminally bled at day 11 (terminal bleeds were necessary for accurate uninduced levels). Group 6 was terminally bled at day 11.

Thus, in this experiment, the pBRES-1 and two plasmid systems were compared as to their MFP- induced and uninduced levels, and also compared was their constantly-induced levels over time.

**Plasmid DNA solutions**: Group 1 mice received 100 ug each plasmid per mouse in a volume of 150 µl. Group 2-5 mice received 200 ug DNA per mouse in a volume of 150 ul.

**Table 7**

| **Group** | **vector** | **description** | **n*** |
|---|---|---|---|
| 1 | pGS1694 | actin pro-GS | 10 |
| | pEP1666 | GS-responsive Epo | |
| 2 | pGT15 | hEpo-RM | 10 |
| 3 | pGT16 | hEpo rev-RM | 10 |
| 4 | pGT17 | RM-hEpo | 10 |
| 5 | pGT18 | RM-hEpo rev | 10 |

| | | | |
|---|---|---|---|
| *n=number of animals | | | |

**DNA delivery**: 25 ul was injected into the tibialis muscle and 50 µl was injected into the gastrocnemius muscle of each hind leg, followed by electroporation with a caliper (8 pulses at 200 V/cm, 1 Hz, 20 msec/pulse).

**MFP treatment**: Mice were administered MFP by i.p. injection of 100 µl of MFP solution (0.083 mg/ml in sesame oil).

**Table 8**

| **Group/ Plasmid** | **n*/ mouse #** | **day 0** | **day 7-10** | **day 10** |
|---|---|---|---|---|
| 1) pGS1694 + pEP1666 | 10 101-110 | inject and EP plasmid | MFP i.p. 101-105 | tail bleed 101-105 |
| | | | | terminal bleed 106-110 |
| 2) pGT15 | 10 201-210 | inject and EP plasmid | MFP i.p. 201-205 | tail bleed 201-205 |
| | | | | terminal bleed 206-210 |
| 3) pGT16 | 10 301-310 | inject and EP plasmid | MFP i.p. 301-305 | tail bleed 301-305 |
| | | | | terminal bleed 306-310 |
| 4) pGT17 | 10 401-410 | inject and EP plasmid | MFP i.p. 401-405 | tail bleed 401-405. |
| | | | | terminal bleed 406-410 |
| 5) pGT1 8 | 10 501-510 | inject and EP plasmid | MFP i.p. 501-505 | tail bleed 501-505. |
| | | | | terminal bleed 506-510 |
| 6 Uninjected | 10 601-610 | | | terminal bleed 601-610 |

| | | | | |
|---|---|---|---|---|
| *n=number of animals | | | | |

**Blood collection and Endpoint Analysis/Assay Procedure**: Mice were bled by tail nick or cardiac puncture (terminal bleed) at the time points indicated in the table above. Blood was collected in Microtainer tubes (no anti-coagulant), allowed to clot, centrifuged, and the serum collected. The serum was assayed for hEpo by ELISA.

**Hematocrit**: Approximately 10 µl blood was collected (aspirated) directly from a tail nick in a capillary tube, sealed with clay, and centrifuged ∼5 min at -10,000 g within 10 minutes after collection. The blood was separated in the capillary tube into 3 layers, i.e.: RBC's at the bottom (40-50% total volume), a small "buffy layer" (WBC and platelets) and the remainder plasma. A sliding gauge was used to read the hematocrit (percentage of RBC to total blood).

**B. BRES-1 Backbone**: Any of the plasmid backbone modifications of the BRES-1 vectors of the present invention, as described herein, that demonstrate a significant increase in the level and/or duration of transgene expression (as determined by the methods described herein) can be incorporated in the BRES-1 vectors of the present invention. Additional modifications to the BRES-1 vectors of the present invention may include the use of a stronger promoter. This type of modification is relatively easy to test due to the modular design of the BRES-1 system.

**C. BRES-1/Vector Testing**: Pharmacokinetics and efficacy studies by the present inventors have employed IFN-β expression cassettes utilizing the CMV promoter enhancer. The BRES-1 expression system of the present invention can be tailored to suit a particular therapeutic need as described herein. For example, changes or modifications of the vectors or expression cassettes of the present invention can be tested in C57BI/6 naïve mice. These studies can be conducted, e.g., to: 1) determine the optimal dose of vector and activator molecule (AM) (e.g., small molecule inducer MFP) necessary to achieve therapeutic levels of a therapeutic molecule (TM) (e.g., IFN-β) systemically either for continuous or pulsatile treatment paradigms, and 2) determine the duration of transgene expression over time, as well as define the optimal time for repeat administration of the vector and inducer. Once these parameters have been established in mice (or other suitable animals) then a superior pharmacokinetic profile of gene-based delivery versus protein delivery (e.g., level of expression, persistence of expression, renewable expression) can be established using these optimized delivery conditions in another species, preferably non-human primates.

### EXAMPLE 8: Selection of Candidate Vector

The selection of the type of vector can be determined by specific studies. For example, for plasmid DNA it can be determined whether electroporation is a desirable component of intramuscular injection to obtain a therapeutic level of the therapeutic molecule (TM), e.g., a therapeutic level of IFN-β. If administration by electroporation of the gene therapy vector of the present invention is desirable, then as described herein and additionally from what is known in the art, a device and protocol that can be clinically feasible and acceptable can be designed. For example, for AAV-1 vectors of the present invention, it can be determined whether repeat administration of the vector is desirable based on potential immunogenic properties reported for AAV vectors (see e.g., Chirmule, N et al. (2000) J Virol 74: 2420-25).

**A. Methods to Improve Transfection of Skeletal Muscle using Plasmid DNA**: Plasmid DNA is a desirable vector for gene-based delivery because it is simple, non-immunogenic, and easy to produce and manufacture. Several different methods have been developed to enhance skeletal muscle (SkM) transfection efficiency of plasmid DNA by intramuscular injection. These methods include the use of enzymes such as hyaluronidase to treat the muscle and surrounding extracellular matrix prior to delivery (see e.g., Mennuni, C et al. (2002) Hum Gene Ther 13: 355-65), various polymer formulations (see e.g., Mumper, RJ et al. (1998) J Controlled Release 52: 191-203; Nicol, F et al. (2002) Gene Ther 9: 1351-58), as well as devices such as electroporation (see e.g., Aihara, H et al. (1998) Nat. Biotech 16: 867-70; Bloquel, C et al (2004) J Gene Med 6: S11-S23) or ultrasound (see e.g., Schratzberger, P et al. (2002) Mol Ther 6: 576-83). Intravascular delivery of plasmid DNA to limb SkM using high pressure and large volumes ("cuff" method) has also been shown to be effective in achieving high transfection and broad distribution of plasmid DNA to this target tissue (see e.g., Budker, V et al. (1998) Gene Ther 5: 272-76). Of all of these methods electroporation and intravascular delivery are reported to be the most effective and have been shown in several animal models to enhance the transfection of plasmid DNA into SkM by two to three orders of magnitude over naked DNA alone (see e.g., Bloquel, C et al (2004) J Gene Med 6: S11-S23; Qian, HS et al. (2004) Mol Ther 9: Supp 1, S91). The present inventors using plasmid DNA have shown that detectable levels of IFN-β the serum (measurable IFN-β protein in the serum, bio-marker response, or efficacy) are achieved when electroporation has been employed with the delivery of plasmid DNA.

Suitable electroporation devices for clinical use in the delivery of IFN-β plasmid DNA can be evaluated and determined by testing in rabbits and other larger animals using methods described herein or known in the art. Electroporation devices that may be suitable for such testing and use may include those developed by Inovio AS, Ichor Medical Systems, Genetronics, Inc. Genetronics has reported testing of a device in humans (unpublished presentation at Gordon Research Conference on Bioelectrochemistry, July 25-30, NH). Inovio has also reported the results of testing electroporation technology in human volunteers (see e.g., Kjelen, R et al. (2004) Mol Ther 9: Supp 1, S60). Ichor Medical Systems has recently reported the development of an electroporation device suitable for the delivery of therapeutic DNA (see e.g., Evans, CF et al. (2004) Mol Ther 9: Supp 1, S56).

Using a plasmid encoding a LacZ reporter gene the present inventors have demonstrated high transfection efficiency to rat hind limb skeletal muscle using intra-arterial delivery of a plasmid solution. Mirus has recently reported an intra-venous delivery method for delivery plasmid DNA with decreased volume under decreased pressure which can be tested for plasmid delivery to SkM using methods described herein or known in the art (see e.g., Hagstrom, JE et al. (2004) Mole Ther 10: 386-98).

Methods other than electroporation or intravascular delivery to enhance the uptake of plasmid DNA to SkM and the subsequent expression of the transgene can be tested and their suitability for delivery of plasmid DNA determined using methods described herein or known in the art. In this regard, certain chemical agents that have been reported to enhance vector uptake to SkM can be tested and may be suitable for use in the delivery of the plasmid vectors of the present invention, including polymer formulations and antennopedia peptides (AP). For example, "F68" is a poloxamer formulation that can be used to formulate and deliver plasmid DNA and has been reported to enhance the delivery of plasmid DNA to SkM by approximately 10-fold (see e.g., Mumper, RJ et al. (1998) J Controlled Release 52: 191-203; Qian, HS et al. (2004) Mol Ther 9: Supp 1, S91). The Antennapedia (AP) peptides and other peptides of similar composition have been reported to facilitate the transport of large macromolecules across the cell membrane (see e.g., Bucci, M et al. (2000) Nat. Med 6: 1362-67; Gratton, J-P et al. (2003) Nat Med 9: 357-62).

**B. Methods to Increase the Level and Duration of IFN-β Expression from Plasmid DNA Vectors**: In addition to evaluating certain chemical agents that enhance plasmid DNA uptake to SkM, various approaches can be used to increase the level and duration of transgene expression from the plasmid DNA vectors of the present invention. For example, it has been reported that the removal of bacterial DNA sequences from plasmid DNA to create circular plasmids containing only the expression cassette ("minicircle DNA") results in 10- to 100-fold higher transgene expression (using factor IX and alphal-antitrypsin as transgenes) compared to standard plasmid DNA following transfection of liver in mice (see e.g., Chen, Z-Y et al. (2003) Mol Ther 8: 495-500). The removal of bacterial DNA sequences that are enriched in CpG regions has been shown to decrease transgene expression silencing and result in more persistent expression from plasmid DNA vectors (see e.g., Ehrhardt, A et al. (2003) Hum Gene Ther 10: 215-25; Yet, NS (2002) Mol Ther 5: 731-38; Chen, ZY et al. (2004) Gene Ther 11: 856-64). The regulated expression systems of the present invention can be modified using such approaches to increase and prolong the level of transgene expression using plasmid DNA vectors. In a preferred embodiment, a BRES-1 plasmid vector encoding IFN-β is modified to increase and prolong the level of transgene expression.

In one embodiment, expression of the IFN-β transgene in the regulated expression system of the present invention was driven by the strong cytomegalovirus (CMV) promoter to constitutively express IFN-β. It has been reported that gene-based expression using the CMV promoter undergoes silencing through extensive methylation of the promoter region *in vivo* (see e.g., Brooks, A et al. (2004) J Gene Med 6: 395-404). In addition the results from the *in vivo* study by the present inventors using the BRES-1 gene therapy plasmid vector pGT26-mIFN-β showed higher IFN-β levels using the BRES-1 expression cassette than the levels achieved using the CMV driven expression cassette (see e.g., Example 6). Given these results the IFN-β BRES-1 expression system of the present invention may generate significantly higher and more persistent expression levels than what has thus far been observed using CMV driven plasmid DNA expression cassettes and therefore it is suitable for examining the delivery of the BRES-1 plasmid vector without electroporation.

In a preferred embodiment, the method of administration is by intramuscular injection of an IFN-β plasmid solution in the absence of electroporation. Detectable levels of IFN-β in serum can be tested by administering plasmid vector by intramuscular injection to naive mice. A complete characterization of the expression level and persistence of the BRES-1 expression cassette can be performed and compared with the CMV vectors previously used. Plasmid formulations including F68 poloxamer and Antennapedia peptides can be tested for their ability to enhance plasmid transfection of SkM and subsequent IFN-β transgene expression. Lastly, modifications of the plasmid vector backbone (e.g., removal of bacterial sequences) can be explored as a means to increase and prolong transgene expression.

**C. AAV-1 Vectors**: Adeno-associated virus (AAV) is a single stranded DNA virus (parvovirus) that was initially isolated as a contaminant in adenoviral isolates from humans. AAV has a number of features that make it particularly attractive as a gene therapy vector. In addition to its non-pathogenic and replication deficient nature in the absence of a helper virus it contains a very simple genome consisting of only two genes, rep and cap. These genes are replaced in recombinant AAV vectors with the desired transgene flanked by characteristic 5' and 3' inverted terminal repeats (ITR's) of approximately 135 base pairs each. The ITR's are the only remaining components of AAV derived DNA required for vector delivery. Studies to date have shown that recombinant AAV vectors without the rep gene do not integrate *in vivo* but rather form large concatameric structures that remain episomal in non-dividing cells (see e.g., Duan, D et al. (1998) J Virol 72: 8568-77; Vincent-Lacaze, N et al. (1999) J Virol 73: 1949-55; Schnepp, BC et al. (2003) J Virol 77: 3495-3504).

AAV has a relatively small capacity for DNA, approximately 4.5 kb, but this is usually sufficient to accommodate all but the largest therapeutic transgenes. AAV2 has been tested in human gene therapy trials and has shown to provide long term expression and minimal inflammation (see e.g., Silwell, JL and Samulski, RJ (2003) BioTechniques 34: 148-59). Recently, alternative AAV serotypes have been shown to have excellent transfection efficiency to SkM in addition to long term expression characteristic of this vector system (see e.g., Grimm, D and Kay, MA (2003) Curr Gene Ther 3: 281-304). The studies by the present inventors using an AAV-1 vector expressing luciferase under a CMV promoter have shown high expression persisting beyond 12 months following intramuscular injection into the hind limb of mice (see e.g., Qian, HS et al. (2004) Mol Ther 9: Supp 1, S60). Persistent expression of hEPO out to five years in non-human primates has been reported (see e.g., Xiao, W et al. (1999) J Virol 73: 3994-4003).

As described herein the present inventors have tested AAV-1 IFN-β expressing vectors (constitutive expression using the CMV promoter/enhancer) to demonstrate robust levels of hIFN-β protein as well as mIFN-β biomarker responses following intramuscular injection into the hind limbs of C57BI/6 mice.

In choosing a viral-based delivery vector for treating a chronic disease such as MS the regulated expression systems of the present invention can be tested, using methods as described herein or as known in the art, for their ability to demonstrate not only long term expression of the therapeutic transgene but also the ability to re-administer the gene (see e.g., Chirmule, N et al. (2000) J Virol 74: 2420-25). In order to determine if AAV-1 is a suitable vector for re-administration, studies can be performed e.g., using the candidate vector, AAV-1, and AAV2, the serotype that is believed to be the most prevalent in the human population. Such an approach can be used to determine: 1) whether pre-existing antibodies to AAV2 or AAV-1 will affect the ability to deliver and express genes encoded in an AAV-1 vector, and 2) whether an AAV-1 vector can be re-administered at a dose sufficient to maintain therapeutic levels of IFN-β in mice. Vectors expressing either the reporter gene luciferase or the therapeutic gene, murine IFN-β, in either AAV-1 or AAV2 vectors can be administered i.m. at different doses and transgene expression monitored. After four weeks the vector can be re-administered and again transgene expression can be monitored, Neutralizing antibodies can be measured *ex vivo* by the ability of immunized mouse serum to inhibit viral uptake in a cell-based assay.

**i. In vivo activity of a pBRES-1-hIFN AAV vector**: A study was performed in naïve C57BI/6 mice using the hIFN-β BRES-1 AAV vector AAV-1 GT58 injected into the hind limb muscles of mice. Mice were bled at day 4 in the absence of MFP, then treated with MFP for four consecutive days, beginning on day 7 after plasmid injection. Blood was collected at days 11 and 18 post-injection. Serum was collected from clotted blood and samples were assayed for hIFN by ELISA (See "Experimental Design" below for details). Mice were then subjected to seven more cycles of MFP treatments and bleeds spaced about six to eight weeks apart. Each cycle consisted of a bleed 3 days before MFP treatment, then 4 days of MFP, then a bleed the day after the last day of MFP, then another bleed 7 days after that. The results show very high inducible hIFN expression, peaking at about 3 months after injection at a level as much as 75-fold higher than what was obtained with the strongest BRES-1/hIFN plasmid (Figure 35). hIFN expression decreased gradually over time, and background expression - MFP remained low throughout the course of the experiment. This demonstrates long-term, persistent (see also Example 9B), inducible expression of a therapeutic target gene from an AAV vector.

**Experimental Design**: Normal adult C57BI/6 mice were injected with an AAV vector carrying the BRES-1/hIFN expression cassettes, as follows. Human IFN expression was assayed through multiple off/on/off cycles of MFP treatment.

**Viral solution**: Group 1 mice (AAV) received 5 x 10¹⁰ viral particles (vp) per mouse in a volume of 75 ul.

**Table 9**

| **Group** | **vector** | **Description** | **n*** |
|---|---|---|---|
| 1 | AAV-1-GT58 | RM-hIFN rev in AAV-1 | 5 |

| | | | |
|---|---|---|---|
| *n=number of animals | | | |

**MFP treatment**: Group 1 was administered MFP by i.p. injection at 0.33 mg/kg (100 ul of 0.083 mg/ml in sesame oil, made fresh) on day 7-10, as indicated in the tables below.

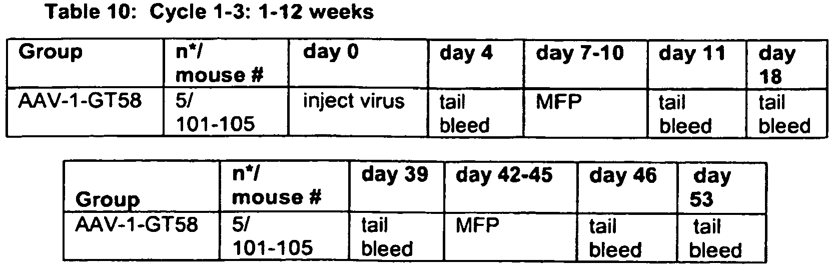

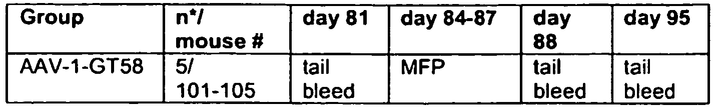

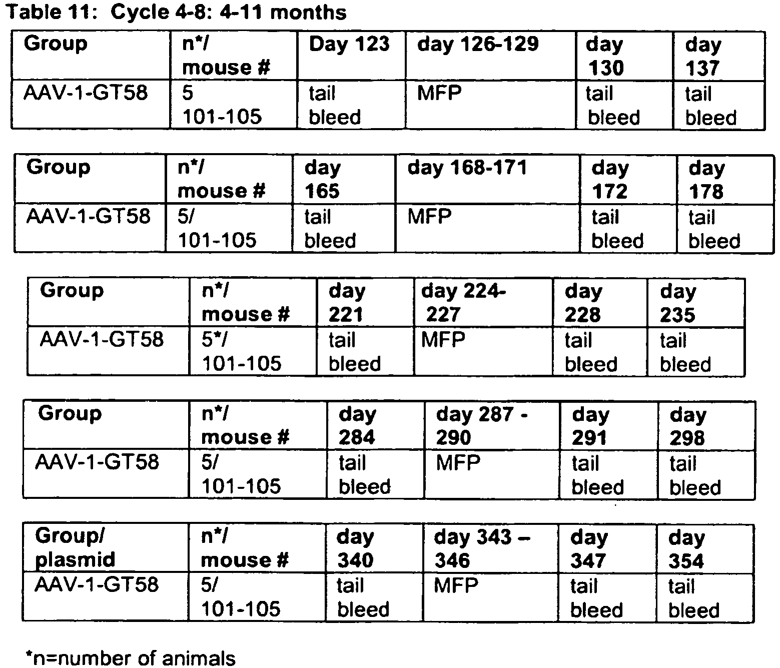

**Blood collection and Endpoint Analysis/Assay Procedure**: Mice were bled by tail nick at the time points indicated in the table above. Blood was collected in Microtainer tubes (no anti-coagulant) and centrifuged for separation and collection of serum. The serum was assayed for hIFN by ELISA.

### EXAMPLE 9: Gene Therapy Using a Regulated Expression System

**A. Dose/Response and Kinetics Studies**: Dose/response studies can be performed to determine the amount of gene therapy vector of the present invention for delivery, whether delivered by plasmid or by AAV-1, that is necessary to achieve therapeutic levels of the therapeutic molecule (TM) (e.g., IFN-β) in mice (or other suitable animals). In one embodiment, the therapeutic level is defined as the induced level of the therapeutic molecule (TM), e.g. a transgene encoding a therapeutic protein, achieved systemically by the vector that is equivalent to the level of therapeutic protein achieved by a therapeutic amount of bolus of the therapeutic protein given in humans on a mg/kg basis. For example, in a preferred embodiment, the therapeutic level is defined as the induced level of IFN-β achieved systemically by a gene therapy vector of the present invention that is equivalent to the level of IFN-β achieved by a therapeutic amount of bolus IFN-β protein given in humans on a mg/kg basis. In humans the current single dose of IFN-β1a is either 30 ug or 44 ug (Rebif).

In addition, a complete pharmacokinetic profile of the expression of the therapeutic molecule (TM) can be performed using an activator molecule (AM) to determine the AM dose/response, as well as the kinetics of induction of TM expression following AM administration. For example, in a preferred embodiment, a complete pharmacokinetic profile of IFN-β expression can be performed with the inducer MFP to determine the MFP dose/response, as well as the kinetics of IFN-β induction following MFP administration.

**i. MFP dose/response and plasmid re-injection with a BRES-1/mIFN plasmid in vivo**: A study was performed in naive C57BI/6 mice using the mIFN-β BRES-1 plasmid vector pGT26 to determine the level of mIFN expression in response to various doses of MFP, as assayed by the level of the chemokine IP-10. pGT26 was injected with electroporation into the hind limb muscles of mice, and the animals were treated with MFP at 0.0033 mg/kg to 1.0 mg/kg on day 7-10 after plasmid injection. Blood was collected at days 11 post-injection, and serum samples were assayed for IP-10 (see "Experimental Design" below for details). The results show an MFP dose-dependent increase in IP-10 levels (Figure 36). There was no increase over background IP-10 levels in the absence of MFP or at 0.0033 mg/kg. IP-10 levels then increase from 0.01 to 1.0 mg/kg MFP. A second and third cycle of MFP induction was performed at the same concentrations at day 39 and 67. By day 67 the level of IP-10 had decreased several-fold from the day 11 level, so the plasmid DNA was re-injected at day 77. Another cycle of MFP treatment was performed and the mice were bled on day 88. The results show renewed induction of IP-10 expression to the same levels as on day 11. Two more MFP cycles on day 102 and 137 were performed, and the MFP dose-response was still observed, with gradually decreasing IP-10 levels over time. Subsequently, the plasmid DNA was injected for a third time on day 189, and the following MFP cycle and bleed on day 200 once again showed a strong MFP dose-response with IP-10 expression renewed to about the same levels as on day 11 and 88. This demonstrates both a positive correlation of target gene expression with inducer dose, and also persistence and renewal of gene expression with repeat administration of the plasmid vector (see Example 9B below).

For safety studies in humans, a complete characterization of the rate at which the activator molecule (AM) and TM expression can be turned "off" following withdrawal of the AM can be performed. The frequency of AM dosing necessary to achieve steady state levels of the TM can be evaluated. In a preferred embodiment, a complete characterization of the rate at which MFP and IFN-β expression can be turned "off' following withdrawal of the inducer can be evaluated. Further, the frequency of MFP dosing necessary to achieve steady state levels of IFN-β can be evaluated.

**Experimental Design**: Normal C57BI/6 mice were injected and electroporated with a single-vector BRES-1 murine IFN expression plasmid and treated with various doses of MFP, and mIFN expression was assayed by biomarker response, as follows.

**Table 12**

| **Group** | **MFP (mg/kg)** | **n*** |
|---|---|---|
| 1 | 0 | 5 |
| 2 | 0.0033 | 5 |
| 3 | 0.01 | 5 |
| 4 | 0.033 | 5 |
| 5 | 0.10 | 5 |
| 6 | 0.33 | 5 |
| 7 | 1.00 | 5 |

**Table 13**

| **Group/ plasmid** | **n*/ mouse #** | **day 0** | **day 7-10** | **day 11** | **day 18** |
|---|---|---|---|---|---|
| 1 pGT26 | 5/ 101-105 | inject DNA | 100 ul sesame oil | tail bleed | tail bleed |
| 2 pGT26 | 5/ 201-205 | inject DNA | MFP 100 µl of 0.00083 mg/ml = 0.0033 mg/kg | tail bleed | tail bleed |
| 3 pGT26 | 5/ 301-305 | inject DNA | MFP 100 µl of 0.0025 mg/ml = 0.01 mg/kg | tail bleed | tail bleed |
| 4 pGT26 | 5/ 401-405 | inject DNA | MFP 100 µl of 0.0083 mg/ml = 0.033 mg/kg | tail bleed | tail bleed |
| 5 pGT26 | 5/ 501-505 | inject DNA | MFP 100 ul of 0.025 mg/ml = 0.1 mg/kg | tail bleed | tail bleed |
| 6 pGT26 | 5/ 601-605 | inject DNA | MFP 100 ul of 0.083 mg/ml = 0.33 mq/kq | tail bleed | tail bleed |
| 7 pGT26 | 5/ 701-705 | inject DNA | MFP 100 ul of 0.25 mg/ml = 1.0 mg/kg | tail bleed | tail bleed |
| 8 uninjected | 5/ 801-805 | | 100 µl sesame oil | tail bleed | tail bleed |
| 9 uninjected | 5/ pool | | | terminal bleed | |

**Table 14**

| **Group** | **plasmid** | **description** | **n*** |
|---|---|---|---|
| 1-7 | pGT26 | BRES-1/mIFN rev | 35 |

| | | | |
|---|---|---|---|
| *n=number of animals | | | |

**DNA solutions**: Each mouse received 250 ug of plasmid DNA in 150 µl PBS.

**DNA delivery**: Adult male C57BI/6 mice were injected bilaterally on day 0 with 250 ug plasmid DNA per mouse in 150 µl PBS. The DNA solution was injected 25 ul into the tibialis muscle and 50 µl into the gastrocnemius muscle of each hind leg, followed by electroporation with a caliper (8 pulses at 200 V/cm, 1 Hz, 20 msec/pulse).

**MFP treatment**: As indicated in the tables above and below, Groups 1-7 received 100 ul sesame oil alone or with MFP at various concentrations by i.p. injection on days 7-10.

**Table 15: Cycle 1**

| **Group/ plasmid** | **n*/ mouse #** | **day 0** | **day 7-10** | **day 11** | **day 18** |
|---|---|---|---|---|---|
| 1 DGT26 | 5/ 101-105 | inject DNA | 100 µl sesame oil | tail bleed | tail bleed |
| 2 pGT26 | 5/ 201-205 | inject DNA | MFP 100 µl of 0.00083 mg/ml = 0.0033 mg/kg | tail bleed | tail bleed |
| 3 pGT26 | 5/ 301-305 | inject DNA | MFP 100 ul of 0.0025 mg/ml = 0.01 mg/kg | tail bleed | tail bleed |
| 4 pGT26 | 5/ 401-405 | inject DNA | MFP 100 µl of 0.0083 mg/ml = 0.033 mg/kg | tail bleed | tail bleed |
| 5 pGT26 | 5/ 501-505 | inject DNA | MFP 100 ul of 0.025 mg/ml = 0.1 mg/kg | tail bleed | tail bleed |
| 6 pGT26 | 5/ 601-605 | inject DNA | MFP 100 µl of 0.083 mg/ml = 0.33 mg/kg | tail bleed | tail bleed |
| 7 pGT26 | 5/ 701-705 | inject DNA | MFP 100 ul of 0.25 mg/ml = 1.0 mg/kg | tail bleed | tail bleed |
| 8 uninjected | 5/ 801-805 | | 100 ul sesame oil | tail bleed | tail bleed |
| 9 uninjected | 5/ pool | | | terminal bleed | |

| | | | | | |
|---|---|---|---|---|---|
| *n=number of animals | | | | | |

**Table 16: Cycle 2: Same MFP concentrations as Cycle 1**

| **Group/ Plasmid** | **n*/ mouse #** | **day 35-38** | **day 39** |
|---|---|---|---|
| 1 pGT26 | 5/ 101-105 | 100 ul sesame oil | tail bleed |
| 2 pGT26 | 5/ 201-205 | MFP 100 µl of 0.00083 mg/ml = 0.0033 mg/kg | tail bleed |
| 3 pGT26 | 5/ 301-305 | MFP 100 µl of 0.0025 mg/ml = 0.01 mg/kg | tail bleed |
| 4 pGT26 | 5/ 401-405 | MFP 100 ul of 0.0083 mg/ml = 0.033 mg/kg | tail bleed |
| 5 pGT26 | 5/ 501-505 | MFP 100 ul of 0.025 mg/ml = 0.1 mg/kg | tail bleed |
| 6 pGT26 | 5/ 601-605 | MFP 100 ul of 0.083 mg/ml = 0.33 mg/kg | tail bleed |
| 7 pGT26 | 5/ 701-705 | MFP 100 ul of 0.25 mg/ml = 1.0 mg/kg | tail bleed |
| 8 uninjected | 5/ 801-805 | 100 ul sesame oil | tail bleed |

**Table 17: Cycle 3: Same MFP concentrations as Cycles 1 and 2**

| **Group/ plasmid** | **n*/ mouse #** | **day 63-66** | **day 67** |
|---|---|---|---|
| 1 pGT26 | 5/ 101-105 | 100 ul sesame oil | tail bleed |
| 2 pGT26 | 5/ 201-205 | MFP 100 ul of 0.00083 mg/ml = 0.0033 mg/kg | tail bleed |
| 3 pGT26 | 5/ 301-305 | MFP 100 ul of 0.0025 mg/ml = 0.01 mg/kg | tail bleed |
| 4 pGT26 | 5/ 401-405 | MFP 100 ul of 0.0083 mg/ml = 0.033 mg/kg | tail bleed |
| 5 pGT26 | 5/ 501-505 | MFP 100 ul of 0.025 mg/ml = 0.1 mq/kq | tail bleed |
| 6 pGT26 | 5/ 601-605 | MFP 100 ul of 0.083 mg/ml = 0.33 mg/kg | tail bleed |
| 7 pGT26 | 5/ 701-705 | MFP 100 ul of 0.25 mg/ml = 1.0 mg/kg | tail bleed |
| 8 uninjected | 5/ 801-805 | 100 ul sesame oil | tail bleed |

| | | | |
|---|---|---|---|
| *n=number of animals | | | |

### Re-injection of DNA on Day 77

Each mouse received 250 ug of plasmid DNA in 150 ul PBS.

**Table 18**

| **Group** | **plasmid** | **n*** |
|---|---|---|
| 2-8 | pGT26 | 35 |

| | | |
|---|---|---|
| *n=number of animals | | |

**Table 19: Cycle 4 Same MFP concentrations as Cycles 1-3 for Groups 2-7. Control groups (1 and 8) treated with 0.33 mg/kg MFP.**

| **Group** | **Day 0/Day 77 pGT26** | **n*/ mouse #** | **day 84-87** | **day 88** |
|---|---|---|---|---|
| 1 | +/- | 5/ 101-105 | MFP 100 ul of 0.083 mg/ml = 0.33 mg/kg | tail bleed |
| 2 | +/+ | 5/ 201-205 | MFP 100 ul of 0.00083 mg/ml = 0.0033 mg/kg | tail bleed |
| 3 | +/+ | 5/ 301-305 | MFP 100 ul of 0.0025 mg/ml = 0.01 mg/kg | tail bleed |
| 4 | +/+ | 5/ 401-405 | MFP 100 ul of 0.0083 mg/ml = 0.033 mg/kg | tail bleed |
| 5 | +/+ | 5/ 501-505 | MFP 100 ul of 0.025 mg/ml = 0.1 mg/kg | tail bleed |
| 6 | +/+ | 5/ 601-605 | MFP 100 ul of 0.083 mg/ml = 0.33 mg/kg | tail bleed |
| 7 | +/+ | 5/ 701-705 | MFP 100 ul of 0.25 mg/ml = 1.0 mg/kg | tail bleed |
| 8 | -/+ | 5/ 801-805 | MFP 100 ul of 0.083 mg/ml = 0.33 mg/kg | tail bleed |

**Table 20: Cycle 5 Same MFP concentrations as Cycles 1-5 for Groups 2-7. Control groups (1 and 8) treated with 0.33 mg/kg MFP.**

| **Group T** | **Day 0/Day 77 pGT26** | **n*/ mouse #** | **day 98-101** | **day 102** |
|---|---|---|---|---|
| 1 | +/- | 5/ 101-105 | MFP 100 ul of 0.083 mg/ml = 0.33 mg/kg | tail bleed |
| 2 | +/+ | 5/ 201-205 | MFP 100 ul of 0.00083 mg/ml = 0.0033 mg/kg | tail bleed |
| 3 | +/+ | 5/ 301-305 | MFP 100ul of 0.0025 mg/ml = 0.01 mg/kg | tail bleed |
| 4 | +/+ | 5/ 401-405 | MFP 100 ul of 0.0083 mg/ml = 0.033 mg/kg | tail bleed |
| 5 | +/+ | 5/ 501-505 | MFP 100 ul of 0.025 mg/ml = 0.1 mg/kg | tail bleed |
| 6 | +/+ | 5/ 601-605 | MFP 100 ul of 0.083 mg/ml = 0.33 mg/kg | tail bleed |
| 7 | +/+ | 5/ 701-705 | MFP 100 ul of 0.25 mg/ml = 1.0 mg/kg | tail bleed |
| 8 | -/+ | 5/ 801-805 | MFP 100 ul of 0.083 mg/ml = 0.33 mg/kg | tail bleed |

**Table 21: Cycle 6 Same MFP treatments as in Cycle 5. Re-injection of DNA on Day 189: Each mouse received 250 ug of plasmid DNA in 150 ul PBS. Group 9 were new control mice, where the age was matched as closely as possible. Group 1 was also injected with plasmid.**

| **Group** | **Day 0/Day 77 pGT26** | **n*/ mouse #** | **day 133-136** | **day 137** |
|---|---|---|---|---|
| 1 | +/- | 5/ 101-105 | MFP 100 ul of 0.083 mg/ml = 0.33 mg/kg | tail bleed |
| 2 | +/+ | 5/ 201-205 | MFP 100 ul of 0.00083 mg/ml = 0.0033 mg/kg | tail bleed |
| 3 | +/+ | 5/ 301-305 | MFP 100 ul of 0.0025 mq/ml = 0.01 mg/kg | tail bleed |
| 4 | +/+ | 5/ 401-405 | MFP 100 ul of 0.0083 mg/ml = 0.033 mg/kg | tail bleed |
| 5 | +/+ | 5/ 501-505 | MFP 100 ul of 0.025 mg/ml = 0.1 mg/kg | tail bleed |
| 6 | +/+ | 5/ 601-605 | MFP 100 ul of 0.083 mg/ml = 0.33 mg/kg | tail bleed |
| 7 | +/+ | 5/ 701-705 | MFP 100 ul of 0.25 mg/ml = 1.0 mg/kg | tail bleed |
| 8 | -/+ | 5/ 801-805 | MFP 100 ul of 0.083 mg/ml = 0.33 mg/kg | tail bleed |

**Table 22**

| **Group** | **Plasmid** | **n*** |
|---|---|---|
| 3-9 | pGT26 | 35 |

**Table 23: Cycle 7 Same MFP concentrations as Cycles 4-6 for Groups 3-7. Control groups (1, 2, 8, and 9) were treated with 0.33 mg/kg MFP.**

| **Group** | **Day 0/77/189 pGT26** | **n*/ mouse #** | **day 196-199** | **day 200** |
|---|---|---|---|---|
| 1 | +/-/+ | 5/ 101-105 | MFP 100 ul of 0.083 mg/ml = 0.33 mg/kg | tail bleed |
| 2 | +/+/- | 5/ 201-205 | MFP 100 ul of 0.083 mg/ml = 0.33 mg/kg | tail bleed |
| 3 | +/+/+ | 5/ 301-305 | MFP 100 ul of 0.0025 mg/ml = 0.01 mg/kg | tail bleed |
| 4 | +/+/+ | 5/ 401-405 | MFP 100 ul of 0.0083 mn/ml = 0.033 mg/kg | tail bleed |
| 5 | +/+/+ | 5/ 501-505 | MFP 100 ul of 0.025 mg/ml = 0.1 mg/kg | tail bleed |
| 6 | +/+/+ | 5/ 601-605 | MFP 100 ul of 0.083 mg/ml = 0.33 mg/kg | tail bleed |
| 7 | +/+/+ | 5/ 701-705 | MFP 100 ul of 0.25 mg/ml = 1.0 mg/kg | tail bleed |
| 8 | -/+/+ | 5/ 801-805 | MFP 100 ul of 0.083 mg/ml = 0.33 mg/kg | tail bleed |
| 9 | -/-/+ | 5/ 901-905 | MFP 100 ul of 0.083 mg/ml = 0.33 mg/kg | tail bleed |

**Table 24: Cycle 8 Same MFP concentrations as Cycle 7. Groups 3-7 were terminally harvested, and RNA and DNA prepared from muscle.**

| **Group** | **Day 0/77/189 pGT26** | **n*/ mouse #** | **day 224-227** | **day 228** |
|---|---|---|---|---|
| 3 | +/+/+ | 5/ 301-305 | MFP 100 ul of 0.0025 mg/ml = 0.01 mg/kg | terminal bleed and muscles |
| 4 | +/+/+ | 5/ 401-405 | MFP 100 ul of 0.0083 mg/ml = 0.033 mg/kg | terminal bleed and muscles |
| 5 | +/+/+ | 5/ 501-505 | MFP 100 ul of 0.025 mg/ml = 0.1 mg/kg | terminal bleed and muscles |
| 6 | +/+/+ | 5/ 601-605 | MFP 100 ul of 0.083 mg/ml = 0.33 mg/kg | terminal bleed and muscles |
| 7 | +/+/+ | 5/ 701-705 | MFP 100 ul of 0.25 mg/ml = 1.0 mg/kg | terminal bleed and muscles |

| | | | | |
|---|---|---|---|---|
| *n=number of animals | | | | |

**Blood collection and Endpoint Analysis/Assay Procedure**: Mice were bled by tail nick or cardiac puncture and the blood was collected in Microtainer tubes (no anti-coagulant). The serum was then separated from the blood, collected, and assayed for IP-10 by ELISA.

**ii. Determination of induction and de-induction kinetics of hIFN from a BRES-1 AAV vector in vivo**: A study was performed in naïve C57BI/6 mice using the hIFN-β BRES-1 AAV vector AAV-1GT58 to examine the kinetics of induction and de-induction. AAV-1GT58 was injected into hind limb muscles of C57BI/6 mice, the animals were administered MFP by i.p. injection for four consecutive days, during which time they bled at various times after the first MFP injection to determine the induction kinetics. They were then bled at various times after the last MFP injection to determine the de-induction kinetics (see "Experimental Design" below for details). The serum was assayed for hIFN by ELISA. The results show that the induction and de-induction kinetics are rapid, with peaks levels of hIFN reached by 48-72 hr after the first MFP treatment (Figure 37A), and diminishing to background levels within 96 hr after MFP treatment (Figure 37B). This demonstrates that the BRES-1 system can be rapidly turned on and off in vivo.

**A. Experimental Design:** Normal adult C57BI/6 mice were injected with an AAV1 vector carrying the BRES-1/hIFN expression cassette, as follows. For each group, five animals (n=5) were sacrificed at the indicated "Harvest Times" in the table below. Human IFN expression was determined in the serum by ELISA in the absence of MFP, or after single/multiple MFP administration.

**Table 25**

| **Group** | **N*** | **Vector** | **Treatment (MFP)** | **Harvest Time** |
|---|---|---|---|---|
| 1 | 10 | PBS | None | 24, 96 hours (post-injection) |
| 2 | 30 | RM-hIFN AAV1 | None | 7, 14, 21, 28, 35, 42 days (post-injection) |
| 3 | 25 | RM-hIFN AAV1 | Day 17 only | 1, 3, 6, 12, 24 hours (post-MFP administration) |
| 4 | 5 | RM-hIFN AAV1 | Days 16, 17 | 24 hours (post-MFP administration) |
| 5 | 5 | RM-hIFN AAV1 | Days 15, 16, 17 | 24 hours (post-MFP administration) |
| 6 | 35 | RM-hIFN AAV1 | Days 18, 19, 20, 21 | 6, 12, 24, 36, 48, 72, 96 hours (post-MFP administration) |

**Virus solutions and delivery:** Group 1 mice received 75 ul PBS and Groups 2-6 mice received 5 x 10¹⁰ viral particles (vp) per mouse in a volume of 75 ul PBS in one hind leg (right leg). 25 ul was injected into the tibialis muscle and 50 ul injected into the gastrocnemius muscle.

**Table 26**

| **Group** | **vector** | **Description** | **n*** |
|---|---|---|---|
| 1 | None | | 10 |
| 2 | AAV-1-GT58 | RM-hIFN rev in AAV-1 | 30 |
| 3 | AAV-1-GT58 | RM-hIFN rev in AAV-1 | 25 |
| 4 | AAV-1-GT58 | RM-hIFN rev in AAV-1 | 5 |
| 5 | AAV-1-GT58 | RM-hIFN rev in AAV-1 | 5 |
| 6 | AAV-1-GT58 | RM-hIFN rev in AAV-1 | 35 |

| | | | |
|---|---|---|---|
| *n=number of animals | | | |

**MFP treatment:** Groups 3-6 were administered MFP by i.p. injection at 0.33 mg/kg (100 ul of 0.083 mg/ml in sesame oil, made fresh) according to the following schedule: Group 3 = day 17, Group 4 = days 16 + 17, Group 5 = days 15-17, and Group 6 = days 18-21.

**Blood collection and Endpoint Analysis/Assay Procedure:** Mice were bled by cardiac puncture (terminal bleed) at the time points indicated in the Table 27. Blood was collected in Microtainer tubes (no anti-coagulant) and the serum was separated from the blood, collected and assayed for hIFN by ELISA.

**B. Persistence of Expression and Repeat Administration Studies:** The data generated thus far has shown that expression using CMV promoter-based constructs persists for constitutive IFN-β at least 49 days using plasmid DNA and 6 months using an AAV-1 vector. The plan is to repeat and extend these studies using the candidate vector with the BRES-1 system to demonstrate both persistent, as well as regulatable expression of IFN-β for at least 3 months. These studies can be conducted in naïve C57/BI6 mice.

The BRES-1 vectors of the present invention can be tested in C57BI/6 mice (or other suitable animals) for repeat biomarker endpoints in response to ELISA or administration using administration of the activator molecule (AM). The presence of neutralizing antibodies against the BRES-1 vector, expressed therapeutic molecule (e.g., a transgene), regulator molecule (RM) or therapeutic protein, can be monitored. For example, the activator molecule (AM) can be administered chronically as well as in a pulsatile manner to evaluate the ability to maintain expression levels of the therapeutic molecule (TM) over time as well as provide renewable expression levels in an on/off manner with AM dosing.

In one preferred embodiment, the IFN-β BRES-1 vector of the present invention can be tested in C57BI/6 mice (or other suitable animal) for repeat biomarker endpoints in response to MFP administration or administration of IFN-β. The presence of neutralizing antibodies against the vector, IFN molecule (IFNM) (e.g., a IFN-β transgene), regulator molecule (RM), or IFN-β protein, can be monitored. MFP can be administered chronically as well as in a pulsatile manner to evaluate the ability to maintain IFN-β expression levels over time as well as provide renewable expression levels in an on/off manner with MFP dosing.

**i. Kinetics of mIFN induction and de-induction from BRES-1 plasmid, pulsatile and chronic MFP treatment:** A study was performed in naïve C57BI/6 mice using pGT26 to examine the kinetics of induction and de-induction with the mIFN-β BRES-1 plasmid vector, compare mIFN gene expression in response to pulsatile or chronic administration of MFP, and examine the persistence of gene expression over several months. Constitutive (pGER101, CMV) or inducible (pGT26, BRES-1) mIFN expression plasmids were injected with electroporation into the hind limb muscles of 20 mice per group, and five mice of each group were bled at day 7 in the absence of MFP. All 20 of the mice that received pGT26 were treated with MFP on day 7-10 after plasmid injection. To examine the kinetics of de-induction, blood was collected from five mice of each group at each of days 11, 12, 14, 16, and 18 days post-injection. All 20 pGT26 mice then received MFP on days 21-24, and to examine the kinetics of induction, blood was collected from five mice of each group at each of days 22, 23, and 25. (See "Experimental Design" below for details). To examine target gene expression in response to continuous treatment with MFP, mice that received pGT26 were injected with MFP i.p. every day from day 35-50, and five mice from each group were bled during this period on day 35 (before MFP treatment), 39, 42, 45, and 51. To examine gene expression after 3.5 months, mice were bled on day 105, then treated with MFP on day 105-108, and bled on day 109 and 116. Serum samples were assayed for IP-10 as a biomarker for mIFN expression.

The results (Figure 37C) show that induction of IFN expression from the BRES-1 plasmid upon MFP treatment occurred within 24 hr, and expression decreased to baseline 24-48 hr following peak induction. Expression of mIFN from the BRES-1 system was higher than that driven by the CMV promoter. All three cycles of mIFN expression over the course of two to three months were at high levels. Continuous MFP treatment resulted in sustained high-level expression of mIFN over two weeks. IP-10 levels after 3.5 months were about one-half to two-thirds less than in earlier MFP cycles, but was still well above the background IP-10 levels and also considerably higher than that generated by expression of mIFN from the CMV promoter, which also decreased over time with about the same kinetics (two-thirds of expression lost after 3.5 months). In total, this experiment demonstrates the capacity of the BRES-1 system for continuous, high-level target gene expression over several months, with the ability to be rapidly turned off and on again multiple times.

**Experimental Design:** Normal C57BI/6 mice were injected and electroporated with single-vector BRES-1 and CMV promoter mouse IFN expression plasmids, as follows. IFN expression was monitored for at least several months, using IP-10 as the endpoint biomarker for mIFN activity. MFP treatment and bleeds are designed to determine the kinetics of the "on" and "off" responses.

**DNA solutions:** Each mouse in all injected groups received 250 ug of plasmid DNA in 150 ul PBS.

**Table 27**

| **Group** | **plasmid** | **Description** | **n*** |
|---|---|---|---|
| 1 | pGER101 | CMV-mIFN | 10 |
| 2 | pGT26 | BRES-1/mIFN | 20 |

| | | | |
|---|---|---|---|
| *n=number of animals | | | |

**DNA delivery:** Adult C57BI/6 mice were injected bilaterally on day 0 with 250 ug plasmid DNA per mouse in 150 ul PBS. 25 ul of the DNA solution was injected into the tibialis muscle and 50 ul was injected into the gastrocnemius muscle of each hind leg, followed by electroporation with a caliper (8 pulses at 200 V/cm, 1 Hz, 20 msec/pulse).

**MFP treatment:** Group 2 was administered MFP by i.p. injection at 0.33 mg/kg (100 ul of 0.083 mg/ml in sesame oil, made fresh) as indicated in the table below.

**Schedule:** Cycle 1 (day 7-18): Determine the "off" kinetics. Cycle 2 (day 21-25): Determine the "on" kinetics.

**Table 28 Cycle 1**

| **Group/ plasmid** | **n*/ mouse #** | **day 0** | **day 7** | **day 7-10** | **day 11** | **day 12** |
|---|---|---|---|---|---|---|
| 1 CMV-mIFN | 10 101-110 | inject DNA | tail bleed A (101-105) | | tail bleed B (106-110) | |
| 2 BRES1-mIFN | 20 201-220 | inject DNA | tail bleed A (201-205) | MFP A-D (201-220) | tail bleed B (206-210) | tail bleed C (211-215) |

| | | | | | | |
|---|---|---|---|---|---|---|
| *n=number of animals | | | | | | |

**Table 29 Cycle 2**

| **Group/ plasmid** | **day 14** | **day 16** | **day 18** | **day 21-24** | **day 22** | **day 23** | **day 25** |
|---|---|---|---|---|---|---|---|
| 1 CMV-mIFN | tail bleed A | | tail bleed B | | tail bleed A | | tail bleed B |
| 2 BRES1-mIFN | tail bleed D (216-220) | tail bleed A | tail bleed B | MFP A-D | tail bleed C | tail bleed D | tail bleed A |

**Table 30: Cycle 3 Continuous MFP treatment**

| **Group/ plasmid** | **day 35** | **day 35-50** | **day 39** | **day 42** | **day 45** | day 51 |
|---|---|---|---|---|---|---|
| 1 CMV-mIFN | tail bleed B | | tail bleed A | | tail bleed B | terminal bleed and muscles A |
| 2 BRES1-mIFN | tail bleed A | MFP A-D | tail bleed B | tail bleed C | tail bleed D | terminal bleed and muscles A |

**Blood collection and Endpoint Analysis/Assay Procedure:** Mice were bled by tail nick or cardiac puncture (terminal bleed) at the time points indicated in the table above. Blood was collected in Microtainer tubes (no anti-coagulant) and centrifuged for separation and collection of serum. The serum from Groups 1 and 2 was assayed for IP-10 by ELISA.

**C. Bioequivalence Studies:** Pharmacokinetic studies can be conducted in normal mice and in one other species, preferably non-human primates, using the candidate vector and BRES-1 expression system, under delivery conditions, e.g., as established in the studies described above. "Bioequivalence" can be, but is not limited to, e.g., to demonstrate that the present regulated expression system of the present invention provides a superior pharmacokinetic profile for IFN-β gene-based delivery over IFN-β protein delivery in both animal models as defined, but not limited to, e.g., in the table below.

**Table 31: Non-Limiting Examples of Bioequivalence Criteria**

| **Criteria** | **Endpoint** |
|---|---|
| Administration | Clinically feasible for IM delivery |
| Expression Level | Equal to or greater than *therapeutic level achieved with bolus protein |
| Persistence of Expression | Greater than 3 months |
| Repeat Administration | Renewable expression upon repeat administration of vector and inducer |
| Continuous/Pulsatile Expression | Optimal MFP dose necessary to achieve and maintain *therapeutic level over time; renewable with MFP dosing. |

| | |
|---|---|
| *In a nonlimiting embodiment, "therapeutic level" in an animal model is defined as the systemic level of IFN-β (as determined, e.g., by ELISA and/or biomarker induction) that is equivalent to the level achieved by a therapeutic amount of bolus IFN-β1 a protein administered in humans on a mg/kg basis. | |

**D. Safety/Toxicity Studies:** If AAV-1 is selected as the candidate vector biodistribution studies following i.m. administration of the candidate vector can be performed. The endpoints will include vector DNA and expressed IFN-β RNA and protein distribution to target tissue (muscle), blood, lymph, heart, liver, kidney, lungs, male and female gonads (testis, ovary).

**E. Gene-Based Delivery for Treatment of a Disease or Condition:** An outcome from these studies is a gene-based delivery system for delivery of a therapeutic molecule (TM), e.g. a transgene encoding a therapeutic protein, for treatment of a disease or condition. In a preferred embodiment, the present invention provides a regulated expression system of delivery of an IFN-β transgene that will provide long term, regulated expression of IFN-β for the treatment of MS. In a preferred embodiment, an outcome of these studies is that the BRES-1 vector of the present invention can provide persistent, renewable expression (e.g., greater than 3 months) through the oral administration of the small molecule inducer, MFP; and is capable of repeat administration by intramuscular injection.

**i. Characterization of BRES-1 mIFN plasmid activity in EAE disease mice:** In order to show a biological effect in an animal disease model of MS, a study was performed in SJL mice with active EAE. Constitutive (pGER101, pmIFN) or inducible (pGT26, pBRES-1/mIFN) mIFN expression plasmids and null plasmid controls were injected with electroporation into the hind limb muscles of SJL mice. EAE was induced in the mice by injection of PLP 139-151/pertussis toxin the day before and after injection of pmIFN, and 7 and 9 days after the injection of pBRES-1/mIFN. Mice were treated with MFP (0.33 mg/kg) by i.p. injection once per day (d) or every third day (etd) after plasmid injection. Blood was collected at day 5 after injection. PBMCs were isolated from the blood and RNA was prepared from and assayed by RT-PCR to determine the level of Mx1 RNA (See "Experimental Design" below for details). The results show about an 8-fold increase in Mx1 RNA levels with pBRES-1/mIFN plus daily MFP injections, and no significant increase over a null vector in the absence of MFP (Figure 38). This demonstrates a biological response in an animal disease model similar to that which has been shown to be efficacious in this model.

**Experimental Design**: Female SJL mice (8 weeks old, Jackson Labs, -20 g) were distributed into 12 groups (n=13 per group), and each group was injected on Days 1 and 3 with PLP 139-151/pertussis, according to the established murine A-EAE protocol, as follows.

**Group 1,** Untreated. No treatment control.

**Groups 2-3,** CMV plasmid plus Electroporation (EP): Two bilateral intramuscular (im) injections of either Null (pgWiz) or mIFN (pGER101) CMV plasmid DNA into the tibialis (20 ul) and gastrocnemius muscles (40 ul), followed immediately by EP on Day 2, as administered.

**Groups 4-9,** BRES1 Plasmid plus EP: Two bilateral intramuscular (im) injections of either BRES-1 Null (pGT4) or BRES-1 mIFN (pGT26) plasmid DNA into the tibialis (20 ul) and gastrocnemius muscles (40 ul), followed by EP one week (Day -7) prior to initiation of disease, was administered. MFP (0.33 mg/kg) was administered daily or every third day (etd) by ip injection beginning on Day 1. The animals receiving MFP "etd" were dosed on Days 1, 4, 7, 10, 13, 16, 19, and 22.

**Groups 10-11,** Buffer Control, mIFN Protein: IFN protein buffer or mIFN protein (100,000 units = 500 ng) was administered every other day by sc injection, beginning on Day 1.

**Group 12,** Prednisolone: Prednisolone, daily bid was administered by intraperitoneal (ip) injection, beginning on Day 1.

**Table 32**

| **Group** | **# of mice** | **Agent** | **Dose** | **Volume/dose** |
|---|---|---|---|---|
| 1 **Untreated** | 13 | None | ------- | ------- |
| 2. **pNull/EP** | 13 | plasmid | 2 x 60 ug | 2 x 60 ul im |
| 3. **pmIFN/EP** | 13 | plasmid | 2 x 60 ug | 2 x 60 ul im |
| 4. **pBRES-1 Null/EP (-MFP)** | 13 | plasmid | 2 x 60 ug | 2 x 60 ul im |
| 5. **pBRES-1 Null/EP (+MFP daily)** | 13 | plasmid | 2 x 60 ug | 2 x 60 ul im |
| 6. **pBRES-1 Null/EP (+MFP etd)** | 13 | plasmid | 2 x 60 ug | 2 x 60 ul im |
| 7. **p pBRES-1 mlFN/EP (-MFP)** | 13 | plasmid | 2 x 60 ug | 2 x 60 ul im |
| 8. **pBRES-1 mIFN/EP (+MFP daily)** | 13 | plasmid | 2 x 60 ug | 2 x 60 ul im |
| 9. **pBRES-1 mIFN/EP (+MFP etd)** | 13 | plasmid | 2 x 60 ug | 2 x 60 ul im |
| 10. **Buffer control** | 10 | buffer | ------- | 100 ul/inj.* sc |
| 11. **mIFN protein** | 10 | protein | 100,000 U/inj.* | 100 ul/inj.* sc |
| 12. **Prednisolone** | 10 | compound | 2.5 mg/kg/inj.* | 100 ul/inj.* ip |

| | | | | |
|---|---|---|---|---|
| *inj.=injection | | | | |

**Table 33**

| **Group** | **plasmid** | **Injection day** | **DNA (mg)** | **mg/ml** | **ml** | **PBS (ml)** | **total vol (ml)** |
|---|---|---|---|---|---|---|---|
| **2** | pNull (pgWiz) | 2 | **1.8** | 5.32 | 0.34 | 1.46 | 1.8 |
| **3** | pmIFN (pgWiz/mIFN) (pGER101) | 2 | 1.8 | 5.36 | 0.34 | 1.46 | 1.8 |
| | | | | | | | |
| **4, 5, 6** | pBRES-1 Null (pGT4) | -7 | 1.8 x 3 = 5.4 | 6.35 | 0.85 | 4.55 | 1.8x3= 5.4 |
| **7,8,9** | pBRES-1 mIFN (pGT26) | -7 | 1.8x3= 5.4 | 5.28 | 1.02 | 4.38 | 1.8x3= 5.4 |

**Group 11** mIFN protein :100,000 units (500 ng) /100 ul inj. x 13 animals x 15 injections = 1.95x10⁷ units (97.5 ug)/19.5 ml. Stock mIFN solution = 100 µg/mL or 2x10⁷ units/mL. Dilution tubes contained 100 uL x 15 animals = 1.5 mL (a total of 15 dilution tubes were made containing 150 mM NaCl, 50 mM Sodium acetate, pH 5, 5% propylene glycol). 75 µL of stock solution was added to each tube. Final concentration = 10⁶ units/mL .

**Endpoint Analysis (Mx1 RNA analysis):** Three animals from groups 1-9 were sacrificed on Day 5 and terminally bled for Mx1 RNA analysis (using purple tubes containing EDTA), and the injected muscles harvested for IFN RNA analysis.

**F. Production:** The process for production of a gene therapy vector of the present invention comprising a BRES-1 hIFN-β expression cassette can be suitable for cGMP-manufacturing. Using methods described herein or known in the art, the BRES-1 vectors of the present invention can be made of sufficient purity, potency, and stability to perform preclinical development studies. The gene therapy vectors of the present invention, and preferably the BRES-1 vectors of the present invention, can be fully characterized with respect to the plasmid backbone, capsid (in the case of AAV as a delivery vector), transgene expression product (IFN-β), and inducer (MFP), using methods described herein or known in the art.

**G. Pharmacology:** Bioequivalence with protein delivery can be demonstrated in an animal model. Dose/response for vector and inducer or activator molecule (AM) (e.g., small molecule inducer MFP) can be characterized and optimized *in vivo.* Repeat administration and persistence of transgene expression can be fully characterized. Immunogenicity studies can be conducted with the candidate vector.

**H. Pharmacokinetics/safety/toxicology:** Pharmacokinetic studies of the expressed IFN-β transgene can be conducted using direct detection of the expressed transgene as well as measurement of IFN-β biomarkers. If AAV-1 is the selected candidate vector, biodistribution studies can be performed to examine the fate of the vector DNA and its expression products.

### Example 10: Identification of New Activator Molecules (AMs) for the Regulated Expression System

### A. Development of AM-RM LBD Complex

New, functionally selective PR ligand analogs for the regulated expression system of the present invention were identified by combining compound screening data with protein engineering to develop a unique AM-RM LBD complex. Proprietary antiprogestin-like steroidal compounds, many sharing significant structural similarity to MFP, were identified and tested using the pBRES-based luciferase screening assay. Structure-activity relationship (SAR) data derived from this directed screen were used to identify compounds which do not activate the pBRES expression system, presumably due to steric or electronic conflicts between the compound and the amino acids lining the ligand binding pocket (LBP) within the RM LBD. Compounds thus identified were further screened in a T47D cell based secondary assay to confirm that the pBRES-inactive compounds also lacked anti-PR activity. Starting with these inactive candidate compounds a functionally selective, i.e. orthogonal in respect to other physiological targets, AM-RM complex was developed using a modeling-guided LBD engineering approach to re-establish pBRES activation.

Since no structural data for the interaction between MFP-like compounds and the hPR-derived LBD were available, to better understand the molecular basis of the interaction between the RM LBD and the compounds tested in the directed screen, a homology model was developed for the MFP-RM LBD complex based on the X-ray crystal structure of the GR complexed with MFP (Kauppi B. et a/. (2003) J. Biol. Chem. 278: 22748-22754). The inactive compounds identified in the directed screen were docked into the model of the RM LBD and the conflicts between each compound and the LBP residues identified. A series of mutations aimed at alleviating the conflicts were modeled and installed into the RM LBP.

The ability of candidate compounds to activate the engineered pBRES expression system was measured in the pBRES-based luciferase assay. Those mutations that improved the interaction between previously inactive compounds and the altered RM LBD were docked into the model and used to propose further mutations designed to better fit the binding pocket to the new AM. Through two iterations of the mutagenesis and testing in cell-based assays cycle, a mutant RM LBD which is 39- fold more active in response to the new AM BLX-913 than the original RM was identified. These studies demonstrate the clinical applicability of the pBRES platform.

### B. Testing of PR Ligand Analogs

115 steroidal compounds with antiprogestin-like structural features were identified. In particular, compounds that showed structural similarity to Progesterone (e.g. *see* Figure 43D) and antiprogestin-like structure (e.g. *see* Figure 43A-C), and contained a bulky substituent at the 11β position, were tested for their ability to activate pBRES in the human MCF10A cell line, which was stably expressing the pBRES constructs, utilizing a firefly luciferase (Luc) reporter gene in place of a TM. The ability of the test compounds to stimulate Luc expression was compared to MFP at 25 nM. Compounds that failed to activate luciferase expression were examined and classified into roughly 3 classes of compounds: 1) those that did not satisfy the requirements for antiprogestin structure, which is required for effective RM activation; 2) those that featured drastic structural changes that would require extensive binding pocket mutagenesis, beyond the scope of this study; 3) those that featured conservative structural changes and retained significant structural similarity (i.e. were structural analogs) to more potent compounds. Eight class 3 compounds were retested in the pBRES-Luc assay in transiently transfected NIH 3T3 cells (Figure 41). Additionally these compounds were tested in the human T47D cell-based assay for anti-PR activity (Figure 42). The compound displaying the lowest level of activity in both assays was identified as BLX-913 (Figure 43A).

### Experimental Design: Stable MCF 10A cell line construction

Human mammary epithelial cell line MCF 10A (ATCC, # CRL-10317) was cultured in 10-cm dishes. Cells were transfected with pGT137 plasmid (pBRES-Luc, puromycin selection) using Fugene 6 transfection reagent (Roche). Briefly, 6 µg of DNA and 36 µL of Fugene 6 were diluted in 750 µL serum-free Opti-MEM. DNA and Fugene 6 were mixed and incubated at room temperature for 15 minutes. The cells were cultured for 2 days before screening with 1 µg/mL puromycin for 3 weeks. Positive clones were selected and verified by luciferase assay for pBRES-Luc expression and activation.

### Determination of pBRES Activation by Luciferase Activity Measurement

All cells were harvested by gentle trypsinization, followed by a wash with PBS. Cells were then spun for 5 min at 800 rpm and the cell pellet was resuspended in 10 ml tissue culture media. The cells were then seeded at a cell density of 1x10⁴ cells/well (volume of 100ul/well) in a 96-well flat-bottom, black tissue culture plate under sterile conditions. Cells were subsequently incubated at 37°C in 5% CO2 for 24 hours. Thereafter, under sterile conditions, the media was removed by careful aspiration and the wells replaced with 95ul fresh media, followed by the addition of 5ul/well of appropriately diluted compound or control buffer, resulting in final compound concentrations ranging from 0.00064 to 50 nM (in 0.05% DMSO final). Cell culture plates were then covered and incubated for 24 hr in a 37°C incubator. Thereafter, media was removed from the plates by gentle aspiration, the plates blotted dry, and the cells lysed with 50 ul/well of luciferase assay substrate reagent from the Luciferase Assay System Kit (Promega, Inc). Plates were then gently shaken for 1 min after addition of luciferase substrate reagent and luciferase enzyme activity subsequently measured at various times (5 minutes, 30 minutes, 60 minutes) using a Microbeta Luminescence Plate Reader (PerkinElmer) using a normalized luciferase reading protocol.

### C. BLX-913

Compound BLX-913 showed significant similarity to an advanced clinical development candidate Asoprisnil (Figure 43B). The most potent pBRES AM MFP is also shown for comparison (Figure 43C). The antiprogestin activity of MFP and BLX-913 is presented in Figure 44 and reveals that BLX-913 is a 30-fold less potent antiprogestin than MFP. The substantial similarity between BLX-913 and Asoprisnil suggested that the extensive safety, dosing and PK/PD data already accumulated for Asoprisnil during its clinical development would have direct parallels to the properties of the BLX-913 compound. The existence of a close analog in advanced clinical stages added to the value of the BLX-913 compound as a potential new pBRES AM.

### D. Construction of Homology Model

To identify the conflicts between the moieties of the BLX-913 compound and the amino acids within the LBP of RM that were responsible for the compound's inability to stimulate the pBRES expression system, a homology model of the RM LBD with BLX-913 was constructed. Since there was no available structure of the RM or its parent hPR LBD with any steroidal antiprogestin, the model of the RM LBD bound to MFP was first constructed (Figure 45), based on the X-ray crystal structure of the GR LBD bound to MFP. RM and GR LBDs share 44 % identity and 68 % similarity in amino acid sequence, thus the resulting model showed a low level of conformational strain on the LBD sidechains and the Root Mean Square Deviation (RMSD) of the conserved LBD amino acids between the RM model and the parent GR structure was less than 0.1 angstroms. The MFP ligand was replaced with the BLX-913 compound in the binding pocket of the RM LBD model. Tryptophan at position 755 (hPR numbering) was observed in conflict with the 11 β-benzaldoxime moiety of BLX-913 (Figure 46) and mutagenesis to alleviate this clash was initiated.

### E. Evaluation of the Effect of Site-Directed Mutations on Luciferase Activity Measurement

A panel of mutations at position 755 was installed and the resulting pBRES constructs evaluated in the Luciferase assay in transiently transfected HEK 293 cells. The RM protein harboring the W755A mutation showed approximately 4-fold improved activation in response to BLX-913 as compared to the original RM protein (Figure 47). The improved interface between the W755A RM LBP and BLX-913 was constructed within the homology model and closely examined for clues for further improving the interaction with additional mutations. Second round mutations were installed to better mold the LBP to the new AM in order to further improve the activity of the engineered RM- BLX-913 complex. The RM construct containing the V729L/W755A double mutation, pGT1017, showed 39-fold improved activity in the presence of BLX-913 as compared to the original RM protein (Figure 48). The response of the V729L/W755A RM mutant at increasing doses of BLX-913 was compared to the first round W755A RM mutant pGT1009 and the wt-pBRES pGT79 construct and confirmed that the V729L/W755A double mutant represents an improvement over original pBRES construct and the best performing first round mutant (Figure 49). To verify that the improved activity seen with the mutant RM proteins was not due to an increase in protein levels, HEK 293 cell lysates from the transient transfections (wt-pBRES pGT79, W755A RM pGT1009 or V729L/W755 RM pGT1017) were resolved on SDS-PAGE and visualized on Western blots using an antibody to the p65 AD (Santa Cruz, sc-372) (Figure 50). The LBD mutations did not affect RM protein levels within cells and thus the improved activation in response to BLX-913 was most likely due to improved interaction between the ligand and the LBD.

### Experimental Design: Primary pBRES-Luciferase assay in HEK 293 cells

To determine the effects of test compounds on pBRES activity in Human Embryonic Kidney 293 (HEK 293) cells (ATCC # CRL-1573), cells were plated in 60-mm dishes at 5x10⁵ cells/dish in 5 mL of MEM alpha with 4 mM L-glutamine and 10 % heat-inactivated FBS. Cells were transfected 24 hours later with pBRES constructs using Lipofectamine 2000 (Invitrogen) at 1:2.5 ratio of DNA (µg):Lipofectamine 2000 (µL) reagent. Medium containing lipid complexes was removed after 4 hours and cells allowed to recover in fresh growth medium for 24 hours. Cells were reseeded into 96-well plates at 10,000 cells/well in growth medium. The next day cells were treated with MFP or test compound using the same medium. Compounds were dissolved in 100 % ethanol vehicle and diluted by using the treatment medium. The final concentration of ethanol in treatment medium was 0.1 %. Each treatment consisted of three replicates. Twenty four hours after treatment, luciferase activity was measured as follows: medium was removed and cells were washed once with Dulbecco's PBS. Cells were lysed with 20 µL/well of 1x Passive Lysis Buffer (PLB, Promega) and incubated at room temperature for 10 minutes with shaking on a titer plate shaker. Plates were frozen at -80 °C for 20 minutes and were thawed at room temperature for 20 minutes with shaking in a titer plate shaker. Luciferase activity was measured using the Promega Luciferase Assay System (Cat. # E1501) substrate (100 µL/well)) using a Berthold MicroLumat Plus luminometer.

### Secondary PR assay in T47D cells

To determine the effects of test compounds on the human Progesterone receptor-induced alkaline phosphatase activity in human breast carcinoma T47D cells (ATCC # HTB-133), cells were plated in 96-well plates at 20,000 cells/well in growth medium (RPMI1640 with 1 mM sodium pyruvate, 0.2 units/mL insulin, 4 mM L-glutamine and 10 % FBS). After overnight culture, the medium was changed to the assay medium (RPMI1640 (phenol red-free) with 1 mM sodium pyruvate, 0.2 units/mL insulin, 4 mM L-glutamine and 3 % charcoal-stripped FBS). The next day, cells were treated with MFP or test compounds in the presence of 200 pM Promegestone. Compounds were dissolved in 100 % ethanol vehicle and diluted using the assay medium. The final concentration of ethanol in assay medium was 0.2 %. Each treatment consisted of 5 to 6 replicates. Twenty four hours after treatment, cellular alkaline phosphatase activity was measured as follows: the medium was removed and cells were washed twice with DPBS. After residual DPBS was removed, 50 µL of cell lysis buffer (100 mM Tris-HCl pH 9.8, 0.2 % Triton X-100) was added to each well and plates were frozen at -80 °C for 30 minutes. The plates were thawed at room temperature for 20 minutes with shaking on a titer plate shaker. 150 µL of substrate buffer (100 mM Tris-HCl pH 9.8, 4 mM p-nitrophenyl phosphate) was added to each well. Absorbance measurements were taken at 5 minute intervals for 1 hour at the test wavelength of 405 nm.

### Homology model of the RM LBD

The homology model of the RM LBD based on the GR LBD/MFP complex was created using the SwissModel feature of DeepView Swiss PDB Viewer ver. 3.70 (Guex, N. & Peitsch, M.C. (1997) Electrophoresis 18: 2714-2723. http://expasy.org/spdbv/). Briefly, the primary amino acids sequence corresponding to residues 640-914 of the human Progesterone receptor (Accession NP_000917) was fit onto the X-ray crystal structure of the human Glucocorticoid receptor 1 NHZ.pdb and was submitted to the Swiss-Prot server for minimization. The fit of the resulting model to the GR/MFP crystal structure was examined using the Insight2 software (Accelrys Software Inc.).

### Modeling of RM LBD-compound interactions

Models of MFP, BLX-913 and other test compounds bound in the RM ligand binding pocket were created using the Discovery Studio Viewer Pro ver. 6.0 (Accelrys Software Inc.) and the active site residues identified via the rlig_view script (Dr. Marc Adler, Biophysics Department, Berlex, unpublished).

### Site-directed mutagenesis

Site-directed mutations within the RM LBD were installed using the Quick Change mutagenesis approach using the following primers with the newly installed codon underlined. The amino acid numbering scheme follows hPR amino acid numbering. Single mutations at positions 719 and 755 were installed using the pGT79 template. Double mutations at positions 729 and 726 were installed using the pGT1009 (W755A) template.

N719Q (pGT1003) (SEQ ID NO: 60) 5'- CTT TGC TGA CAA GTC TTC AGC AAC TAG GCG
AGA GG -3' and 5'- CCT CTC GCC TAG TTG CTG AAG ACT TGT CAG CAA AG -3';

N719A (pGT1004) (SEQ ID NO: 62) 5'- CTT TGC TGA CAA GTC TTG CCC AAC TAG GCG
AGA GG -3' and 5'- CCT CTC GCC TAG TTG GGC AAG ACT TGT CAG CAA AG -3';

N719G (pGT1005) (SEQ ID NO: 64) 5'- CTT TGC TGA CAA GTC TTG GCC AAC TAG GCG
AGA GG -3' and 5'- CCT CTC GCC TAG TTG GCC AAG ACT TGT CAG CAA AG -3';

W755F (pGT1006) (SEQ ID NO: 66 5'- CTC TCA TTC AGT ATT CTT TCA TGA GCT TAA
TGG TG -3' and 5'- CAC CAT TAA GCT CAT GAA AGA ATA CTG AAT GAG AG -3';

W755L (pGT1007) (SEQ ID NO: 68) 5'- CTC TCA TTC AGT ATT CTC TGA TGA GCT TAA
TGG TG -3' and 5'- CAC CAT TAA GCT CAT CAG AGA ATA CTG AAT GAG AG -3';

W755V (pGT1008) (SEQ ID NO: 70) 5'- CTC TCA TTC AGT ATT CTG TGA TGA GCT TAA
TGG TG -3' and 5'- CAC CAT TAA GCT CAT CAC AGA ATA CTG AAT GAG AG -3';

W755A (pGT1009) (SEQ ID NO: 72) 5'- CTC TCA TTC AGT ATT CTG CCA TGA GCT TAA
TGG TG -3' and 5'- CAC CAT TAA GCT CAT GGC AGA ATA CTG AAT GAG AG -3';

V729A/W755A (pGT1015) (SEQ ID NO: 74) 5'- GAG GCA ACT TCT TTC AGC CGT CAA
GTG GTC TAA ATC -3' and 5'- GAT TTA GAC CAC TTG ACG GCT GAA AGA AGT TGC
CTC -3';

V729G/W755A (pGT1016) (SEQ ID NO: 76) 5'- GAG GCA ACT TCT TTC AGG CGT CAA
GTG GTC TAA ATC -3' and 5'- GAT TTA GAC CAC TTG ACG CCT GAA AGA AGT TGC
CTC -3';

V729L/W755A (pGT1017) (SEQ ID NO: 78) 5'- GAG GCA ACT TCT TTC ACT GGT CAA
GTG GTC TAA ATC -3' and 5'- GAT TTA GAC CAC TTG ACC AGT GAA AGA AGT TGC
CTC -3'.

V729F/W755A (pGT1025) (SEQ ID NO: 80) 5'- GAG GCA ACT TCT TTC ATT CGT CAA
GTG GTC TAA ATC -3' and 5'- GAT TTA GAC CAC TTG ACG AAT GAA AGA AGT TGC
CTC -3';

L726V/W755A (pGT1020) (SEQ ID NO: 82) 5'- GGC GAG AGG CAA GTG CTT TCA GTA
GTC AAG -3' and 5'- CTT GAC TAC TGA AAG CAC TTG CCT CTC GCC -3';

L726A/W755A (pGT1021) (SEQ ID NO: 84) 5'- GGC GAG AGG CAA GCC CTT TCA GTA
GTC AAG -3' and 5'- CTT GAC TAC TGA AAG GGC TTG CCT CTC GCC -3';

L726G/W755A (pGT1022) (SEQ ID NO: 86) 5'- GGC GAG AGG CAA GGG CTT TCA GTA
GTC AAG -3' and 5'- CTT GAC TAC TGA AAG CCC TTG CCT CTC GCC -3';

L726I/W755A (pGT1023) (SEQ ID NO: 88) 5'- GGC GAG AGG CAA ATC CTT TCA GTA
GTC AAG -3' and 5'- CTT GAC TAC TGA AAG GAT TTG CCT CTC GCC -3';

L726F/W755A (pGT1024) (SEQ ID NO: 90) 5'- GGC GAG AGG CAA TTC CTT TCA GTA
GTC AAG -3' and 5'- CTT GAC TAC TGA AAG GAA TTG CCT CTC GCC -3';

### Example 11: Third Round Mutations

Every round of mutagenesis and the subsequent evaluation in cell-based assays contributes to our understanding of the SAR between RM ligands and the amino acids lining the binding pocket. Third round mutations that take into account the results from first round W755A and second round V729L/W755A mutants and are designed to further modulate the RM LBP have been installed: V729F/W755A, L726V/W755A, L726A/W755A, L726G/w755A, L7261/W755A and L726F/W755A. Contributions from individual mutations are being evaluated and where appropriate, are combined into a single construct to obtain the best BLX-913-engineered RM ligand-receptor pair. Since simply enlarging a portion of the binding pocket to accommodate a bulky compound is not sufficient to yield a potent new AM-RM complex, careful molding of the pocket to the ligand to minimize unoccupied space and thus exclude any adventitious water is required.

Further improvements to the unique AM-RM complex may be accomplished in combination with a chemistry-based diversification approach to generate analogs of BLX-913. More potent AM-RM ligand-receptor pairs may be developed using bulkier (i.e. more selective) derivatives of BLX-913 at the 11-position. Since the orientation and the required compensatory mutations for the aldoxime moiety at position 11 of the RM-bound ligand has been identified and validated, more substantial disruptive groups can be installed to ensure absolute selectivity against the parent PR. The larger substituents on the AM can be accommodated via the modeling-directed mutagenesis approach to yield a highly selective AM with no unwanted physiological effects. Additionally, the combined chemical synthesis-mutagenesis approach can produce analogs that take advantage of naturally occurring or synthetically introduced hydrogen bonding networks. Other regions of the ligand-RM interface, besides position 11, may also be amenable to chemical modification and commensurate mutagenesis. Existing MFP analogs with modifications at positions 15 and 16 (Figure 13) have been identified and screened for antiprogestin activity (Figure 14). One of these compounds, BLX-899, has approximately 5-fold weaker anti-PR activity than MFP in the T47D cell-based assay. This compound was also tested in the pBRES-Luc assay and demonstrated 2-fold weaker induction as compared to MFP. The compound was modeled into the RM LBD homology model and conflicts between the methyl group at position 16 and the binding pocket residues were identified. Side chains of amino acids Leu 887 and Leu 797 define the topology of the RM LBP in the region where positions 15 and 16 of the ligand bind. The leucine residues may be scaled back to valine, alanine or glycine to accommodate the structural bump on BLX-899 to restore pBRES activation. Any improvement in the stimulation of the pBRES construct containing position 887 and/or 797 mutants in response to BLX-899 is not significant enough on its own for use as a functionally selective regulated expression system, but would prompt a chemical synthetic approach to incorporate both the position 15 methyl group and the 11 β-benzaldoxime moiety in a single molecule. W755A and V729L mutations may be installed alongside position 887 and/or 797 mutations in a single pBRES construct to accommodate the new ligand with two selectivity features.

In a similar approach to the position 15 and 16 analogs, compounds with modifications at positions 4 and 7 (Figure 15) have been tested for antiprogestin activity in the T47D cell-based assay (Figure 16). Compounds BLX-952 and BLX-610 are much weaker antiprogestins than MFP or any other compound tested to date: even at 100 nM concentrations, these two compounds are unable to completely inhibit the progesterone receptor in this assay. If this weak antiprogestin activity successfully correlates to weak pBRES activation, conflicts within the binding site may be identified and ablated. The hydroxyl and ester moieties at position 7 appear to be in conflict with Met 756 within the LBD of RM homology model. This methionine may be scaled back to an alanine to introduce a simple hole in order to accommodate the bumps on the ligand, or installation of a more advanced hydrogen bonding network may be attempted using aspartic or glutamic acids. Any beneficial compound-mutant RM interactions discovered for individual positions around the binding pocket may be combined into a single system via chemical synthesis of the ligand and site-directed mutagenesis of the binding pocket. This combination of several individual specificity-enhancing elements into a single RM-AM complex may exponentially improve selectivity against the PR and other endogenous proteins.

The current 39-fold selective AM-RM complex will be tested in animal model studies. The previous animal model work carried out during the optimization of the pBRES expression system provides a robust protocol for the testing as well as the criteria to be achieved by a successful new AM-RM regulated gene expression system. Existing animal models include live imaging of Secreted Alkaline Phosphatase (SEAP) or luciferase reporter genes in C57BU6 mice and the Experimental Autoimmune Encephalopathy (EAE) disease model for Multiple Sclerosis (MS) in SJL mice. Briefly, pBRES DNA is delivered via intramuscular (i.m.) injection into the tibialis and the gastrocnemius muscles of each hind leg followed by electroporation with caliper electrodes (Inovio, San Diego CA, 8 pulses at 200 V/cm, 1 Hz, 20 msec/pulse). AM (MFP or BLX-913) is delivered via i.p. injection at 0.33-1.00 mg/kg in sesame oil on days 7-10. Expression of SEAP or luciferase reporter genes is monitored via live imaging of mouse hind limbs and allows comparison of the pBRES/MFP and V729L W755A pBRES/ BLX-913 regulated gene expression systems. The murine EAE disease model allows further characterization of the new pBRES/ BLX-913 complex in the context of existing efficacy data for the pBRES/MFP complex.

**Summary:** An advantage of the new AMs and IMs of the present invention is that they selectively bind to a unique RM having modifications that enhance this selectivity. Therefore, the new AMs and IMs of the present invention have improved properties that selectively bind or otherwise interact with a unique and/or specific RM of the present invention, and diminish or eliminate cross reactivity with endogenous proteins, particularly endogenous human proteins, e.g., an endogenous PR or other endogenous steroid receptors. In some embodiments, a new AM or IM of the present invention is a PR ligand analog, more particularly a modified antiprogestin, e.g., a modified MFP. In one embodiment, the PR ligand analog selectively binds to an RM having a modified LBP, and has diminished or no side effects relating to e.g., cross reactivity with endogenous PR or other endogenous steroid receptors, abortifacient activity, or contraceptive activity.

In certain embodiments where a modified MFP is the AM, the dose of the MFP used to activate an RM of the present invention is 100 fold lower than the report abortifacient activity of this compound. However, the present invention also provides new AMs that are selective and specific to a unique and new RM such that the new AM has little or no pharmacological effect on an endogenous protein within the body, e.g., a PR and glucocorticod receptor (GR). As described herein, compounds were identified that have little or no activity against the PR *in vitro* (e.g., as measured in the T47D-based secondary assay). In one embodiment, where the PR and RM Ligand Binding Pockets (LBPs) are identical, a compound that is found to be inactive against the PR is also inactive at stimulating the pBRES system, which is a non-limiting aspect of this embodiment. In some embodiments, the level of activity of a newly identified compound in the pBRES system can be used as a source of SAR, but is not limited herein as a selection criterion.

As described herein, in some embodiments, the pBRES-Luc (pBRES-luciferase) assay can be used to identify inactive compounds in a directed screen. In one embodiment, the correlation between pBRES activation and antiprogestin activities, was used to identify compounds which do not activate the pBRES system, and also likely lack antiprogestin activity. The lack of antiprogestin activity was confirmed for each promising compound in the T47D-based assay for antiprogestin activity. Therefore, the data from this secondary anti-PR assay was used to confirm the identity of the promising compounds of the present invention. In this embodiment, the original RM (pGT79) construct was used as the control in the luciferase assays of the engineered RMs with new AM candidates, to provide a point of reference regarding improvements to the induction levels in the engineered RMs in response to a novel AM compound, which has poor activity against the original RM and more importantly against the PR. The equally-inactive original RM was included as a reference or control.

The above approach can be used to identify compounds that do not bind or inhibit the human PR due to a structural feature on the compound. Thereafter, an engineered RM molecule can be developed to accept this new compound as the AM. Using this approach, a key feature of the engineered system is that the AM compound does not interact with PR. Further, the endogenous steroids do not activate the engineered RM due to a 19 amino acid truncation of the LBD within the RM as compared to hPR. Once a lead compound is identified for further characterization, it can be screened using in *in vitro,* cell-based and *in vivo* assays as described herein or in Fuhrmann, U., et al. (2000) J. Med. Chem. 43: 5010-5016.

Figure 55 illustrates an exemplary and non-limiting embodiment of the pBRES expression system. In this embodiment, binding of MFP to the RM activates the RM and results in the subsequent expression of the TM from the pBRES expression system. MFP is a known antagonist for several endogenous human steroid receptors, which can potentially result in unwanted side effects.

Figure 56 illustrates an exemplary and non-limiting approach to screening for novel activator molecules and regulator molecules. (1) New AMs are identified from a directed screen of PR ligand analogs that do not interact with the endogenous human PR due to the presence of steric or ionic perturbations on the compound. (2) Modeling guided mutagenesis is used to introduce compensatory mutations into the LBP of the RM, thereby creating engineered RM molecules. (3) The new AM is used in conjunction with the engineered RM to produce an active AM-RM complex with improved properties e.g., a diminution or elimination of unwanted side effects on or cross reaction with endogenous proteins, particularly endogenous human proteins, e.g., endogenous human steroid receptors.

Figure 57 illustrates an exemplary and non-limiting approach for developing a highly a selective AM-RM complex using the SAR from two or more moderately selective AM-RM pairs. (1) New AM1 is identified using approach outlined in Fig. 57 and compensatory mutations are installed in region 1 within the LBP of the RM to create a moderately selective AM1-RM1 complex. **(2)** New AM2 is identified using approach outlined in Fig. 57 and compensatory mutations are made in region 2 within the LBP of the RM to create a moderately selective AM2-RM2 complex. **(3)** Using chemical synthesis, selectivity-imparting modifications from AM1 and AM2 are combined in one compound, AM3. The compensatory mutations in regions 1 and 2 within the LBP are combined within a single RM3 construct using site-directed mutagenesis. Taking advantage of two or more selectivity-imparting features, AM3 is used in conjunction with RM3 to yield a AM-RM complex with greater selectivity as compared to AM1 or AM2.

### MATERIALS AND METHODS

**A. Efficacy of Gene-Based Delivery of Murine IFN-β Protein in Mouse Acute EAE**: Seventy 8-week old female SJL mice from Jackson Labs were immunized with a 0.1 ml SC (divided between base of tail and upper back) injection containing 150 ug Proteolipid Protein (PLP)139-151 in Incomplete Freund's Adjuvant (IFA) supplemented with 200 ug M. tuberculosis H37Ra. This emulsion was obtained by mixing saline containing 3 mg/ml PLP 1:1 with IFA containing 4 mg/ml ground M. tuberculosis. Immediately after immunization, all mice received a 0.1 ml IP injection of pertussis toxin. Two days after immunization (day 3 of study), all mice received a second IP injection of pertussis toxin.

Mice treated with IFN-β protein or its vehicle (20 nM NaAc, pH 5.5, 150 mM NaCl, 5% propylene glycol) were dosed with 0.1 ml, SC once every other day beginning on the day of immunization until the end of the study. The positive controls used for this study were 9 mg/kg Mesopram (ZK-117137) and 2.5 mg/kg Prednisolone. Both controls use a dose volume of 0.1 ml/injection and are administered IP, twice daily, beginning on the morning of immunizations until the end of the study.

### Experimental Groups (n=10):

1. Vehicle
2. 10 K units murine IFN-β
3. 20 K units murine IFN-β
4. 30 K units murine IFN-β
5. 100 K units murine IFN-β
6. Mesopram, 9 mg/kg IP
7. Prednisolone, 2.5 mg/kg IP

**Clinical Scoring of Mouse Acute EAE**: The mice were scored daily based on the following scoring system:
0 = normal
1 = limp tail
2 = difficulty righting
3 = incomplete paralysis of one or both hind limbs
4 = complete paralysis of one or both hind limbs, or hind limbs mobile but drag
5 = complete paralysis of both hind limbs & weakness/paralysis of forelimbs, moribund, or dead

Moribund mice were euthanized. One half scores are added to mice exhibiting borderline clinical symptoms. Mice treated with 100K units of IFN-β developed significantly decreased clinical scores of EAE compared with vehicle treated mice (p=0.0046). Mice treated with 30K units of IFN-β also developed decreased clinical scores compared to vehicle treated mice, although this decrease did not reach statistical significance. The positive controls in this study, Mesopram and Prednisolone, also significantly decreased clinical scores. See Example 5 and Figure 13.

**B. Efficacy of Gene-Based Delivery of Murine IFN-β in Mouse Acute EAE:** One hundred thirty 8-week old female SJL mice from Jackson Labs were immunized with a 0.1 ml SC (divided between base of tail and upper back) injection containing 150 ug Proteolipid Protein (PLP)139-151 in Incomplete Freunds Adjuvant (IFA) supplemented with 200 ug M. tuberculosis H37Ra. This emulsion was obtained by mixing saline containing 3 mg/ml PLP 1:1 with IFA containing 4 mg/ml ground M. tuberculosis. Immediately after immunization, all mice received a 0.1 ml IP injection of pertussis toxin. On day 2, mice in the plasmid + electroporation groups received appropriate intramuscular injections followed immediately by electroporation. Mice in the plasmid + PINC groups also received the appropriate intramuscular injections. Two days after immunization (day 3 of study) all mice received a second 0.1 ml IP injection of pertussis toxin. On day 5, mice in the plasmid + PINC groups received the same treatment as on day 2.

Mice treated with IFN-β protein or its vehicle (20 nM NaAc, pH 5.5, 150 mM NaCl, 5% propylene glycol) were dosed with 0.1 ml, sc once every other day beginning on the day of immunization until the end of the study. The positive controls used for this study were 9 mg/kg Mesopram (ZK-117137) and 2.5 mg/kg Prednisolone. Both controls use a dose volume of 0.1 ml/injection and both controls are administered IP, twice daily, beginning on the morning of immunizations until the end of the study.

There were a total of 10 groups in this study. Each group had 13 mice. The last 3 mice in each group were bled via tail nick on day 6 of the study for Mx 1 RNA analysis. The same 3 animals that were bled on day 6 were bled via cardiac puncture on day 13 of the study for Mx1 RNA analysis from PBMC's, and injected muscles were collected for analysis.

### Experimental Groups (n=13):

1. PBS control
2. pNull + EP
3. pmIFN-β + EP
4. pNull + PINC
5. pmIFN-β + PINC
6. IFN-β protein (100K units) SC
7. Vehicle, SC
8. Mesopram, 9 mg/kg IP
9. Prednisolone, 2.5 mg/kg IP
10. Untreated

**Clinical Scoring of EAE**: The mice were scored daily based on the following scoring system:
0 = normal
1 = limp tail
2 = difficulty righting
3 = incomplete paralysis of one or both hind limbs
4 = complete paralysis of one or both hind limbs, or hind limbs mobile but drag
5 = complete paralysis of both hind limbs & weakness/paralysis of forelimbs, moribund, or dead

Moribund mice are euthanized. One half scores are added to mice exhibiting borderline clinical symptoms. Mice treated with 100K units of murine IFN-β protein had significantly decreased clinical scores of EAE, compared to the vehicle control treated mice (p=0.045). Gene delivery of the murine IFN-β + EP also significantly decreased clinical scores, compared to gene delivery of pNull & EP (p= 0.0171). Gene delivery using the PINC formulation of IFN-β did not statistically decrease clinical scores compared to pNull & PINC. Both Mesopram and Prednisolone, the positive controls for the EAE model, significantly decreased clinical scores.

### C. Regulated Expression of mIFN-β In Vivo

**IFN15-GSS: In vivo transfection of BRES-1/IFN plasmids:** Demonstration of mifepristone (MFP)-regulated murine interferon-beta (mIFN-β) expression from a BRES-1/mIFN-β plasmid electroporated into mouse muscle.

**Experimental Design**: Normal C57BI/6 mice can be injected and electroporated with a BRES-1 single vector of the present invention and control plasmid DNAs as described in Tables 34 and 35 below.

**Table 34**

| **Plasmid** | **Description** | **date** | **ug/ul** |
|---|---|---|---|
| pGT4 | Empty BRES-1 vector. Negative control for pGT26. | 4/23/04 | 6.35 |
| pGT26 | RM/mIFN-β reverse. Experimental | 4/14/04 | 4.73 |
| | BRES-1/mIFN-β plasmid. | 4/16/04 | 4.80 |
| pGER101 | pgWiz/mIFN-β (CMV/mIFN-β). Positive control for mIFN-β expression. | 2/18/04 | 5.73 |
| pGT31 | SEAP/RM. Positive control for RM function. | 4/23/04 | 5.78 |

**Table 35 Groups (n = 5/group)**

| **Group** | **plasmid** | **time points** | | |
|---|---|---|---|---|
| | | **day 7** | **day 11** | **day 18** |
| **1** | **none** | - MFP | | |
| **2** | **pGT4** | - MFP | + MFP | |
| **3** | **pGT26** | - MFP | | |
| **4** | **pGER101** | - MFP | | |
| **5** | **pGT26** | | - MFP | |
| **6** | **pGT26** | | + MFP | |
| **7** | **pGER101** | | - MFP | |
| **8** | **pGT26** | | - MFP | - MFP |
| **9** | **pGT26** | | + MFP | - MFP |
| **10** | **pGER101** | | - MFP | - MFP |
| **11** | **pGER101** | | + MFP | |
| **12** | **pGT31** | - MFP | + MFP | - MFP |
| **13** | **none** | | - MFP | |

mIFN-β expression can be assayed by biomarkers and RNA levels in muscle at 3 time points. On day 7 after DNA injection Groups 1, 3, and 4 can be terminally harvested and Group 2 can be tail bled to determine uninduced background mIFN-β expression and biomarker activity in mice receiving GS/mIFN - MFP (Group 3) in comparison to uninjected mice (Group 1) and mice receiving empty BRES-1 vector (Group 2). CMV/mIFN (Group 4) serves as a positive control. Group 12 can be tail bled to determine uninduced background levels of SEAP expression.

Mice in Groups 2, 6, 9, 11, and 12 can be treated with MFP on days 7-10 after DNA injection. On day 11, Groups 5-7 can be terminally harvested to determine induced mIFN expression and biomarker activity in mice receiving RM/mIFN + MFP (Group 6) in comparison to mice receiving CMV/mIFN (Group 7). Uninduced levels in mice receiving RM/mIFN - MFP (Group 5) will also be assayed. Group 2 (empty BRES-1 vector) can be terminally bled to determine whether MFP stimulates the biomarker response. Groups 8-10 can be tail bled to determine if biomarker activity is detectable from small volumes of blood and to provide an induced time point in the same mice with which to compare the uninduced levels on day 18. Group 11 (CMV/mIFN + MFP) can be terminally harvested to determine whether MFP affects mIFN expression or inhibits the biomarker response. Group 12 can be tail bled to determine induced levels of SEAP expression. Group 13 will provide a negative control for SEAP expression.

On day 18, eight days after the last MFP treatment, Groups 8-10 can be terminally harvested to determine if the mIFN-β RNA levels and biomarker activity in the RM/mIFN -/+/- MFP group (Group 9) have returned to baseline in comparison to RM/mIFN mice that never received MFP (Group 8). CMV/mIFN (Group 10) again serves as a positive control. Group 12 can be terminally bled to determine if SEAP expression has returned to baseline.

### D. Reagents

**DNA solutions:** Each mouse in Groups 2-11 can receive 250 ug of plasmid DNA in 150 ul PBS. Each mouse in Group 12 can receive 25 ug of plasmid DNA in 150 ul PBS (see Table 36 below).

**Table 36: Prepare DNA solutions for 5 mice/group plus extra**

| **Group** | **Plasmid** | **# of mice** | **Prep solution for** | **mg DNA** | **mg/ml** | **DNA** | **total ml** | **PBS** |
|---|---|---|---|---|---|---|---|---|
| 2 | pGT4 | 5 | 8 mice | 2.0 | 6.35 | 315 ul | 1.2 | 0.89 ml |
| 3, 5, 6, 8, 9 | pGT26 | 25 | 32 mice | 8.0 | 4.73 4.80 | 1.2 ml 0.48 ml | 4.8 | 3.12 ml |
| 4, 7, 10, 11 | pGER101 | 20 | 25 mice | 6.25 | 5.73 | 1.09 | 3.75 | 2.66 ml |
| 12 | pGT31 | 5 | 8 mice | 0.2 | 5.78 | 35 ul | 1.2 | 1.17 ml |

### E. Animal Procedure

**1) DNA delivery (Groups 2-12)**: Adult male C57BI/6 mice (5 per group) can be injected bilaterally on day 0 with 250 ug (Groups 2-11) or 25 ug (Group 12) plasmid DNA per mouse in 150 ul PBS. The DNA solution can be injected 25 ul into the tibialis muscle and 50 ul into the gastrocnemius muscle of each hind leg, followed by electroporation with a caliper (8 pulses at 200 V/cm, 1 Hz, 20 msec/pulse).

**2) MFP treatment (Groups 2, 6, 9, 11, and 12)**: Mice in Groups 2, 6, 9, 11, and 12 can be administered MFP by oral gavage at 0.33 mg/kg (100 ul of 0.083 mg/ml in sesame oil, made fresh) on days 7 through 10 post-injection as indicated in Table 37 below. Group 12 mice can be bled prior to MFP treatment on day 7.

**Table 37**

| **Group** | **day 7** | **day 8** | **day 9** | **day 10** | **day 11** | **day 18** |
|---|---|---|---|---|---|---|
| **1) uninjected** | Terminal bleed + muscles | | | | | |
| **2) empty vector (pGT4)** | tail bleed, then MFP | MFP | MFP | MFP | terminal bleed + muscles | |
| **3) RM/mIFN (pGT26) - MFP** | Terminal bleed + muscles | | | | | |
| **4) CMV/mIFN (pGER101)** | Terminal bleed + muscles | | | | | |
| **5) RM/mIFN (pGT26) - MFP** | | | | | terminal bleed + muscles | |
| **6) RM/mIFN (pGT26) -/+ MFP** | MFP | MFP | MFP | MFP | terminal bleed + muscles | |
| **7) CMV/mIFN (pGER101)** | | | | | terminal bleed + muscles | |
| **8) RM/mIFN (pGT26) - MFP** | | | | | tail bleed | terminal bleed + muscles |
| **9) RM/mIFN (pGT26) -/+/- MFP** | MFP | MFP | MFP | MFP | tail bleed | terminal bleed + muscles |
| **10) CMV/mIFN (pGER101)** | | | | | tail bleed | terminal bleed + muscles |
| **11) CMV/mIFN (pGER101) -/+ MFP** | MFP | MFP | MFP | MFP | terminal bleed + muscles | |
| **12) SEAP/RM (pGT31)* -/+/- MFP** | tail bleed, then MFP | MFP | MFP | MFP | tail bleed | terminal bleed |
| **13) uninjected** | | | | | terminal bleed | |

| | | | | | | |
|---|---|---|---|---|---|---|
| *pGT31 was constructed by digestion of pGER75 (CMV/SEAP) with Nhe I and Not I, and insertion of the resulting fragment carrying the SEAP gene between the Spe I and Not I sites of pGT1. | | | | | | |

**3) Harvest of blood and muscle (Groups 1-11):** On the appropriate day after DNA injection as indicated in Table 6 above, mice can be tail bled or terminally bled. When mice are terminally bled, the injected muscles can be collected.

**Blood**. Blood can be collected into Microtainer tubes (containing EDTA) at RT and then PBMCs can be separated and collected. The leftover plasma can be stored at -20 °C for cytokine assays.

**Muscle**: The injected muscles of both legs can be harvested, pooled together, and cut into pieces no larger than 5 mm on one side. Approximately one-fourth of the chopped muscle can be placed into 1.5 ml of RNA-Later solution in a 2 ml tube. The remainder of the muscle can be stored at - 70 °C. The DNA and RNA can be extracted from the muscle samples in RNA-Later solution. The samples can be stored at 4 °C for at least 24 h and then transferred to -20 °C if they can be stored for more than 5 days.

**Blood (Groups 12 and 13):** Mice in Group 12 can be tail bled on day 7 and day 11 and terminally bled on day 18 into yellow Microtainer tubes (no anti-coagulant). The mice can be bled prior to MFP treatment on day 7. Mice in Group 13 can be terminally bled on day 11 into yellow Microtainer tubes (no anti-coagulant).

### 4) Endpoint Analysis/Assay Procedure

**Results of Biomarker Assays**: The Mx1 RNA and both chemokines (IP-10 and JE) showed little or no activity with BRES-1-mIFN-β (pGT26) in the absence of MFP at 7 days. All biomarkers were strongly induced, to levels higher than with CMV-mIFN-β, in the presence of MFP at 11 days. At 18 days, in the absence of MFP, the chemokine levels had returned to baseline and the Mx1 RNA had decreased nearly to baseline. See Figures 18 and 19.

**Mx1 RNA from PBMC:** RNA can be prepared from the separated PBMC's and assayed for Mx1 RNA by TaqMan.

**JE and IP-10 protein from plasma**: The plasma can be assayed for JE and IP-10 cytokines by ELISA.

**mIFN-β RNA from muscle**: RNA can be prepared from the injected muscles and assayed for mIFN-β RNA by TaqMan.

**Plasmid DNA from muscle**: DNA can be prepared from the injected muscles and assayed for plasmid DNA by TaqMan. Primers and probe specific for the CMV promoter can be used for DNA from Groups 4, 7, 10, and 11. Primers and probe specific for the GAL-4 DNA binding domain of the regulator protein can be used for Groups 2, 3, 5, 6, 8, and 9.

**SEAP protein from serum:** The serum can be assayed for SEAP expression by the chemiluminescent activity assay, using the serum from the Group 13 mice as a diluent.

### F. Construction of Plasmid Vectors

**pGER101 (pgWiz/mIFN):** The mouse IFN-β (mIFN-β) gene was amplified by PCR from the plasmid vector pbSER189 (Figure 20A) with the mIFN signal sequence placed on the 5' primer and Sal I and Not I restriction enzyme sites added at the 5' and 3' ends. The fragment was digested with Sal I and Not I and inserted into the Sal I and Not I sites of plasmid vector pgWIZ (Figure 20B) resulting in plasmid vector pGER101 (Figure 20C).

**pGER125 (pgWiz/hIFN):** The human IFN-β (hIFN-β) gene was amplified by PCR from plasmid vector pbSER178 with the hIFN signal sequence replaced on the 5' primer and Sal I and Not I restriction enzyme sites added at the 5' and 3' ends. The fragment was digested with Sal I and Not I and inserted into the Sal I and Not I sites of plasmid vector pgWIZ resulting in plasmid vector pGER125 (Figure 21).

**pGene/V5-HisA:** Plasmid vector was purchased from Invitrogen and contains 6 GAL-4 binding sites upstream of a minimal promoter (E1b TATA), a 5' untranslated region (UTR) that is UT12 derived from CMV, a synthetic intron 8 (IVS8), a multiple cloning site (MCS) and the bovine growth hormone (bGH) poly(A) site. Genes inserted at the MCS can be regulated by a regulator molecule (RM). For example, a gene inserted at the MCS can be induced by the activated form of the modified progesterone receptor (e.g. comprising the amino acid sequence of SEQ ID NO: 22 or encoded by the nucleic acid sequence of SEQ ID NO: 21) upon binding of the activated RM to the GAL-4 sites (Figure 22).

**pGene-mIFN (pGER127):** Plasmid vector pGER101 was digested with Sal I, filled in with Klenow, ligated to Hind III linkers, and digested with Hind III and Not I. The mIFN-β gene fragment was inserted into the Hind III and Not I sites of plasmid vector pGene/V5-HisA resulting in plasmid vector pGene-mIFN (Figure 23).

**pGene-hIFN (pGER129):** Plasmid vector pGER125 was digested with Sal I, filled in with Klenow, ligated to Hind III linkers, and digested with Hind III and Not I. The mIFN-β gene fragment was inserted into the Hind III and Not I sites of plasmid vector pGene/V5-HisA resulting in plasmid vector pGene-hIFN (pGER129) (Figure 24).

**pSwitch:** This plasmid vector was purchased from Invitrogen and encodes the modified progesterone receptor (e.g. comprising the amino acid sequence of SEQ ID NO: 22 or encoded by the nucleic acid sequence of SEQ ID NO: 21) linked to an autoinducible RM-responsive promoter (4XGAL-4 DNA binding sites and thymidine kinase (tk) promoter) upstream of 5' untranslated region 12 (UT12) derived from CMV and synthetic intron 8 (IVS8) driving expression of the gene for the RM protein (Figure 25).

**pGS1694:** Plasmid vector pGS1694 was provided by Valentis and contains the chicken skeletal muscle actin promoter (sk actin pro), 5' untranslated region 12 (UT12) and synthetic intron 8 (IVS8) driving expression of the gene encoding the modified progesterone receptor (e.g. comprising the amino acid sequence of SEQ ID NO: 22 or encoded by the nucleic acid sequence of SEQ ID NO: 21) (Figure 26).

**pLC1674:** Plasmid vector pLC1674 was provided by Valentis and contains a "RM-responsive" promoter (i.e., a promoter responsive to the activated form of the modified progesterone receptor (e.g. comprising the amino acid sequence of SEQ ID NO: 22 or encoded by the nucleic acid sequence of SEQ ID NO: 21), 5' untranslated region 12 (UT12) and synthetic intron 8 (IVS8) driving expression of the gene encoding the firefly luciferase gene (luc) (Figure 27).

### G. Construction of Vectors for Producing Virus

Vectors for producing virus (e.g., shuttle plasmids) and methods of producing virus (e.g., AAV-1 virus) are known in the art and can be used to produce the virus of the present invention. In some embodiments, the viruses of the present invention are produced from shuttle plasmids (e.g., see Table 38) and used for the delivery and expression of a molecule of the present invention (e.g., a TM and/or RM encoded by a sequence contained in the vector) in the cells of a subject, for treatment of disease.

**pGT2/mGMCSF and pGT/hGMCSF:** Shuttle plasmids pGT2/mGMCSF and pGT/hGMCSF were constructed as follows (Figure 28). A fragment encoding mouse GMCSF (mGM-CSF) was excised from pORF9-mGMCSF (Figure 30) by digesting the vector plasmid with Agel and Nhel. This fragment was then blunted. Similarly a fragment encoding human (hGM-CSF) was excised from pORF-hGMCSF (Figure 30) by digesting with the vector plasmid with SgrAl and Nhel. This fragment was then blunted. The excised and blunted fragment encoding either mGMCSF or hGMCSF was inserted into the EcoRV site of pGT2 vector plasmid. The orientation of the insert was then checked by restriction digest mapping. The resulting shuttle plasmids were named pGT2/mGMCSF (encoding mouse GMCSF) and pGT2/hGMCSF (encoding human GMCSF) (Figure 28).

**pZac2.1-RM-hGMCSF and pZac2.1-RM-mGMCSF**: Shuttle plasmids pZac2.1-RM-hGMCSF and pZac2.1-RM-mGMCSF were constructed as follows (Figure 29A). A fragment encoding a mouse GMCSF was excised from the plasmid pORF9-mGMCSF (Figure 30) by digesting the plasmid with AgeI and NheI and blunted, and the resulting blunted fragment inserted into the EcoRV site of the plasmid pGT2, resulting in the plasmid pGT2/mGMCSF. Similarly, a fragment encoding human GMCSF was excised from the plasmid pORF-hGMCSF (Figure 30) by digesting the plasmid with SgrAl and Nhel and blunted, and the resulting blunted fragment inserted into the EcoRV site of the plasmid pGT2, resulting in the plasmid pGT2/hGMCSF. The inserts in the resulting vector plasmids were each checked and verified by restriction digest mapping.

The vector plasmids pGT2/hGMCSF and pGT2/mGMCSF were then each digested with Fsel and Srfl. These BRES-1-GMCSF fragments were then blunted. The plasmid vector pZac2.1 was digested with Bgl2 and Clal, and blunted. The blunted BRES-1-GMCSF fragments were each ligated to a blunted pZac2.1 vector. Positive clones were verified by restriction digests. The resulting shuttle plasmids were named pZac2.1-RM-hGMCSF (encoding human GMCSF) and pZac2.1-RM-mGMCSF (encoding mouse GMCSF) (Figure 29A).

**pZac2.1-CMV-mGMCSF and pZac2.1-CMV-hGMCSF**: Shuttle plasmids pZac2.1-CMV-mGMCSF and pZac2.1-CMV-hGMCSF were constructed as follows (Figure 29B). A fragment encoding a human GMCSF and a fragment encoding a mouse GMCSF were each separately cloned into the plasmid vector pGENE/V5HisA (Invitrogen) resulting, respectively, in the plasmids pGT723-GENE/hGMCSF (encoding human GMCSF) and pGT724-GENE/mGMCSF (encoding mouse GMCSF). A fragment encoding mouse GMCSF was excised from pGT724/mGMCSF by digesting the plasmid with Kpnl and Xbal, and a fragment encoding human GMCSF was excised from pGT723/hGMCSF by digesting the plasmid with KpnI and Xbal. The resulting fragments were each separately inserted into the KpnI/XbaI site of the vector pZac2.1 that had been digested with KpnI and Xbal, and treated with calf alkaline phosphatase (CIP). The resulting shuttle plasmids were named pGT713 (or pZac2.1-CMV-hGMCSF) encoding human GMCSF and pGT714 (or pZac2.1-CMV-mGMCSF) encoding mouse GMCSF (Figure 29B).

Additional shuttle plasmids were constructed as described in Table 38 below.

**Table 38**

| **Shuttle Plasmid** | **Description of Shuttle Plasmid** | **Construction of Shuttle Plasmid for Producing AAV-1 Virus** |
|---|---|---|
| pGT61 | AAV-1 shuttle plasmid encoding mIFN-β operably linked to a CMV promoter | The SpeI-AscI fragment of pGT26 encoding mIFN- β gene was inserted into the NheI-MluI site of pZAC2.1, resulting in the shuttle plasmid pGT61 that produces the AAV-1 GT61 virus. |
| pGT62 | AAV-1 shuttle plasmid encoding hIFN-β operably linked to a CMV promoter | The SpeI-AscI fragment of pGT30 encoding hIFN-β gene was inserted into NheI-MluI site of pZAC2.1, resulting in the shuttle plasmid pGT62 that produces the AAV-1 GT62 virus. |
| pGT54 | AAV-1 shuttle plasmid containing BRES-1 encoding mIFN-β | The SwaI-SbfI fragment of pGT53 was ligated to the SwaI-Sbfl fragment of pGT26 containing the BRES-1 sequence, resulting in the shuttle plasmid pGT54 that produces the AAV-1 GT54 virus. |
| pGT57 | AAV-1 shuttle plasmid containing BRES-1 encoding hIFN-β | The Fsel-Swal fragment of pGT53 was ligated to the Fsel-Srfl fragment of pGT28 containing the BRES-1 sequence, resulting in the shuttle plasmid pGT57 that produces the AAV-1 GT57 virus. |
| pGT58 | AAV-1 shuttle plasmid containing BRES-1 encoding hIFN-β | The Swal-Sbfl fragment of pGT53 was ligated to the Swa-SbfI/ fragment of pGT30 containing BRES-1 sequence, resulting in the shuttle plasmid pGT58 that produces the AAV-1 GT58 virus. |
| pGT714 | AAV-1 shuttle plasmid encoding mGMCSF operably linked to a CMV promoter | The fragment encoding mGMCSF from the plasmid vector pORF9-mGMCSF (Invitrogen) (Figure 30) was inserted into the multicloning site of pZAC2.1, resulting in the shuttle plasmid pGT714 (Figure 29B, pZac2.1-CMV-mGMCSF) that produces the AAV-1GT714 virus. |
| pGT713 | AAV-1 shuttle plasmid encoding hGMCSF operably linked to a CMV promoter | The fragment encoding hGMCSF from the plasmid vector pORF9-hGMCSF (Invitrogen) (Figure 30) was inserted into the multicloning site of pZAC2.1, resulting in the shuttle plasmid pGT713 (Figure 29B, pZac2.1-CMV-hGMCSF) that produces the AAV-1GT713 virus. |
| pGT716 | AAV-1 shuttle plasmid containing BRES-1 mGMCSF | A blunt ended fragment encoding mGMCSF was inserted into the EcoRV site of pGT2 resulting in the plasmid vector pGT712, and the Fsel-Srfl fragment of pGT712 containing BRES-1-mGMCSF was blunt ended and inserted into pZAC2.1, resulting in the shuttle plasmid pGT716 (SEQ ID NO: 42) that produces the AAV-1GT716 virus (see Figure 31 B). |
| pGT715 | AAV-1 shuttle plasmid containing BRES-1 hGMCSF | A blunt ended fragment encoding hGMCSF was inserted into the EcoRV site of pGT2 resulting in the viral vector pGT711, and the FseI-SrfI fragment or pGT711 containing BRES-1-hGMCSF was blunt ended and inserted into pZAC2.1, resulting in the shuttle plasmid pGT715 (SEQ ID NO: 41) that produces AAV-1 GT715 virus (see Figure 31A). |
| pTR-mIFN-β | AAV-1 shuttle plasmid encoding mIFN-β operably linked to a CMV promoter | The blunted HincII-BsrBI fragment of pgWIZ/WT IFN encoding mIFN-β was inserted into the blunted AgeI-SalI site of pTReGFP, resulting in the shuttle plasmid pTR-mIFN-β (SEQ ID NO: 43) that produces the pTR-mIFN-β virus, resulting in the shuttle plasmid pTR-mIFN-β that produces AAV-1TR-mIFN-β virus. |
| pTR-hIFN-β | AAV-1 shuttle plasmid encoding hIFN-β operably linked to a CMV promoter | The blunted HincII/NotI fragment of pgWIZ/hIFNb encoding hIFN-β was inserted into the blunted AgeI-SalI site of pTReFGP, resulting in the shuttle plasmid pTR-hIFN-β (SEQ ID NO: 44) that produces AAV-1TR-hIFN-β virus. |
| pGER75 | AAV-1 shuttle plasmid encoding SEAP operably linked to CMV promoter | A fragment encoding SEAP was amplified via PCR using pSEAP2 DNA (Clonetech) as a template and the amplified fragment was inserted into the Nhel-Xbal site of the vector phRL-CMV (Promega), resulting in the shuttle plasmid pGER75 that produces AAV-1GER75 virus. |

In Table 38 above, for the AAV-1-BRES-1 constructs, the pZAC2.1 shuttle plasmid was modified at the MCS resulting in shuttle plasmid pGT53, in order to enable the insertion of the fragment containing the BRES-1 sequence into the vector. To insert the fragment containing the BRES-1 sequence into pGT53, the appropriate pGT plasmid (as described above in Table 8) was digested with restriction enzymes that resulted in a fragment containing the entire BRES-1 sequence encoding the respective IFN, and this fragment was inserted into a compatible site of pGT53. The resulting AAV-1 shuttle plasmids were used to make AAV-1 virus preps as described herein using standard methods for producing AAV-1 virus.

For the CMV-promoter-containing shuttle plasmids, a fragment encoding the respective IFN gene was isolated from the appropriate plasmid vector via restriction enzyme digestion and inserted into the pZAC2.1 plasmid vector at (a) compatible restriction site(s) as described in Table 8 above.

For the BRES-1 hGM-CSF shuttle plasmids, an SrgAl/NheI fragment of pORF9-hGMCSF (Invitrogen) encoding hGMCSF was blunt-ended and inserted into the EcoRV site of pGT2 resulting in pGT711. The FseI/SrfI fragment of pGT712 containing the entire BRES-1 sequence was blunt-ended and inserted into pZAC2.1 resulting in pGT715 (Figure 31 A).

For the BRES-1 mGMCSF shuttle plasmids, an AgeI/NheI fragment of pORF9-mGMCSF (Invitrogen) encoding mGM-CSF was blunt-ended and inserted into the EcoRV site of the pGT2 vector resulting in pGT712. The FseI/SrfI fragment of pGT712 containing the entire BRES-1 sequence was blunt-ended and inserted into pZAC2.1 resulting in pGT716 (Figure 31 B).

The mouse and human GM-CSF genes from the respective pORF9 plasmids (Invitrogen) were cloned through the plasmid pGENE/VSHisA plasmid (Invitrogen) so thatthey could each be excised with KpnI/XbaI and cloned into pZAC2.1.

### H. Stable MCF 10A cell line construction

Human mammary epithelial cell line MCF 10A (ATCC, # CRL-10317) was cultured in 10-cm dishes. Cells were transfected with pGT137 plasmid (pBRES-Luc, puromycin selection) using Fugene 6 transfection reagent (Roche). Briefly, 6 µg of DNA and 36 µL of Fugene 6 were diluted in 750 µL serum-free Opti-MEM. DNA and Fugene 6 were mixed and incubated at room temperature for 15 minutes. The cells were cultured for 2 days before screening with 1 µg/mL puromycin for 3 weeks. Positive clones were selected and verified by luciferase assay for pBRES-Luc expression and activation.

### I. Determination of pBRES Activation by Luciferase Activity Measurement

All cells were harvested by gentle trypsinization, followed by a wash with PBS. Cells were then spun for 5 min at 800 rpm and the cell pellet was resuspended in 10 ml tissue culture media. The cells were then seeded at a cell density of 1x10⁴ cells/well (volume of 100ul/well) in a 96-well flat-bottom, black tissue culture plate under sterile conditions. Cells were subsequently incubated at 37°C in 5% CO₂ for 24 hours. Thereafter, under sterile conditions, the media was removed by careful aspiration and the wells replaced with 95ul fresh media, followed by the addition of 5ul/well of appropriately diluted compound or control buffer, resulting in final compound concentrations ranging from 0.00064 to 50 nM (in 0.05% DMSO final). Cell culture plates were then covered and incubated for 24 hr in a 37°C incubator. Thereafter, media was removed from the plates by gentle aspiration, the plates blotted dry, and the cells lysed with 50 ul/well of luciferase assay substrate reagent from the Luciferase Assay System Kit (Promega, Inc). Plates were then gently shaken for 1 min after addition of luciferase substrate reagent and luciferase enzyme activity subsequently measured at various times (5 minutes, 30 minutes, 60 minutes) using a Microbeta Luminescence Plate Reader (PerkinElmer) using a normalized luciferase reading protocol.

### J. Primary pBRES-Luciferase assay in HEK 293 cells

To determine the effects of test compounds on pBRES activity in Human Embryonic Kidney 293 (HEK 293) cells (ATCC # CRL-1573), cells were plated in 60-mm dishes at 5x10⁵ cells/dish in 5 mL of MEM alpha with 4 mM L-glutamine and 10 % heat-inactivated FBS. Cells were transfected 24 hours later with pBRES constructs using Lipofectamine 2000 (Invitrogen) at 1:2.5 ratio of DNA (µg):Lipofectamine 2000 (µL) reagent. Medium containing lipid complexes was removed after 4 hours and cells allowed to recover in fresh growth medium for 24 hours. Cells were reseeded into 96-well plates at 10,000 cells/well in growth medium. The next day cells were treated with MFP or test compound using the same medium. Compounds were dissolved in 100 % ethanol vehicle and diluted by using the treatment medium. The final concentration of ethanol in treatment medium was 0.1 %. Each treatment consisted of three replicates. Twenty four hours after treatment, luciferase activity was measured as follows: medium was removed and cells were washed once with Dulbecco's PBS. Cells were lysed with 20 µL/well of 1x Passive Lysis Buffer (PLB, Promega) and incubated at room temperature for 10 minutes with shaking on a titer plate shaker. Plates were frozen at -80 °C for 20 minutes and were thawed at room temperature for 20 minutes with shaking in a titer plate shaker. Luciferase activity was measured using the Promega Luciferase Assay System (Cat. # E1501) substrate (100 µL/well) using a Berthold MicroLumat Plus luminometer.

### K. Secondary PR assay in T47D cells

To determine the effects of test compounds on the human Progesterone receptor-induced alkaline phosphatase activity in human breast carcinoma T47D cells (ATCC # HTB-133), cells were plated in 96-well plates at 20,000 cells/well in growth medium (RPMI1640 with 1 mM sodium pyruvate, 0.2 units/mL insulin, 4 mM L-glutamine and 10 % FBS). After overnight culture, the medium was changed to the assay medium (RPMI1640 (phenol red-free) with 1 mM sodium pyruvate, 0.2 units/mL insulin, 4 mM L-glutamine and 3 % charcoal-stripped FBS). The next day, cells were treated with MFP or test compounds in the presence of 200 pM Promegestone. Compounds were dissolved in 100 % ethanol vehicle and diluted using the assay medium. The final concentration of ethanol in assay medium was 0.2 %. Each treatment consisted of 5 to 6 replicates. Twenty four hours after treatment, cellular alkaline phosphatase activity was measured as follows: the medium was removed and cells were washed twice with DPBS. After residual DPBS was removed, 50 µL of cell lysis buffer (100 mM Tris-HCl pH 9.8, 0.2 % Triton X-100) was added to each well and plates were frozen at -80 °C for 30 minutes. The plates were thawed at room temperature for 20 minutes with shaking on a titer plate shaker. 150 µL of substrate buffer (100 mM Tris-HCl pH 9.8, 4 mM p-nitrophenyl phosphate) was added to each well. Absorbance measurements were taken at 5 minute intervals for 1 hour at the test wavelength of 405 nm.

### L. Homology model of the RM LBD

The homology model of the RM LBD based on the GR LBD/MFP complex was created using the SwissModel feature of DeepView Swiss PDB Viewer ver. 3.70 (Guex, N. & Peitsch, M.C. (1997) Electrophoresis 18: 2714-2723). Briefly, the primary amino acids sequence corresponding to residues 640-914 of the human Progesterone receptor (Accession NP_000917) was fit onto the X-ray crystal structure of the human Glucocorticoid receptor 1 NHZ.pdb and was submitted to the Swiss-Prot server for minimization. The fit of the resulting model to the GR/MFP crystal structure was examined using the Insight2 software (Accelrys Software Inc.).

### M. Modeling of RM LBD - compound interactions

Models of MFP, BLX-913 and other test compounds bound in the RM ligand binding pocket were created using the Discovery Studio Viewer Pro ver. 6.0 (Accelrys Software Inc.) and the active site residues identified via the rlig_view script (Dr. Marc Adler, Biophysics Department, Berlex, unpublished).

### N. Site-directed mutagenesis

Site-directed mutations within the RM LBD were installed using the Quick Change mutagenesis approach using the following primers with the newly installed codon underlined. The amino acid numbering scheme follows hPR amino acid numbering. Single mutations at positions 719 and 755 were installed using the pGT79 template. Double mutations at positions 729 and 726 were installed using the pGT1009 (W755A) template (*see* Table 39).

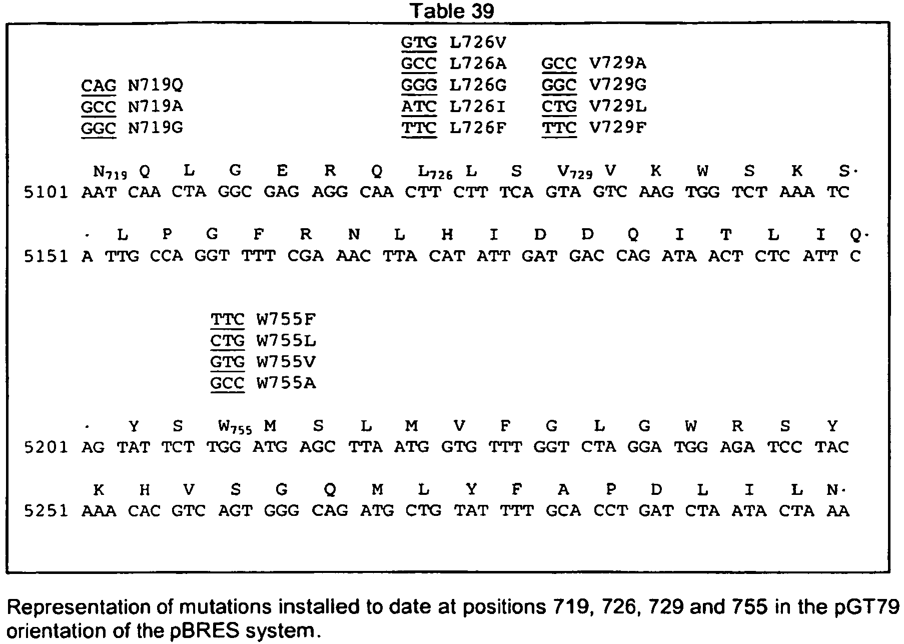

N719Q (pGT1003) (SEQ ID NO: 60) 5'- CTT TGC TGA CAA GTC TTC AGC AAC TAG GCG
AGA GG -3' and 5'- CCT CTC GCC TAG TTG CTG AAG ACT TGT CAG CAA AG -3';

N719A (pGT1004) (SEQ ID NO: 62) 5'- CTT TGC TGA CAA GTC TTG CCC AAC TAG GCG
AGA GG -3' and 5'- CCT CTC GCC TAG TTG GGC AAG ACT TGT CAG CAA AG -3';

N719G (pGT1005) (SEQ ID NO: 64) 5'- CTT TGC TGA CAA GTC TTG GCC AAC TAG GCG
AGA GG -3' and 5'- CCT CTC GCC TAG TTG GCC AAG ACT TGT CAG CAA AG -3';

W755F (pGT1006) (SEQ ID NO: 66 5'- CTC TCA TTC AGT ATT CTT TCA TGA GCT TAA
TGG TG -3' and 5'- CAC CAT TAA GCT CAT GAA AGA ATA CTG AAT GAG AG -3';

W755L (pGT1007) (SEQ ID NO: 68) 5'- CTC TCA TTC AGT ATT CTC TGA TGA GCT TAA
TGG TG -3' and 5'- CAC CAT TAA GCT CAT CAG AGA ATA CTG AAT GAG AG -3';

W755V (pGT1008) (SEQ ID NO: 70) 5'- CTC TCA TTC AGT ATT CTG TGA TGA GCT TAA
TGG TG -3' and 5'- CAC CAT TAA GCT CAT CAC AGA ATA CTG AAT GAG AG -3';

W755A (pGT1009) (SEQ ID NO: 72) 5'- CTC TCA TTC AGT ATT CTG CCA TGA GCT TAA
TGG TG -3' and 5'- CAC CAT TAA GCT CAT GGC AGA ATA CTG AAT GAG AG -3';

V729A/W755A (pGT1015) (SEQ ID NO: 74) 5'- GAG GCA ACT TCT TTC AGC CGT CAA
GTG GTC TAA ATC -3' and 5'- GAT TTA GAC CAC TTG ACG GCT GAA AGA AGT TGC
CTC -3';

V729G/W755A (pGT1016) (SEQ ID NO: 76) 5'- GAG GCA ACT TCT TTC AGG CGT CAA
GTG GTC TAA ATC -3' and 5'- GAT TTA GAC CAC TTG ACG CCT GAA AGA AGT TGC
CTC -3';

V729L/W755A (pGT1017) (SEQ ID NO: 78) 5'- GAG GCA ACT TCT TTC ACT GGT CAA
GTG GTC TAA ATC -3' and 5'- GAT TTA GAC CAC TTG ACC AGT GAA AGA AGT TGC
CTC -3'.

V729F/W755A (pGT1025) (SEQ ID NO: 80) 5'- GAG GCA ACT TCT TTC ATT CGT CAA
GTG GTC TAA ATC -3' and 5'- GAT TTA GAC CAC TTG ACG AAT GAA AGA AGT TGC
CTC -3';

L726V/W755A (pGT1020) (SEQ ID NO: 82) 5'- GGC GAG AGG CAA GTG CTT TCA GTA
GTC AAG -3' and 5'- CTT GAC TAC TGA AAG CAC TTG CCT CTC GCC -3';

L726A/W755A (pGT1021) (SEQ ID NO: 84) 5'- GGC GAG AGG CAA GCC CTT TCA GTA
GTC AAG -3' and 5'- CTT GAC TAC TGA AAG GGC TTG CCT CTC GCC -3';

L726G/W755A (pGT1022) (SEQ ID NO: 86) 5'- GGC GAG AGG CAA GGG CTT TCA GTA
GTC AAG -3' and 5'- CTT GAC TAC TGA AAG CCC TTG CCT CTC GCC -3';

L726I/W755A (pGT1023) (SEQ ID NO: 88) 5'- GGC GAG AGG CAA ATC CTT TCA GTA
GTC AAG -3' and 5'- CTT GAC TAC TGA AAG GAT TTG CCT CTC GCC -3';

L726F/W755A (pGT1024) (SEQ ID NO: 90) 5'- GGC GAG AGG CAA TTC CTT TCA GTA
GTC AAG -3' and 5'- CTT GAC TAC TGA AAG GAA TTG CCT CTC GCC -3';

### In vitro validation of BLX-913/engineered RP system

Furthermore, the LBD double mutation V729L/W755A that was shown to rescue BLX-913-dependent activation of luciferase reporter gene in HEK293 cells was tested in C2C12 mouse muscle cells using luciferase, murine Secreted Embryonic Alkaline Phosphatase (SEAP) and human interferon beta (hIFNb) TM. It was previously reported (Szymanski P et al., Mol Ther 2007; 15: 1340-1347) that the best orientation (i.e. one showing the lowest level of TM expression in the absence of inducer and the highest level of TM expression in the presence of inducer) of the RP cassette and the TM cassette depends on the identity of the TM. We thus evaluated the new inducer and the new engineered RP LBD in the four orientations within each group of TMs, using either MFP or BLX-913 to induce expression.

Results are shown in Fig. 58 - 60. In every orientation with the three transgenes tested above, the engineered RP retained full activity when induced with MFP.

In virtually every orientation and with every transgene (with the exception of pGT1050 and pGT1058, the combination of the new inducer BLX-913 and the engineered RP (*) is as good or better an inducer of TM expression as the original GS with MFP. These studies further validate BLX-913 compound and the V729L/W755A RP pBRES as the new inducer-pBRES combination with improved selectivity over the human Progesterone Receptor.

### In vivo validation of BLX-913/engineered RP system

Upon the conclusion of in vitro testing, the divergent orientation of mSEAP and RP in pGT45 and pGT1048 were tested side-by-side in a mouse study. Results are shown in Fig. 61. Mice were injected with plasmid DNA in the hind limbs and electroporation was used to deliver the DNA into the muscle cells on day 0. This protocol has been previously used with pBRES DNA and MFP inducer. On days 7, 8, 9 and 10, the mice received inducer compound (either vehicle alone, MFP or BLX-913) via intraperitoneal (IP) injection in sesame oil vehicle in 24-hour intervals. Six hours after the last injection on day 10, samples of blood were collected from the tail veins of the mice. mSEAP levels in the serum were measured to determine the level of this reporter expression following induction with compounds. Additional blood samples were collected prior to injection of inducers on day 7, as well as on days 18 and 25 to monitor the drop off of mSEAP expression.

From Fig. 61 it is evident that mice which received pGT45 (original RP) responded well to MFP and either the 0.33 mg/kg or the 1 mg/kg concentration. When BLX-913 was used as the inducer, these mice did not respond at either concentration. When the new engineered RP DNA was delivered to mice in the pGT1048 groups, they responded to BLX-913 better than the mice that received the pGT45 DNA.

### Example 12: Generation of further vectors

### Mutagenesis of pBRES RP LBP

All site-directed mutants were constructed by QuikChange mutagenesis of pBRES plasmids and confirmed by sequencing. Plasmids pGT77-80 (pGT1044-1046, pGT1017 for LBD mutation V729L/W755A) contain the four orientations of the pBRES-luciferase cassettes, pGT44-47 (pGT1047-1050 for LBD mutation V729L/W755A) contain the four orientations of the pBRESmSEAP cassettes and pGT27-30 (pGT1055-pGT1058 for LBD mutation V729L/W755A) contain the four orientations of the pBRES-hIFNb cassettes. The respective orientations can be obtained from Figs. 58 - 60.

Primers used for site-directed mutagenesis:

5'- CTT TGC TGA CAA GTC TTG CCC AAC TAG GCG AGA GG -3' and 5'- CCT
CTC GCC TAG TTG GGC AAG ACT TGT CAG CAA AG -3' (N719A);
5'- CTT TGC TGA CAA GTC TTG GCC AAC TAG GCG AGA GG -3' and 5'- CCT
CTC GCC TAG TTG GCC AAG ACT TGT CAG CAA AG -3' (N719G);
5'- CTC TCA TTC AGT ATT CTT TCA TGA GCT TAA TGG TG -3' and 5'- CAC
CAT TAA GCT CAT GAA AGA ATA CTG AAT GAG AG -3' (W755F);
5'- CTC TCA TTC AGT ATT CTC TGA TGA GCT TAA TGG TG -3' and 5'- CAC
CAT TAA GCT CAT CAG AGA ATA CTG AAT GAG AG -3' (W755L);
5'- CTC TCA TTC AGT ATT CTG TGA TGA GCT TAA TGG TG -3' and 5'- CAC
CAT TAA GCT CAT CAC AGA ATA CTG AAT GAG AG -3' (W755V);
5'- CTC TCA TTC AGT ATT CTG CCA TGA GCT TAA TGG TG -3' and 5'- CAC

CAT TAA GCT CAT GGC AGA ATA CTG AAT GAG AG -3' (W755A);
5'- CTC TCA TTC AGT ATT CTG GCA TGA GCT TAA TGG TG -3' and 5'- CAC
CAT TAA GCT CAT GCC AGA ATA CTG AAT GAG AG -3' (W755G);
5'- GAG GCA ACT TCT TTC AGG CGT CAA GTG GTC TAA ATC -3' and 5'- GAT
TTA GAC CAC TTG ACG CCT GAA AGA AGT TGC CTC -3' (V729G);
5'- GAG GCA ACT TCT TTC AGC CGT CAA GTG GTC TAA ATC -3' and 5'- GAT
TTA GAC CAC TTG ACG GCT GAA AGA AGT TGC CTC -3' (V729A);
5'- GAG GCA ACT TCT TTC ACT GGT CAA GTG GTC TAA ATC -3' and 5'- GAT
TTA GAC CAC TTG ACC AGT GAA AGA AGT TGC CTC -3' (V729L);
5'- GAG GCA ACT TCT TTC ATT CGT CAA GTG GTC TAA ATC -3' and 5'- GAT
TTA GAC CAC TTG ACG AAT GAA AGA AGT TGC CTC -3' (V729F).

One embodiment of the invention is a vector of the group pGT77, pGT78, pGT79, pGT1044, pGT1045, pGT1017, pGT1046, pGT44, pGT45, pGT46, pGT47, pGT1047, pGT1048, pGT1049, pGT1050, pGT27, pGT28, pGT29, pGT30, pGT1055, pGT1056, pGT1057, and pGT1058.

One further embodiment is the use of a vector of the group pGT77, pGT78, pGT79, pGT1044, pGT1045, pGT1017, pGT1046, pGT44, pGT45, pGT46, pGT47, pGT1047, pGT1048, pGT1049, pGT1050, pGT27, pGT28, pGT29, pGT30, pGT21055, pGT1056, pGT1057, and pGT1058 for any purpose described herein, in particular for the use in a regulated expression system of the invention. Also an embodiment of the invention is a vector of the group pGT77, pGT78, pGT79, pGT1044, pGT1045, pGT1017, pGT1046, pGT44, pGT45, pGT46, pGT47, pGT1047, pGT1048, pGT1049, pGT1050, pGT27, pGT28, pGT29, pGT30, pGT1055, pGT1056, pGT1057, and pGT1058 as a part of a regulated expression system of the invention.

Also part of the invention is a reglated expression system os the invention comprising a vector of the group pGT77, pGT78, pGT79, pGT1044, pGT1045, pGT1017, pGT1046, pGT44, pGT45, pGT46, pGT47, pGT1047, pGT1048, pGT1049, pGT1050, pGT27, pGT28, pGT29, pGT30, pGT1055, pGT1056, pGT1057, and pGT1058, wherein the activator is selected from the group consisting of a modified PR modulator, a PR ligand analogue, a modified antiprogestin, a modified mesoprogestin, a modified progestin, a modified MFP, a modified asoprisnil, BLX-93, BLX-899, BLX-952, BLX-610, BLX-117, BLX-784, or BLX-913.

One skilled in the art will readily appreciate that the compositions and methods of the present invention are well adapted to carry out the objects and obtain the ends and advantages described herein, as well as those inherent in the present invention. Changes to the compositions and methods of the present invention, and other uses, will occur to those skilled in the art

## Claims

1. A regulated expression system comprising at least a vector and an activator molecule (AM), said vector comprising: a first expression cassette comprising: i) first nucleic acid sequence encoding a therapeutic molecule (TM) having a therapeutic activity, and ii) a first promoter and a first poly(A) site operably linked to said first nucleic acid sequence, wherein said TM is expressable in cells of a subject, and said TM expression or activity is dose-dependent and regulated or induced in the presence of a regulator molecule (RM); and a second expression cassette comprising: i) a second nucleic acid sequence encoding said regulator molecule (RIv)7, and ii) a second promoter and a second poly(A) site operably linked to said second nucleic acid sequence, wherein said RM is expressable in said cells and activated in the presence of an activator molecule (AM), thereby regulating said TM expression or activity, and wherein said AM is a modified steroid receptor modulator being a modified mifepristone (MFP), wherein said modified mifepristone is a 11-beta substituted ring selected from the group consisting of BLX-913, BLX-899, BLX-952, BLX-61 0, BLX-117 and BLX-784 and has no or limited effect on human hPR.

2. The regulated expression system of claim 1, wherein said vector can be administered by contacting said cells with said vector in vivo or ex vivo.

3. A pharmaceutical composition comprising at least one expression system according to claim 1.

4. A kit for delivering a therapeutic molecule (TM) to cells of a subject, wherein said kit comprises at least one regulated expression system according to claim 1 and instructions for its use.

## Patentansprüche

1. Reguliertes Expressionssystem, umfassend wenigstens einen Vektor und ein Aktivatormolekül (AM), wobei der Vektor Folgendes umfasst: eine erste Expressionskassette, umfassend: (i) eine für ein therapeutisches Molekül (TM) mit einer therapeutischen Aktivität codierende erste Nukleinsäuresequenz und (ii) einen ersten Promotor und eine erste Poly(A)-Stelle in operativer Verknüpfung mit der ersten Nukleinsäuresequenz, wobei das TM in Zellen eines Patienten exprimierbar ist und die TM-Expression oder -Aktivität dosisabhängig ist und in Gegenwart eines Regulatormoleküls (RM) reguliert oder induziert wird; und eine zweite Expressionskassette, umfassend: (i) eine für das Regulatormolekül (RM) codierende zweite Nukleinsäuresequenz und (ii) einen zweiten Promotor und eine zweite Poly(A)-Stelle in operativer Verknüpfung mit der zweiten Nukleinsäuresequenz, wobei das RM in den Zellen exprimierbar ist und in Gegenwart eines Aktivatormoleküls (AM) aktiviert wird, wodurch die TM-Expression oder -Aktivität reguliert wird, und wobei es sich bei dem AM um einen modifizierten Steroidrezeptor-Modulator, der ein modifiziertes Mifepriston (MFP) ist, handelt, wobei das modifizierte Mifepriston einen aus der aus BLX-913, BLX-899, BLX-952, BLX-610, BLX-117 und BLX-784 bestehenden Gruppe ausgewählten 11-betasubstituierten Ring darstellt und keine oder eine begrenzte Wirkung auf menschlichen hPR ausübt.

2. Reguliertes Expressionssystem nach Anspruch 1, wobei sich der Vektor verabreichen lässt, indem die Zellen mit dem Vektor in vivo oder ex vivo in Kontakt gebracht werden.

3. Pharmazeutische Zusammensetzung, umfassend wenigstens ein Expressionssystem gemäß Anspruch 1.

4. Kit zur Zuführung eines therapeutischen Moleküls (TM) an Zellen eines Patienten, wobei das Kit wenigstens ein reguliertes Expressionssystem gemäß Anspruch 1 sowie Anweisungen zu dessen Verwendung umfasst.

## Revendications

1. Système d'expression régulée comprenant au moins un vecteur et une molécule activatrice (MA), ledit vecteur comprenant : une première cassette d'expression comprenant : (i) une première séquence d'acide nucléique codant pour une molécule thérapeutique (MT) ayant une activité thérapeutique, et (ii) un premier promoteur et un premier site poly(A) liés de façon opérationnelle à ladite première séquence d'acide nucléique, ladite MT étant susceptible d'être exprimée dans des cellules d'un sujet, et ladite expression ou activité de la MT étant dépendante de la dose et régulée ou induite en présence d'une molécule régulatrice (MR) ; et une deuxième cassette d'expression comprenant : (i) une deuxième séquence d'acide nucléique codant pour ladite molécule régulatrice (MR), et (ii) un deuxième promoteur et un deuxième site poly(A) liés de façon opérationnelle à ladite deuxième séquence d'acide nucléique, ladite MR étant susceptible d'être exprimée dans lesdites cellules et activée en présence d'une molécule activatrice (MA), régulant ainsi ladite expression ou activité de la MT, ladite MA étant un modulateur de récepteur de stéroïde modifié qui est une mifépristone (MFP) modifiée, ladite mifépristone modifiée étant un cycle substitué en 11-bêta choisi dans le groupe constitué de BLX-913, BLX-899, BLX-952, BLX-610, BLX-117 et BLX-784 et n'ayant aucun effet ou peu d'effet sur l'hPR humain.

2. Système d'expression régulée selon la revendication 1, **caractérisé en ce que** ledit vecteur peut être administré en mettant lesdites cellules en contact avec ledit vecteur in vivo ou ex vivo.

3. Composition pharmaceutique comprenant au moins un système d'expression selon la revendication 1.

4. Kit pour délivrer une molécule thérapeutique (MT) aux cellules d'un sujet, **caractérisé en ce que** ledit kit comprend au moins un système d'expression régulée selon la revendication 1 et les instructions pour son utilisation.
